# EUROPÄISCHE PATENTSCHRIFT

(11) **EP 1 666 610 B1**
(45) Veröffentlichungstag und Bekanntmachung des Hinweises auf die Patenterteilung: **22.04.2009**
(21) Anmeldenummer: 04106291.0
(22) Anmeldetag: 03.12.2004
(51) Int. Cl.: C12Q 1/68

(54) **Nachweis der genetischen Anlage für Farbverdünnung beim Dobermann Pinscher**
Detection of a genetic susceptibility for diluted coat colours in the Doberman Pinscher
Détection d'une susceptibilité génétique à la dilution de la couleur du pelage du Doberman Pinscher

(43) Veröffentlichungstag der Anmeldung: 07.06.2006
(73) Patentinhaber: Stiftung Tierärztliche Hochschule Hannover, 30559 Hannover (DE)
(72) Erfinder: Leeb, Tosso, Prof. Dr., 30559 Hannover (DE); Philipp, Ute, Dr., 30163 Hannover (DE)
(74) Vertreter: Taruttis, Stefan Georg

(56) Entgegenhaltungen:
- DATABASE EMBL [Online] 5. September 2001 (2001-09-05), "Mus musculus melanophilin (Mlph) mRNA, complete cds." XP002329419 gefunden im EBI accession no. EM_PRO:AF384098 Database accession no. AF384098
- DATABASE EMBL [Online] 3. September 2004 (2004-09-03), "Rattus norvegicus melanophilin (predicted), mRNA (cDNA clone MGC:93800 IMAGE:7110013), complete cds." XP002329420 gefunden im EBI accession no. EM_PRO:BC081894 Database accession no. BC081894
- MATESIC L E ET AL: "Mutations in M1ph, encoding a member of the rab effector family, cause the melanosome transport defects observed in leaden mice" PROCEEDINGS OF THE NATIONAL ACADEMY OF SCIENCES OF USA, NATIONAL ACADEMY OF SCIENCE. WASHINGTON, US, Bd. 98, Nr. 18, 28. August 2001 (2001-08-28), Seiten 10238-10243, XP002955946 ISSN: 0027-8424
- DATABASE EMBL [Online] 19. Juli 2004 (2004-07-19), "Canis familiaris chromosome 25 cont_37811, whole genome shotgun sequence." XP002329421 gefunden im EBI accession no. EM_PRO:AAEX01037812 Database accession no. AAEX01037812
- KERNS JULIE A ET AL: "Characterization of the dog Agouti gene and a nonagoutimutation in German Shepherd Dogs." MAMMALIAN GENOME : OFFICIAL JOURNAL OF THE INTERNATIONAL MAMMALIAN GENOME SOCIETY. OCT 2004, Bd. 15, Nr. 10, Oktober 2004 (2004-10), Seiten 798-808, XP002329417 ISSN: 0938-8990
- BARRAL DUARTE C ET AL: "The melanosome as a model to study organelle motility in mammals." PIGMENT CELL RESEARCH / SPONSORED BY THE EUROPEAN SOCIETY FOR PIGMENT CELL RESEARCH AND THE INTERNATIONAL PIGMENT CELL SOCIETY. APR 2004, Bd. 17, Nr. 2, April 2004 (2004-04), Seiten 111-118, XP002329418 ISSN: 0893-5785
- 'ClustalW2 results' INTERNET CITATION 01 Januar 1900, XP007903787
- ABSTRACT ARTICLE: 'Homo sapiens cDNA FLJ12145 fis, clone MAMMA1000395' EMBL-EBI, [Online] 09 Januar 2008, XP007904975 Gefunden im Internet: <URL:http://www.ebi.ac.uk/cgi-bin/dbfetch?d b=embl&id=ak022207>
- ABSTRACT ARTICLE: 'ClustalW2 Results. 3 sequences' EMBL-EBI 18 Juni 2008, XP007904991
- ABSTRACT ARTICLE: 'ClustalW2 Results. 4 Sequences' 17 Juni 2008, XP007904980
- VITELLI R. ET AL: 'Molecular cloning and expression analysis of the human Rab7 GTP-ase complementary deoxyribonucleic acid' BIOCHEMICAL AND BIOPHYSICAL RESEARCH COMMUNICATIONS Bd. 229, Nr. 3, 01 Januar 1996, ACADEMIC PRESS INC. ORLANDO, FL, US, Seiten 887 - 890, XP002365365

## Beschreibung

Die vorliegende Erfindung betrifft einen genetischen Test für die genetische Anlage zur Farbverdünnung beim Dobermann Pinscher durch Genotypisierung des Melanophilingens.

Von Newton et al. (Mammalian Genome 11, 24-30 (2000)) wird berichtet, dass das Zusammenspiel der Genprodukte Agouti und Melanocortin-1-Rezeptor (Mc1r) bei Hunden der Rasse Dobermann für die Fellfärbung verantwortlich sei und auf Mutationen untersucht wurde. Mittels RT-PCR wurde die cDNA des Gens Mclr mit degenerierten Primern auf Basis der homologen Sequenz bei Mensch und Maus amplifiziert. Die molekulare Ursache für die rote, gelbe oder cremefarbene Fellfarbe verschiedener Rassen wird einer Mutation des MC1R-Gens zugeschrieben, die zu dessen Funktionsverlust führt.

Es ist bekannt, dass die Farbverdünnung bei Pinschern, insbesondere Dobermann Pinschern zu einem als blau oder isabell bezeichneten Pigmentierungstyp führt. Dieser blaue Phänotyp zeichnet sich durch eine silberblaue Schattierung der schwarzen Fellbereiche aus. Diese Farbverdünnung hat klinische Bedeutung, da Dobermann Pinscher mit dieser Farbverdünnung zur Farbverdünnungsalopezie neigen, die auch als "blauer Dobermann Syndrom" bezeichnet wird. Diese Alopezie zeichnet sich durch progressiven Haarverlust aus, der gelegentlich durch wiederkehrende bakterielle Infektionen der Haarfollikel gekennzeichnet ist. Die von der Alopezie betroffenen Hunde haben oft trockene und schuppige Haut und sind gegenüber Sonnenbrand oder extremer Kälte empfindlich.

Es ist bekannt, dass die Anlage zur Farbverdünnung autosomal rezessiv vererbt wird, sodass bei der Anpaarung zweier heterozygoter Hunde, die eine normale Fellfarbe aufweisen, die Wahrscheinlichkeit für einen verdünnten Farbschlag bei den Nachkommen jeweils 25% beträgt.

Das für die Farbverdünnung verantwortliche Gen wird als "dilute" bezeichnet, wobei das Wildtypallel D dominant ist und das mit der Farbverdünnung gekoppelte dilute Allel d rezessiv. Daraus ergeben sich die nachfolgend in Tabelle 1 zusammengestellten Genotypen und Phänotypen:

**Tabelle 1: Dilute Genotypen und Phänotypen**

| Genotyp | Phänotyp |
|---|---|
| DD, frei von der Anlage für Farbverdünnung | Wildtyp (schwarz-rot oder braun-rot) |
| Dd, heterozygot Träger für Farbverdünnung | Wildtyp (schwarz-rot oder braun-rot) |
| dd, homozygot Träger für Farbverdünnung | verdünnte Fellfarbe blau, isabell |

In der Übersicht von Tabelle 1 wird deutlich, dass nur bei verdünnter Fellfarbe auf den Genotyp geschlossen werden kann, während bei Fellfarbe des Wildtyps ein Tier dennoch heterozygot Anlageträger sein kann.

Es ist erwünscht, Dobermann Pinscher auf ihre genetische Veranlagung bezüglich der Farbverdünnung testen zu können, insbesondere um festzustellen, ob zumindest heterozygot die Erbanlage für die Farbverdünnung getragen wird. Denn die verdünnten Fellfarben blau oder isabell entsprechen nicht den kontinental-europäischen Rassestandards und sind unerwünscht, während sie im angelsächsischen Raum teils gezielt gezüchtet werden.

Ein genetischer Test für die Anlage auf Farbverdünnung beim Dobermann Pinscher ist derzeit nicht bekannt.

### Aufgabe der Erfindung

Es ist daher die Aufgabe der vorliegenden Erfindung, einen genetischen Test zur Verfügung zu stellen, mit dem der Genotyp von Dobermann Pinschern in Bezug auf die Farbverdünnung festgestellt werden kann.

### Allgemeine Beschreibung der Erfindung

Die oben genannte Aufgabe der Erfindung wird durch die Bereitstellung des Melanophilingens des Hundes nach Anspruch 1 und einen genetischen Test nach Anspruch 2 gelöst, mit dem Punktmutationen im Hundegenom identifiziert werden können, die zumindest beim Dobermann Pinscher mit dem Phänotyp der Farbverdünnung gekoppelt sind.

Bei der genetischen Analyse kann daher unmittelbar der Genotyp eines Dobermann Pinschers bezüglich der Farbverdünnung festgestellt werden. Insbesondere können die heterozygoten Anlageträger (Dd) für die Farbverdünnung identifiziert werden, die anhand ihres Phänotyps nicht von reinerbigen Hunden (DD) zu unterscheiden sind.

Es ist gefunden worden, dass das Melanophilingen des Dobermann Pinschers Punktmutationen aufweist, die mit dem Allel der Farbverdünnung gekoppelt sind. Entsprechend kann der erfindungsgemäße genetische Test eingesetzt werden, um den Haplotyp von Dobermann Pinschern daraufhin zu analysieren, ob phänotypisch nicht betroffene Tiere zumindest heterozygot ein dilute Allel tragen, dass für die Farbverdünnung verantwortlich ist.

Die Database EMBL vom 5. September 2001 "*Mus musculus* Melanophilin mRNA" beschreibt die mRNA-Sequenz des Melanophilins der Maus (AC AF 384098).

Die Database EMBL vom 3. September 2006 "Rattus norvegicus Melanophilin mRNA" beschreibt die mRNA-Sequenz des Melanophilins der Ratte. (AC BC 081894).

Matesic et al. (PNAS 10238-10243, Bd. 89, Nr. 18 (2001)) beschreiben das Melanophilingen der Maus. Dabei wurde gefunden, dass bei der Maus eine Mutation im Melanophilingen zu fehlfarbigen Individuen führt. Auf molekularer Ebene führt die Mutation zur Unterbrechung des Transports von Melanosomen in Melanozyten, was zur Folge hat, dass die Melanosomen nicht zu den benachbarten Keratinozyten gelangen können, um ihren Farbstoff übergeben. Die identifizierten Mutationen des Melanophilingens der Maus wirken sich laut Matesic et al. so aus, dass in Exon 2 eine neue Spleißstelle erzeugt wird, wodurch das Transkript um 21 bp verkürzt ist, das reife Protein entsprechend um 7 Aminosäuren.

Die zentrale Rolle des Melanophilingens bei der Entwicklung der Fellfarbe und dessen Orthologie und orthologe Lokalisierung mit dem Melanophilingen des Menschen und des Hundes spiegeln sich jedoch nicht in einer entsprechenden desaktivierenden Mutation im Melanophilingen des Dobermann Pinschers. Vielmehr ist bei der Vorbereitung der vorliegenden Erfindung gefunden worden, dass die Aminosäuren kodierenden Bereiche von dilute Allelen des Melanophilingens beim Dobermann Pinscher mit dem Phänotyp blau oder isabell, die Peptidsequenz des Wildtypallels des Melanophilingens identisch kodieren. Des weiteren wurden im Vergleich mit dem Wildtypallel keine Mutationen des dilute Allels gefunden, die eine Veränderung der Spleißstellen verursachen könnten.

Überraschenderweise hat sich bei der weiteren Analyse des Melanophilingens beim Dobermann Pinscher jedoch gezeigt, dass einige Punktmutationen im Melanophilingen identifiziert werden können, die mit dem dilute Allel der Farbverdünnung gekoppelt sind. Diese Punktmutationen liegen mit einer Ausnahme in nicht-kodierenden Intron-Bereichen des Melanophilingens; die Mutation innerhalb eines Exons führt nicht zur Veränderung der kodierten Aminosäure gegenüber der Wildtyp-Sequenz. Daher wird gegenwärtig von den Erfindern angenommen, dass die Punktmutationen nicht für die Farbverdünnung ursächlich sind, jedoch hinreichend eng mit dem dilute Allel für die Farbverdünnung gekoppelt sind, sodass die Punktmutationen als Marker für das dilute Allel der Farbverdünnung verwendet werden können.

### Genaue Beschreibung der Erfindung

Die vorliegende Erfindung stellt einen genetischen Test für den Genotyp bezüglich der Farbverdünnung von Dobermann Pinschern bereit, der als Marker für das dilute Allel verwendet wird, das für die Farbverdünnung kodiert. Die erfindungsgemäßen Punktmutationen sind in der nachfolgenden Tabelle 1 zusammengestellt; die Numerierung der Nukleotide bezieht sich auf die als Seq.-ID Nr. 1 enthaltene Sequenz des Melanophilingens des Hundes und wurde auf CFA25q24 lokalisiert. Die Seq.-ID Nr. 1 ist eine Sequenz eines dilute Allels (d) des Melanophilingens, das mit der Farbverdünnung gekoppelt ist. Ein entsprechendes Wildtypallel des Melanophilingens (D) kann ausgehend von der erfindungsgemäßen Seq.-ID Nr. 1 in den Genomsequenzen des Hundes lokalisiert werden. Der lokalisierbare Genomabschnitt ist revers komplementär und ohne Annotation von Genen unter der Zugangsnummer AAEX 01037812 in GenBank veröffentlicht (Version AAEX 01037812.1 vom 9. Juli 2004).

Die Nukleotide der Wildtyp-Sequenz sind in der Tabelle 2 angegeben, wobei Intron 1 die Nukleotide 157472 -164131 (**7693** - 14352 in AAEX 01037812), Exon 2 Nukleotid Nr. 164132 - 164262 (14351 - 14221 in AAEX 01037812) und Intron 2 Nukleotid 180796 - 181071 überspannt.

**Tabelle 2: Punktmutationen des Melanophilingens beim Dobermann Pinscher, die mit dem dilute Allel der Farbverdünnung gekoppelt sind im Vergleich mit Wildtypallel**

| Nukleotid, Numerierung in Seq.-ID Nr. 1 | Wildtyp Allel (D) | Dilute Allel (d) |
|---|---|---|
| 163882 | T | C |
| 163889 | C | A |
| 163936 | A | G |
| 163983 | A | G |
| 164012 | A | G |
| 164049 | A | G |
| 164075 | C | T |
| 164258 | C | T |
| 164397 | A | G |

Zur Bestimmung des Genotyps in Bezug auf Farbverdünnung eines Dobermann Pinschers reicht es erfindungsgemäß aus, dass eine der in Tabelle 2 aufgeführten Punktmutationen typisiert wird. Erfindungsgemäß ist bevorzugt, dass zwei oder mehr Punktmutationen im Melanophilingen gemäß Tabelle 2 typisiert werden, um den Haplotyp eines Dobermann Pinschers in Bezug auf die Anlage zur Farbverdünnung zu bestimmen, um so eine noch sicherere Aussage über den Genotyp treffen zu können.

Genetische Verfahren zur Bestimmung des Vorliegens einer Punktmutation sind bekannt, wie beispielsweise die Sequenzierung des Genombereichs, in dem die Mutation lokalisiert ist, mit spezifisch hybridisierenden Primern und Vergleichen der sequenzierten Basenabfolge mit dem der Wildtyp-Sequenz. Die Sequenzierung der genomischen DNA kann unmittelbar aus isolierter DNA des Hundes vorgenommen werden, oder nach Amplifizierung eines genomischen DNA Fragments, das den Bereich der identifizierten Punktmutation umfasst und anschließender Sequenzierung eines Teilbereichs des erhaltenen Amplifikats.

Ein weiteres Verfahren, das zur Identifikation der Punktmutationen verwendet werden kann, ist die Ligasekettenreaktion (LCR), bei der genomische DNA oder ein spezifisch amplifizierter Bereich der genomischen DNA, der die erfindungsgemäßen Punktmutationen umfasst, als Matrize für die Ligation zweier einzelsträngiger Oligonukleotide verwendet wird, die mit der genomischen DNA hybridisieren und im Bereich der Punktmutation mit einem 3' bzw. einem 5' Ende unmittelbar aneinander grenzen, sodass eine Ligase diese Oligonukleotide miteinander verknüpfen kann, wenn sie mit der genomischen DNA im Bereich ihrer gegenüberliegenden Nukleotidenden hybridisieren.

Wenn die Oligonukleotide der Sequenz des Wildtyp-Allels des Melanophilingens auch im Bereich der Punktmutation entsprechen, können diese hybridisieren und zu einem größeren Oligonukleotid ligiert werden und zeigen das Vorliegen ein Wildtyp-Allels zumindest heterozygot an.

Wenn Oligonukleotide verwendet werden, die im Bereich einer möglichen Ligation mit der Sequenz der Punktmutation hybridisieren, so zeigt die Ligation der Oligonukleotide das Vorliegen des dilute Allels für die Farbverdünnung zumindest heterozygot an.

Weitere Verfahren sind RFLP (Restriktionsfragmentlängen-Polymorphismus), Massenspektrometrie (MALDI-TOF), SSCP, DGGE, die Pyrosequenzierung, dHPLC, die allelspezifische Hybridisierung mit immobilisierten Oligonukleotiden (z.B. Genchip), die allelspezifische PCR und real-time PCR mit Schmelzpunktbestimmung des Amplifikats durch Messung der Fluoreszenzveränderung beim Denaturieren.

Für die Ausführung dieser Erfindung durch Nachweis der Punktmutationen eignen sich daher Oligonukleotide, die mit einem Bereich der Sequenz AAEX 01037812, vorzugsweise im Bereich den Melanophilingens hybridisieren, noch bevorzugter in einem Bereich des Melanophilingens, der eine oder mehrere der erfindungsgemäßen Punktmutation umfasst.

Für den Nachweis der erfindungsgemäßen Punktmutationen durch Sequenzierung oder eines der nachfolgend aufgeführten Verfahren eignen sich Oligonukleotide, die mit einem Bereich der Sequenz des Melanophilingens oder angrenzender Bereiche im Hundegenom hybridisieren. Im Falle der Sequenzierung können ausgehend von dem Ort der Hybridisierung die erfindungsgemäßen Positionen zur Bestimmung der Punktmutationen sequenziert werden.

Als ein Schritt bei der Identifikation der erfindungsgemäßen Punktmutationen im Melanophilingen kann die PCR eingesetzt werden, um einen spezifischen Bereich der genomischen Hunde-DNA, insbesondere des Melanophilingens zu amplifizieren, der zumindest eine dieser Punktmutationen umfasst. Entsprechend sind bei der Ausführung der Erfindung auch Oligonukleotide geeignet, die mit einem von der erfindungsgemäßen Punktmutation entfernten Bereich der Sequenz hybridisieren, um einen Abschnitt der genomischen Hunde-DNA zu amplifizieren oder zu sequenzieren, der eine Punktmutation entsprechend Tabelle 1 enthält.

Die Erfindung wird nun im Detail in Beispielen erläutert:

### Beispiel 1: Nachweis des dilute Allels für Farbverdünnung durch Sequenzierung

Zur Identifikation des Haplotyps von Dobermann Pinschern, die phänotypisch dem Wildtyp entsprechen, wird aus der genomischen DNA ein Bereich amplifiziert, der die erfindungsgemäßen Punktmutationen umfasst. Durch anschließende Sequenzierung des Amplifikats kann der Genotyp an allen polymorphen Stellen des Amplifikats exakt bestimmt werden und bei Identifikation der erfindungsgemäßen Punktmutationen kann gefolgert werden, dass das Tier zumindest eine Kopie des dilute Allels d trägt. Wenn die Sequenzierung sowohl die Basensequenz des Wildtyps (D) als auch die Punktmutation des dilute Allels (d) ergibt, ist hat das Tier den heterozygoten Genotyp Dd.

Genomische DNA wurde aus einer Blutprobe eines Dobermann Pinschers mit Hilfe des QiaAmp Blood Kits (Qiagen, Hilden) isoliert.

Das spezifische Fragment dieser genomischen DNA, welches die erfindungsgemäßen Punktmutationen umfaßt, wurde durch Amplifikation mit den Primern Seq.-ID Nr. 2 (5' GTC ACC GAC ATT ATG TCA CAG 3') und Seq.-ID Nr. 3 (5'CAG GCT GGA AGG TCA GAT C 3') durch PCR amplifiziert. Für die PCR wurden 2 µL genomische DNA (ca. 10 ng), je 1 µL der beiden PCR Primer (je 20 pmol), 2 µL 10 x *Taq*-Puffer, 0,8 µL 5 mM dNTPs, 0,2 µL *Taq*-DNA-Polymerase und 13 µl H₂O vermischt. Folgendes Temperaturprofil wurde zur Amplifizierung durchgeführt: 5 min 95°C zur initialen Denaturierung, gefolgt von 35 Zyklen mit 30 s 94 °C zur Denaturierung, 30 s Hybridisierung bei 57 °C sowie 50 s bei 72 °C zur Elongation der Amplifikationsprodukte. Anschließend wurden die Proben weitere 10 min bei 72°C inkubiert und dann auf 8 °C gekühlt. Bei der PCR entstanden spezifische Amplifikate von 598 bp Länge.

Für die Sequenzierung wurden die Amplifikate mittels des Exo/SAP-Verfahrens (Shrimp alkalische Phosphatase, Roche, Mannheim) und Exonuklease I (New England Biolabs, Frankfurt) aufgereinigt. Hierfür wurden 5 µL PCR-Produkt mit 1 µL SAP, 0,05 µL Exonuklease I, 0,2 µL 10 x SAP-Puffer und 0,75 µL H₂O für 15 min bei 37 °C inkubiert und anschließend zur Inaktivierung der Enzyme für 15 min auf 80 °C erhitzt. Von diesem Ansatz wurde 1,5 µL, was etwa 100 ng DNA entspricht, in der anschließenden Sequenzierungsreaktion eingesetzt.

Die Sequenzierungsreaktion wurde mit dem Primer Seq.-ID Nr. 2 unter Verwendung des Dynamic ET Terminator Cycle Sequencing Kit (US81060, Amersham Biosciences, Freiburg) durchgeführt. Es wurden 1,5 µL aufgereinigtes PCR-Produkt, 1 µL Sequenzierprimer (10 pmol), 1,5 µL ET-Mix, 3 µL H₂O und 0,5 µL Verdünnungspuffer (400 mM Tris/HCl, 10 mM MgCl₂, pH 9,0) zusammenpipettiert. Dieser Ansatz wurde in einem Thermozykler mit folgendem Programm inkubiert: Initiale Denaturierung 90 s bei 94 °C, dann 35 Zyklen mit 20 s bei 94 °C, 15 s bei 50 °C und 2 min bei 60 °C, danach Abkühlung auf 8 °C. Die Sequenzierungsreaktionen wurde durch Gelfiltration (Sephadex^{™} Superfine, Amersham Biosciences, Freiburg) aufgereinigt und auf einem MegaBACE Kapillarelektrophorese-Sequenzierautomaten (Amersham Biosciences, Freiburg) analysiert. Zur Bestätigung von unsicheren Ergebnissen wurde das PCR-Produkt zusätzlich in einer weiteren Sequenzierreaktionen mit dem Primer Seq.-ID Nr. 3 sequenziert. Diese Sequenzierung wurde völlig analog zur Sequenzierung mit dem Primer der Seq.-ID Nr. 2 durchgeführt.

Als Ergebnis der Sequenzierung wurde festgestellt, dass bei Dobermann Pinschern mit phänotypischer Farbverdünnung (homozygot für dilute Allel) und heterozygoten Tieren, auf deren Heterozygotie für das dilute Allel durch eine Analyse der Nachkommen rückgeschlossen werden konnte, ausschließlich die in Tabelle 2 aufgeführten Punktmutationen mit diesem dilute Allel gekoppelt war.

Im Unterschied zu den mit dem dilute Allel gekoppelten Punktmutationen von Tabelle 2 wurden noch die folgenden Sequenzabweichungen, die jeweils den angegebenen Basenaustausch bzw. die angegebene Mutation betreffen, identifiziert. Diese sind in Tabelle 3 zusammengefasst, zeigen jedoch keine Kopplung mit einem dilute Allel für die Farbverdünnung.

**Tabelle 3: Punktmutationen des Melanophilingens beim Dobermann Pinscher, die nicht mit dem dilute Allel der Farbverdünnung gekoppelt sind im Vergleich mit Wildtypallel**

| **Laufende Nr.** | Mutation | Nukleotid, Numerierung in Seq.-ID Nr. 1 |
|---|---|---|
| **1.** | A/G | 157471 |
| 2. | A/C | 157486 |
| **3.** | A/G | 180908 |
| **4.** | T/C | 180994 |
| **5.** | A/G | 180998 |
| 6. | T/C | 184650 |
| 7. | G/A | 184751 |
| 8. | G/A | 184818 |
| 9. | C | 186072 |
| 10. | C | 186091 |
| **11.** | G | 186184 |
| 12. | A | 186329 |
| 13. | InDel GGAGGA - | 187448 - 187453 |
| 14. | C/A | 187456 |
| 15. | A/G | 189560 |
| 16. | A | 189575 |
| **17.** | C/T | 189719 |
| **18.** | G | 189728 |
| 19. | A/G | 195548 |
| **20.** | G | 195720 |
| **21.** | A | 195808 |
| 22. | G/A | 195888 |
| 23. | G/C | 195891 |
| 24. | A/G | 195900 |
| 25. | InDel G +/- | 195901 |
| 26. | A/G | 195932 |
| **27.** | A/G | 196097 |
| 28. | T/C | 196354 |
| 29. | C/T | 197343 |
| 30. | C/T | 197510 |
| **31.** | C/T | 197673 |
| **32.** | A/G | 197730 |
| 33. | A | 198080 |
| 34. | A/G | 198126 |
| 35. | A/G | 199847 |
| 36. | C/T | 202837 |

Für die in Tabelle 3 aufgeführten Punktmutationen wurde festgestellt, dass die laufenden Nrn. 1, 3, 4, 5, 7, 11, 17, 18, 20, 21, 27, 31 und 32 (fett gedruckt) in Exons lokalisiert sind, die übrigen in Introns.

### Beispiel 2: Nachweis des dilute Allels für Farbverdünnung durch RFLP

Die genomische DNA wird isoliert, wie in Beispiel 1 beschrieben. Ein 598 bp großes PCR-Produkt wird gemäß der Beschreibung von Beispiel 1 erzeugt.

Die folgenden Restriktionsenzyme können eingesetzt werden, um einige der mit dem dilute Allel gekoppelten Polymorphismen zu genotypisieren:

**Tabelle 4: Restriktionsfragmente bei RFLP**

| Restriktionsenzym [Erkennungsstelle] | Diagnostische Position in Seq.-ID Nr. 1 | Restriktionsfragmente dilute Allel (d) | Restriktionsfragmente Wildtypallel (D) |
|---|---|---|---|
| *Sac*II | 164049 (A/G) | 190 bp | 598 bp |
| [CCGC↓GG] | | 408 bp | |
| *Aat*II | 164397 (A/G) | 60 bp | 598 bp |
| [GACGT↓C] | | 538 bp | |
| *Mae*II | 163936 (A/G) | 27 bp | 47 bp |
| (oder *Hpy*CH4 IV) | 164075 (C/T) | 47 bp | 128 bp |
| [A↓CGT] | 164397 (A/G) | 63 bp | 167 bp |
| | | 193 bp | 256 bp |
| | | 268 bp | |

Im Folgenden wird die Genotypisierung am Beispiel des *Sac*II-RFLP beschrieben. Für die anderen aufgeführten Enzyme wäre analog zu verfahren. Es werden 10 µL des PCR-Ansatzes mit 2 µL Mikroliter 10 x Puffer, 1 µL *Sac*II (Puffer und Enzym von New England Biolabs, Frankfurt) und 7 µL H₂O vermischt und für 1 h bei 37 °C inkubiert. Anschließend wird der Ansatz durch Gel-Elektrophorese auf einem 1,2% -igen TBE-Agarosegel mit Ethidiumbromid aufgetrennt. Die DNA-Banden werden unter UV-Licht sichtbar gemacht und die Größen der entstandenen DNA-Fragmente werden durch Vergleich der DNA-Banden mit einem Größenstandard bekannter DNA-Fragmente beurteilt. Bei dem Experiment wird durch das Mitführen geeigneter Kontrollen (DNA von Tieren mit bekanntem Genotyp, jeweils 1 Tier DD, 1 Tier Dd und 1 Tier dd, sowie eine Probe ohne genomische DNA) sichergestellt, dass nicht experimentelle Fehler zu falschen Ergebnissen führen. Im Falle des *Sac*II RFLPs sind drei folgende Untersuchungsergebnisse möglich:

**Tabelle 5: RFLP mit SacII**

| | | | |
|---|---|---|---|
| Bandenmuster auf Gel (bp) | 598 | 190 + 408 + 598 | 190 + 408 |
| Genotyp des Tieres | DD | Dd | Dd |
| | (Wildtyp, reinerbig) | (Anlageträger für dilute Allel) | (Merkmalsträger) |

## Patentansprüche

1. Melanophilingen des Hundes nach Seq.-ID Nr. 1 mit mindestens einer Punktmutation, ausgewählt aus der Gruppe, bei der Nukleotid 163882 T anstelle von C ist, Nukleotid 163889 C anstelle von A ist, Nukleotid 163936 A anstelle von G ist, Nukleotid 163983 A anstelle von G ist, Nukleotid 164012 A anstelle von G ist, Nukleotid 164049 A anstelle von G ist, Nukleotid 164075 C anstelle von T ist, Nukleotid 164258 C anstelle von T ist, und Nukleotid 164379 A anstelle von G ist.

2. Analyse des Genotyps eines Hundes bezüglich der Farbverdünnung durch einen genetischen Test zur Identifikation mindestens einer Punktmutation in Seq.-ID Nr. 1 des Hundegenoms, wobei die Punktmutation aus der Gruppe ausgewählt ist, bei der in Seq.-ID Nr. 1 Nukleotid 163882 T anstelle von C ist, Nukleotid 163889 C anstelle von A ist, Nukleotid 163936 A anstelle von G ist, Nukleotid 163983 A anstelle von G ist, Nukleotid 164012 A anstelle von G ist, Nukleotid 164049 A anstelle von G ist, Nukleotid 164075 C anstelle von T ist, Nukleotid 164258 C anstelle von T ist, und Nukleotid 164379 A anstelle von G ist.

3. Analyse nach Anspruch 2, **dadurch gekennzeichnet, dass** die PCR eingesetzt wird, um einen Bereich der Seq.-ID Nr. 1 zu amplifizieren, der zumindest eine der Punktmutationen umfasst, die aus der Gruppe ausgewählt ist, bei der in Seq.-ID Nr.1 Nukleotid 163882 T anstelle von C ist, Nukleotid 163889 C anstelle von A ist, Nukleotid 163936 A anstelle von G ist, Nukleotid 163983 A anstelle von G ist, Nukleotid 164012 A anstelle von G ist, Nukleotid 164049 A anstelle von G ist, Nukleotid 164075C anstelle von T ist, Nukleotid 164258 C anstelle von T ist, und Nukleotid 164379 A anstelle von G ist.

4. Analyse nach einem der Ansprüche 2 bis 3, **gekennzeichnet durch** Amplifikation eines Fragments der genomischen DNA mit den Primern Seq.-ID Nr. 2 und Seq.-ID Nr. 3.

5. Analyse nach Anspruch 2, **dadurch gekennzeichnet,dass** Punktmutationen durch Sequenzierung oder die Ligasekettenreaktion bestimmt wird.

6. Verfahren zur Bestimmung der genetischen Anlage zur Farbverdünnung eines Dobermann Pinschers, **gekennzeichnet durch** die Verwendung des Melanophilingens des Hundes nach Seq.-ID Nr. 1 mit mindestens einer Punktmutation, ausgewählt aus der Gruppe, bei der Nukleotid 163882 T anstelle von C ist, Nukleotid 163889 C anstelle von A ist, Nukleotid 163936 A anstelle von G ist, Nukleotid 163983 A anstelle von G ist, Nukleotid 164012 A anstelle von G ist, Nukleotid 164049 A anstelle von G ist, Nukleotid 164075 C anstelle von T ist, Nukleotid 164258 C anstelle von T ist, und Nukleotid 164379 A anstelle von G ist.

7. Verfahren nach Anspruch 6 zur Auswahl eines Dobermann Pinschers zur Zucht.

## Claims

1. Melanophilin gene of the dog according to Seq ID No. 1 having at least one point mutation, selected from the group, in which nucleotide 163882 is T instead of C, nucleotide 163889 is C instead of A, nucleotide 163936 is A instead of G, nucleotide 163983 is A instead of G, nucleotide 164012 is A instead of G, nucleotide 164049 is A instead of G, nucleotide 164075 is C instead of T, nucleotide 164258 is C instead of T, and nucleotide 164379 is A instead of G.

2. Analysis of the genotype of a dog in respect of colour dilution by a genetic test for identification of at least one point mutation in Seq ID No. 1 of the genome of the dog, wherein the point mutation is selected from the group, in which in Seq ID No. 1 nucleotide 163882 is T instead of C, nucleotide 163889 is C instead of A, nucleotide 163936 is A instead of G, nucleotide 163983 is A instead of G, nucleotide 164012 is A instead of G, nucleotide 164049 is A instead of G, nucleotide 164075 is C instead of T, nucleotide 164258 is C instead of T, and nucleotide 164379 is A instead of G.

3. Analysis according to claim 2, **characterized in that** PCR is used for amplification of a region of Seq ID No. 1, which contains at least one of the point mutations, which is selected from the group, in which in Seq ID No. 1 nucleotide 163882 is T instead of C, nucleotide 163889 is C instead of A, nucleotide 163936 is A instead of G, nucleotide 163983 is A instead of G, nucleotide 164012 is A instead of G, nucleotide 164049 is A instead of G, nucleotide 164075 is C instead of T, nucleotide 164258 is C instead of T, and nucleotide 164379 is A instead of G.

4. Analysis according to one of claims 2 to 3, **characterized by** amplification of a fragment of the genomic DNA with primers of Seq ID No. 2 and Seq ID No. 3.

5. Analysis according to claim 2, **characterized in that** the point mutations are determined by sequencing or by the ligase chain reaction.

6. Process for the determination of the genetic disposition for colour dilution of a Dobermann Pinscher, **characterized by** the use of the melanophilin gen of the dog according to Seq ID No. 1 having least one point mutation, selected from the group, in which nucleotide 163882 is T instead of C, nucleotide 163889 is C instead of A, nucleotide 163936 is A instead of G, nucleotide 163983 is A instead of G, nucleotide 164012 is A instead of G, nucleotide 164049 is A instead of G, nucleotide 164075 is C instead of T, nucleotide 164258 is C instead of T, and nucleotide 164379 is A instead of G.

7. Process according to claim 6 for the selection of a Dobermann Pinscher for breeding.

## Revendications

1. Gène de la mélanophiline du chien d'après la SEQ ID N° 1 avec au moins une mutation de point, sélectionnée à partir du groupe dans lequel le nucléotide 163882 est T au lieu de C, le nucléotide 163889 est C au lieu de A, le nucléotide 163936 est A au lieu de G, le nucléotide 163983 est A au lieu de G, le nucléotide 164012 est A au lieu de G, le nucléotide 164049 est A au lieu de G, le nucléotide 164075 est C au lieu de T, le nucléotide 164258 est C au lieu de T, et le nucléotide 164379 est A au lieu de G.

2. Analyse du génotype d'un chien au niveau de la dilution de couleur par un test génétique destiné à identifier au moins une mutation de point dans la SEQ ID N° 1 du génome du chien, sachant que la mutation de point est sélectionnée à partir du groupe dans lequel dans la SEQ ID N° 1, le nucléotide 163882 est T au lieu de C, le nucléotide 163889 est C au lieu de A, le nucléotide 163936 est A au lieu de G, le nucléotide 163983 est A au lieu de G, le nucléotide 164012 est A au lieu de G, le nucléotide 164049 est A au lieu de G, le nucléotide 164075 est C au lieu de T, le nucléotide 164258 est C au lieu de T, et le nucléotide 164379 est A au lieu de G.

3. Analyse selon la revendication 2, **caractérisée par le fait qu'**on a recours à la PCR afin d'amplifier une zone de la SEQ ID N° 1 qui comprend au moins une des mutations de point sélectionnée à partir du groupe dans lequel dans la SEQ ID N° 1, le nucléotide 163882 est T au lieu de C, le nucléotide 163889 est C au lieu de A, le nucléotide 163936 est A au lieu de G, le nucléotide 163983 est A au lieu de G, le nucléotide 164012 est A au lieu de G, le nucléotide 164049 est A au lieu de G, le nucléotide 164075 est C au lieu de T, le nucléotide 164258 est C au lieu de T, et le nucléotide 164379 est A au lieu de G.

4. Analyse selon une des revendications 2 à 3, **caractérisée par** l'amplification d'un fragment du DNA génomique avec les séquences primaires SEQ ID N° 2 et SEQ ID N° 3.

5. Analyse selon ls revendication 2, **caractérisée par le fait que** la mutation de point est déterminée par séquençage ou par la réaction de ligature en chaîne.

6. Procédé destiné à déterminer la prédisposition génétique à diluer la couleur d'un Dobermann Pinscher, **caractérisé par** l'emploi du gène de la mélanophiline du chien selon la SEQ ID N° 1 avec au moins une mutation de point, sélectionné à partir du groupe dans lequel le nucléotide 163882 est T au lieu de C, le nucléotide 163889 est C au lieu de A, le nucléotide 163936 est A au lieu de G, le nucléotide 163983 est A au lieu de G, le nucléotide 164012 est A au lieu de G, le nucléotide 164049 est A au lieu de G, le nucléotide 164075 est C au lieu de T, le nucléotide 164258 est C au lieu de T, et le nucléotide 164379 est A au lieu de G.

7. Procédé selon la revendication 6 destiné à sélectionner un Dobermann Pinscher pour l'élevage.


### SEQUENCE LISTING

<110> Stiftung Tierärztliche Hochschule Hannover
<120> Nachweis der genetischen Anlage für Farbverdünnung beim Dobermann Pinscher
<130> N1017EP
<160> 3
<170> PatentIn version 3.1
<210> 1
   <211> 212696
   <212> DNA
   <213> Canis familiaris (dog)
<220>
   <221> startcodon
   <222> (164153)..(164155)
   <223>
<220>
   <221> exon1
   <222> (157302)..(157471)
   <223>
<220>
   <221> exon2
   <222> (164132)..(164262)
   <223>
<220>
   <221> exon3
   <222> (180796)..(181071)
   <223>
<220>
   <221> exon4
   <222> (181137)..(181249)
   <223>
<220>
   <221> exon5
   <222> (184662 )..(184786)
   <223>
<220>
   <221> exon6
   <222> (186177)..(186293)
   <223>
<220>
   <221> exon7
   <222> (187395)..(187581)
   <223>
<220>
   <221> exon8
   <222> (189595)..(189740)
   <223>
<220>
   <221> exon9
   <222> (195626)..(195811)
   <223>
<220>
   <221> exon10
   <222> (196074)..(196229)
   <223>
<220>
   <221> exon11
   <222> (197573)..(197665)
   <223>
<220>
   <221> exon12
   <222> (198205)..(198282)
   <223>
<220>
   <221> exon13
   <222> (200004)..(200058)
   <223>
<220>
   <221> exon14
   <222> (201833)..(201936)
   <223>
<220>
   <221> exon15
   <222> (202756)..(202792)
   <223>
<220>
   <221> mutation
   <222> (163882)..(163882)
   <223> T= Wildtyp (D), C = Farbverdünnung (d)
<220>
   <221> mutation
   <222> (163889)..(163889)
   <223> c= wildtyp (D), A = Farbverdünnung (d)
<220>
   <221> mutation
   <222> (163936)..(163936)
   <223> A= wildtyp (D), G = Farbverdünnung (d)
<220>
   <221> mutation
   <222> (163983)..(163983)
   <223> A= wildtyp (D), G = Farbverdünnung (d)
<220>
   <221> mutation
   <222> (164012)..(164012)
   <223> A= wildtyp (D), G = Farbverdünnung (d)
<220>
   <221> mutation
   <222> (164049)..(164049)
   <223> A= Wildtyp (D), G = Farbverdünnung (d)
<220>
   <221> mutation
   <222> (164075)..(164075)
   <223> C= wildtyp (D), T = Farbverdünnung (d)
<220>
   <221> mutation
   <222> (164258)..(164258)
   <223> c= wildtyp (D), T = Farbverdünnung (d)
<220>
   <221> mutation
   <222> (164397)..(164397)
   <223> A= wildtyp (D), G = Farbverdünnung (d)
<220>
   <221> stopcodon
   <222> (202790)..(202792)
   <223> A= Wildtyp (D), G = Farbverdünnung (d)
<400> 1 
caaacatgct tggcgagctc cctctgcaga tgcccaaacc ccttccacgc gatgacattt 60

tgcaaccgcg gcctgagggt tttcacagac acctctgatt taaggcagga aaataatatc 120

tattttccag gttgagtatt acttggaagg tgctctcgtt atgcacagag gccctttctc 180

ccttggctgt ttcccattac ccaccaccaa atgtctccgg gctacaaccg gacttgaagt 240

actccgtctc tggctggtat ttcgctcctt ggcaatttta cgttgaccat gtgggttaca 300

aaatcaggaa caaggtggaa aggcatccct gaaccggctc cacacagggc ctctgaacat 360

ctgaaggact gtcccaactc tggagtcatg attccaaatg gacagaagac cccctgggaa 420

tccaatggtg ctcggaggaa ggtgagaggc acagtcagcg ttaaagctca gaccagcaaa 480

ctgagatcgg gaaagaagca ggccctgggc agccccggat ggtggctcag cggctcccac 540

accaccagca gcccccaccc tctctggaag ccgcacccag atgtctgggc cccagatacc 600

gtggatcccg aggaacaagc aggtccagct ggaaatctga caacaaggag aatttggcag 660

ggaaatctga tccattccct gaaaatgaat caccacggga gaagaaattt aaaattgccc 720

tcctgggttc atgatattgg cttcctatga aaagactggt ttccattcgt gcttattatg 780

atttcatttc atttcatttt atttatttat ttatttattt aatttttatt ttcacttttt 840

ttacatattt tttaattgga gttcaatttg ccaacatata gtgtaacacc cagtgctcat 900

cccatcaagt gcccccctca gtgcccgtca cccagtcacc ccatccccct gcccacctcc 960

ctttccacca ccccttgttc gtttcccaga gttaggagtc tctcgtgttc tgtctccctt 1020

tctggtattc cccactcatt tattattttt tagttctgta ggaagttgtt ggcgagaggg 1080

gagcagaagg cacttatgtg cacaaatcca gcaaagatga agatgaatgg actgaaaggg 1140

gctaatactt gggtccaaac agatgtcccc atgatctggc cacagaggct caaacaggcc 1200

acccactact cacgccctaa acttcagcca aatatccacc ttctctgagt ccttccctga 1260

gaagagcagt gctggataat gagagcgaga tgagaatagt tgtaataaaa ggaaggagga 1320

cagatggaga gagactctct gaccaagaag agtaacacta ggtcttgggc agggtcctgg 1380

gtagggcgtg ctttgtggag gaagaagata gagctacagc tgaaatccag cacccaggga 1440

agcagtccga agctcctgga gaatgatggt gagggtcccg taagaaccaa cacgtcattg 1500
 
gaaggtctgg agggcttcca ggtcacaatg atacagcccc cagtagattg ttagctggat 1560
gaactggacc actctgaccc cccgtttcat catttgtaaa actagggaag tgcctgctac 1620
cagacagcac agccttgggt gaagcaccca atacattgcc tgacacacag ccactgctgg 1680
atagaagtga attcaatttc acactagctg ctaggaggga ccaaaccctt agggcatcgc 1740
cccgagagaa gattaggtct tgctcaccaa atccagaaca ggagttgcaa gccacatcta 1800
cacggccaga ctggaagttc tttgaagcag ccccagaaag aagctcatgg ccattcgggt 1860
caggggcaca cccagggtgt cagggagggc tgcagggtct gtggcatggt ctggcaaaag 1920
acaagtcttg aaaagaagtt ctcagctata aggggtcagg tttcctgctg ctcagaccac 1980
actttgctga atgaaaagtc atcacagatg atgtaaaaca agaactttag tccctctgtg 2040
agaataaatg tgaaaaactg aacacaaagg ttatatcaat ttttgtatgt tttggatcaa 2100
aatgtaccca gagcctgtac ttcatctcct ctgctatttc ttggccttgg ccactgttgg 2160
cctctcacgg tcacctgaat ttatgccaca gtgtcgtcac tggtgccacc atctcattgc 2220
tctgatgtcc tgtcatgcac aggagctagg gggcctgtta gaattcttgg cagagcatgc 2280
atttgccgct gagcagtggc tcccttgtca ccgagcgaaa gctagccagc ttacgtggcc 2340
accagtgcac tccctggcct tcccttcact tgtcacagcc caacagtggc cttcccgggt 2400
gctcttccta cactagagat gcatctgggc tctaggtccc cctctgtcca gaaccctcct 2460
cggttagcca cctggccacc tcccttacca tccccatgtg tttttagttt tttaattttt 2520
tctcacagtg gactccccag ccaggtcccc gtggtcaccc tgctggaatt cggtggccac 2580
ctccacctcc agcacaacca gctcccatag tagttgggca cgggcttcat gtcacctttc 2640
aactcttgtc tgtctcccgc catgagaaga cgagcccctt ggggacacac acgatatcct 2700
cagtatctat caaatgaatg aacataactg attgtctctg ttgaacaaaa cagaattaca 2760
tgaaaatggg atctttgtac taaaattgag aatctggcgt ggctgagctc actgatggct 2820
gctcaactcc attttgtaat taatatgggg gcaaaaggca acttggagtt tagggtctag 2880
gtggaaagga agggacagca tggtagctgg ccaggaaaat ttcgtttcat tttcttttaa 2940
tatgttttct ctttgttaga aagaaacaca tgtttgtgtg tgtgtgtgtg tgtgtgtgca 3000
tgttatatat gcaatcattg tattaaacat acagatttgt gtcctttttt tttaacattc 3060
taccataaac cacaattgat taatggcatg ggccatttgt caacattatg acatatttgc 3120
cagctactta tttttgttgt tgttggcaaa gcaagctgaa tttaagcatt cccttgttgg 3180
gcattttgat cacctcgaat tgatttttat catctcaaat aaaatcgcat ggaatacttt 3240
tgaaagagaa cctttgcgca tccctgattg ctggcaaagt tgtggaaact tgcacacact 3300
ccgccagttg ggattataga aattttgcac atcttgagtg tgcggagggt aaggtaggta 3360
cgttcatttg tcaaaactca tcaaacaata cacataaatg gatacatttt atcgcatgta 3420
aagttgagtt tttaaaaaat gtgatggggt gaatagatgt tccttgaata gtgcaagaag 3480
catgacaccc accgttcgtc ttcttgggct tttgttttcc cttccaaagt acccacacca 3540
 
aggagtgggc tggaacgtcc aagggtatgg agggttacaa gcagctgggt gagggaagag 3600
gaaggagaga tttgaggcag ctgctggggg tgaggtcatc tggggccaag gcagcttgtg 3660
caatggaatt tgtgtgaagc aaaccatgga acatgtgaga aacttctctc cagacccctc 3720
aacgtgtctt cagcaaaatg tccacctgtc actaacgtgt ggtgaggcca gcttcgcatg 3780
ctattggggt aaggacctgg gggtgaggag ggaagtgcaa tgttcagccc atcacaaaag 3840
ggggccagag cccacagctc tgtgctcatt gcatgcctaa ggaggctggt gttgcctgcc 3900
tggtagatcc cgcgtgctca cagtggatca ccaaaggctc agctcagaac acactgccct 3960
gaggactcca cagtcactgc tgaggcagaa gaggaccatg ggataggtcg cctttagagg 4020
gcaaagcagt gattgctttt gtcacctcca ttggggaagg gaaggaactc aattccaaag 4080
gtgacaggaa ggggcaggaa ggggcagaaa gggtggtggg aagaggcagg aagggaggaa 4140
gaagaggcag gtagggtggc aggtagaggc aggaagggtg ggagtaaggg acaggaaggg 4200
taataggtag aagcaggaag agtggcaggt agaggcaaga atggtggtag gaagaggcag 4260
gaagggtgga aggaagagct aggaaagttg aatgaaaggg gcaggaagga tgggagaaga 4320
gtcaggaagg gtggaaggaa ggggaaggaa aagtggcagg aagaggcagg aagaggtagg 4380
aaggggcagg aaggctgcca gaagccacaa caccctagct gggccccagt ttcccccggt 4440
gaacagcacg tgtggccacg agcccacctg ggagcctgct gcggagcgcg cccagggcgg 4500
ggtgggcgac agccgggcag cagccgaatg ccagcactgc ccagcccggg gacccacggg 4560
gtgctctagc acggatctcc ccgggcctgg ggacagaggg gacttgggga gagagcaagc 4620
cccgggctcc tccactccga ccctatgagt gtagcctacg ctgtggctca cagtaaaagg 4680
ctgtggagag tatctgggat tttatttttc tttttacttt actttttaa ttttaactat 4740
ttagtcatgg ggacacaga gacagaggca gagacacagg cagagggaga agcaggctcc 4800
ttgcagggag cctgacgtgg gactcgatcc cgggacccca gggtcacgcc ctgagctgaa 4860
ggcagacact ggaccgctaa gcccccaggg catcctggga tttaagtttt aaaatgaatg 4920
ggccccagcc ttgagatcca tctgtgacgg ttttcataac ctcaagtgac ttgacagtga 4980
taggattcag ccgaaagccc tcactggggg agccgctggc cccggagcat cgggccgggg 5040
ggcgggtggg ggcaggtatg gaaatgctaa cgctgtctta tgggctcaat tgtgagctcc 5100
cttgccctcc tcacccccca aaaaagagat agatcagaag tcctaatccc ccagtaggtc 5160
aggctatttg caaatagggt caacgcagct ctaactagat ggggtgaggc caccctggtg 5220
tagggcaggc cctgatccca tacaacccat gtccttgtga gaagacagcc atgtgcacac 5280
gggggcagaa tcggggggag gcagctgcaa gccccggggt cgccagaaag ccaagaagca 5340
agaccgggtt tcctgcagat ttccggggag cacggccctg ccaactcctt gattttggac 5400
gtgcagcccc caaagctgtg agagtccagc tctgtcgccg tagctgccga gcccatggtc 5460
accgcctaca gcagccccgg cgacggccca cctcgcaccc gggcccaggg atctcccctc 5520
cccagtcccc actgcaaacc ttcttgcccc catgcatgac ctcggcttgc acacagcttg 5580
aggggaggtg caggcgtatt tacaaggaaa aggggctgtt cccccggggg cggggaactt 5640
 
tgggggcgct gtccctgaga acttcgcaga gtgcacattc cccccacccc accccaggct 5700
gaacatgcac ttgaagggca tttgtgacca tcgccgagct ctgtgtaagt cccctgggct 5760
ggccttatct tcaaaagggg cccttaaaga ggagggggtg tcagagtgac ctcaagggca 5820
gagagagccc ctgggaagga agggttgctc acccccagta gggaagggga gaggaacccc 5880
acagggctcc tgtgcaggat ggagcagtcg tgtctggggg gcaccctgat ggtgaaagca 5940
tctgaggagt tcccgaagct ttttggttac tcggaagctc ccctgatgtc caagcaggct 6000
ctcagtccca aagaaggcag gtaaactcac ttccccgatg tcctatgaaa tcctgagtca 6060
tccctcaaat ttctgccaat tccccatggg ggaggggggt tgggggagct cctgcaacca 6120
ccccgtcctt ccccttctct gtctccgcgc taccttgaca tggccacaaa gctcagttct 6180
tcccccgctt tctaggcaga tgggccaccc tgagttcatt ttgagaattt agaaatttca 6240
cccaaagatc tgggtggaag tcacagggag tggatcacag ggggctcggc acactctctg 6300
acctcctgtc tttccactcc gtagatggac tgacggacac atccccatcc tacataaggc 6360
ggggctgccc acgcccaagg tccctgcatc tgtcgggagc atttgtcaga gcaggtgtgt 6420
cactaacgca gcctctctcc taagtaggct tcagatgcca cctaaacatg tatttattta 6480
tgtgttactt cttttatttc ttttttcttt tttttttttt tttttttttt tttttaggat 6540
tttacgtgtt tatttgagcg tgagaccaca agcaggggga gggagaggga gaagcagact 6600
ccccgctgag caaggagccc ggcgtggggc tcggagtcag gacctgggga tcacgacctg 6660
agccgtgggc aggtgcttca tcaatggagc cacccagcgc ccgctcttgt gttacttctg 6720
aagatgggag gaaaaggtct ttaaggaaat aaataaataa ataaataaat aaataaataa 6780
ataaataaat aaataagtaa gtaattttgc acttggtctt tgagactata gaaaagggct 6840
agtgtttcct ggccaatggt ttctcatctc tcagccaccg tggggcggga gcaaacccaa 6900
ccctgggaaa catgactaag aagaagcagg tagttattct gtatgttggc aaattgaaca 6960
ccaataaaaa ataaatttat tattaaaaaa aataaaacaa aattacaata ccaatatcac 7020
tcctaaaaat aaaactatta acagaggaaa agggttaaaa aaaataagaa gaagcaggta 7080
aagagcccat ggctgtgggt tcaaccagaa ggtagagcac aggataaaag ccacctggtt 7140
ctgaagatca caaagattcc agaaagggtc ctaagctggg aaggtgaccc accgcgtccc 7200
agagccctgc agggccaggg ggctgagccc cggtctcccc tcactccctg atggagcaga 7260
gtcccctgtc cgccctggga acaaaaggat agatgccagg ctgggcccca ctcccctggg 7320
ccgggggtca tcgtgacagc aggtgctatt atagggaaac aagacagcca gtcacaccaa 7380
acccatgaca ctttcaatat actttattaa aaagtttctt tgtataaatt tcctaaaatt 7440
tttaaaatta aaattaatca ccctcccccg ccccgcacaa aaaaaaataa taaaccacaa 7500
cacacaacat tagaggaatg ccaaaaatat tctctattat gactttcttt tttttttcat 7560
tctttaatga aggcattggt ctcacaacag tgcacaaaga tgaagggatt tttgcttcct 7620
tctaactggg tcatttgtca gaggctttgg aaaaggaaaa atcggcttga agtctaatct 7680
 
gggaactggg gagggtgggg tggcggcggg aattgaaaag cgggactctt gtttgttccc 7740
catgcgaggc tttccaccta acgtcgtgcc tactggagag ggaagggatg aaccaagcag 7800
aagcatacag agccgcggtg agtggggagt cctattctag accttgcgct cgccccaaag 7860
tggcatagtg tgaaactccg gggtggggag gcaggggccg gggggcgggt ctgagtttat 7920
agaaatgttc acataaacac cagcagtggg taactattac acaacgttca aagtatacga 7980
tataactgcc tggagacgga gagcacggag tccacagaca catttagcac catatcgata 8040
ggtcatgctg cagatcggtt cctcccaagt tcgagggcaa atcaaagcag gaaatggaac 8100
agtgcgcgga tgtctatacc cagagcttct acagagacga ctcgccacgg ctgactcctt 8160
cttcttcaag aggtatgtgg tcccggtcgg ccacgcttag gtccccatgg cagcgatgac 8220
cccggggttg acgagcgctg agcgtcggtg gaggggcagg ccggggcggg gggggggggg 8280
gtgagagaga agtgcagggt gatattattg atggaaaagg atgcagcatt tcttaccaca 8340
actgtcccgc ggcctctggg tttcccccct aaccttgctt ttctgctgag tgccttgcgt 8400
ggcccccctg gttctccctg gccatgggtc tggggccttg tgggcagtct gagcctgtct 8460
gctgcctcgc ctggcccccg ggcctttgca cacactgcgc tttccctggg ctctcaggga 8520
ggccgccctt accacatcag cagtcaggct gcccaacgtc acggagatca tgggtggccc 8580
ctgtgccaac attagtatca ctcaaagcgt gatcatttaa cgatgaagag ggagggatag 8640
ctgggccctg tgtttcttgg tcggcaagaa tccaagtatt acagggatgt ttttaggaaa 8700
tgatactagt tcagaaaatg aatttcctgt gttctttttg agtatcttcc ttctctaaag 8760
acaccaccga cctcctctgt catctctctc tctctcgtaa tcatcgtcgt tcaactatct 8820
taaccgaggt gcatagtcgg cccggatcgc ttctctgatt tcgcaaaagc agcaaacacc 8880
aaagaagaat cagccactta ccaggagtgc aaaccgtttc acattctttc tgtgaatcga 8940
atctgttttc gttgccgctg caacctccgt accagaatct catgcagctt ttcgtctcca 9000
cgtcgtagta ccattttaac atgaatttcc tgcacgtgcc ttcttctttg ggcaacttgc 9060
atatgtcttt aatgacagga ggaggaaaga gcgtttatga ggacatgcta gggacctcac 9120
ccccagcgca tggggctgga ggtctcaaca caaggctgtc aggacgagga ggaggagagt 9180
tccgaggagg gaacccctct tggcctcccc gaggccccca tctccgccct tgcaccatgg 9240
ggtgcacctg attctcaccc acagcaataa tacaaccacc tccatggcag gagctcacca 9300
acctccctcc gatcccaaac gccagccatc gttattggat aatttcaggt aaaatgagct 9360
acatagcttc cctttccggt cccagggcca tctgccttcc ggtactttca atatgaacac 9420
cccagcgaag aaactgcaac tcaaagctat ggcgctctct actcctccgc aaaatagcag 9480
ctgtgttctc tcagaatata gctcacaaat gaggccgccc atttcaccac gagccgagca 9540
gatgggaaat aaaagttaaa gtggaaaaga gagatccttt ttgagtgtgt gcatctgcgt 9600
gcacaagtgt gtgtctgtgt gtagctgagg gccttcaaga gcagccaagt taaacaagta 9660
agtaaaagaa gtgtcgccac tgataaaaaa cccacgacgg caaaaatgtc acagagcaaa 9720
gctattttga agacacagaa gcattctttt tccagttaag gaagtttatg ttttcaatgc 9780
 
atacttctga tgcagcccag ctgcaaagca aaagccacgc tctctccatg atgcctggaa 9840
agaaaagttt acaggccttc cacgataaaa aaaaaaaaaa aaaacagctc tgtttacaga 9900
gttttctggc aaactctaga tcatgctggg aaagagaggc attaatgtca accattatct 9960
gaaagcttga aaactgggaa ccttcctccg acagaactct ctggagtgga cgacgctaca 10020
agtttatggg agcggccaac atgctctccc tgcgcacggc tgacatctcc atgggcctat 10080
tcacgtgggc ggctcacagt tgcagaagcg gtgagtgtct gcagatctgt tacaccaaga 10140
agtaaaagga aaccaaagcc ttccgctaga gaaccaaagc ttgggtcaac tgtcaggcac 10200
cagacgcaga cgtacagagg actgaggctc agaattgacc taacacactc catgatcatg 10260
tggggaggcc atcacccagg cacagagact gggccgcctc ctccccctgg tatctcctca 10320
gtgctgacac tctcagaagc ccagaaagct agaggcccta ccaagagcct ttggaaggac 10380
cagtggagcc agaataaaat gatggcaatt cacaaaaata actttttccc cttcaaatgt 10440
gccaataact gaaggaacat gcctgggtct caagaaatgt catacaaggg actgagaaag 10500
gcaaaattat tcaaccacca aaactgactc tttgtttgcc ggtgggttcc cattttagtg 10560
tgaagctcaa gccaaatggt tgcctcatcc cctctctttc tgcccccaag catcccccac 10620
atggctcatg ttcgtcctat ctttgttgca aaagctctca gtcctggcca cttgtgttta 10680
gctatttcat tcccactcct ccgtgttctc cagaaatgga catgggaaga cttcccagac 10740
ctgaggacac acaacctgga ggctgccctg tggaaggaca ctctgcaccc tgaatgggtg 10800
gcttttccag cttcctagga attgcacctt aactgtccag catcaaacta ttgctcttgc 10860
attccaaacc ctatatgcca atgacagtcg ctgctaaggc cctgtagctc ctactaacca 10920
tgacccaagg gcgctggcac tgtggacacc cagatatttg ttagaccaag aaaaacatat 10980
taacgagggc cccacactgt taaaagacca aaatgtagga ttcttcattt ttaagaaaga 11040
aaacgggagg cttggtcctt aagaagcctt gtagttttaa actcccatca tcttcatttc 11100
tgaaacgcat cgtgtagact ggactcacca aagctgtgtc tatgctgaaa caaccattaa 11160
ttttaaagac ataaaaatca aaatattttg tttccaaaga agtgtggttg gacagacatc 11220
atggagctct tccccaggac aagtctttgg acacaacagt agctcatgtt ctcactccaa 11280
aatgggcttg agcccggagt cctctccaat gtgagaggag ggtctggctg cacatgcact 11340
aattttctcc tccttatact gagaacccaa gaggagccaa aaggacaata aagagtgaaa 11400
tttatcaata acattttctg attcactttt gccccaaata acaatgtaag tacgtttcat 11460
cttaaaatat agacttgggg gctgcctcat tccaaccacc aatgggtgaa actttctgtc 11520
caaatagtga ttggtcactg ggcaccagtg agcatccaca cacaagacat ctggaaaacc 11580
ctgccaggta atcccacctg ttcacagaag ccccaggagc caacctagga gtcctagaac 11640
agcagccatg gggtggaagg caccctagga aacacggtct atttttgtaa tggcaacgtg 11700
tgtgtgtgca cacgtgtatg tgcaagccca cgtgtcagat ccgtggaaga atcaagaggg 11760
atattcattg ggtcattact ggtggtcatc agtgctgcaa ccatgggaat cacattttct 11820
 
taggttttcc tgtgtgttcc gggtcctcca caatgaatat ctaggatatt actctttgaa 11880
acagaaaacg tattccattg gaaaatgttg cctattttta tagtttcagg tttctatagt 11940
gtctaagggc agcattttag aagttatcag tctggacatt tcccaagcca acaagtgatc 12000
agtatttaag tgcttccttg agcaaaactg gtgtgtttga tggggcgggg gcaggtgttt 12060
atgctacaat tgtaaatacg cttctcatag atttaagggt tttaaacaca agaaggttta 12120
acggtcttca gacccagcat tgttgatatg gaatcaggtg cttccggccg cagaagcagg 12180
tacaatttgc ctggatgaat gaatgcctga gtctacattt tgagcatcaa ctgactcaaa 12240
aggtaccaag gaaactttat tttaaatgta gttgcagtta ggtttgagag cctttgcttt 12300
ttttttacat cttggctcgt agatttttat ttttcacaat taaaaaaaaa aaaaaaaaag 12360
gcaaacaccc tgtgaggctc atcatgaccc aaacactgcc atggatatag acacattctc 12420
acatggtaac aagcacacag ccttgcggat agctatttct cttttaccaa gcagagaaca 12480
tatttctagc tatttaatta acctcaatct gtgaggttaa attagttcta aatgaccgtt 12540
gacataaagt caactgtttt tccctattat atcattttgt gctagccaat gggatagagt 12600
aggacacagc caaccaaata ctgtatttcc tcagatctaa gataccttca ttttattgtg 12660
cactattatt ttatatcctg ttaaatagga caaaacactg tcaataagcc atgacgtgtc 12720
atcaattttg agtcacactt caatttcaga agcgttacaa gggcaaaata gtgaatccta 12780
gaatcaagaa aataagtaaa acatttaaga acttccttcc ttcagagcta tgcagttgac 12840
tcagtaatgc ctgcccaaag tggtcatttc cacagattat taatttctag aataagtaaa 12900
agagaatgtt acacacttgg ttagtcatct gggctctctt ctgcattgaa acttttctcc 12960
aactcatacg ggtaagccag taatttagga ttcgctttgt aatatcggaa aaagtaaata 13020
tcctgagtaa gaagaatttt gcgttattta gaaacttcag agtcctgcct ttagtactaa 13080
catgcattga aaatatctat ccaagtcttc tttagtgaga aatggggagg agagagatgt 13140
gccaaaaacc taaagccaaa atgagcaatt ctgtagcatt cccaaagcta gccgccacaa 13200
agtgtgatac caacacgact ggcacactca atgcgtttta aaggctgtga aaattgccga 13260
aaagattggt ttaatggttg ctaactgccc ttaggggcag caagtgcatc atagaaaagt 13320
ggatgtgatt cttttagatc ctgaggaaag cataaagtgg gatgggattt tttggaatgt 13380
ggggattttt ttttgtttgt ttgttttgct gaagaaaata caagtattaa ccatttaaaa 13440
cggaatcatg taatattgca aagcctggga tcctgagtga tgagtaatga tgccgtggct 13500
ctgtcaccat aatcagtctt cacctcaaag tctagacttt actgctacac cacagctcca 13560
tgctgagcag ggccaggcct tacttggggg acaaataatc ccctcgctag cccccctccc 13620
attatgtgtc tgtatcaaag aagtgtctga taaagaaata tctgttaatc ataatttcaa 13680
aatagtccaa cattaatcca tgccgacaaa aagttttaaa acggctcctt cttctttccc 13740
tccaccagat gacacagatg gggagctttt cattttatgg gattttgttc acaaatgaaa 13800
cactgattca aacactggtc gccgtgattt ctaaatccaa aatgctaagc tataacaaaa 13860
tacaattcct taagggagaa tcaaactcca gaaaatcttt tgttcatgga gtttaaaaag 13920
 
aaaaaaaaaa aaaggcaagg aaaagtatct aacatttaat cctgggacag ggacacatct 13980
ctgtaactat aaaccaggtt tctaattaac tggtactgca gaaaataaca gaaattaaac 14040
tgcactcaaa tctacagaga aaaactaact tttaaagtga attttgcctg cattgtctag 14100
aaaatacata tttggagaaa tactgcagag tatggtaatt gatacttgga tgcaaaatac 14160
acaagcaact actgaatatg aaggcagtat attttttgaa gttactgaat aaagtgattc 14220
gtggtcgctc ttttctacct gcaaaaatat gaatattata gaaatgttct tccccctggg 14280
ggacatttcc taaaataact taattatagc agggaggtgg gggagaggga cgaggttgag 14340
agggagggga agctttcctc tcctgattct gaatcctgat tccagtatga ggtttaaaca 14400
ttttaaagta aatatctaat tgatttcctt caaaaagagc tctcaaaaaa tggtgtgtgt 14460
gcacacgtgc acgtgcacac gccatacact tacactgcat atgttcattg agaaaaatgt 14520
gtatgaaggg ccttctccag taaagccctg atttgttact gtgggacaag gggtggtgat 14580
tgattttgca gggtcttatt cttgagcact gggtgttctc tatgcaaatg atgaatgacc 14640
tctacctctg aaactaatga tttactacat gttggctaat tggatttaaa tttctttttt 14700
taaaaaaaga gcaagttatg tcctgacact catttgaacc accagctggg aaatacacag 14760
ctatgcatgt gtacacatgg gggtgggagc aggggcacac acaggggtac acactacgat 14820
gctctgcagg tctgcacaca gaggagggtg aaagagctgc taagtcctgg gcttgaggtt 14880
atgtagtgac cacaataagg gagaaaggtt agaatcatct gcatctgact ccatgtagcc 14940
atgaaagact cacaagcccc aacaaaaacc cttttctcgg ccacatggtg agaggagacc 15000
cagtgttaga ggtctggact aaaaggactt ttcctaactg tgaacctatg actcagcctt 15060
aaacaacaaa cataacagcc cagccagatt cttatctgaa gaattgaatg attaagaatt 15120
caatcattct ttaatgatta tgttgtttat attaaagaaa cactgtttat agatttgtgt 15180
ccagaccaca cataagaaaa tcagaaacat ccctaaatct caccatggaa cttactctta 15240
gccattttct ctcaacgaat caccggattc gcaggaattt cacattcctt acaaccctgt 15300
gagcggtaaa attccatgga gtgggggaaa caatagacag atttcagtga ctttgaaggt 15360
agaacttcac tatttcttag tgaaagggac cataagggaa aggaggggaa ctgagtggga 15420
aaaatcagag agggagacaa accatgagag acctctaact ctgggaaaca caaagggttg 15480
cagaagggga ggtgggcggg aggatggggt gactgggtga cgggcactga ggagggcacg 15540
tgatgggatg agcactgggt gttatactat gttgtactat atgttggcaa atcgaactta 15600
aataaaaaca aatgaaaaac aattttttta attaaaaaaa aataaaacct agtatttcat 15660
ctgtcctgac ccatatcttt ggggaaatcc tagtgggttc tttgcagctt ccctctgagc 15720
tcaagaacca agttaggaac ccagtccttg tggaggtgat tcgggaagag agccagtggg 15780
ggtagggaac gtgggggtgg gggtagggga gggggtgttg gggcagggtg ggggagtggg 15840
aaaatgagtt agaagctgca catgtcccag cacaggggac aaaggctcat ccacgggctc 15900
atgatgctgg actcagaaga ctgcttaagt gcagagatcg aagcgacaga ggcttgggta 15960
 
tcttctccat cccaaaccct acttccattt tgttagcttg agttggaaat tagaacatta 16020
aaggttttta gaaaatcttt ttacactatt cctaacaaat tcaagtttca ttatccaatc 16080
taccttgaat tccggaatgg ttcccataaa ttcatatcaa gagaggggaa tcccctgttt 16140
ttctgtcaat ctttccaatg ggaaggctgc gagggtttga aaaacacatg gctgtctcta 16200
ccacaggtca gttcccaaac ttgtttccaa aagcgaatga atgatgtgag aagtgattcg 16260
tttgcttaca gtaattgcac agctacgtgt tgagcagttt cacgttccct gcattgactt 16320
aattgctttg cttctagtaa ctcctgatgc cacatgtcag gatgggttgc aaccgaacta 16380
aggaatgata gacagacaca gggacagatg gacacagaca gagagataca gtacagatac 16440
acatcagaca gagataacaa acatcaacgt acacacacac acggctgggg gggacgtcca 16500
ctcatggaat aagccatcca agcaacagag aaggttctct tcagaaacaa caaaaagaat 16560
caagaggcct ttcctcccaa gtggccacca agagatgggt gtgtggagtc agaaatcctc 16620
cgatttcgtg taaacagaga tgagatagga cacggtctcc actaaaaaaa agggggtaag 16680
atggagaaac agacatcatt tttgaaactg acagaggaaa gagcaaaacg aggaatcgtc 16740
acaaatggct ggggaagcag ggtgccttcg cttctgacaa cgtggacggc accgagaaag 16800
cacagggctt tcaaatccag catcaaagcg gcgagcctca gcaaaactca gcaaatgcct 16860
tttatcagag ccagttacag aaataaacac cataaagagg catgccttcc ttctccggac 16920
agaggccaag acatcacaga ctgggacttg tcccccgagg tgcaaccctt cagcactggg 16980
ctctataaag caatacgatg cgtaggtgcc cacggaattt tgactttgga aattagtgcc 17040
aacgcctttt gcagaacatt catcagcaaa gcgcaccgac ggtatcaaca gtcaatgtct 17100
cgtgggcgaa caatctaagc ccacgctctt ccgagtgagc gggttgaggt cacacagagc 17160
cgcagagtcc cacggcccag gaacacaagc gtgcgacagc ggtgagtgcc aggaacaggt 17220
gtgactggtt gctgattgaa atgggcacac acccagctca ggcacacaga cggggggtgg 17280
ggtggggtac gacagactga tccagggtgt gtgcctaggg gattgagacc agaacatcca 17340
tcccgagggt cacctccaaa cccacgtatg tcatgggctc tgatctgtgt tgctttgtgt 17400
tcttgtaagc agttattatt agactaagcc actgagggga tgaatccgga cgcttccagc 17460
atcacaagcc acaaattgac tctagcccca aaaggaggca caggggaatc ttatcaacag 17520
gcacatgaat tcctggttcc ggccccaaag caaaacccat ccggttttcc ttgagagtct 17580
ggtaacagga aaggaaaatc gccttttaca acttgggact caggcaggac aagaccacgt 17640
tggaccccaa agcgccaact gggaatttgc ccgtagaagt caggcttgcc tgagaattag 17700
aaacctcagc ggatgaccca gaatccaggc ttcgagggct tgcaaacgtc tggaagggtg 17760
ctctcttagt gcccgaccta acaccttcac gactcacctg tctcaccgcc agccaacggc 17820
tctgtgctca ccattagatt gacagttgaa ctagaagcca accttgcttg tgaaggtgga 17880
ggttgagctt tctctataaa agaaaaatac ataggactag taggaaacac agaaattaaa 17940
gggcttagga attctgaggg accgaggcaa gctgccttcc tgggttaaga gctcgcacgt 18000
aagggcgagc cacggacaca cggagtccca caaagtctct acagacacca ctaacttcta 18060
 
tcgtctgcaa gttccattca tctcaacaag cgaatggggg aaatctctga actttgaaca 18120
aatcctctca tttgccacat aacctctcct gctgctcatc caggcaaatg gagccctggg 18180
ggcggaaggg tcttttcact cgggtcggca tggattacgt ggtgactggc gggcattccc 18240
tttcctcaag cagggctcct tgatctgaat tccacgggcc cggggaggaa ggcatgtgag 18300
gcctcctcat catcccagga gcagcctggg tctagaagga cgcacggtgg gggaatcgag 18360
gagagaatca gggggtgtga gtaattaacg gagaccgcag gtgcacgtgg gaacagatgc 18420
caggctccaa ggccgcagag gagccagggg gacatcgggc cacggagggc agggtcccag 18480
cacggtggac ccgggagggg aattcggatg ccctgcgtgc agcggctgtg gacacccctg 18540
gccgtggggt ggtgaggccg cacggactcc tcggttactc tcggagaagc cagcgaccct 18600
cggtggtgtt ttctgctcac ccaacgcgga gacatacttc agaaacacgg tcttggtggg 18660
cagtggagac cccagccagg gcccacggac gtccttcctt ctctcggaga aggggatgca 18720
tgcaggccac gtggatttgg cttcctgcct aggggaccct gggaccccga gggcagggca 18780
gcagttcacg aggaatcccg ccttccaccg cgctcccttg tgaaaccagg tgtcaccgaa 18840
acacccggga catggcctta accgtgtcct tgcaggttaa ccttgtccac aggtcaattc 18900
aacggccagg aagcccgccc tttctgtccc accagctgtc gcacgtggca gtcagtgccg 18960
actgctgggg gctgaccgtg tactcacttg tgctgaagct tccttggtag acggccctgg 19020
cctgcgcccg caagtagcac accaccgcca cgtggtacgt gtggccggcg agcaggcccc 19080
cgatggcgcg ctccgtggcc gagaggttct gccgcagaac cagggactgg tcgtgggccg 19140
aggtgacggt gaggtcgtaa aagtaaggac tggggggctc gggcctctcc cagtgcagtt 19200
tggcgctgtt ctcggtgatg tcggacaccc ggacctctcg ggttgcctta gctgcaagac 19260
aggtggatgc agagggcgga gataaggctc aaagccccac agccgcgaca cagaggtgac 19320
acccagccac ctgaggagga atcggcaggc aggggagcgg aaatgaggtg agcgcgggca 19380
actaacttct cgttcgggga acgtcagtct tggaagcgac cgacatccac gcaacgtggc 19440
cgtgcctcgt ctcgtgactg ctcagcaagg tgcaaagcta ttttcttttc tgttgacaat 19500
cacccccagt aaaggagaaa cttggaaggc attgaaagaa gagcgagccc cacgatttca 19560
aatgtgttaa gtcacgagac tctgggcccc ggcgggaaat tctttcaaag gaatccccgc 19620
ctcctctctg tgcatcgaag gaagcccgtt gccgcctttt cacaaacatg tacctgccgc 19680
ctgcaccttt ccacgtgccc tgtgactccc tggtacgttc cagcgtccgg aggaaagaac 19740
taaattacca actctgggtc tcgcgaagcc cggctgccct cctatctaat tcggtccgga 19800
aggtgttttc gccacgggca gtgttagcaa gaactcggtc tcagggggcg ggggtatcac 19860
ccaggctctg acctgccagc atgctcagca gagcacccca ctagggcccc atcctatggg 19920
aaccccaacc ccagggtttg ggacttgggg cttggggcgg cattgggggg aaggaggcat 19980
cagggggagg gagatgcttt gtgtggagct caccagcctc acagaggtaa gagaaagact 20040
gctttctaac aggaaaaaaa taaataaaat aaaataaaat aaaataaaat aaaataaaat 20100
 
aaaataaaat aaaataaaat aaaataaaat aaaacaaaac aaaacaaaac aaaacaaaac 20160
aaaataaaat aaaataaaat aataaaataa aataaataaa ataataaaat aaaataaaat 20220
aaaataataa aataaaataa aagcaacgtt tgacaattgt atgccctgac agcaccttct 20280
caaacacatc acctagatgt ctaaacccca gattagtgac tgtgttccag ctgctttata 20340
aacaggctcc tggggcacct gggtggctcc atggttgagt gtctgccttt ggctcagggc 20400
gtgaccctgg ggtcccggga tcgagtccca catcaggctc cccaggggga gcctgcttct 20460
ccctctgcct gtgtctctgc ctctctctgt gtgtctctca tgaatagata aataaaatat 20520
ttaataaata aataataaat aaacaggctc cagggcactg gccctgaaca caggaaacga 20580
ggatgtcccc ggctggcagc gctatgcgtg cttcttaccc acgggtttcg aagtggctgg 20640
cttggtggcg gccggtttgg ctccctgggg cttgggggcc tcgggcttgg cggccaccgg 20700
cctggctgca acgggggtc tcacagctgc tggcttcgct gccaccggct tcgctgccgc 20760
tggcttcacc gccactgcag gtttgaccgt ggccgtcttt gtggcctcgg gcttggcggc 20820
ctcgggcttg gcggcctcgg gcttggtggc cacgggtttc gcggccatgg gtttcgcggc 20880
ggccggtttg ggcggggctg gcttggctgc ggccggcttt gaggccggca ggtttaccac 20940
agttactggt ttcgtcgtgg tggtcactgg ttctgtggtg gtcattggct tggctgtggt 21000
cactggtttg gatgtaggac ttgaagtagc attattcgga gtatttctgt taaaacaaat 21060
taaataccgt atcaatccat ccatcatttt gcctgctcta aaatttacaa aaacacgctc 21120
cccatgagct atgtgaaatt gctgctcgat caaaatgcat gcagtagttt ttatattttg 21180
catacagcat tgcacatatt aaattccttc cttttagaat ctgtgctgca gcaaagcaca 21240
taggtaatag aatcattaaa aatgaagctt agggacgtct gggtggctca gtggttgagc 21300
gtctgccttc agctcagggc atgatcccgg gatcctggga tcgagtccca cattgggctc 21360
cctgcatgga gactgcttct ccctctgcct gagtctctac ctctctctga gtgtctctca 21420
tgaataaata aatacaatct ttaaaaaaaa taaatgaata atttaaaaat gaaaaaaata 21480
aaataaaaat gaagcctcta gtataactat aaatacacct gacatcttgt ttttaattta 21540
taaaatataa taaatgtttt aatttttata aacacaaaca ataataaaat aatactcaaa 21600
cgacaccctt aattataaaa aatggccaac tgaaacatat acacccaaaa ttaaataaag 21660
ccacctaata gctactctca ttgtaagaaa gtatccactc aataaaatgt agatcggtgt 21720
gcttctagca tcaccaccat gacctgtgtc atcatagcat taatcattcc ctcccggagc 21780
gtattgtgta tgtttgttgg cattaatttg ggggtgaggg tgttttgtgg ttttgttttt 21840
tttcacaatt tcttgatctg ctacaaatcc ctgaaaagaa cagcaacata atgagacaca 21900
gcagcacaga ctcagccctc gttcgcttgt tgccgagacg caccctcatt cattcttagg 21960
tctgtcacgc tctacgactc cagcagcaag ttttcaactt ggtgtcatag agcttcagat 22020
attatgttac gtcattcatg tggtttccct aagaataagg ttgaggacag gccagggaat 22080
gtttgagagg aatgacaaca agagttaagg ccaggaagaa agaacctctg gacctgggtg 22140
acacaggaca cacctcgcag ccccagaaag gggcacagtt ccctcagctg tgattgtcgg 22200
 
tctgcccggc tgagcatgga gcccgccctc caaggacacg ggcaagggct gaagggctta 22260
ggggtgagac agggaggcgg gctctctgtc cccacgctgg aggtcagcca ggagtaagcc 22320
acatactcgt ttgtcaagga taattcaagt ccacatctcc tctaggtgct atcgtcagaa 22380
ccctctccta cccaaagagc atggcagtct gtggcactcc cttgggagtc tggcacagcg 22440
cctggactca gtttctgcca ccatgtcatc atcacctagc cctctgtcac acagccacac 22500
agccctcctc tccgctcctg gagttggcac cagagatgga gctgggccag catccctgtg 22560
agtacaacta ccccagcccc cacctgctcg ttagcctttc tagtagaaca tgcccggtgc 22620
ctcctagtgt caccaccatc acctgtgtca tcgtagcact aattatttta agatttggaa 22680
tgcttaatgt agtcaatgta ctcaaccctt ctgtgtgaga tcatttcata ccaagcgtag 22740
tagacacatg gccccaattt cccatcctct ccattcctgt gcccttcaca gtgtaatgtg 22800
atagggcctc ctcttgagtc tagcccccaa ctcttgaatt ggcctggctg gccattggat 22860
gtgcattggg catggaatgt gacaggagta acagcgtgct ggttccagcc tagcctcaag 22920
aggcctcatg ccctcggact tctttcatgg aaccctgccc attcaccagc gagcaaacct 22980
ccaaccctgc ctcctggaga atgagaggcc atgtggagca gaggtaagcc aaccactgag 23040
accttcccga ccaccatcca ctgtggacag gcctccgacc acaaacatat ctgtgagccc 23100
agcagggacc agaggactgc ccagctgagc ccaagccaaa aaaaaaaaaa aaaaaaaaaa 23160
aaaaaaccac acagaactgt agattaaata aatcgttgct ttaagccact aaattttgga 23220
tagtttgcta tgcaataaaa gcaaatggat acaacagatg gaaagaaatg ctccatgtga 23280
ccttttggaa gtcacaattt ctggatttca atgtctttac ctataatgtt gggcgttgtc 23340
tggaggacct ctgcactccc cctacactat actttctaag acaatcatta caatacttac 23400
aattgtttgt gaccaaactt cacaggattc tttgtcagtt ggtccccttg gaaccaatcg 23460
cactgtttcc tgatatctgg ggacaagtaa aaagcatttt cacctggaga gaaacagaga 23520
aaaaaaaatt taagtgcatg tgaatcccat gcaaatgaag attaatctgc tgatgagtta 23580
aatggctcac acagcaggtg gtccataaat attggttgaa ttcactcatt cctccagaca 23640
agcggaatct agtcaattta aacattaagc acaatggtac ttgagccaca aagcatctcc 23700
agccaaaatc ctccatttgg caaaatttgg gtgattaggt tgaaggaaat gttgactcag 23760
cgtaaggggg agacaggaac agaagatcaa gcacggggtt taacaagttc caagctcatg 23820
aacctcaaac tgcaatcaaa ttacttttaa tgttttctca atgttgtgtt taatagaact 23880
tttgttttta atctttggaa ggcagcgatg gtggctccca ggtgaactgg ctggcttacc 23940
ttggcttcat gggttttctc tagcactttt gagggacaga cttccacaca tgggaacact 24000
gtgttcatgg tcctcacctt tcccatgaat aataaactct ctgtacacgc atgtttgttc 24060
ctattgtcta gatgtctctc gttagttaga taactcgcct ggtctgactt ggggctgtgc 24120
actgggccag gctctgagca cagggtgaac aatgcacctc ttcacggacc atgcagtcta 24180
ctgggaccac ggactccaaa ggaataacca gcgggccatg aaatagatac agtaagtagc 24240
 
gcccagatta acgatgagaa agccctggaa gtggggtgct ttcatggtga ccaggggcag 24300
ctatggatat gttttgaagg tgaaggtgag catcccaggg aggagattca cattaacaaa 24360
ggcccaagag tttccaagtg tggggttgtt ggctaagccc aggtcaacac acctccttct 24420
aggagcttcc ccatctatca tgtcatgcat aatacatatg cgctggatcc actgttcatt 24480
ctcagagaca cacggtctat ggctgggagg tgctcagtca gatttggttg aatcagtgag 24540
taaacaggga caggatctcc tgctgtgtgt tcactgccaa ccctggctcc taaaatatgt 24600
ctaggatatg gtaggtatca atatatgtgt gttgaatgaa caagtcaaca tcagcacact 24660
agatgtcttt tctgtggccc atgtcccagg taaccccagg cagaaagccc tggcccaggc 24720
acacactgtc ccatgtagga cttactgcca acgaaggatg gtaacaacct cccaaagcgc 24780
atcagaggct cttcgttaag ctcagtcggc ttatccaata gtttgaagaa gacgtcattc 24840
ggctcactgg cgaagctgta cacctccttg acgttcacct tcctgccaat gcccagaatc 24900
acgaagaaat aacctttgca tttggcctgc aagatgactc tatgggcctc ttccagctgc 24960
tccttctcca cctcacctgt cagcatcaga actatgattt ttaagtcccg tgggttgggg 25020
gcgctttcaa agacattctc tatagtgtat tcaatggcac tgcccaggtc cctggtccct 25080
tccagctgtg tcattcttct gctgagaaag tccaccagct tctccttgga gccatagtca 25140
gtcagggaga actccacctt cacgggcggt aggctgggat tccccaggga ctcatagggc 25200
gcatgctgca caactgccac tctggcaaag tgctgagagg ccttggggtc agggcttatg 25260
tccagctgcc tgaccaggta ccctatgtac ttcttcatct cattgaactg gaacagagag 25320
gtggactcag agctgtctaa gatgaaagcc atgtcgatgt ccacatcgct gcccgctgct 25380
ctcctatccc tgaaggaagg cttccagttg ccaaatccac aggccgggtc aatgttgcag 25440
atgtctagaa aaaagcacgg taccccattt attctgtgac ccctcacagt aagatattgt 25500
acatcaatgt gctgggagtt gggggtagtt gaaatcatcc cataatatag aaacctactt 25560
atagcttgct gccactttgt ggccatattt cagttttcat cagctaacac taaatgaaac 25620
aaatcagtaa gctaaagtcc ttgttagtat actatggtat tggcattaaa agaagaggaa 25680
aaggaagaga ataaagagga ggaacagtgg gaggaggagg gaggacatag gggtaggagg 25740
agtggggaga aagaaggaaa agaaaagaga aaaaagaaaa gaaaagaaaa aagaaaagaa 25800
aagaaaagaa aagaaaagaa aagaaagaaa agaaaagaaa aagaaaaaga aaaagaaaaa 25860
gaaaaagaaa aagaaaaaga aaagagaaaa gaaaaaagaa ggaaaggaaa aaaaactatt 25920
tcttcaccac tcttcgaata aatgcgttcc agaaagtcac aaagacagat cattgaaatt 25980
tggacgccaa ggtcaaatag aagcagagcc tactttctcc tttatttccc ataaaatgaa 26040
tcattccctg tcaaagagaa gctctgttta aggggttcca ctttcagtta ggaaactact 26100
tatatgggaa ttacaccggt aaacgtgagg ctttatgaat cttcctttaa ctctagccct 26160
ttatatggat ggcgaggatt cattcgaatg ctactccgtg gatagcacag tacgcgttgt 26220
aacggatgtt cctgctaggg aagcccctta cccaggcaaa cgtggcaggt caggacattc 26280
ttcaagaagt ccgtgaggtc tctcctagcg ggtaggacga gcgcgtgccc caccgctgtg 26340
 
ttattgatct ggtttaaaca aaagaaaatg cttggtagga gtgtccatgg taagtaacta 26400
ctggaaaatg tagctgagga agagagtcac atcagtaaaa taaactttcc aacagatcag 26460
tatctcctct gggcaggtgg ggggttgggg aggaggtact gagcctaatc ggacaaccca 26520
gattttcctc tgtggagatg agctgcagtt cagatcccac tcttgcactt cctgccaggc 26580
ctgcaggaag attatttaac ttgtctgggg tccaatttcc tcagctgtga aatgaggaga 26640
ctcctaaact cctcatgtcc aattagataa tgcatgtaaa aggcctgggc tagcctctag 26700
gaaataatgg gcacttctgt agaatggttg ttttcctcaa tcattccagc cactgacaat 26760
cattagctcc atcctctccc actctcccag gcccagaaag ggatttcccg acaaagcaag 26820
taataacatc acatctagaa gattcaagaa gtcttgctta tcatcatggc acctaggaca 26880
ccatccctaa gcagtcattt aatgaataat tacatttaat taatgaatga ataattgact 26940
tttcaaactt gctgaaacaa tgctgtcaag aaccctgtga ttctgggttc ttcagcccag 27000
gggagggcca atgtacataa atgacccctt gcgagggtag acagctggaa gcccaagctc 27060
agtacgttgt gctcaaactg gcttatggtt tggcctgaga gatggcacca aaaaccagac 27120
tgtctttcac tgacacaggc agtggtaaga ccatagaata cccagtctag aatgacagat 27180
gctagagacc atcccttccc gacctcgtct ttcgcagtca agaaatctaa gacccagaca 27240
agttaaagtg acgtgaccat attacttgtg gtgacttgaa gctataaagt ggatgggttg 27300
gatctagggc tcttgcccat catgcccacc accccactca tcttgagcaa tgccaccaga 27360
atgggcattg aataaatatt cattgattaa ttagccaatc atatttatta ttgtaacaat 27420
taaacaagtt atttaagcga atgacaaggg agcactctgc agcttcgcag caaaggctga 27480
cccttggctg ccatcctggc tggagaaaga ctcagagagg ggggtgtaga tgggggtgct 27540
gggatcactg acacttgggg gacctcattg gaagactgta ggcctcaggg tcagccctgg 27600
gagggtctcc tccagcatgt gaggggggac cctgtggcaa taaatcacaa gcctgtggct 27660
gtcgctctag tgccagagcc acccggctgt cctggaaatc tcactagagt agtcgccaat 27720
cacaacagcc tcctccagga gcttttggaa accatggcat gaattcatca aggaggtacc 27780
aggtgcccga gaagcttctc agagcgcaag aagaaccgag ggaagagtca acaatgcccc 27840
cagggcacag acctgcaacg cgttgaccag ctgccggtcc tcctggctcg tgaggaacaa 27900
gggtgtgatc cctgcgtccg aaagctttag cacagcctcc ctgagctgtg gggaggccct 27960
cgtgggctta ttgctgaaga aaacagccac ttttcgcatc aggaatccgt ttctcactcg 28020
cttaaacgtg tttctggcca caaaggacat ggccgtttcc aggctctgct gcttggatgt 28080
cagcgccacc tgaaggttct tgatcttgtc cagaaggact gacttcttct tggagtcggc 28140
aaaccggatc tccgtggtca cctcgttgtt gtaggtgacc acggccacac gcgccccccg 28200
cgggcagttg ctctcggcaa tggtcaggtc gcccacaagc ttcaggacca cgtccctcat 28260
ccggctgaag gtgtcttggg tgaccccctc tgaggtgtct aaagcaaagg ccagctccgt 28320
cgggaagacg gggcattcca ggggccctgt gggaagtggt gggtttccaa agacaagctt 28380
 
tacttactgg aaaagggagg gtcttctttc agcaggaggg tagtcacacg gctggaatca 28440
acttaccata gcagcaagct acggaagaaa ggaagagaac agtgaatatg gtgaccacat 28500
gagaatgtca aacgtcctac caaatggggt cgcggagagt ggaaaatcat gtacttacga 28560
catttatctt tgatgctctg gacaagggca cattgcttta aaaggaaaag aaagaagcat 28620
gagggggtgg accagggact cacagcgcaa ggacgggagc tgtacacatg caacttacat 28680
ccatggagtc acctctgttg cctttgggac cctgcggaat aaggaaagag gggatgagaa 28740
ggagcacagg taggccggat gctggggctg aggaaccacc ttcggatccg agtaaacccc 28800
atggaggaag caaaggtaga ccctggccaa ccttggtcat tttcaggtcc cctcttagat 28860
ttgactttac tgctcaagga ccaataggga gataactttg aaggcaagag tgtttccctt 28920
gcaagataaa tagccctgca ccccagaaac ctggaagctc atgacaaatc aaagggatca 28980
catatttggg cttgaggcta cacatcgctt ctcctaagaa gcacttcctt tgcacctgct 29040
agcttagtac ctcctgttca cagaagggac cgtgaggagg agtcatttag ctgaccctgg 29100
gctccagagg ggaagcacgc cagctggagt ccatcccgga ggagccccaa gaacagaccc 29160
acaaccaccc tcaatccctc ctcccaacct gtggcctccc tgacggtcaa gcagtcatct 29220
tgtgggccca tcaagtgtct ttccacggga ctagagactg gtttactttt aaacacttca 29280
cttcttaaag gcagcaatca cctgactcag ccttcaccca agttcaacca gtagaatccc 29340
tcaacctttc cttgtaagaa tgattgtcca tcattctttg aacctcatcg aatttaatgt 29400
agggagacaa tacagggaaa gcttcaaata aggaaacaaa gacattcgta gtgaagagac 29460
gtcctggaaa gcctgcagaa ggtcaccctc ctggcccccg gtggcagggc actcgtgggt 29520
tttaaaccac aaatcagaag ctctctcggg cattttataa accaaaggaa gtagtccaca 29580
gaccgacatc tgtcataaat tatactactg ggatccctgg gtggcgcagc ggtttggcgc 29640
ctgcctttgg cccagggcgc gatcctggag acccgggatt gaatcccaca tcgggctccc 29700
tgcatggagc ctgcttctcc ctctgcctat gtctctgcct ctctctctct gtgactatca 29760
tgaataaata aataaaatct ttaaaaaaaa taaataaaaa taaataaata aattatacta 29820
cttctacgat atgcaaccaa gaagtattaa aacttgtact ttttttttgt cctgtacaca 29880
aacctcaaac aaacctatac caggaagtta taaaggcaaa ttacaatata ccctagacca 29940
caaggttcca taagaaactt ctttttacag cttcttgtta ctctaaaact caaaacctct 30000
tttctccatt gtggtatcaa aagaaaagtg gtttacttga aaaaaaaaaa aaaggcaata 30060
acaatacaaa tgcagcagcc ctctcttgag gagaagggac agtggaaagt ttataaagag 30120
gcatgtgtaa agcatccagt ccagtggcta gatcagaatt cggggggtgg ggaggaggga 30180
aggtacttac agatggtcct gggtagccag ggtctccctt ctgcccaatt gcgcccggag 30240
gtcctgaatt tccctagaaa ggacagaaca aacattgctc agctatggca ggagatgagg 30300
aatccccatc tcctatggtt ggaagggggc agtgaaggtg atctaattca gcgttccccc 30360
gtcaccatga gaaatctctc cgtcagatgc ttgcccaaga accatcaata agactatccc 30420
tggatggaca agaaaccaaa aaatggcaat taatcagatc actattcagt ggagacaacc 30480
 
aatctaatgt attgaaaccc acctcgaata gtatgatgat ggagtctggc tgtcttcact 30540
gagcaaagcg acaggataag atgagggttt taactgtatt ttaagctaaa aacaacagtt 30600
tcccacatcc agaccacaat ttgctaagtc aagactcggt gtaatcttaa gagggtgagt 30660
aaacatccat tgaagagagt aattcagagc taaaagtagc ttagggaaga ggactctaat 30720
ggtggggagg agggcagcgt gtggtctcgt gatctctgag atgtgttaga ggaaggcgtt 30780
ctcagagcct cctcacgagg acggcatggc tgacggagga cagagcagac aaccacagag 30840
ggtcctgagg acactgctct ggggcttagg attccttctc ttcttcccta aactacaact 30900
agctaggttg caagtgaaga agaatcattt ttactacaca tgttctcatt ccccacctgc 30960
ctgcaggtgg gcagactcac ccttcggcct ctgatgcctt tgggtcccgg tggtccgcct 31020
gtccctggct caccagggac tccctgggaa gagatccaga aagaaatgca ttaggctctg 31080
aaatggaggg tctagctgag ggtcccaggg gcatccggac catacttcct ggggtagcca 31140
agtggctcac tctgatttac attccactgc cttcctagtc catttgtgtc ccttgcagtc 31200
agccttagct tgttgaaccc gtggtgacaa taccacaccg atcacagcat tttgagttta 31260
caaagtatgt tactgctgcg tcatttcatt taaccctcac gggtctcgag gcaagtgcac 31320
actgtcccgt ccacctaaca gatgagggca tacctcagag aggccgaggc cttccctggg 31380
tcctggttag taagcagctg acccaacact agcacctcag tgttctgatt tccagcccat 31440
gaacacacca gactcaggca caaacaacag tgacatttca ccattagggg aatatttatt 31500
tacctttgga cctgggtatc cagggaatcc tctttctccc tgtaagagag aaataagtaa 31560
ctcagcattt ggtcccaatt taagtgcatc taaaatacat tattcttgag ctgaaatata 31620
tatatatata tatatatata tatatatata tatatatata tcacatatat acacacacat 31680
atatataata tatataatga aatatatatt tatatattat gaaatatatg aaaatatata 31740
ttatgaaata tatatatata tccctggagt catcaggaga aaaaaattct tgatattaca 31800
ataaatatat tatgaaatat atgaaatata tattatgaaa tatatatata tatccctgga 31860
gtcatcagga gaaaaaaatt cttgatatta caataaaatt ttgagaaata aatctctaaa 31920
aatataagtt gtcagaagtt caaagaacct agcagataca tacttttttg cctttgcgtc 31980
cttcactgcc aaccccgtct ctgccatcgt cgccctaggg gtggaaagac agcaatactg 32040
caggccgcca tgcggggcac tgcacagcca cctctgtcta gaggcctgag cacagggtct 32100
ttgggtccct gaagtgttcc cagatcaagg acgtgttcct gctcccatcc catcctccag 32160
gtacaaacaa gactgcatct ggaaaaggat gtgcttaatg ggctcctttg gcgaaatggt 32220
gctggtgtgg caccaatata aacaattgac agccactgga aagacaccgc ttgggcatag 32280
aatcacagac ccactgaacc caagactctg aaagtggaaa ggattcccta aggccaccct 32340
gactctgcat ctttgggtct ttgattctac ttcaaagcaa gaatgcagtc tctcatgcct 32400
tcccgagtgc tcctgtgctc agactctgtc ccctcccctc ctaacagtca tgccgtagac 32460
tactttcatt cccaactccc ccttcctgtg ctcttggatt ggtcccatca cccccaccat 32520
 
gggagatctt cagaactgga ttccattaac gatttcaaaa gatcagaaga atgcatgtaa 32580
aatatacatt aatatctcta aaatgtacat ggagcgcact acatagttaa tttacgcttt 32640
tgagcaaact ttaacagtga aagagctgtt gtccaaaaat ttatggacac aatcaagaag 32700
agattccctg caatggtctg aagagatttt aaagtctgat ttttttcgcc ctctgctcct 32760
gaaactaaca gtgacagaaa tggatctttt aaatgccaga aatggatctt tgccatagag 32820
aagttgcata caccccaaaa aatattttaa agtttgggat tatacatcaa ataaatctgg 32880
tccattttag ctatactgcc tcattcttta aaaattctca atattctgta gacttctttt 32940
ccctggatag aaatgtatat tacgaagata gaatgtatat tattcgtgag caaatagatt 33000
attattatta tagattagtt ttaaatgaca tttttaaaaa tgagttatcg aggtagtaac 33060
ttttattcct aggatgtctc attcaaagga aatggtttca atctggatcg agacctgagc 33120
agatccaatt ccattttaac caaaggatat ttatttcttt gcggaaaagc tcctgcaaga 33180
ctgaaaatga aagtatatcc cacttcatcg tgtccccaca ttcacttagg aaaaaaatgg 33240
ctttcctcag tcttctctga tggaatgaaa ctgatttgaa attcacttgc tgtttataag 33300
cagaaggttt tgctgtgttc aatcggatcc caaggacaca agaagcaact atcaagttac 33360
ccttatttgg aacccagtta atgggcaatg atgaagccta gcccagagca aggcccccgt 33420
ctccccacct cccgccttct agcgaaggat gagccacagc acgtcttgat cttaccgtct 33480
cccctcgagg gccccggctc ccaatgcctc ccttctgtcc cggctctcca ggctcaccct 33540
acacaaaaga aaatttaaaa aataaaaaaa taaaaaataa aaagctgaga cagcggtggt 33600
tccaaggcca acttaaaatg tgattctaag ccattcatgt tctatgcaat gctcatggta 33660
gggttgctgc aagctgctcc cttcatcttt tagattttga taaaaatgat tttttttttt 33720
ttaaatcgca gtgcttgtgt gtacatgcca ggaaaagaat ccaggaaaag tctgtgtttg 33780
tcccttgcat tagcatggca tggggacatg ctgacgggga agagagaaac agaaacgggg 33840
aaagaagcca ggaaagtcag tgggcattga attcagcagg gaaacccaac aacaccagca 33900
agagacgcag agacaaatgt ggattcacat gaacacattt cctttgtgga gaaaatgctg 33960
cccttccaga ctggttctga taaattgtgg ttttcttaag gcttacggaa agtctgttct 34020
cagacagcct tcatgtaaca agctgccgtt atccaaaaca gtcaggcatc tgggcctctt 34080
ggataagcta aatatggcca gcagagctcc gtgtgtgcac tgccagtgtt gaaaggatgc 34140
gtgtgcaatg cagcacagaa gctgtgctag gcatccccaa tctgctcgga tggctctgag 34200
gagccacaaa ccacgcatga atagaatcag gtcatccacc tgctgctgga gggttatggc 34260
catgagatag ccaagccact gagtacagcc cacatgttcc cttggggtgg ggacaagaca 34320
gggatcccac gggccaacct ggctggcgac aggggaagct gggtgcaagc aaagcaggag 34380
ggcacaggga caggagagag agagagagag agagagtgag gccacggagg gtctggctgg 34440
gcctgggcct caggaaatga gtcctacttt tgtcaagacg cacctttctt cccaggggac 34500
cgatcctgcc acgctcccca ggaacgcctg tggtccctcc gcctccctgc agcacgagga 34560
gagaggagcg tgtcgatgaa acattcaccc tgcaagccca agggcctgaa ccctgccctc 34620
 
ccccgacccc gctgcactca gggccacgca cggtgctgga gacccaaatg cacgcatgca 34680
cagacaccac ttatgcaaac tgaaagtatc acctgaccgc tcagcacctg tttccccgga 34740
ggaacacatg ctgctgttac cagtcacata ccccgaggcc ctgaccagag aggaccagca 34800
cccggatatt atgtggcttt gtttccctgt ccaygcctgc acactgaccc ctgggtacac 34860
agttgtggaa tcctgtgtcc ctgtatccac tgagggagca aataaataat gaagaaacag 34920
ctcttcaaat ggagcgcagc ccccagagcc cggaaatgac cagaacaaag atgccaacaa 34980
cagggggtag cagctacttg gcattggtga ccgtttgaag agcttcacct cacccccact 35040
ccaccgtaag ctctcgacca accagagctt aaatgatgtg aacagaacca caggagcttt 35100
gaaaataggg atttctcagt ttgcagaccg gggaaggatg tctaagcgaa cagaggttct 35160
cacaaagttt ctgcttcact gtccgacctt ttgttccttt aaggagcaac aggtcaattc 35220
tttgccagtt ttcggtcact atcgttttcc ataacaaacg acaggggagc tcctgtgtca 35280
gacaggaggg acgcccgagg gtgcagcgct gggtgctggg tccccactca gctctgacac 35340
atttctgctc atcgtcacca accacagaga tgatgttcat gcctgtggcc atgcaagtat 35400
aaggagcagc ttctgtgtca gacctgagga cggggaacgc ctcacacaaa acaccgaacc 35460
ccaaggcaat gtcaaggggc cacctgtgaa acaacttggc attagcggag aatgtgggca 35520
gattgagtta gatcttaagt gggtctaatc ctcagacttt ggagatgcag ggcggcccct 35580
ctgtgctcac cacccagctg ggccctgttt ggaaggagca gcactaggat ttagaaaccg 35640
ccggaggagc acccgggtgg ctcagcagtt gagcatctgc cttggagttc caggatcgag 35700
tcccacatca gggtccctgc aaggagcctg cttctccctc tgcctgtgtc tctacctgtc 35760
tctttctgtc tctttatgaa taaataaata caacctttga aaaaaaaaga aagaaagaaa 35820
agaagccccc agagcaccaa gcaacagtgt ctcttacttc ttcggggaga ctggccagtc 35880
gccgtgagga ccgaggctga ctcagggtgt gtgttgccag ggatgaggcg agggggagtc 35940
tatctacagt gacaccatcc acgggacctc agcctgtgca ggccctggct ctctgcttag 36000
ctaaagacag gggtgcgatc gaaccgactt acccgaggcc cagaaatgcc ttgttcaccg 36060
atcagccctg gaggacctgt gggaccaggt ggaccctgaa gagataaaat gccaacgtaa 36120
gtgctgtgtg gaggtggcgc tccatgagga agctcactca gggtgacacc tgcacatggc 36180
acagagccca gagccccgtt ctttttggcg cagccagaaa gcccgctgga gaccacgctc 36240
ctgtctcagc tgttaacagc agctccaacc caggacacaa atggggaagt taattaaaaa 36300
aaaaaaaaat acaaggaaca aggggcgcct gggtgactca gtcagttcag cgaccaactc 36360
ttgatttcag ctcaggtccc gatctgagtc atgacatgga gctccgcgtc aggctccgtg 36420
ctcagtgcgg agtctgcttc agtctctctc tgtcttcctc tccctctgct cctcccctcc 36480
tcactccttc tccctttctc tctttctttc tctctttctc tttgtctctc tctaacataa 36540
agaaataaaa atcttttaaa aaattacgag gaacaagggt tgaaacaaaa gcttgtacac 36600
agatgtcccc ggcagcacca ttcacaattt ccaaaaggtg gaaagagtct gagtgtccac 36660
 
cagccgacga ctgggtaaac agattgtggt ctgtccacgc cgtggaatat tcctcagcca 36720
tcgaaaggaa ggaagcgcag acacacacca ctacgtggat gaaactccaa agcatgatgc 36780
tcagtggagg aggccagaca caagaggccg tatattgtgt gactgtgttt acatgaaatt 36840
tccagaagag acaaacccac agagacagaa gggaaatgag tggttcccag gagctgaggg 36900
aggggagatg ggagtgactg cgtaacgggt aaggggctcc tctggggaag gtggacacgt 36960
gtagagctca gggtcacagc attgccaatg gactatctgc catggaacgg ttcgcttcac 37020
gatgattagt tttgcatccg attttgtaaa attgactctc acctcattta aagacaagca 37080
actaagcatc cgggaaacat cacaaggcat gcagccacac cttgggcggg ggtggtgggg 37140
gtgggggtgg ggttactgac ctgcgcacct cgggccccct gctctcccgc ggagcccggc 37200
tggcccgggc cgcccttgtt gcccttggaa acaaaagaag atggaggggg tgtcaggatg 37260
gttttcacca atgtcagcca tctgctctac agtcacacct ggacaaggcg acaggagaac 37320
agccctatgg ggtgggggtg ggggggtgcg gaggccatgg aggaggcggc tccgggacga 37380
gacgttgggc aggtacagcg gtgtctctct gcactcttag gatggttttc cactcttttc 37440
accctctgct caagatggct ttggaaaaca aggtttgttt ttttcttttg ttttgttttt 37500
ttcatccaaa cctgtcttgc ccgggtgagc agatggacac aggaagatgc tgagactggt 37560
caatgacctg acaagtgggc ccatcgggga taagggatgc cggacatcgc ctcctccatc 37620
tctcgccttc gcttttggtc cctgtcccca tggccccttg gtctcacaag ccgtttagaa 37680
gagcctcacg ttaaggaatt ggtagagaag gtggctgcag ctgatcgagc gccacgcctc 37740
gctggcaaag ggacagggca catggggagt catgatgtga tgctgggaaa gaggcaccaa 37800
gttagagacc taccttcgtt cctgctggtc cccttcggcc aaagccaccc tgtggagaag 37860
tcacagaaag tgagagggag cctggggacg gagccgctgt gcacctggcc ggccccgagc 37920
agacgtgcac gcgccaccct tcctcctgcc ctcatctccg cgagcttttg cttccgtgct 37980
gccctgttca ccacccagat ggaagctttc agagtgactc cagacctgcc aagcaaaaca 38040
atgactcgtt ctggctggat gatcccagcc agtctcacac aaggatgtga aatggtaatg 38100
tcaacctgtc tgctttacga ccgagttcct aactgcaggg cgaagtcatg aaatatgttc 38160
attctcgtgg aagggagtcc caagctctcc agatgctccc ggccaaatat aacacatagt 38220
tggttaagta aagtattggg attgtgcata tgtggaccta aaagcccaac tgtgcttagt 38280
ctgggctcat gacttggcaa cgtctccctc gatgttctag aagtgaccct taattcaaat 38340
aaacgtggac ctgctgcatt aagtcatcct ttaatcccaa ttttgtgatg cttcctctcc 38400
agagcaaagg ttcaggatcg gaactcacgg aggtcctggg tgtatctttg cgaggtaagg 38460
gctggtattc tggagactgt aacacaatag ttctgttacg ggcttgacgc atctgctgca 38520
tcaaccaaag aaacacactc ttcactccca atggacaggc ccagagattt tctggggtga 38580
cggctttgcc gttattcact gtgatggcag cttctttaag ggaagacctt ggcctccttc 38640
tatggatgac acatggacaa gtggaggggg aagggacggg ccaggccact ccttcagagt 38700
aaacaatctg gctggcctcc cccaaccccg gcgaggggac atcctagagt atgactagag 38760
 
gccaccagtg ctggatgacg tacaactgag gccagagaca gcctgcagaa gcctgagtgg 38820
tagactgagg catgaggtaa cccagtggga aggacacagg tcaccctggc atgagcctgg 38880
cacagtgagg tcctaaggga aggaggagga agctgacaac caggccttcc ccggccatca 38940
agggaagagg ctatgagagc ccttgacccc gtatcaactt caaacccaag tgtctacctt 39000
gcatgtagaa atcaaacaaa ttggctgcat ggctctatta ataatcattt catgaaaaca 39060
aggtttggct cccctcacga ccggagccgt cagcacgtca tgacagacaa tagagtattt 39120
cctcatcgct tcgtcatcac acagctcctc ggcattattc aaatataatc ctcagaacat 39180
ttaagacagc atcttcaaat tttgcttgcc tctgaattat aaaagcataa cctaaaagat 39240
tatgcctagg ggcgcctggg tggctcagtg gatgaagcgt ctgccctcgg ctcggatcat 39300
gatctcgggg gtcctgagat cgagcttcgc gttggggtcc ctgcttttcg gggagcctgc 39360
ttctcctcct ccctccaccc ctacccttgc ttgtgctctc tcttgctctc tctcaaataa 39420
ataaaggctt taaaaataat aataaataat aaataaataa atgtgcccaa ggcatatcat 39480
gccatcatga gtaagagtca ctcctcccca aaagcgccca gtgcagagca cacggctgat 39540
ggtgccacca ccggtccagc tcccaatgcg aggctggcgc cagggaacag gcagaggtgc 39600
tattgttggc tacgcagccc agcccggggt gctgtgctgt gccgcccgct gagcagagcc 39660
accctgacgg cccctgaaga tttatccgcc tccagaccgc tcctccccga gcagggactc 39720
tctccccact tccagagccg agctctgggt accccacccc acccccccgc ccgggcccag 39780
agcttgtata catagctttg gcttcagcct tccgcaagcc tcagagaacc tgcatgcaac 39840
gtgccgtctg gcagcagaca tttgtgaaat gtcttgaaaa agcagctgca agatgcgatt 39900
cacgagaaaa cagaatccgg ttccgttgct tgaactacag cagcaccgac caaccaacca 39960
accattatga cgccaacacg tacacgaccc gtcggccctc ctccccctaa cgccctcatt 40020
cccctctgta ctcctgcttc agtttggatt ttaaaaatat ttagtgggat taaaacctag 40080
ccaaatacgt gaaagcagcg aaggcatcgc ttttaaaagc ctctgttgga tgctgtgctt 40140
tggacaagag gcaaggagaa ccccggggcg ggaggaggga tggggcacag aacaaatggt 40200
gagcatgaaa cacgtccata agcaccaaga tttgggtgac agcagtttca agaatccctt 40260
taaagtccct aaatataaag ctgctgagta actctgcccc agagcgtgtt ggtgggccac 40320
tcaggcggcg gcacttgctc aatctcccca tggaagcccc caagccccaa gatgcactca 40380
cttccttccc ggggtctcca ggacttccag acaccccgtc tctcccaggg agaccctgat 40440
gggaggaaaa atcggtgtaa gattctcagc tgcaaagaag accatcaaac tctgcacact 40500
gcaccagagg ccgcggggaa gggcgcacac acacagggtg gtgggctgct tggggggacc 40560
ctgagacgtg ggccgtgccc ggcttcggaa gcctctggct tcacaagcac ttttcactga 40620
tatctaaggg gaacaaattc tgcaggtaag tagcagttgc tcccactccc tgttggagac 40680
tcccttcacc aaggggtctg caccccgcgc gtgggcccca cagccttgaa gaccacctcc 40740
gtggcctctg cggcggcggt gggcatgtgg ggagggggcc aagcactcag ccaagggggt 40800
 
ctgggattgc agggtgtcgt accatggggc cgatgtctcc agtttctcct ttaggtcctc 40860
tctgctgact gtcccgtcct gagtcaccct gcaaacaaca caggtcaggg acatgagctg 40920
catgtgcaag caaaggatgc tttcagttca ctttagggaa agagatggaa aagaaaggct 40980
tgtggcttct ctcgttctgt gactctataa aaccccatgc caggccagcc tcctctcctc 41040
cgggccagaa ccaggcaaga gtgaccctgg gggatcatcc ttctcactga ggaagggcca 41100
ccgcccaccg tccagggcca agagtggttg gtgaccccat gggcaggctg tgggtcgtgg 41160
gtgggatatg gccgaggggc ggcccctcag ggtctgacgt gttggtcacc cctaatactt 41220
tcctgaccac ccagtccagc tggatgaaag cgccctgagg ccacctttta ggaatacttc 41280
tgaggtcttt gtaggaaagg ccctgcaaac actttatctc tgctggtaga gagcaaacac 41340
atctccaaaa ttgtaggaag aggacaaacc atgtgtgtta ccaacacatg ttaaggccat 41400
ttcagaattc tggtttaaaa ttcttccctt caacccacgt cgccgaaacc ctcttccccg 41460
agacgcggct ctcacgctgc gggtgaggac gtccccttac cgggtcacct cgaaagccaa 41520
cgtctcctct ctctcctttg acccctttag gtcctttgtc accctaaata aaaagcccaa 41580
gcggagacaa aaattaggta gggtttccaa tttgtatgtc acccttaaaa tcacgtactt 41640
taggtcgtaa atctttaaca ggatagcatt tcccatttgc gcatttacag aaaggtaaaa 41700
tcacttccag atatttctga tttgttctgg taatcccaaa tatttactgg gacctattcc 41760
gtaccagaca ccgggggttt atttaaacag atcacactca gctcgtgccc tatgagacat 41820
acccttcaaa caaacaaaaa tatatgatgt aattgtcaca gctgatagag gacagagtgg 41880
atgccaaccc cggaggacat cagagaaggc tgcctggagg aagcagtctt cacagacatg 41940
aattaaacaa gtatgtattc cttaggtaaa atgtttctag tttttccatc atgacaacgc 42000
tatggaaaca tacttaccct tctgcccgga ttccctttct ctccggaaga cccgggcaat 42060
cctttgtctc cctgtcaaag acaatgagaa aacgtcaccc ccggcacaat ttccaccgta 42120
gcccgagcta ggagcagccg ggatgttcag gatgaggaca cggtgtggac tccgagcgcg 42180
aagtcactta cgtcttcacc gtcgagaccg tccagcccga tttctcctaa ttcgccctgc 42240
ggggaaagca agacacacgg tgagaaagga aaacccacag atcctgagaa tgcgatcaat 42300
ttataaaaag ctaaaacaaa gcgtaggtac cttctctcct gggaatccgc gagagccctg 42360
gaaggcaggg gaaaggggga agattaggtc ctccctggag gccacagaga aaggcggggg 42420
gaggggggga atccctcctc gacacctgga tctgaaatcc agtgctctca ggactcatct 42480
gtctacactg ccctctctcc atcccggcgg ggtggggtgg gggggatcct gaagctaatt 42540
tccctgggcc tgagcacctg ctgctctagg ctccctgggc aggagggcac caggctccaa 42600
ggtccctttg tgactttacg gcccggtggc ctcttacctt cagcacctgc tttcaaacat 42660
cagcaaacgg aggagacagc gcctccaagc acttcccaca aggaaccttc cagcgtgtgc 42720
ctggcaccct ggccccccaa cacttctctg gctcctctcg gggcccgggg ctagaggcac 42780
aggccaccgg cccttccccc acctggcctc gtgaccgccc cctgacttcc tcaggctccc 42840
tgcccgcggc agcctgggct gctggacccg ccagaccttg gacccggcct gcagttggag 42900
 
cccctggctg ctgaggcctc cggggccttc ttccccaggg aagcatttgg ggacagcgtc 42960
tcacttctgc acgtgccact gaacgcctct gcctgctggg gccctggtgc tgtggtcaca 43020
cacatgcaca cacatacaca cacacacgca cacacacaca caccctgtac ctttattcct 43080
ctctgcccgg ggcagccctg gaaaccttga gtgccgttca cgccaggagg accgcgctca 43140
ccctgccggg agagacaaag ccatttacag gttggagctc cttgtcacca tgcctccact 43200
gagttcgctt caccaggaga ggcgttttct agaaagtaga tttccccggc agggatgctg 43260
ttcgatcctg gtcaagcctt cggcacccct gcacccccag ccctgggagg ttcccacccc 43320
ttgagggagt gggccctgga aggtctgcgg ctcagaaggt acttccctgg aggaccaagg 43380
atgggcaggc ctctgggtac caggtgctag gagaaccctg ggcctgatgg ctcctgcaca 43440
ccgtccctgg ttcctagctc cccagagggt cccggggttg gcactgcctg ctgggacccc 43500
atgacttcat ctgctgtcca ccggccttgt cccccgatct cagggctggg cctctttcta 43560
gtgtccccaa gcctctcaga cctcatctgt cccaggacct gcttcccgag cccccagctg 43620
cacccttagg tcacccaagt cggcaaacct cccttaaagg ccgtaacccc gacaacagcc 43680
tgrttaggct ccctccgccc tgccccagct cactcccgtc tctgcttgga ctggacatct 43740
gcatttgctg gaccccaggc atggcctatt tggtgtcccc cggcctcaga ggccatcgag 43800
tctctccctc tgaaatagaa cgaggaagag ccctgcacag cacgtccacg tgtgtcacgt 43860
ccagtcatcc ccctgtgccc tgtgccccac agcgtagaga tctgagttgt cacagaagtc 43920
agaaacgaag gttttcctgg taggaaaatc actggaaatg caacagatca tgagccccac 43980
aattcaaccc caatgtgtga ggaacaaaga gggaagacaa gcttaaagaa caaatcatct 44040
tgtacttacg ggtccaccct cgtcgccagg ataacctcga tagccatctt ccccgggaat 44100
accctaaaac agaaagagtg attcctccgt gaaagcctct agttttcttt gttctacctt 44160
caagcatctc cccgaccccg ttcctttgat agagcagtgc caccacttag cacggcgtta 44220
gctgttccct tgaacttggg cttccattca tcattccagc gccattcatc tatcaggatc 44280
ttgaaggctc tcaaaatcct gtccgcttcc tttaacttag atgcaaaggc taagattccc 44340
tgctgaccct ccgcggagag gctgccagta cgtgatgccc accgatatga actttatttc 44400
ataaaatgaa gaaccaaagc actaaagaac agatcccctc ttcctgagga aagaaagaag 44460
gcagcgggca tccagtgtcc ctcaggcctc ctgttgcagg gactctgctg agagctggcc 44520
ccaggtgtaa gaggcacagc caccttacct ttggcccgat gctgccaatg ggcccgcggt 44580
ctcctctttg cccggagcat ttgcagggaa ctccgcagca agctttctca gcgatgttgt 44640
cctaccaaga agaaaagaag ttcaagtcct aacaaatgcc aagaaaaaaa aaaaaaaaaa 44700
aagcatatat aggccatgaa gggaaaaaaa aaatctgatg tgcgtggatt ccacagtgag 44760
aggtgaagct ctgcccacgt ggccctccct ctgagacggg cccagcagtc agatggcagt 44820
gggaagtcac acacggggac gtgaagagcc accaaagtaa aatagatctt attcatggca 44880
tcactttggg ctgggcctta tttctaaaag tttccaatgt ccatcttcaa atttcacaca 44940
 
ttaagcatga gagacatatt ttcaaactct aaagagcaag acctaagaag caagtygcat 45000
atggcccgga tggaaggatc ccccaaatct cctcagaacc atggtcagag cacagaccag 45060
ggtgccagga cggaggaaaa tagggaagag ccacatgtgg ggcaaacaca agtggtccct 45120
gccaccaccc tgggtcacca aggaggccac actcagcaga caccctcgat ggtcccttcc 45180
catccgagcc cctgacttta cggggagagt gcatgcccaa gaaacatttc cagctgctac 45240
actcatggtg gtcaaacact ttccttacag tgggcttctc agagttttca ctttcttcca 45300
cgttccagat gtgtcaacta acacaacctc ctaccaccca attaaaaaaa aaaaattcta 45360
attccagcct tgggaagaat gcaagagcga aaaagggaca ttcaattcat cccacggcct 45420
gctattgcca ccagcaaaca gtccctgagt tgaccactca gccccacgtc ccccaaaggc 45480
ccacccactg ccaaccctgg agctagcatc tgcagccaag agaggctgaa cccaagcatt 45540
aggaaacctc aggggtcccc tccacctgct ccaaaggaaa ctaggagaga ggccatggag 45600
atgcttctct gcccagaaat gatctctctt ctcctgcgtt gccttgtttt ctcatgcaag 45660
ctttctgatg ctgacaacct gattcccatt tgcggacagt cactctgatg ttttgccttc 45720
ccgacagtct tatgaagggc aaaatgctcg caaaagggaa ctgaggaaat cgccgggtga 45780
tgccaccagc gtacccagag aaccactgtc ccgctggcca ctttcccagc tcttgggtac 45840
ctccggtccg agggctcaca cacaggatgg caacaaggag gtccctccca ggtgaattaa 45900
acaacttgaa tgattgagtg acccacccaa agggcccctc tgccattcat ctggcaaaaa 45960
aaataaaata aagtgccttc gttatcttaa ggtgacaaag aacttggtat ctagaccagt 46020
ttgtgcgtag agacataaaa actcacgagc tgctctgcta gttcataatc gaggtccagc 46080
aagttaagcc tcaggggcct gttgtatagg aagcctcgcc cgaactccaa ctgcatcagc 46140
tgctccaagt tggctacacg ttcaaggccc acgaggataa gagcctggac acctgcgcgg 46200
agaggcacat tcagtgcagc ttgcctggcc ctccacagcc accacctctt tgccctcctg 46260
actttttatt ttattttatt ttttttaaag atttattttt atttatttat ttatgataga 46320
catagagaga gagagagaga gagaggcaga gacacaggca gagggagaag caggctccat 46380
gccgggagcc cgacgcagga ctcgatcaca ggaccccagg atcgcgccct gggccaaagg 46440
caggcacgaa actgctgagc cacccaggga tcccccctga ctttttattt taaaagaaga 46500
cttcagtgta aaatgtggcc tttacctaag acatattgaa tcaaactagt tagccactct 46560
gaagggtcaa gatcctggaa attttgacac ctctgagcaa ttgctccagc tacatagcta 46620
aactcatcag tagaaataca ggaagtgaca tcatgaaaca cattcataca gaaattctca 46680
aactctggcc ttgtgaaaca tgggggtgct gtgggaagag ctgagatgca cacctcccaa 46740
accagttgag tcacagtctc atcttcttcg ctcaggaaaa gccagctaag tcatagaaca 46800
ttcttaggat aaattttgct tctgtaaacc tctggtgttg gcattcactt tctgccagtg 46860
ctatgaattc caaaggaaac gaatatccag atattatatc tgataatagg catccctgtt 46920
tatctaattg ccctaaattt ggaaaatcta tgttcattat ttggtgtgta gtagagcata 46980
tttttgctta aaagtatggt atttgtgagc tgtgatcaaa actacatatt cttttgcaaa 47040
 
agcatgcctg tgtcaggact tgctgataaa gccatctgtg gtacgagggg aaccccgata 47100
gcaccaggca ggtgtcagag ggccaggctg cagggaggat gccccaagga tcccccagag 47160
gactctagct gggtagtgtc cctgcctcac ccaggccaac ttctcacatt tagaaagccc 47220
acgacctacc ttcttgtcgg agttcctcag atgccttttg taaatcagcc aggtttccat 47280
ccactccatc cgtaaaatga atgaccacct ggaaaaaaaa aaaaaaagat gtgtcaaacc 47340
ccaaccaaaa ttagaaacat cagacttcca gcagggagac cagtccattc aaatcctcca 47400
tcttagagtc gaggacaccg gggaccaaca ataagaaaca gtcatcctca gtttatagct 47460
ctttgatact gtgcaataag tgagggataa aataaagggg gatactgtag tgcttgattt 47520
actaaaaaaa acaaaaaaaa aaaacaaaaa aaaaacctca gaataaaaat aaaaacagtt 47580
tatctcattt tcctatttat gtgatgtagt ttctagaaat tgtgtgagtg tgtgtccgtg 47640
ggtgtgttat gggctgaagt atgtccctcc aaatatcagc tgttgaagcc ttcaccttca 47700
atacctcaga agataactat atgtggcgat agggctttta aagggtcctt aaattcagtg 47760
aggtcattgc ggtgggctct aacccagtgt gactggtcta cggagaagag attgggacac 47820
agacacaccc agagggaaga tggtacacag acagaaggag aaggtggccg cctgcaagcc 47880
aaggagagag gcctccgaag aagccaaccc agcagacact ctggtctcag actttcagtg 47940
tctccagaac tgtgacaatc aatttctgtt gtttctactg tttcagccac ccagcctata 48000
cacatggaga gaaagagagg gaaacagaaa ccatgatgta cttaaggggt tggactataa 48060
ttcaacatga attaatcata acttcaactc agtcaagctt acttactgat ttccaggatt 48120
ttttttttta gagcattagt aaaactacta caatgaggaa gataattacc tttcagacta 48180
ttatgatcag ctgacggtaa ggcacttttt tattttttgg gcactgccaa ccccatcaaa 48240
cgggaagcgt gttcatagaa gaacactttg ggaaaagggg cttctggaca tagaggtgtg 48300
cctcctgacc actagaagtc cagaaactgc atgggaagct gaaagaagga ggagcccatg 48360
ctcaccttta cattgtctgc tgaagactgt ctgaacttgt tctgatagac cttcagtgtg 48420
tctgcagtga ggacataggg gtgctggttg cgcatgttcc ggaacttctc aaacagttct 48480
ggctggtact cggcaaagtc aaaggcctcc acgggacctg agggtgtgtt ggccaccacc 48540
gacacccgca cggtgggcat ctggctgccg ctgcagctga ttctttgcat ctggctgatt 48600
ctattcaaga tggcatccat cttggactca aggcccttct gggtcacaaa gacattctga 48660
tcccttgatc cgtcaaaccc cagaatcaca tccagattac agactgcaaa agggaaagta 48720
ggcaatgagc tctaggttga ggtggctgag atgagtccaa tacacaggca cagcccccag 48780
atggggacca ggcatcgtgc tcaagggcaa aatgagatcc tctatgtatg ggcttggaga 48840
agcaatttgg tctgcctctc tctcaaggca cgccagagct gactcttagg aactgagagt 48900
gtagaccatt tcaatgtgtt tgcttttctg gggtatttgt cagttttggc ctgcaataca 48960
gttggtgtgc acgtgcaccc caagattgta cttggaatta aacgtgcttg agaactcgac 49020
tactcttttc tcaaatagtt gatctaataa acctatcaac agaaacaggg atcgattact 49080
 
ctgaattatt tgggagatcc aagaatagag ggaaaaagta aatcttgtaa gagcaagaaa 49140
atacagactt ccactcttac cccaaaatat aacatatcct tattgttaac aggggccagg 49200
cggttgccag gacccaaccc acgtggatat aaggaaaatg actgaagcaa ccaaaggctc 49260
aaggacccca cgggccagca ccaggcacgt aaataaccaa acaggtccta aatgttggca 49320
caacaggatg aaaatgtacg tataagggga ttggttacct ttggaaatat cggtcacacc 49380
cggacataag gtttcatgca ttgcatcgtg cagagtttcc agaacttgct cactcaattc 49440
agacagctcc tggacgttcc ccactcggaa cgctgtagca ctgttggacg ctattttccc 49500
aacctcctct gagtcgatgt tcttcacacc cacggcaaac actttgacac ccttctgggt 49560
gagtgccagg ctggcctcct gggcatcctc caccgacttt ccacccgtga tcacaaacgc 49620
tatctgaggg accctctgat ccaggcggct gcccgcctcc ggcacgaagt gattcagcct 49680
caggtgctcg atgcccacct tggtatttgc gtgtctcccc cctttgtaga caactttgtt 49740
gatggcatca ataatctgct gcttggtgga aaagtccttc aggaagaact cgtcagtggg 49800
gtcagagttg tactggacca gccccacttg gatggagtca cccccctcat acagcgtgtc 49860
cacaatttca gacacaaaac ggagcacttc ctggaaactg tccctcctga aattgatgga 49920
gccatccaat aggaacacaa catctgcttt ctttttctct gaaagtgatt gaaatgcaaa 49980
tgaggagaaa tttggtttcc atatccataa caaatataca aagtgtaaaa tagctggaat 50040
gatgctaaaa atgctgtgac ttattcacag caaatacctt tgctttattc catgcctcta 50100
tggttcccaa attcacagcc actattttgc taagaacaag aagataaatg acaaaccatc 50160
tgaatgaaaa gataagagac agtaggactt ggggaaactg agaattcagt ctgattatcc 50220
cacacaaaat ggtatcatgc cacagattca acgatccaaa aactcagatg ttttaggaaa 50280
tgccactaag cacttccctt ctggttaaaa ataaacaatg atgtctgaca ggagttgaag 50340
gaaatgtgcc agaggcagat caaaaaaggg gtgggggaaa cagattggaa aatgggaagc 50400
agaccccgta cacctttgga agatcgcctg ccatcaggct aagagcttcg tggaagcctg 50460
ccaaaagtca atcacatttg ggggtaattt cctaattata atcaatccat caagactgga 50520
gtaaaagagg agcatggatt agccagtctc agattctgtc cttttctgag agccaacgaa 50580
tctagagtta accctcaatg caccttcata ggaactttca gtgaggttct aactagtctc 50640
agagaagcct ctggaggaat caatgaccat gttctctggg atatgtttcc ttttacagtt 50700
tcaactgaat catttttcca gtatttagtt tctaatatta tgtgcagcaa caaccttaac 50760
agatgaaaat tattaagata aatactaact atattttttc aatgaaagga taattgatgt 50820
cacgctctgt agttacaggt cacgcaagtc atacctggcc gtgagggaga aggtgtgtcc 50880
actcctggag gtgcaggcgt gaccccagag ggcccaaagg cgtgcatgac cctgtcttct 50940
atgttgggga gctctctgaa ctctcgcacc gtgaagacca gtctggggtt gttggtgatg 51000
gtctgcagct ctgctctgtc gatatttcgg tttcctactc ctaaactcac aatccctgag 51060
gagctgatca cctgcgaaaa tctggaagta tcatcctggg attttccacc cagaaacaga 51120
accaggtgtt ggggcacccc gtcttctatc cggctcccag cagacttgac aaagagattt 51180
 
cttgccacaa attccagagc tttgccagtg ttcagtgggg acccccctct gtgcctcagc 51240
tgacgcaaag cattgatcac ggaagcctga gatttatggg tcttcaggta aaatactggg 51300
aagacctcat tgctgaactg tacagcccca atcctcactt tattggggcc aatgttgagt 51360
ctgcgaacaa tcttgataac aaagtcccga atatatgcaa tgtcatcagg cttgacgctg 51420
tcagagctat cgattaggaa gataatgtct gctgcatcac tctcaatacc tgtggataga 51480
aagaaaagaa gaggaaaact tgggacacaa atggagtcat tatttggttt tatttctgct 51540
ttcatttctg aactttgcct tatacggcac cacaatatca attaacatta aaattataag 51600
ctgatgtgtc tgtgtttatc tcttgagctt tttaaatata atcactaaaa gttgagcctt 51660
ttctcaccaa aatcatttct gctttttgaa ctattcgaag ttgtccaaaa ctttctcttt 51720
ctaagtttac tctagtttca ttttggcttt gcggtctggg ttacaactag tttttaaaaa 51780
atgtaaacag catttagaaa ctggattatc agaatctaca gaatcatgag ctgttactac 51840
caaaagggat caagatcacc cagaataaca tctttggtat acaaaaagag agagacaact 51900
gacgtcagag gtcctctgtc tgaggccgcc acgtaggtag tgacacctag agaatgccaa 51960
ctctctccta gtgcacctcg acagctgccc aggtgtttga ctcatggaat ttcctcctgc 52020
ccacatatcc ctagcattta tacaatttgt tttattttca ctgttatttc ccctgttttc 52080
tttatatgag tccactttta actacaaaat taatcaaaaa gtcttgagaa agatgaacca 52140
tgttttcctt ttagttagga ttctaagtac ttcttggata ccattttggg gtttttttgt 52200
tttttttttt aaagattcat ttatttattt atgatagaca gagagagaga ggcagaggca 52260
caggaggagg gagaagcagg ctccatgctg ggagcccgat gtgggactcg atcccgggac 52320
tccaggatcg cgccctgggc caaaggcagg cgctaaacca ctgagccacc cagggatctc 52380
cccagatacc attttgttga gctactcata tgttgccact caacaaattc agcctctttt 52440
gcctcatcaa tcaccaggct acttataatg gaaacacagc acagcaaaag ctgagcaagt 52500
catggaactg agccattaat actgagaagg acaagaaact ggcccatcac acacagccag 52560
gccttgggtt ctcttgtaga acacaaaaga tttcacacaa catcagcatc agaatcatac 52620
acatacagac aaaaacgaag acaaaaataa ctaaatacct gccaccctag ctaatacgaa 52680
tgacttctgc ttctttacca gttacagtgt tagccataag atgtagaaag ataactaatc 52740
agaattattt tcaggccaaa ttgccatttc cttaagcctt ccacaaaatt gtccaccaca 52800
agctcaaatc ctatcctttc taacaccctc tccctaagat gctctatggt tcaccatcgg 52860
gtacatccac ccttcttgca acaagcaata gacacaaatg gttcaatagc agtgtccctt 52920
tattttaatt taacttatca tgagtaccat ggatctctga acctgttatg tttttatata 52980
tatttttaaa aatgcctcag agaacaacaa aaaatacccc atcttacctg gagaaggata 53040
gcgagtgctg gccaggatct gctgtatctg ctctgaagtt agggttgtga tgggggtcag 53100
cagtttctgc tccaggctgg gcagctcccg gaatgtgttc actgagaaca catattcagg 53160
gctcagggag atcttaacca actgttcctg gtctgcgtcc cttgcgatgg taaatggtgc 53220
 
aacaccaaac tgcttgagtt ctgctgcaga atcatctact tcatcgtcag actttccaga 53280
tgagatgagg accaggaact gagggacgcc ctcctcaatc ctgcttccca ggggcctctt 53340
gaagatattc cttgacacgt actccagggc actcccaatg ttgatctgcc tcccaccttt 53400
gggcctcagc cgcctcaccg cattctgcac ttcatcctta ctggaatggg cattcagcag 53460
gaattccacc ctggggtcct cgctgaactg gatgactgcc acccgagtcg tgtcaaagcc 53520
cacgtccagg tagtcaacca gtctctcaat gagggtgcgg atgtactgaa attcagggct 53580
agcattttgg gacccatcga tgaggaagac cacatccttc ttgcttccaa cacctgaaaa 53640
tcacatgtaa tcatgggctt cacctttgcc ggcaccaaat ccttagtgtt aatgaaccac 53700
ctggttcatg gtttgtcatg gaatcatcac ctgacgggcg acataacctc attctgacta 53760
ttacagataa aataccagtc ccaggagtgg gttctggctt tgcagtgatt ataagtcagg 53820
gagaagaaac cagcaaagga caaagagtaa ccctgaggga gggggaaagt agacacataa 53880
gagccatgtc tagaccaagt aggggtggtg tttcaggaag gagatgatct caactgggac 53940
atgcatcagc tcctcataga tcaagcaaga tggcatctgg gaactgatca tgggggaacc 54000
ttgtcaagag cgctttctgt ggaatggtga aagtgaatga cagatagggg tgggttcaga 54060
gaggaattgc aaaggtttca ataactcttt taaaagagga ctgaggtatt cctggagggg 54120
aagcaggcat caagagggtt tctcagaaag aagaaaaagt gatgtgtctg tatcgtaaag 54180
ggaatcatcc ttcccatcct tggtggagaa tcaatgaatc aggagaaaga atgatggatg 54240
gcgtccttga ataggtgaga gtgggcaggt gcctaggagg cagagacggc tcacctgggc 54300
tgagctatgg acagcagtgc tgggaagaag gaaagcatag aagaagcagg ctaggaggta 54360
gacaaaggta gaaaatagtt attttgaaaa gtgggggagc aaatatgttg cagaaatatg 54420
tgcttgtatt taaagtcagg aactcacatg tgcactatga caagacttcc agagtgcatt 54480
cttgagaacc ctgcaccata tagaaggacc ctggagtcaa atagattttg gaactgtggc 54540
attcccttat tgcaatacac atcaattgtg ggaggacctc tttggcttta tcccactgtt 54600
ccccagattt atttgatcac aaacccttta taacattcca cagaagcctg caggacacgg 54660
tgtactctag caaggaaaag ccagccttgc ttctactctt cccttccttt tgttcccctc 54720
tgcctttctt tttcccccct ccacaccatg agacttcgga tggtactctg taaaatgtaa 54780
gccacaaggg agcaaggacg ctgccgcctc ctcaactact gcaaccctct ccacctgagt 54840
gcccagggtg ggtgagccct cgtgggtatt gtgcagggca cggatagatc tcagccccct 54900
tcatccccag cagccaggat cccactgttt tctagcttat tgtaaccagg tctacaaaag 54960
gggaacagga aagacgaaat agaatgaact acagttataa gagcaaggtt ggctcctgcc 55020
ggttaagaca tttacttctc agcaccagcc acattcaaag gcagtcatca tgctttggct 55080
ctagaaatac caatatttca gagactgagc cccagcatag gggaccctgt tgtcaagttc 55140
cctccctcat cacagcaggc tgccagctga accccccagt ggaaccttag aactgatatc 55200
aagccggtgc caccagtgag gtggccccat ccttttcaac caacattata cagaagcctc 55260
tacatggact taggggtcta cattaaagcc aagcatatgc acatctttga aacttttata 55320
 
atttggccca aaacatgaat catagtatgt ccccaggcct ctgccactcc tgatcactga 55380
gaccaaaggc acatagttcc taattcttca tgaaaggcca gttttcacca catatggtag 55440
agaaaaccaa cagagaatac tttggagccc acagtccaaa caagtagccc catccaagga 55500
atctcctcct tacctggact cgttggggga agcaaaactg gctgcaaccg gctcagttct 55560
tcccggctga gctggatcac tctgtcggag accacctgct ggatggttcc cagctgccgg 55620
aaggtaggaa tggccacggc aaagtcaggg atgaaggaga tggtctgcat ctctgtgatg 55680
tcagcattcc cgatgccgat gccgatgggc actgcccctc ccctcttcag gaccactgag 55740
gggcccctca catcatcccc tgacctgtca gccgtgagga caatcaagag ctgggggaca 55800
ccttccgcta tcctgcttcc agctgagctg atcaggatat tcctcaggac aaagtccagg 55860
gcagccccag tgttgggggt tggccctccc aggatggtca gcctgcggat ggcgctgacc 55920
acactctgct ggtccatgta tgaattcagg tagaactcag gtctggtccg gtcgctgtac 55980
tgcaccacgg ccacacgaac ccgatctggg cccacatcca ggctctccac tactctctgg 56040
acaaattcct tcagcagagg gaagccactt ctgacacctt cagagccgtc aatcagaaac 56100
accacatcct tctcacctga aactgggagg gagacaccct cagtgacaca caggaccaaa 56160
ggactgtctg ccttggatca aagtgggcag cataatggcc ccacacaggt atccagttag 56220
gaatccctag aaattgtgga ggagactctg cagggatgat tacattacat gtttcaggga 56280
tgaggaaact gtcctaggtt atccaggtag atccaggata atcaaccatg tgtccttcta 56340
agagggagac aagacaatgg aagttcaagg gagagctcta agagaggaat cagaggccag 56400
aaagaagatg atctgctgct gctttgcaga tacaggaagg ggcttcaggc caaggactat 56460
ggtccacatg aggcaaggga gtgtgtgacc catgaggacc tccaaaagga atgcaatgtg 56520
gttgacacct tgcttttaac ccagcgaaac tggttttgga ttgctgacct ctaaaaccag 56580
aagacaatga atccattttg ttttaagtca ctaaatttat ggtaatttgt tacagcagga 56640
atcaaaaatg aatatagcaa gatagataca tgattgatag atggatagat gatggatagg 56700
tagatgatag atagatagat agatagatag atagatagat agatagaccc tttattttag 56760
gatttctcaa tgcttgagca gtatggtcaa ctttcaaagg tgtactcagg aaacagtgtt 56820
tgctgaggga gagatgagac agcataacct gcataaccat cagcacgtta ctgagatggg 56880
agggtttgag atttgccgta tgtcacagcg aatcaacagg gaaaaatgga gaccagatgt 56940
tggggttaca atccaaaacc aacagtgtat ggcccaagag tcaatgtaga agttctaaac 57000
cctcggtttt taatcaaaag ggctgtattt gaaactaatt gctcttttct tgctgcattc 57060
ttatcgacaa gtgtaaagtg gttaaacaca aagtcttggt aaaatggatc agcacttgga 57120
gattccagtg aagtgcttat ggtgagaatt cacccacctc ttgtcacact ttatattctc 57180
tatgaatgaa ttgtggatga attagagcat agggagaccc acttaaagta agacattagg 57240
ggaccatgaa gttgaagaac tttttagcta taatgtaccc ctggtatgtg gcttttatta 57300
aacatctagt aaaaaatagt gttttcaaaa agatttttaa ctactgatgt gtctaaatct 57360
 
ttataatctt aattatgaaa tatatatcca attatgaaat attctaatgt taccctgatt 57420
ctcctgtttt gccttgagag cagttagaca attaaagtga tggaaaaata ttccatgctc 57480
atggattgga agaattaata ttgtgaaaat gtcaatgcca cccagggcaa ttaatacatt 57540
taatgtaatc cctatcaaaa tatcatggac tttcttcaga gagttggaac aaataatctt 57600
aagatttgtg tggaatcaga aaagacccca aataggtgga gtaatattga aaaagaaaac 57660
catatctggg ggcaaaacaa taccagattt caagttgtac tacaaagctg tggtcatcaa 57720
gacagtgcgg tactggcaca aaaacagaca catagatcaa tggaacagaa tagagaaccc 57780
agaaatgggc cctcaactct atggtcaagt aatgtttgac aaagcaggaa agactatcca 57840
ctggaaaaag gactgtctct tcaataaatg gtgctgggaa aattggacat ccacatgcag 57900
aagaatgaaa ctagaccact ctcttgcacc atacacaaag ataaactcaa aatggatgaa 57960
agatctaaat gtgagacaag attccatcaa aatcctagag gagaacacag gcaacaccct 58020
ttttgaactt ggtcacagca acttcttgca agatgcatct ctgaaggcaa gggaaacaga 58080
agcaaaaatg gactattggg acttcatcaa gataaaaagc ttctacacag caaaggatac 58140
agtcaacaaa actaaaagac accctacaga atgggagaag atatttgcaa atgatgtatc 58200
agatcaaggg ctagtttcca agatctataa agaacttatt aaactcaaca gccaagaaac 58260
aaacaatcca atcatgaaat gtgcaggaga catgaacaga aatctcacag aagaagagac 58320
atggccaaga agcacatgag aagatgctcc gcatcactgg ccatcaggga aatacaaatc 58380
aaaaccacaa tgagatccca cctcacacca gtgagaatgg ggaaaattaa caagacagga 58440
accaacagat gttggagagg atgtggagaa aggggaaccc tcttgcactg ttggtgggaa 58500
tgtgaactgg tgcagccact ctggaaaact gtgtggaggt tcctcaagga gttaaaaata 58560
gagctaccct aagatctagc aattgcactg ctggggattt accccaaaga tacagaaatg 58620
ccaaaataca cctgcacccc gatgtttcta gcagcaatgt ccacaatagc caaactgtgg 58680
aaggagcctc ggtgtccatc gacagatgaa tgtatacaca agctgcggtc tatgtataca 58740
atggaatatt cctcagccat tagaaacgac aaatacccac catttgcttc agtgtggatg 58800
gaactggagg gtatgatgct gagtgaagta agtcaatcgg agaaggacaa acattatatg 58860
gtctcattca tttggggaat ataaaaaata gtgaaaggga ataaagggga aaggagagaa 58920
aatgagtggg aattatcaga gagggagaca cagaacgtga gaaactccta actcggaaac 58980
gaacaagagg tagtggaagg gtaggtgggt tgggggatgg gatgactggg tgacagcact 59040
gagagggcac ttgacaggat aaacactggg tgttatgata tatgttggca aattgaactc 59100
caataaaaaa aaataaacaa aaatgattaa taaggaagaa agaaagaaag aaagaaagaa 59160
aagaaaagaa aagaaaagaa aagaaaagaa aagaaaagaa aagaaaagaa aagaaaagaa 59220
aagaaaagaa aagaaagagc cagcagccta ccgcataccc acgcattgca gaactgggcc 59280
gcggaagcaa ccagaatttc tatgccatac ctggtgccgg tgccccattt tgcactgatt 59340
ttaagagatt gacgatctgt ggttgaaggt ctccaatctt gggaagtgac tcggcggcca 59400
ggataaagac aggcgacggc accatgagct ccagctcagt aggatctgcg ttcttggcct 59460
 
gcaagatgaa agtcaccacc ccgctctgtt tcaggttgag ggcaggtgtg tccaaacgat 59520
ctgaggacct tcctgccacc agcaggacca ggaactgaag cactccatct tcgatccggc 59580
ttccagcgga cttgacaaag atgtatctcc gtgcgtagtc cagcgcgtag cccagattga 59640
gggttttgcc agtcttgatc ttcattctct tcacaagatt caggatttcg ggcttattct 59700
ggtgctcatc gaagcgagac tccaccctga catcatcgct gtactgagcc actgcaaccc 59760
gggtcccctc tggtttcaca tccagctcat cgataacctt gtagagaaag tcgcggaccg 59820
cagggaactg gcccacgaga ttggctgagc cgtcaaagag gaatagaatg tctcgcttgc 59880
tctctacaac aaaagtaggt tgggacacag tgagggacga gaaccttcca ggcaccagtg 59940
tgacttcagc ccagcaccac tagcatttgg taatggggag tcacacaatt atttttgatg 60000
atcactgtac cagcaaggaa ggagaaaacc cactgtcaaa acctgaagca ccagccatca 60060
gtaaaatgct ccatttcaca gcagtgcctc ttggcaaaga accggatggg tctacccaaa 60120
ctagtgtcat gcttcttcaa caaagcaaga cagtgtaagc tccagagtgc cctgttcttg 60180
ggtcaggagg tcgtcactct aggagaacag tgttttccaa tcattgtaaa ttaaaccact 60240
taaaatgtgg cccaaattat ccttttgctt caaaattgac cagtcctttg caacaaaggc 60300
tgacatgggt taaccatcca aggataactg agttgacacc tatctgctct aagtggatcg 60360
tcttttcatt aactttgagg ttatggacta ggaccccaaa gaatacccac cattgctctg 60420
tggtacagtc ccaatcctct gtacttagtc cacagaaaac agggatgaca gcccagaggg 60480
ggctaccaca tgtaccgtga acattttcag ttcaaacagg attaggatgc agttgtctcc 60540
cccttgctac ccatctgggt gatgtcagca agtggtagag ccaacgtgtc ctcatctcac 60600
cactgtactg atttgcagtg acaacaacag tccagacttc ctttggaaat cattttatcg 60660
tcacataaag atgcagccct tgtggggttt attaaaaata ctaatttcta ccttctgcag 60720
attttgaaca ttaaaaaaat catctttaga tgacctcagt ggattatccc atatgaaagg 60780
gaggccgtcc ctctcactct cactgccgtc tggatctaag ctccagaaac cgtcacagga 60840
ggcaggttga gatgggagga acagaagctg gagtttctag actttctttc acaaatgaac 60900
ccccttgaga gagaatcaaa acttgctggt accgtgtctc aagcccccat ccaaaacatt 60960
tccaccgcag aaggccagtc tcctttctac agttcagcac gctcctaggt tcagactggg 61020
ccacctgcmc ccgggggacg tgctggggga agggagcccc gcgcaggtgg ggagctttac 61080
ctggctgggc gagtgggacc tcctccacgc ctccactaac ataagtggtt aggggctgaa 61140
tcagctgctg aggcaaagct ggcagggagc tgaaatcatc catgagatac accaggctcg 61200
ggttaaaagc aatctgctca agctctgccc tattcgcctg gttagccccc acgcagaagg 61260
tgaggacgcc cgcgcgcgct agggcattgg cagctggcag ataggcgtcc tcagaccgcc 61320
cggctgtgag caggagcaag agctgcggca cgtgttcgcg gatcctgctg ccgccagctt 61380
cggtgaagtg gttggcatgg acatagctca gggccgagcc agtgttcagg cctgaccccc 61440
ccttgagctg cagctgcctc agatgagcaa gcaaatctga ctttgtctgg tatgtgttta 61500
 
gggagaactc tgttactgga gtgtcactaa attgcactaa accgacacga atgttgtcgc 61560
ttccaacatc aaggttgtta actacgttca ttacaaagtc ccgcacataa gggaaattgg 61620
cctttccaac gttgaacgat ccatccaaaa gaaagatgat atcccttctg tttgcgtgaa 61680
ctgcagtgga gcacagaaaa caaagtgaga catacacaac cgcgagggcc acatcatgcg 61740
tgcccgcacc tcgggcacgc atgccaacat tgagaacaca cagacggtca cagtgaggca 61800
aagcatgtcg gtctcgtaaa atatggcagc aggctgcata ttttcctcta gtggcggctg 61860
gtgcttgtga ggcatattgg tagccaagtt gacgaaacca agttggcttg tggagagata 61920
cagtgccaaa gttagaatat ttgcaaccaa caaaaatgac cggaatcctg gaggtggaac 61980
attccttccc tggaagtgcg ttcattccct gcgagggaag ccaccagggc actgcggctt 62040
accgcctcac tcatgtttat gaccgcggtc gcggccaggt tcacacgggt gagacagggt 62100
cccccggggc ttcaccccta cacatctgca gagagacgtc cactgacaga agtgttagcc 62160
caagttcacc tgataataga agttcctcaa ggctagaaaa aaaatgaata aaatggctct 62220
attctcatcc tgaagtattc ccaggaccac ccctttgtca ctctctattg atactcttct 62280
cttgggagcc acagcatcgc ctggcagtaa tccagcgggg tgcccgtggg aatgcctcca 62340
gagtgagata ataggagcat ggaaacgggt gaaaatgcaa aaggattgtt gaccatgctt 62400
gactgaagac aggcacgtga agattcaaaa gtataatttt caaattagag aatttgtagc 62460
agcacatata gtactacatg cctttccaaa aaaaaaaaaa aaattacctt ccatcttttc 62520
cctggttatc tcactcaact tttggcctaa caactaattc taccctatgt cctattaaat 62580
ggctttggct aggagagtgt ggactggaag tctgaaggaa gtaggagaga gggaggcttt 62640
cagaagagca gagagcctcg ggagaaagca tgcagcatgc aacatgggtc atgcatccaa 62700
cagtcttcca gggggtgagg ccgtgaggtg gaaaagctca tttccgtgat attcatcttc 62760
attaaggacg gcgcatggta ctcatccagc tcaacagaat ttgaaaataa cttttcactg 62820
atcatcacat caaaggaaag attttagaaa ccacttgggt ctttatctcc atgcccattc 62880
ggggactgaa gggaaacccc ttagagagag gagagaaggc caataacatt ctgcgctggt 62940
cagggctggg ccactgtcgg ctcaggaggg caagagccgg gccagatgat gctggtggca 63000
gaagcaggct ggacgggagt caccagtagg gacagaaccc tgggatctga caagaggtaa 63060
caactaccaa gtgactccct aggctaggcc agaagctgta aggacaggtg acccctggcc 63120
ctatagtctc tgtcatagga ttctgtttga tgacatgtga agtcatgcaa ttctcatcct 63180
atgacactgg cagacatacc attgtatcat atggcattgg ggtcacacaa tctccaactc 63240
agggtaaaag gaagcctgaa gtaaatctca tttgtggctc ttgtttccat catttaacac 63300
ctatctctca gctgagcaca aatcagactt ggttttgttc tgttttgctt ttctcaaagg 63360
ctctgcctgc agaccaccct gccgtgctct gacttcctcc ccatcatacc ttcagaggtt 63420
ccggagaggg tcctgagagg cgccagcaag ccgggcagca ggccctgcaa aggggcggct 63480
cggaactcgg tggggatgaa caccagggag gaatcaaatg caatctcttc cagctcagcc 63540
tggtcggccg ccttgctccc aacggcaaag gacatgatgc tgtttctctt cagctcctgg 63600
 
gcgggctggc tgatttcatc tagggactta ccgcctgtaa tcagcaccag aagcttaggg 63660
accccctcgg cggcccggta gccagccgaa ctagtgaaca ggttgtcccg agcaaagtcc 63720
agagcggagc ccgtgtacag gaccgagcct tccatgggtt tcattctccg cacggcggca 63780
atgacttccc ttttattggg gtagctattg aaataaaact cgggcctcac agtgtctgca 63840
tactgagcca ctgccacttg gattagatcc tgtccgatct ccagcctctg gatgactttg 63900
gcaatgaagt cgcggattgc attgaagttg gccagtccca gtgcggatga gccatccacc 63960
aggaaaacta tgtctctctt gttgacttca atgactgtag gtagcaacat cataaggtga 64020
gtagaatggc cttacctatc gcatccaact aagaccctct atacaaccaa ctccactcca 64080
agggcattcc cgtttgctcg ggccagcgtg aagtcagtgg gacccccctg aagaatctgt 64140
cataacacac acattagtgc cagctgcatt ctccctgtat gtggtggcag gatgggctcc 64200
tgaccctccc acccccaccc actcttagtc catttcacaa aatagttgca agaaaacaaa 64260
ttgagtttta gtaaccgcct gtttagcgca ttatcagtaa aaaaaagaaa aagaaaaaaa 64320
aaagccagga agctcttttc tgccaaaagt ctataggtta gatttgaaag tcagaatgaa 64380
gccatagcag cttaaataat taaaggaggc atcagtgtct actcagaggg cccttcattt 64440
cacaactgtg atgtcatgaa catctgccaa gctgatggca caaacatccc cattccctcc 64500
cgttctactc ttcaagcctg ggtttttcag gcgaagaaaa gagaaacaat tacttggcat 64560
cataaaactt acacaaagga aactgatgtc aaacccagca agtgacattg aagtatttcc 64620
attcaagaga cctgcgttgg ggaggtgcta ggactgtgat aaaccacaaa gcaaaagcac 64680
ctgagcaagg tcactgcctc atgcctgtga gcttgtgtcc acctgccatg tgagcaggtg 64740
gcctgagcca tgaccaccag gaactgggtc agacacgaca ccccatctaa acacctgcca 64800
gggcttttag gacttataag aacatcagtg gttaaagctg aggcaggaat gcttgtcatc 64860
tattaaataa ttatctggac gtgagcaggc ctctaccgct ccacaccgtc aggtggatta 64920
tgtgtcttga cgtttgacta tcttattact catccaaatt gtcctctttt aagagaaaac 64980
atgtagatgc gcacagacac aaagcacccg gggacaggca tgcagggaca ttgctctggc 65040
atcagtttgt ggtgtgggaa cgatgagttc caagtcctac tctccataga gatgtgcaga 65100
tgtgtggaat tctcactaca tgcgtgtgca aaagacactg cgtgtgaccc aggcctgcct 65160
tctcctcccc tccacatgta catagaagac atacatacgt acagacagct aaccacacgt 65220
gtacgtttaa tgcacacaca gatagtgagc ctgcccggat gcacgcactc acacactccc 65280
ctcctccata ttgatgtaca agtagttctg acctgaaaag cagagattcc tgcattgtgc 65340
gtgtgtgtct gtgtgtgtgc gtgtgggtaa catacatggt ggatgagcag aaagacaggt 65400
gaactgtcat cctcgatcac cgggctgact ccctcagcag gttgacgggc agcctcttgt 65460
cccccttcag tgcgtgacat cctccttcct atcatgtgat gccaacccag atggagctgt 65520
cttttgctgg gcctgggttc aaaataccct ggttgggagg tgaacctaag gtcctgctga 65580
tctgagggct tttgctctcg cctccggagg tagccgccac cagcacaccc tgaggacatc 65640
 
acatcctccc agacctcaca agcccgggac cggggtggct gcccttgctc tgccgtgctg 65700
ctatttgcaa atgtccagac ccctcctgtc acttcttccc aggggctgtc tcctcaccgc 65760
cctactccca tcatgctcaa tgactcagga ccttggcccc cagtaaatcc tgtcctcccg 65820
tgccgcctct caccccctac cctgcaactg cttctcaggc cccacctgtc cttaccatct 65880
ccggagactg ccatccccca ccctcggcct ggcctcacct gcagctcact caccctccac 65940
ctgcctccac catgccaggc cccagatctg cccacccagc tccgcagccc accttcgggg 66000
gccttgcctc ggttcctgac aaagcccccg tccttctccg ggctgtgctg ctcgggtaat 66060
ctgcaccagc ccacgagggc cagtgctgtt ctggaagctt tcctctccca gcctccagaa 66120
tgactacttc atcctctgct cttggctcac acctggcccg ggcccaagcc caatctcacc 66180
acatgggtca ctgagaaaac agaagccact ggaggacatg ccttcagctc cacaggcacc 66240
tctacctccc tcggagctcg tcctctgccc tccctgtctc ctcctgcctc tcctccccac 66300
cccccaccag cgctacagtg acaaggatct gccaaggcca ccaatgacct ccatggggac 66360
acatgcagtg gagccctcgg tctcatccaa ctgagcttct cagcagctct ggacataagc 66420
cacctctccc gccctcgaga cacaccatcc ttgccaggct tccaacattc cactccggca 66480
tctccctgca ctgaccgccc tcccaggtca ctttcgagag cattcctttc cttacccagc 66540
tggcctggcc ccctaggtcc tgtctctctc ccagccgcac tcccacacct ctcctctgaa 66600
cccacatgtg actgtctgtg acggtcagcc cccagtgccc atgctcacct caaacatgac 66660
acggtgaaga caaagatgtt tgtgtttacc cccaaactag ttgcttctgc agccttccgt 66720
ggatcaagtg agctccagcc ccactacctt ccaacagtcc tggaaagatg ccaggctcct 66780
ttgcgttgca gggccaccat cccttggaac actgtccctc tgctcttaat ctgatgtccg 66840
ctttctatcc tgcagcttct ctaacaggcc ttcgctagcc accctgtctg aagctctctc 66900
cccctagtcc tccccttaca gccccagggc accctcatct accttctcac actcccaata 66960
ctgtgtctgg tcgttggctt gcccttccac catctgccca ttatcctgta acaggccagt 67020
ggccgtgtcg ctctcgtcca ccagtactgt tgtgtccagt gcttggcgta ctgtccacac 67080
cagaaagcca agaatctttg ttgagtattc aaagtagtca caaaggtcaa agatgtttgt 67140
gagtgaggac ctaacgctct cgctagggaa aactgaactc agagcatgtg ctgcccagga 67200
agaagaagag tgtggagctc ctcctgggac agaaaatgtc ttcatctagt gtagctcctt 67260
tgtcataaat tcaagaaatt ggagcccagg gaagttgtgc ggcctgccca ggatggcaca 67320
gctggttcac ataagaccca ggatcaaagg ccaggtcccc cggctacagc cgtggtgttt 67380
tctcaatgtt gaagaaacct atggaaacca tctgtttgtt tccaaaagtg tacaaatcca 67440
gagaaacctt cgtgggtcgt attccaatca gaaggatgtc tagaaatatc aaggggattc 67500
tcaggatgga atttggcatc aattctgatt ttccctaccc agcaataatg cggaataccg 67560
ttggtaatct ctggcggaca gatgagatca ccagttgagg taacttggta cagttggatc 67620
tgcctaaacc ctaactgcca aagcctcaag cacagctata ttcttcttta cattttgaaa 67680
acacattctc acctctcaca tttgttaaat gcatatttgt taggtgatgt gatttgtttt 67740
 
atgtttataa attactccaa tcggtctctt tcgccaaagg ccctccattt gaatgtacgt 67800
catgatttca ccacttgaaa gaactactca gtaatctgac aagcacagga aaacagcatc 67860
tcataaatat cccaccaagt gagtgctttg ttgtttctta ccttcagaag tgtggctgtc 67920
agtacccagc atagggtctg ccctcacata cctacaagca cacctggtaa cacactggct 67980
tagaggcagt gccaaagtcc agggactaac tttctcagaa ttgcaatgag accctgagat 68040
ccatttttca gatgactgga tcgtaagtaa acaatacgat gatgctcagt agcctcagag 68100
taacagcttt cttgaattcc tcttaaaaag cacactttct tgacaatgca tgcattaaca 68160
ggagcttccc actcactcac agatcttgtg tctttcccaa cactgcgaca agcacggaca 68220
tcacatccaa gagctggtgc atgtcagaag ggctccactg tgggtactta caggttctcc 68280
tgaggtggat cattctgtag gtgcctgcct tacttctaca aaaaccaaca cacaaaacac 68340
cccactccca cacggatggc gagaagagca tacgtacctt ctgtgacaat ggttggcggt 68400
tgtaagacaa tgtgcctttg ggccacgcca acaatgtacg gcagtaattg ctcctggagg 68460
tccccaaagc tacggaattc cgggacagta aacaccaagt tgtcattggt agctatgtgc 68520
tgaagctctg ccctggaggc ggcctgggct ccgaggccaa atgagaacac gctagcctgc 68580
ttcagcgcta ccaccccgtc gcggatctcg tcactagaag gcccagcact tatgaggacc 68640
agcacctggg gaaccccttc ctccactcgg ctgccccctg cccgggtgaa gtggttctcc 68700
accacgaaat caagggcaag gccgacattg gccagctccc caccagtgaa cgccagggct 68760
ttcactgcat ccagaacctg ggccttggtg gagtaggtgt ccaaggagaa tacggttctg 68820
ggctcatcac tatactggac cacccccact cggatctgct gagctccaac tgagagtctc 68880
tcaaggagat ttacaaggaa gtcgcgaatg actgcgaaat gggcacttcc ggtgttgttt 68940
gatccgtcaa taaggaaaat aatgtctgca gaatcttgtg ctttaaaaga aagaaatgca 69000
aaaaatgtga aagcgttaga acaagtgacc acatgcattg ttcagtgttc tgacatgttt 69060
ctttggaaac caataagaaa cacgaaatat ggacaaacaa aacacatgtt gattaaggaa 69120
tgtgaaattc tctttcagtt ctgcattctg aatggataag aggcaacttc ttaggaacca 69180
tggtgtaaaa cggggcactt atatcggatt ttcatttgcg ccacctctgt agttacactt 69240
caaaacattg tagtcacctt tctgagaaat catgggcaca tattctagaa atttctatga 69300
aatttggtgg gagatgtcat tgcatggaat tgtgcattac tatacccaca ggagaagata 69360
agggggtgcg tgctatgaac agaggtccaa acatggtttc taaattttgg acatttctct 69420
tataatcctc aagtaattat aggaattttc tttgtccaaa tccacagcta ggaaacagaa 69480
tctcaggttc acggtttggt gacttatcat cccagtatgt tagacacttg attcctttga 69540
gcaacggttt cttctctttt ctttaaaaaa acaaaatggc agcagccatg caactctgtg 69600
aatttgaaag gaaaaactca agccaacgta aaagttttca tcttaaaaat actttcttag 69660
atgtttggtt gatcttttct tttttttaat aattgatttt atttaaattc aattagttaa 69720
catacagtgt atcattagtt ccagacgtag agtccagcaa atcttcagtt gtatataaca 69780
 
cccagtgctc attacatcaa ggtcttttct tttttcatat gatgtttagt tgcatggaat 69840
tcacatgcag gtctgggatt aggctagatg tcttgctcat gtgtttttgc tattgcctct 69900
gtattcaatc acataatgca gaacaatgtt aaggagagtg atatgaaacc agtcatgaca 69960
taattatcca accttatttt tatgacatcc taaactgatt gcctacacag atcacaagcc 70020
tctaataaac cctgaatcag aattagctct ggggctatac ggatggacat gagatcggag 70080
acctaggcct ctcttctccc cacaatggca agctagagac tcccatcccg ttacagagaa 70140
tgggggttac acagcagcac tgtgtaagga gtggcaatca ataatttata aacctaagta 70200
gaatatttca aaaggtcagc tagaacattt aatcagactg atttaaaact agaaaaaaag 70260
ccaaatcagt ttggtgaatg ctttttaata actgaaatgt gtgtcaggaa aagattctat 70320
gtgagcatat atgatattta tattctgaga ttttaggaag ctgtatttag ggtataatca 70380
caacatgata cataaaactt ttccttctga ccttgttagt ggtcagtggt cagttcttac 70440
atgtaaaatg aatttttcct tatgccagca aaattgcttt tatctttgct atggaaacag 70500
gatataaatc taatatcata aaactcaaga attttgctat agtgatagtc tgttctttcc 70560
caaatactct catagtgtca gatgagccag ggattactag aatcagaaca ggtctttctt 70620
cccccactta cacaaaactc ctgacatttt tgcagatgag ttccttgtgt agggaaagat 70680
atcagcttgg aaatatgaga tctattgaaa gggttaattt gctacagtga ttaggatctt 70740
gtatttttta taaatgtcac acaaaagtat gaaataacag ggggcccttg ctaagcaggc 70800
cagagcattt gctactcttt agccaaaaca tacgattcct gagggatctt tgggaggatg 70860
cagtgccaca gaaatggccc atggtgtcat ttgagtcaga ggcctgaggc tctgccaatc 70920
acaccactat attcctcaac attttccagg cctgaaccaa tgccccagag agacaatggt 70980
agatctgcca ctcactcagc acaccaagtc ccttaaaaga agggttagct atgagcctag 71040
ctagggtcag taagagaaaa atgatgccta aaaccaggct gacacattcc ttgttagttt 71100
tactttttca ttaatttatc ccactattat tctgttttgg gtgcaatttc caggaaattc 71160
tctttatggt acataaagag aagccctatg agaaaatcct tgaacttttc tctcctaaaa 71220
gctgaagagc agcaggtcat aaaaaaatgt tggataattt gtcatcactg aaaaaacaac 71280
attgtcctct aatgcacaca atgatggaca cacaagggca tgctgtgtta tccaggagag 71340
ccgaagcctc agacagacac gggagctatg ggttcttgtg tacagcaagc cgtaccaatg 71400
accaagatta accctaagga taaccaggtg tcactaaaag taaaaacatg acgttgtctg 71460
gcttgctgct ttaacggctc atgtatcaca ccctctgtgg cattttccat tgaaaacttt 71520
caggatgtcc atggaaaaat caggctcctt taacgctgtt ggaaacactg ttttcctgag 71580
catatttgtg tgggtacaat gccttcctta gctcatgaga tgcttcctct tttaaataaa 71640
aataaaaaga tcacatacat actacctatg caaacattaa attgtagatt gatttcgtga 71700
gtgctttgaa tctgtatttt acaaagaaaa gaaggctcac accaaaatcc tcactgaata 71760
cagaccactt ttagctttcg cttcagtgca aagcttttgt ttcttagtgg tatgcatatg 71820
caaaagaatg gcaaagaaat ttttaaaaac acaatctcca gagactgagt acttgagtag 71880
 
agttcattta agaacactgg ggacttgttg gcaaattgaa tttaaataaa attaaaaaaa 71940
aaaatcctct ccaactaata ttttatccac caagctgcca tgatacatca gtacatttgc 72000
tgatttatta ggcaaaagtc actttctggt tgtgcaaata ctgagaatca aacttaatga 72060
gtttgcagaa gtaaacctat gatatttcat tcacgtacgc ctgtgtagaa tgacatttac 72120
ccgaataaag ttgtagtcta ataaaaagag aatagcgggg accattagca ttgtatacac 72180
atccatattt tatgatcaag aacactctat gggtagaagt tacctggtgg acaggtaacg 72240
ggaagaaata gcgctaactt actagcaatc tctatactgc cctctgacta cctctcccaa 72300
aaaccataag cagagaatga ttattatttt actttttgct gtggaacatg cagtgggatg 72360
tgatactgga ggaattgcaa agtgaagtta atcagaaaag gcaacataaa cagattaatg 72420
ttccaccctg gagctgctag tggcttggat actagaagac agagatgctt tcttggcata 72480
aaaacaaagg ttttatcacc tgtgtggaaa cgtactctat tgacaggctc attgttcgtt 72540
taaagaatgg tcaaactcac aaaggcaaac tcatcccgag tcggccaaac cagcaataga 72600
gaaaacacgt cccgtttctc tactgatccc ttcactgttt ccgtatggat gagttctcat 72660
ccctccattc agaaaggtga tggtcggtga actactgaca ctgagactat cggagagaat 72720
aagaggtcat catagatact ctcatgtcgt ttccttgtgg cccagagcat ttggtttggg 72780
atgctcttgg gggtggttcc ccagatcctt ctcttataat ctggcaggat cgacacgtcc 72840
gtcataaaac atacgtgagg cgtgtcactc atagcagaga acacgttctg gtgtcaaatg 72900
taaataatcc aggccgctgc ttgctcccct gtcagaagta gacctgctgg caagggcctt 72960
agagttccca cacctgtcac agctgagcgt ataggtgacc aatggggaga ccctcgatga 73020
acaacttggg gttggtcagt ccttggaacc cttctctcct ctcaactcca tctgcccaag 73080
acgactgttc cttctcccga tatccatcaa ctacttctcc agcgctcgct ttctgaatct 73140
gtttcgggaa cctgacaccc gtactgtgtt cctacaattc ccccattacc ctgatctcaa 73200
tccagaaagc tacacctttc tggaataaga ccagactcca catgccctcg tgtctttcca 73260
catgcccctc ccacgcggcc ctttagattg gccaagttat gcctcaaaat ctcaactctt 73320
ttcttctcca tgctccatcc aacttgttga attaacaaga gaggccgact tttgacttcc 73380
tatctctgag cccctgggag ggggtggggg tgaccacttt ggaaagctta ggaggaacgg 73440
gcggatcttg ctcatagggt catttctggg gtgcagagcc tgctttctga ggctgctccc 73500
cactcactcc tcttgttcgg aggatcctcc acctcccggg cagagcaagt gccctgaagg 73560
atctttgtcc ttcctaaggg actccttcac atcacgctca gcctgcgccc accccacaca 73620
ccctgtgttc acctccaggg actttaacgt ctgtgtggta accctggtaa gcgccagcct 73680
ccctggtctc agctgcccct ccccaggggc cctccatccc ctggacgctc tgcacacgag 73740
ggccgccctg agcagccctc aaaactacca cacccccggg tttgccccca gtctcccccg 73800
tgctcagtgg ctcacacacc aggctgcctc tagggccctc tctccctccc ggtccacccc 73860
gctgtcccca tcttccccag ggcgggaccc ccgcgtgggc tgttgcaggg gctgactcgc 73920
 
cccctcctgg gggttccttc ctgagaaggg cctcttgcct ctccatcact gtctctcttt 73980
gcaagttctg caggcacttc cagcaccggc cttcgctgcc aggaagcctc cgttcccgga 74040
acaggtgcca gggatctggg ttagatgcct gcctcgtagg acccggcaag tgcctcccat 74100
gggatcctaa tagcagacag gcgttcttta gagtccagat ttacctgttt cacagaacac 74160
ccccttggag gcaggtgaaa gctccagttc aggagactcc agagcacggt caaacctttg 74220
tttttagccc cgcgaagagc catctcctcc cgccccccgc ccgccctagc cacgggcttg 74280
ctctaaggcc agcggttgcc aagcttacgt ccaaagaaca gcgaggcggg cagcagcctg 74340
gcaatgttgc cattacctgt gatgtcttta agggtctccg tgcctccagc cccttccgga 74400
gccacggatg attgcacaca ggacactaag tttcctacta tgtcatgaag tgaggtaaaa 74460
ttctctaggt tgaacacatg catattgggc ggttcgcttg ctatttcttt taaggctcct 74520
tcatctgcat cctcaactcc aattgcaaac acgttagcgt cagcagactt aagttccgct 74580
gagggcagag caaggcggtc gtcggagtgt ccatgggtta acactacgat aacctgaggg 74640
actccgtccc cggcccggct tccggcagcc tcagtgaggt ggtgctgcat tacgtattct 74700
aatccttttc cagtctcatt gcttccccca atataagaca tgttggaaat gtgggagagg 74760
acttcttgct tagtacggta ggtatttaac aggaactcgg tatgtgggtt gccgttgaac 74820
tggaccagag caaaacggaa atcactgtct cccacagcta aagattttat aacatcatac 74880
agaaactctc gaacaagttg gaaatgttcc tttccaatgc tccaagagga atccactaga 74940
aatattatat cggcagcagc accatttttg acatctttaa aaaaagacag tggtttagat 75000
gtttctcctg ttcaggcaag gacttcctag cggcgcaaag ccttcctccc tacgctgccc 75060
ggccttgtac tgcactgctc cccgggagcc ccgcccggag gccgacttac ctgagccacg 75120
ggcacctggc cgcggggacg cgacaccagc tggtggccac tcgctggccc cagtcccata 75180
tgcaatgacc ccatatgtga gccagtagca ggctccagaa tggggagggg gacagtccct 75240
ggagtgtctt tcaactcagg cgtgattctc tgtgacagtt tgagccccca agacccacct 75300
gatgagacca gaagcctatc tcccatcaga gacagccaac cagcgtttta cgagttcatc 75360
cttcagtttg ggctctgagc cccgagaaga aggaacagtg agaacttcta ctgctttaca 75420
ttagctagcc tccagcactt tggataattt gcaagaaaaa taaatgtaat caacctgtct 75480
tccttacatg aagactcgtg aaactgtaga gaaaggcagc gtctcatttt tctcagactt 75540
tttagcagga ggttagtgct ctaactactt cccatgtatt tttcagggcc aggaaagcct 75600
ttgcagatat ggaacactat ttatggtttt caaagatgaa tccagtcctc tctcaaaagt 75660
ggaagctgga ggctcagagt gccaggctgt gggtggccaa agagtgccct tctgcactgt 75720
aggccaggct ggctgcacac agcaagctcc tggactctac ttacctcagg ctgccctgac 75780
gcttcaagca atgtccccct ttccttaagg tagtgctcac ctccctgcca tctaaggacc 75840
atggacctca ggcaggagga tccctgccag aggcccaacc ctccaggcaa aggggaggaa 75900
gacccgaggc catgcctccg agctcataat gcacgaagta ttcctaagtt ttctaaaact 75960
ctgcaagcaa actcttaaga gggttaattc ggatctatgt tttcaaatag ttttgagaaa 76020
 
ctgatttttt aaagatgtga ttctacatga aaaaaatgtc tttttgcatc tagaattaga 76080
gaggaagggt gatccatgtg ttctgttttc aaggaagaat aatgttcatt ctcagtaatt 76140
gtcttgtcct ggttttatat ggcgtgagac caaagcagtg ccagaaatgc ggaagagctc 76200
ttcatttggc aaaatgtttg tgatacagag tttctgctca tgcccaatgt aaaaacaaac 76260
cacctgcaaa acaactccaa gatatcaaac ccaagtttta gcaaaaataa gccatctctc 76320
actggataca gagcatctgg aaaagtctgt acactgaatg aacatcccat aacaaggggc 76380
ttcaccattc cttcaaagca agtctaagtt ctatctcaag ctcatgaggt ctaagatcta 76440
caatcagaga acatattcct ttgaataaaa gtaagaaata tttccggatc ttacctgctt 76500
gctgctgttg ggcacgggta agagaaaagc ctgagagaaa aagacaaaag atggccacca 76560
agggcaaatg tcgatgtttc ctcattttga atgtgtctaa gcaccaagtg ggaacctgaa 76620
cgagaaaata gggcaatgat cagttttgat actcagatta caagctagtg catcaaggat 76680
ttatcccttt tgtgaaaact ggttttgact aaacaataaa tgagactctt tgagtctagt 76740
gattatatcc cttttgtcgt agcttaaaaa aatgcaatta aattttcctt cctctcagat 76800
taaggaggac agcaagtcac caaacaaatg gcagaaagag tcaagagtca aggaacgtgt 76860
gggcacagaa agggacaggt ctgtgatacc cagctgaatt ttgtagaccc agtttgtagg 76920
aatccctact gtttacagaa aaaaatggac tttgcccaaa tcagactgta tgcaaaacac 76980
aactgtgact tctgggacca cttcaaatct tgcccttact ctgtggtggg caattttttc 77040
cccaagaaaa cctgctgtgt gccaaccacc aatctcagaa tggaacaagt gcagggacgc 77100
tgaattagca agcctgtgaa ggcagacctg agggttgacc ttcttcctac ctggtctccc 77160
attcagctgg tggctacaca cgtgacttcc aattggccaa gctcatgttc agatgggctc 77220
agtagcctgt gtcaccagac ctttgccctg caaactgcat catctccaca ccccagctca 77280
gaaaacaccc ccactctaac aaaatcccaa tggcattttt tttttcgtag aaatagggga 77340
aaaaatccaa aaatcatatg gaagttcaaa agacccagag gagccaccac tgtcttgggg 77400
aagaaaaata tagctggagg tgttgcattt cctgatttca aaacatctta caaagctata 77460
gtaatcaaaa cagaatggca ctagaataaa gacagacata tggaccgatg gaacaaaata 77520
gaaatcccag aaataacttc aatcccatgt atgtgtcaaa tgatccttca tcagggtgcc 77580
aaagctacat gataaggaaa gaatagcctc ttcaacaaat ggtgccagga aaactgtata 77640
tccacacgca aaagaacaaa attagatgct taccttacaa catgtacaaa aaccatctca 77700
agatggacta aagacctaaa cattaagaac tgaaagtata aaattcctgg aagaaaatgt 77760
aaggatcttc ataacattga atttggcaat gatttcctgg atacgacacc aaaagtgtgt 77820
gcaacaaaag caagaataga caaattggga ctagatcaaa caaaaacttc tgtgcagcaa 77880
agggaactat caacagagtg aaaagcaatc tacagaaggg agaatatatt ctcaaatcat 77940
gtatctgaca aagggttcaa atctggaata tataaagaaa tcccacaact caacaacagt 78000
aacaaataat aattgtctga cttaagaatg ggaaaagcgc ttgaatagac atttctccaa 78060
 
agaagataca ccaatgacca ttaagcacat gagaagatgc tcatgactca tcatcagaaa 78120
aatgcaaacc aaaaccacaa tgagctatca cctcacaccc attagaatga ctactatcaa 78180
aaaatagtta acaacaatta tcaatgagga ggcaggaaaa ttggaaactt ggtgccctgt 78240
tggctagaat gcaaaatggt gcagccacta tagaaaacta tatggaggga tccctgggtg 78300
gcgcagcagt ttagcgcctg cctttggccc agggcgcgat cctggagacc tgggatcgaa 78360
tcccacgtcg ggcctccggt gcatggagcc tgcttctccc tctgcctatg tctctgcctc 78420
tctctctctc tctctctctg tgactatcat aaataaataa aaatttttta aaaatcttaa 78480
aaaaaaagaa aactagatgg agtttcctta aaaagttaaa aatagaatta ccatgtaacc 78540
cagcaatccc acttctgagc atatacctaa aataactgaa acggggttgt gaagaaatat 78600
ctgtacaccc aaatttgttg cagaattact tacaataacc aagaggtaga agcaatatag 78660
atgcccatca atggatgaat agataaatat gagacatgca tacagtggag tattactcag 78720
ccttaaaaaa atgggggggg ggattctatc ctatgtgaca tcatggctta accaaccaca 78780
ccacccaggt gccctgagga catacacttt aaaacaatgt aaaagtacat tgcaagtgtt 78840
ctatccttga ttctttctat tcaaattaaa cattggattt ttctatcatc aaaacgcatc 78900
ctactggaaa actgttttat ggaaagattg tccacgtcat ttagattcta ctggattctt 78960
tcaaaaaata tagccagttc tttctaaaac aacctgcaat attgtaatag gtcaaaacct 79020
tttttttaaa atgtggcatc cacaaatttc aaaagacctg tcaacctctc acaaatgtgt 79080
gcacaattgt atctgtgtat aaagggtttt ctgagagtgg ggtccatggt cgcatgtgac 79140
tctgaaagca tctagtctac ctccccacta aagtgtaagc accatagcat cactgcttcc 79200
atgataaatt ttaatgcaaa tctttcaggg tgtccagctc agttgtttta aatatttgat 79260
tgatgcacaa gatacatgat taagaattag agcaagagaa aaatcatagg gcctcatcag 79320
taatcactgg acccatgcac tccatgtgga aaagagcagg aagggagaca agatctgccc 79380
acccactgct gggtgtggga tgggagtatc accggtctcc cgaaggcaac cttcataaac 79440
aggggacact gggtgttcag cggtttgcca acagcttttt aatcatccct ccatcttcca 79500
cagcatcaca tctaagaagt cagattctca gaggaatcca cgaatcctct catacattaa 79560
accctgagta gggtctctat tgagaaactc caaaataatt ttctcatctt cgataaggct 79620
caaaaaaaaa aaatctaaac tctaatgaga tgaaatatct aacatcttcc agaggcatta 79680
ctcacttcaa ctctcctcta aggaaaatga attttattta cttatttatt tattttagga 79740
aaatgaattt taaattggtt gaaaatgaca tgtaaaattt cttttatttc cgagatattc 79800
atctttagaa gagcaacccc ataaaagaag tgctacccat tgagaacaca aagtaacgaa 79860
gtgagtgtgc agggcgcaag aattatcttt tctaaaatct acccagatat taaggagaag 79920
agaagggagg ggcggggaga gggggggcat gaggacagag ggtaaaagct cttcctgtcc 79980
tagagacgat gcttgtgttg cccaagcccc actcatgacc gatgagctca cgtggtttgg 80040
ccgagctgat tcccaggtgg ggaaactgcc ttcggtccca cacacttttc ttgccatcat 80100
gcttgggagc cagtcatgtg gttcaagtat tcctagccca gctgctgaat atttgttaat 80160
 
gtttctgttt gcaattaaat ctattctatt tatcatgtca tgcagtccaa gttttttaag 80220
tgaagttgtt aaaagatgtc aaacctcatc ttcccacatg atcaccgggc ttgctcatat 80280
taataaggga aaggatgcag ccactccctc atcacccccc tcgcaaacct ccccatcacc 80340
ccagcaaata gagacaagag ggcacaagta gtatttaggg cacttctatc tcgcctgtgg 80400
gatttcatgt cctccttcca acaccccaag ggcagcaccc cgcccacccc cctgcccatt 80460
ttatagccgg gaaactgaaa ccaggtgagg caacgcgctg tgcccaaggt cacatggata 80520
aaacaagtta gaaccagaat cctcaccaaa gtctgcattt gcaaagccca tgggaagaat 80580
cccaggaaac cctgagagac catgccaggt ctggccccaa tcccgaggaa actccccatc 80640
tcttctaggg aaaaaataga cttccctggg gacagagtct gtgtcaatag aatccatttg 80700
tgtgccacca ttcttttaaa tatgaaagaa aaaccagaat tggccgcttg caacccttgg 80760
gttgggtggg gtgaggccct gcttcttcat gtcacagatt ctgtgtccag aaggcaacag 80820
atttgatcat agaacttcat tttcactctt atctttccat ttggcattag taatgccttt 80880
ccccgctctg cctcattctg aagaggattc gaggtgcctt tccatttgaa gcaaagatac 80940
ctcttcttac aaagattgcc tcacagagac ctctgactcg caatcgaact gggtcgtatt 81000
ccaaagactt tcccaagcta ttttgcaact ccatgaaatt agttttcata aagtctactc 81060
aaaagctttt tcattttcca tgtctggaaa tcttttttta agaaagagac acacacatgc 81120
acacaaggaa atcattttga aacaaaggct taaatatatc cagaggcctc attttgaggg 81180
catttattgg gtcttctccg agcacggctg ttttaaagtc aggttggtcc tcctatcaag 81240
ttctgggacg tctgttaact ctgatgtcct gtgtaaagtg agagctacgg aaatcgttga 81300
agaccaggcc ggcatgtgtc ctatctgctg tgagagaaga agtcacactt caggaaaccc 81360
taagggtctt gagttggcag tcactctcct tctccttcct tctcttactc cctttccctt 81420
cttgtaggaa tcctgggtaa ggagtcctcc tcacctgccc ctcactggga ggaggaagta 81480
aattgagaat tttttttttt ttaagattta tttattagag agagagagca cacagagaga 81540
aagggagagg gaaaagggag cccgacgtgg tgctcgatcc caggacccca ggatcaggac 81600
gcaagctgaa ggcagactct taaccaactg agccactcaa gtgccctgaa aagaggactt 81660
atcaggtcct ctgttaggaa aggaagaagg tgggatttaa tggattcctg ccacttgaaa 81720
gaactttggg ggaatcctca aaagcaaatg tattggaaca tctggagaaa ccgccctgcc 81780
cccaaatcgg aaggtggtct tttaactgtt tattttacaa cttgaacctc atctttccct 81840
tctgtgaaaa tgcacaagag agtatcagcc gcacatatgt atctgtaaac cattatcggt 81900
ttgacccagg ctctgaacgt gtttcctgtg atagcgaaat gttaactctt ctgattatgt 81960
gtgttggctt ggccttgctg ccacccacca tgtccttgtt agacactcag tagccaaccc 82020
aagatctttc atctctggct cctcctggta aaatcaagta aaacgatacc cgttcagcag 82080
ctcatgcatt taaacagagc aatacagaaa gggggaggct gtttggatgt cccccctgca 82140
agtggcctcc ttcctgcatg aggtcacgtg tgagtgtgtg ctgagcacta tcggggtgag 82200
 
cttccttccc cccttaaagg caacctgaag aacctctcga tgagctgcca gatatttgga 82260
aattgattta aaagacagat tcctagggta caaggtaata accaagaagc agaagtttct 82320
acgtctgctg cagttggaaa agcctgacac caccatgcac atgtagaaaa catcttgccc 82380
tcttgaagac aaggcagcca ggcacggctt tttctccatt tatactgcta atgataaaca 82440
ttcagcagag gctagatcag atttaacatc aaggcagaga gtgccttcga aaacacttca 82500
aaagaaattg cacaaacgtc cgtcccgctc caagagacac ggggctgctg ccccaggctg 82560
gcactggcct ccgcaccccc ccaaccccgc cttccctggc cacggactct gctgtgccac 82620
tgcagaatga ctcaaagagg ctaaaacgtt ctcactctag taaggacaaa ttaaacattt 82680
ttgctttcta ctctgaatca ccatcaagtc tggaagagaa aattatttta aaatagactt 82740
tataaaaaaa attaagttga aacatctaat tcaatattat ccacactgga tttagggtaa 82800
cagcctggaa aaattctact tccttacctc tctaccagta atggtcagct aatctcccca 82860
ggctcgctta atttatgtgt aaaataaggc taactgatag acactcggac agatggggtc 82920
ctgctacgat ttctttccat ctgtttcccg ggactgaccg gggctgggtc tccccctgac 82980
cccatgacaa acagagaacc ccccaggggc acatggggca atctgtgagc ctcggcccag 83040
ctggctctag ctctgggtgc tggacccagg ctgctgaatc aactgatgaa gattgcagaa 83100
gagaaaaacc agtttcctgg aatggcttcc cttgagacct gagtcatctc cagctgctgt 83160
tcaattttta attttcaaat taaatggacc catgagctct tgccttccag tggccaattc 83220
tcgacgctct ggatccgtga aaactaaatg tatagtgcac cccagggaaa tttctcagag 83280
ctgcttccca ctgtgctttc ccctggccgc ccctaactcg tgaagcacgg ccgtgagtct 83340
gagcattcag ggaacgcagc aggatcacac tacgctagag gcggccggac ccccccacaa 83400
aagactcaca atttaccttt gggaaagggt ttggcaaaat ttttcatgtt tgtcttattt 83460
ttctcctctt gaagatttaa aaaaaaaaaa aaaaaacaat gactctctgc taactcaact 83520
tagaatacac tactccgtag aagactggtc aatgggaggg ggtgcctttc ttcacatggg 83580
agaccctaaa atattctcta acgactttta cttccttgct atccctggtc ccgggtatct 83640
gcttacgggc tcacattaat atgggcagga gcgcccagaa atgctgactg tgctctgcta 83700
ctagagctat gacctcaggc cagtaaatca cctctctgag cgtccgaaat gtcaagggcg 83760
tggaaagcat ccaccaaagg agtcattttt tttgatattg aaaatagcca agtggaaatc 83820
acatatttgc caagcagagg gtggagtctc catctcagtc tccccccccc ccacccccgc 83880
agccctcctc aaaccccaca gcccccatgt acacccgggg agcacagcag ggcttatcaa 83940
gcttgaacaa atatgggacc acggggaccc actagagggt taggtacctt ctctttgagt 84000
tctgttgctc aggagcctga ctgtggctag ctcaggccaa aagcctaggc taaccctatg 84060
tctgagagtc agtaatatat atataatata tataatatat tattattatt aacaaagatt 84120
taaaatggga agttcataat gatagtctgc aagaattctt taaaacaggc tcaatcaagt 84180
acgttgccta ttgggagaaa gtagcaaagg gatggcagtg aaaagtctgg ggatttgggg 84240
ggaatgacag aatcagctca tcttaacgtc attcctgcca gttttaaagt tcaggaatcc 84300
 
tacctggaga ccgcacggcc tgaaagtgta aacttgcttg ggttctgaaa ccacttactg 84360
tccatttaaa cttttctcag gagatgaggg caaaatgttt ctcttataag ctcttatttg 84420
gttagtgtcc gtgtcatgaa attattcgag agggcagagt gcctcatttc aaggcataat 84480
ttcacctcag cgtctgagca cagaaaaaaa atgtaaaaat cctaaattta gttctacttc 84540
agatattaca gtcccattta aaaatacatg tgtttaaaca gtctgaagtt acccgtgagc 84600
cctgctgaga tggctatgga cctactgcct gcaaacccag aaccccaccc tgaaggactc 84660
ccaccccttg gagatttttc tagagcttgg aaggatacac cgccctctag aggaaagaaa 84720
acccggctgc gttttcagga taggctcccc ccggggatga gggtgctcgc tcgaaatttc 84780
atctccctcc cagaattgag ctctgaaacc agaaggtgtt aaacgggcac tgaggggggc 84840
acttaatggg atgaacactg ggtgtgatgc tatatgttgg caaatcaaac tccaacaaaa 84900
taacacacac aaaaaaagca aaaaaaaaat tatgcttcca aaaatggaca taatgtggag 84960
agcccccata aaccaggacg ctgtgcgtgc tgagtggggg gcgatcatcc cccatgcatt 85020
agaggaagca agcagcatct tttccttaaa gcggaggaga accttccaga atttttacac 85080
tggaaagaca gacatcatat ctggaaatgc gggggaagaa gagaagcgtc cttgtctagt 85140
tgcaagcggt ctgaattagc agcaattttt taaagcttcc ctgccttgaa attggaatta 85200
tgttgggcct ccatttcctc agctagagct actattggga tttttttttt tttaaatgtc 85260
aggtcactcc tggagctgtc gactgttggt gccacagcag agtggatttt cctgcctgcc 85320
tgctttccac agcgaaggca ccatgagagc ctgtgggcca ccgtggattc ttctccaaga 85380
cctaacaaat cctctcgggt tcctccgaga agaacagtga ccgaaatgaa ttgtcaaata 85440
aaactttggg ctgaggtcat tggcatgcct gcgaggcggt cccatgggac agactttgtg 85500
gcttgctggt gacttataaa ccctaactgt ggaatttcac atgcaagtgg gtggtgacac 85560
aggctgctgg taaggaccat actttatctc cttgtctaaa catgctgagc aagctcacag 85620
gccggaagct ccccagcagg aactctctag caactttcaa aagggacacg ggacggagga 85680
cccgaatggc aagccggctt ccagcagtgc cccctgcagc cccctgaccc aaaactaccg 85740
tcagacaggg agcttcattt gagccacaaa tcaatcatta gaggggtttc ttcctacttg 85800
atcatcagtg atgtgttagg ttctaagcta caaaaatgac cgaaggggct cctgtggaca 85860
gaataaaggg gggtgcagaa ataaaggcat ttctatccag aaagatctca tatcaaaaat 85920
gccctgaaaa cctctcgttt ggacatttgc catatttcca tcggctgaga cccacaagca 85980
tggtggggtg gggcacacat ccttccaaga gctggaagcc aaaggctaac ccttctagag 86040
gcttccccgg ccacctccca ggaaggtggg gtggccactc cctccaattc ctccatagga 86100
aaaaggcggg atggccacac ttctccagct tgctgtggcc ttctccccca gaccctgttc 86160
tccacccaat gtcttcctgg ttctctcact gctggagttg gggaaagaga aaaataagcc 86220
tcacggtaat gacaacccct ggactcaaca aagatggtca agaccacgag accattccta 86280
tatgttgagt tcctacaaaa gcccctcacc ctccacatcc ctgtgtcttc accctcggca 86340
 
gcaagtccag gttaagaatg aagtaaagta caaataccac cccggtggcc cccagcccca 86400
gaggtggcct gagtgaggga agcccactcc ggtccagcac tccagtcctg tagcccacgc 86460
ccctgcctgc cagcaagttc ctagcacctt ttctccacgt cttggagagc ccgagggagc 86520
ataaacccat ttccccatta gttagagtag aataggacag aagaacgctg tcctctgggg 86580
ggaacctaac acatggccct ttccaatcca gatgtaaatc agcaatggga tcctccattg 86640
cttggccatc tgcttccctg aaatggtgaa gatctcagcc acccactggt tctgcagcct 86700
ctgcaagtga tttcgcatct cagagcccgc gcctcggatt ctgcatcttc caaaccaggg 86760
agccacacct cagtggcttc caaccaagaa tctctgccat tgtctttggc ccagcaaaga 86820
actcagaagt tctgctaatg tccagcagcc aactgctttg caatataact tcggtcagga 86880
cccaagaata ttttattttt aaagccattt ctgtctcgat ttttttgaag tcttaagaat 86940
gggtgccctg ggggacacct gggtggctca gtggttgggc atctgccttt ggttcagggc 87000
atgaccccaa ggtcctggga tcaagtcctg catggagctt gcttctccct ccgcctgtgt 87060
ctctgcctct ctctatgtgt ctcacgaata aatgaataaa atctttataa ataaagggca 87120
aggggaagaa tgggtgccct gggaggttga gaaggatctg cccaagggct ctgagcacct 87180
ttgcagctct cagatcctac acttcttggg tggagagagc tgtgtgcggg gtgagaagct 87240
ggctggcatg ggtcagcacc cctgcgctcc ctctgggagc aggacacacc cctgcgctca 87300
cagcagcttg ttcttggact ccagaccagc ctctaagtgt gaaatgctga tctgtgtcac 87360
actcccccgt actcccctgg gccctcccag ggaccccacc ctggtccact ctctaatgct 87420
tgaaaccagt acccaaggca ggctggcagc tgtcagggag cactttccat cctgggcgtc 87480
ccagatcggc aacaggcctt gggctcccag cttggcaccc gcaccccatc tgggcaacct 87540
ggggcggcct ctcaacccca ggagccctgc cttatccacg gggcctgcca ccccaactcc 87600
gaacaccttc tcctcctctg acctcggcag ctcctttctg caaccgcccc tctcccggcc 87660
cagggcagag actccaacgg tgagctcggg ttcctgaaaa gaggaagccc gggtggtacc 87720
ggtcgaggaa ctcttgacct ggccctgagc tgagacacca aagtggcccc aagcccccga 87780
ggacagcgca cgcgaggccc cgggggcagg gaagcccacc tgccgcaccc acctggggga 87840
cagcggctcc ccaagggctg ctgctccaca gggagctctt gaaccctcgc caaggcgctt 87900
ctgctaacca agcatattcc cccggagtct gcgagggcga ggggacgtca gagggccagc 87960
cgggcatccc ggcggagggg caagcgcagg atgggcacag caagccccgg gcgccccgcg 88020
acggccgccc tcggccaacc gggggagccc cggggccaca gagtgagagg aacacccagc 88080
tgatccccgc agggccagga acacgtctgg aacagcgacc gcgagggccc cccagaaatg 88140
ctctcccggg ccgtctcctt gttgcccgca ggctccagga agcagacggg cgcgcactta 88200
ccctgagcaa atctgaagaa ggctacgtcc ctgggcctct atagtcacct aggttcgcat 88260
ggctttcccc actcgcggcg gctgctttcc tgtctgcagg agaggaaacc ctggcgtcct 88320
cgccggagcc tggagcgcag gagaactgag tggcctttgg gggaagactg agctccgaac 88380
tggaccagat caaggatccc aggcagctcc tcctcctcct cctcctcctc ctcctctccc 88440
 
ctcccctcct cccgacccct cctctccccc tccccactgt gggcctgacc caactgtaaa 88500
tgaaaaccag actccctgtg ctctgttgct tctggccccg gctgcaggct gatgtcaccc 88560
accgaatact gtcttcaccc tacaagcctt tagccggact cctgcaaacc ccaactcttg 88620
aaaagaaatc cacctcttcc actcagtcgt caccttttgg gctgggcccc tggtggcaaa 88680
gggaatgaag tttagaggtc cccccccccc cgccccgcgg gtatccatgg ttttcctgca 88740
gtgttttcca acaccatctg cagggaaaac aggaaatgca gtctccccct ccacccttac 88800
cacgccctcc catttctcct tgaagtttct gaataatcga gactgctcct aataacctcc 88860
ctttcaaata gaaaaaaccg gcaattcaca tcgcctcggc cttccgccaa agcaaagctc 88920
tgtgcaccag tagtgaaagg acaaggatct gaagctactt ctttttgaaa agttgtctac 88980
acaaggattc tgcgcgcgaa gccggtggtc ccgttctagg acacggacga ggtttccttg 89040
ctcagagtta caagaaaatt cttgagaaac aaattgccag gctttgcacg tttctcggca 89100
ctttgactca cattatctca tttctcccaa acatctacca ctgcagcccc ttaaatagat 89160
atatttgtct ctctctcttt tttttgttag atgagattat ggtctcattt tacaggtgag 89220
aaaaaccagg gttcatagca gcacttggtc agaaccccag ccttggacac ccctgcacct 89280
cccctgctct ccggcacgga cccttggtga gattcacagg aaatatcaag atccctctga 89340
tggatacttt ggactttgag atctttctgg agccgagtgt ggacagtaga ggtatcatta 89400
agtcctttcc atagactatc ccattcaagt ttggggaaat cagagacaca gcacccggag 89460
accatgcctg ctcctggacg gggtggacag gtggtcggac aggagctgca cgtagaagcc 89520
cgagggcaca acatcctggc ttcgtgggtc taatacctgg acaaggatcc ctgggtggct 89580
cagtggctga gcgcctgcct tcagcccggg gtgtgatcct ggagacccgg gatccagtcc 89640
cgcatcgggt tccccgcacg gagcctgcgt ctccctctgc ctgtgtctct gcctctctct 89700
ctgtgtctct tgtgaataaa taaataaaat cttaaaaaaa aaaaaaaaaa aaaaaaaaca 89760
cctggacaga caaagagcta caggtgtgca ataggagctc tcctcttcaa cttccgccta 89820
ggaggtcccc tgggatcact atggtgcagt tgcatgggtt tgcccccctc accccccatg 89880
gagcaaggct gccaggacca cccagtataa acctagtagg gtccaaacct gcccttccaa 89940
ccaggtggga ggagtcctcc tgatgacggg caggagcccc cagcagagat cacactgtcc 90000
gtgacccaac agccccactt acataagaag aagagacttg gggcgtctgg gtggctcagt 90060
tggctgggcg tctgccttgg gctcaggtca tgatcttggg gtcctgggct ccagccctgc 90120
gtcgggctct ctgctgagtg ggaagtctgc ttctccctct ccctctgccc cctgccctgg 90180
ctcatgctct ctctgtatct ctctctctct cttaagaaaa agaaaggaaa ggaaaggaaa 90240
ggaaaggaaa ggaaaggaaa ggaaaggaaa ggaaaggaaa ggaaaggaaa ggaaaggaaa 90300
ggaaagggag gaaagaaaag aaaagaaaaa gaaagaaaag aaaagaaaaa aagaaaaaaa 90360
aagaaaagaa aagaaagaaa aaagaaggga aaggaaaaga aaagtaaaga aaagaaaaaa 90420
gaaagaaaag aaaagaaaag aaaagaaaag aaaagaaaag aaaagaaaag aaaagaaagg 90480
 
aaaaaagaaa aggaaagggr agggagaaaa gaaagaaaag aaaagaaaga aaaaagaaag 90540
gaaaggaaag gaaaagtaaa gaaaaaagaa aagaaaagaa aggaaaaaag gaaaggaagg 90600
aaaggaaagg gaagggagaa aagagaaaag aaagaaagga aaagaagaaa agaaaagaaa 90660
aaaagaaaag aaaagaaaaa aaagaagaaa aaagaaaaaa ggaaaggaaa ggaaaggaaa 90720
ggaaagaaag gaaaaagaaa aaagagaaga cccttgtagc ctcaggaccg ctgcccatct 90780
gggcctgccc tgtcccctcc ttgagaacac actgtccttc agaagcggct tagggcttgc 90840
tgccccccac ccccgggctg cctctatccc agcgcaggtg cccgagggga acctgagaac 90900
cgacccctct attcacccga ggcagcagca ttccgctccc gcagcgccgg ggcctcaccc 90960
cttctcgggg gccctagggt tcagctcgca gaatgtctgg ctggggcgac agggacagag 91020
gcccggattg tcccatttcc ctgcccacca cctgtagatg aagaggctct gagcggcaac 91080
cacaccgtcc atgcagcgcg tgggaaggcg acatcccacg aggggtcccc tctgcaccgg 91140
gcaggtgcag acttgggtcc cgggcggctg ggacacctgc tgcacctcac tggttggcgt 91200
ctgcaagctc atcactcggg agagccgtgc acctggggca cattggctgc tgccaggaag 91260
gcaggctctg gagacaactt cccgtcccag gcatccctgc tcccaaaggt tagtgagaca 91320
agtgtgtcct agttgctccc tttcggtggt tccttttcct gatcaagaat acaactagtt 91380
cttctcaaaa tagctaagaa tgtgcattct tcggtcttcg gaataagtgg cttttttatt 91440
tattttttat ttttattatt tttttttgag gaaatctgtc actttattta tttatttttt 91500
attggagttc aatttgccaa catatagcct aacaccaagt ggctttttta attgatttca 91560
tttaacaaag cacggttgac acacaatgtt ccacgacttt ccggtttgcc acatagcgat 91620
tggacaagtg tgtaccttaa gctgtgctca ccacaggagt agctgccgtc cttcacagac 91680
aaccccgttc aaagccgttg accctactcc ctatgccgta tcttttatcc ctgtgactca 91740
ccccataatg accatcattt aaaaggcaaa ggtgacacat catttagaac aagatttaaa 91800
gaagtaaaaa aaacaaaaat ttaaaaaccc tgttcaatca ggaaccgtcc tctataatat 91860
tgagctgtac ctgccattgg atctagaccc tttccaacac tgcttgtgac taaagaaatt 91920
tctagaagct ggtggctgtg ggtggcaaac ttccagcatc ctcccttgta ctcatcagag 91980
cattttggct cctatggttt tctcacttaa tcacttggat ttgagattcc cactgaaacg 92040
tctaagttcc tgtctgccac acggtcccag gaggaaacca cctcagtgtc agcacccacc 92100
tgaagactga ggatcctggg tctgggcccc ctgagggacc tcataagcca cagagtctct 92160
cagctcagct tccccttaaa tgttttcttg cacttggtct ggcgcctctt cccacatctt 92220
tccgcacaag ttagtggggt tcagggccgt ggctaggaat tcggtttccc tacttagttg 92280
cgctgatgtg aggcagctaa agcggaggca cccgctccca aggtgtctct gctggtgttt 92340
cttctgctgg ccagcagagc atgcaggtgc tggggaccct ctcctagacc ccttcagggg 92400
ctggggggca cctgccccat ccagtgtggg tgtcagctgc taatggctcc acctgcaccc 92460
aggttggaag cttgcccttg gccgctagga gctgtgtctt ctggagatgt cggagtatgt 92520
gcagtacgca gacttctaag ggaaacccca gcttcctggg tgccaagcag cagtgggggc 92580
 
gggggcggcc gggctgggcc ccgcagagtc ccatcgcagg cctgggagtt taggggaagt 92640
cgtgaaggcg gtagcagcag cctgacccca ggttccaggc agtagctagg gagtgtgtcc 92700
atgtccctcc ccaccccctg tcctcggctt accccggggg gtggctgtgc aggctctccg 92760
gcgcagactc ttggctcccc accttccaga acgggccaga agagaggcga ggaagtcctg 92820
ggagccgttc ctgcaggccc agccccaagc ccgctccact gctcccacct cataattccc 92880
cacaatggat ccctttctgc tcacaatagt tagagccgtt tctctttcct ctacagaccc 92940
ccgaccagca cagacctatt acatcagttc tgaactttct ttcaggtcta acagctgtgt 93000
ttatatatat catatgacag accaaaagaa ataatgctgg gccccaatgg gactgcagat 93060
gccattttgt atctaactag gccccagagc aattatgcgt aggacccctg tacctgggtg 93120
cctatcaggc ttccagcctc ttctccaaag attgtctctg ttactgctcc ttctgtacct 93180
ctcccttggc atatcttctg gcaaattcca tctgccattt gcagtctcca tcaaactagc 93240
aaacacataa aatcaaccca cacagacata gactattaca gtgaattgca gcatctcgtc 93300
tgcgctctaa aacaatatga aaaaaatacc cttaaataga attcttttta agatcttatt 93360
tatttattca tgagacacac agagacagag gcagagacac cggcagaggg agaagcaggc 93420
tcctgcgggg agtctgatgt ggaactcgat ccctcaagat catgccctga gccgaaggca 93480
gttgctcaac catgaagcca cccacgcgcc cctcaaattt taacttgttt ttaacgtgaa 93540
atgatttatc ctcaactaca gttttcattc aacaaagcaa gtccatgctg atggaaaatc 93600
aagtgtaaat aacacaactg acacaaccct gaagctgtta atggcacctc agttgagatg 93660
gagactcatt tagtttggga atcttaagaa atgttatggg tagacacatg ggcatcagat 93720
gaatctcaac cagctcaata aatccagagc taacctattt ccatgttaca taatcatccc 93780
agataggttc cctaccacca tcctccatcc cctttgccat aataaccacc tgataggact 93840
aacggagctg agggaaccca gaaccacgtc tatgtgacct ttatccccag ttgagagatg 93900
agctcatgat cccctggcaa gtcaaaacgt gcctcactgc cttccactct tgaaaaggcc 93960
ttttctggga cacagccgag acccccaaca tctcggtccc aaggacattg ctcaagacca 94020
aagggatctt ccccaaaaag catcagtcga tttggcaaat aacaagcaga catgaaggct 94080
acagcattat aggaagagaa gaatcccact tctgcttaat tggccctact cttaagatcg 94140
cgatattcct aaaataaaag ggcaacacac cagttgcaca tttacatcca caaagacgta 94200
aactggagag ccagatagtt gcatcatccc tgggtgaggg tggagaaggg tggcttctaa 94260
ggtcagggca cacagttgaa gcgcgtttcc tggccagtga agtgagcgct cctgagttgg 94320
cacagacggt cctgtgcatt gaatcccgag cttgccgttg cttggaaggc agggaggaag 94380
gaagaaatgg aaagttaacg gacatttctg ccttgaagat aaaaaccaaa ttcctagaag 94440
agactgagca cgggtggagg ggagagcctc aaggctttct gcttgcttgc agggttcgcc 94500
accttcagag ctcatcgcga gcatctgcat ctgggttcca tgcgcagatt ggcccgaggc 94560
ctggagccta cctccctgga caggggccac gtgagattcc agaggcagcc ccgtgtaaaa 94620
 
gcctccgaag acacaactct actatttcaa gagaagaagg atagtgagcc tgaccaaaga 94680
ggaggatttg aggtttccac tgaccccaca gccagagggc agctggcatc cctcattcca 94740
ccctggtgcc tgtttcaacc ctcctctggc agcatcgtgg gaatgaggcc ccagtgcctt 94800
ctctgccgct gagaactgcc gggcgcatgc aagtcagctc cgccttctgt ggctcagttt 94860
cctcgtccgc agacccagcg gtagctgcgc acagcaatgt cagggcaggc ccagcactga 94920
gtccccggga ccagggccac atcaaccgta cttgcctgtg cctgacttcc agcctcggct 94980
tctttccccg tccccagcta tcaacagctg actctccgga ttcagctcca cccaacccca 95040
agctcagagg caaggagggg aaatactcga tgggccagct accctctccc gtgttcccca 95100
ggcacctgct acaagcttat ggacgcaggc cttccactgc cccgggccac tgctgccata 95160
acgccatcta gcaatgtttg aggatctgaa gctaaatgac atttcatctc caatcactga 95220
agcacaccag gctatgaggc tacgaaggga gagactgtgg tcagcatcag gtgcttggtc 95280
ccctctcatc aggtgcttca agagggctgt catgttcctg cagaaccttc tggcaccacc 95340
tctgcgggct gacccaggga tgggcatatg acacaggtct tccaaaccga gctcttctcc 95400
agggtttcca gcttggacct ggagccagcc agtctcaggc catctctgat atgatcccag 95460
aagcttccac atttgcagcc atttggagga agccagcccc tgagaacaaa ccagagaaga 95520
gagaaaggga gagtgaggac catcagcttc ccggctccag atgtttgaaa ccaagtctac 95580
cccacacttt ggtcccaccc catccatctc aactttccct ttgattatgt gagccaaaaa 95640
ttattcattt tttacactaa tttgagtcag gtttccaaca cttggaacca aaggagtctt 95700
gcccaatgca cgaataaaaa ctgctcacag ggcaacttta tttttttttt ttaaagattt 95760
catttattta ttcatgagag acacagagag aggggggcag agacacaggc agagggagaa 95820
gcaggctccc tgtggggagc ccaatgtggg acttgatccc aggaccccgg gatggcgccc 95880
cgagccgaag gcagacgctc aaccaatgag ccacccaggg gcccctcaca gggcaacttt 95940
ctaatgtgag tcctttgcta gcggatcagt cctgcataat tactgctgga attcgtgatt 96000
acttctgcta tagacaaaat gtaccccgtc ctgattttcc atgatttttc ttaaaatcat 96060
ttttctaatc aaacagagag gaagcgaatt ctttgtcact gattggcaca tggacccaga 96120
agtctaaaat agaaattagg cattcactaa agcatccact gggcgagaca tgagggaggg 96180
ctttgagtcc taagcaagtc tttgactttc agtgctcggg aaataccttg taacaatata 96240
tacattcttg aaaaggaatg ccttagagga ggaattttat atttatcttg accgagtaac 96300
ttcgggggaa catgttagcc ccctctgtct gggggaatga gaagatctca ttgagcacaa 96360
gtaacatgcc agcattgagc tggctctagg aaaatttgtg taaaattgcc cggtgaaaaa 96420
tatagcattc ttcccagaat tttgtgagct ataaaccatg ctcagccgat tagttttaag 96480
gatgtcaaac ccaaatgtga gacaagccaa gaaccttttg cagttgattg gaattcaaaa 96540
atcttagcaa ggagatgttt ggttttataa ttatcagact acaccaaaat tccaattttg 96600
actcattttt ttaaacgttg aaatataaat ttccatttat aaagaatggg tggactggtt 96660
ctttcttttt cttttctttt tttttaattt tttttttttt gtttgtttgt tttagcgatc 96720
 
ttaacctttg gattgatttt ctcttgggcg taaagtcaaa tgtctactca tttcagcgtc 96780
aatgtctgct cagttccttc atttattcca catgtgtgct taggggctct tgtgtgaaaa 96840
cgctgtgcca ggaggtgggg gtgatgccca gatgcatcag acatagagac ctcgctctag 96900
gaccttctta caccccaggt gagatgcagg ccaaaggagg agggaggtct gggggaagca 96960
cagagcagat gcttggggac cacaagaggg caccctcact cccatgggga gaatgcaggg 97020
gtttccggaa gcctgacctt gggctgaggg acttgatcaa tggactttta atgcacggcg 97080
cggtcctcag gaacaagagg ttccagttat ttctcaagag tgaactgagg atgaaaacac 97140
aaggattagt tgggagtctg gatgggaagg acgcaggcac cgagacccct gccctgcgcg 97200
gatcacccct tcctctcctc agaggaacca ggaggttcct ttcaagagca atgaaaccaa 97260
agaaggtcca gaccctaggg acaggtggca cagagtagtg caaggtgaga agctacatcg 97320
accacagagg tgatcagtga gggccactga gtaccgtctc acagtcctta cgtgtccctt 97380
gccagcccct tgccccttgg cctccacagc aagcagagtc attctcctgt actctctgaa 97440
actgtgtcct ggaacttctg tcacaaggag ccacagacag gaggctctga acaccagaat 97500
tttagtgtct ctcagtgtta gaggctaagg tgtcagcatg gccatcctcc ctctcaagtt 97560
cccagggaag aatccttcct ttctccttcc agctccggag acagcgggtg ctccttggtt 97620
tgtggcagca tggccccagg ctctgtctct gttgtcacat tgcctctccc tggacatgtc 97680
tgtctcctga ggacaccagt tatgttggat cagggtccca cccaatccag agtgccctcc 97740
cactagccaa tcacacctgc gacaacacta cttgcaaaga tcacattctg aggtcctagg 97800
ggcttggact tgaacatgtc ctttttggaa gatacatttc aacctataac actgtcccag 97860
cagaaatcag aaggttactc catagagaaa ctcactgagc taaaagaaag catattcaga 97920
gactaatata ttgagtccca cagaaaaaca ggcaggtcct catgctgccg tgaagctcat 97980
tacccgacaa gctccacctg tgaacacaga gcttccaaac agattttcat ggtccattct 98040
taaatatgga caaccagtca agccctctag gaaatctcat aaataagaaa cagacgctta 98100
aacgacaaca gctgtcaatt aattggttag ttaattgatt aaggaactta gaagcaacag 98160
agacaatgca ggaagcagag aaaaacttcc agggggtaat aatgaacatt catagaaggc 98220
aaattaaaaa tatggcaacc atgaaacaag aaagggatgt tattttttaa atgagaatat 98280
ttactgaata agaaatctcc tagaagttaa aaacaccata agaaatctca cttacatgtg 98340
gattctaaaa aagccaagct cacaaaagca gagaatggtg gtagccaggg gctgagggga 98400
tggaggacct ggagagaatt ggtgaaaggg cacagacaca gttttaagat aagttccggg 98460
atctgtggtg ccacatggtg atgatagtta ataacaccct gtatatttgg aattttctgg 98520
agagttggtc ttaggtattg tcaccttaca cacacacaca cacacacaca cacacacaca 98580
ctggttacta tgtatggtga tcaatgtgtt aatgagcctg attgtggtca ttttaaatgt 98640
acacatatat caaaacatca cattgtgcac caggaatatt acaatattat ttgcaaaaaa 98700
aatctggggg aaaatgtcat agcaagactg aaaaaaatca gtatcaatat aagattaact 98760
 
aaaggacctc cctcagagaa tataactaaa actccaagac agagaaaaca aaacaaggaa 98820
gacaagaaaa ttataggccc acccaagaag tccaacatct gaaaaacaga atcacagaat 98880
gaaagaacag aaagccatag ggaggaaatt acctgataag taatgttttt taaaatccca 98940
gaattggagg gtatgagttt ccagcacaaa catcaaaatg gtgaatgaag aaggaccctt 99000
gtcaaggcac attgctgtga catttcacaa acattgacaa taaagaagaa agatcctaaa 99060
acctcagcaa gaggtaaaat gtgaaatgaa aaatgggagt tttatacaaa gcattaagga 99120
tcgaaaaacc tgtcagacat tctcagaagc aatgctagaa acaaggcaac agtggggcaa 99180
tgccttcaaa agcctgcggg aagtaaacat ctccatccta gaatgccacc cctgaccaac 99240
tattacccaa acataagggt agaatagaga cacctttggg ccagatctca aataattatt 99300
tcccagttcc ttttctcagg agtctgctaa aggctgtgct ccaccatgtg gagaaagaaa 99360
accaaaaaag aggaaaacat gagctctgcg agatggggat ctaacagaag atgcagggga 99420
aggatatttc caggtaaatg atgaaggaca acagcttata cagcaagcct ggaaatcagc 99480
atgctgggac aagacggaga ggctggaggg aggccccggg aacatagaaa gcacatggat 99540
tagtgtaggg cttgttgatg attaaagaag agtggggagg aagcatgggt cagcccctag 99600
acaaccaaac caaagaagaa gccattagta gctctcatca tcatgtaagc agaattgttg 99660
agccaaaaca tgtgacatga ttatcagcag agagggagga actgtgcagg gtaagagagc 99720
aaagcactca tgtccataga aagagaggtc agatctttgt agtgaaaatg ctaaggaccg 99780
aatgggcatt tcacacagat ggacgaatgt agaacaagaa agaacaaaaa gagttaaaat 99840
ggtttcggga agggtgggac agagggttgc tgagctttag tttataaaag aaaaacatct 99900
ttaaccaaaa aagaaaaaga aaaaaagaaa tgaaagaaaa agaagaaaga aagaaagaaa 99960
gaaagaaaga aagaaagaaa gaaagaaaga agaaagaaag aaagaaagaa agaaagaaag 100020
aaagaagaaa gaaagaaaga aagaaagaaa gaaagaagaa agaaagaaag aaagaagaaa 100080
gaaagaaaga aagaaagaaa gaaagaaaga aagaaagaaa gaaagaaaga aagaaaaaga 100140
aaaaagaaag atcagaaaat caaataattc agcagctctg ggcttaatac ttagtttcca 100200
aattttctga ccataatgaa agactggaga agtgggaagg ggatctctaa caacagcatc 100260
tatctgacat gtcccaaagc atgacttttc aatttggaaa aatgggtctt ttgcctcaaa 100320
attaaaaact caaattcatg gacgcatgat tgtccagttt tcatcaatga aaaaagaaga 100380
agaagaagaa gaagaagaag aagaagaaga agaagaagaa gaaggaggag gaggaggagg 100440
aggaggagga ggaggaggag gaggaggaag aagaagaaga agaagaagaa gaagaagaag 100500
aagaagaaga agaagaagaa gaagaagaag cagcagcagc ggcagcagca tggacactct 100560
ttcttgaaat caacaatgcc ctgcaaagca gcagaagtcc tccttcgagg acttgcccca 100620
cagaaagcag aagcaaggag aatcttcatt taaaatgctt ctctctcatg tcttgctcta 100680
caacagagtc tctctcctga ttcaaacgtg ttgtgttttc tgaaacggat tccagtctta 100740
tcacaaatgg ataagcaaac ttcaccctcg tgtgaagcga aggcttgtgg cttgccaggg 100800
acactgcccg tggagcccgg gacacaccag acagcgatgg tgtttgcaag ccctgcctga 100860
 
ccccggcgta gatggagacg tctcagctgt gctgtgccat tctgccttcc agaagtgttt 100920
caatcattct tcgaagacaa agggcttatt taaggaagag ttccacttct aaatattgct 100980
aaaaatgtct ggtcttcatt aatgccaaag aactgccatt tctgcgttcc aagaaatgtt 101040
ccctgcttgg aagagaagtt caaaagacca ttttgagagc tgtgccccaa aggtaccttg 101100
ttttgtcaga aaaacgtgct tcagcttttg ttttcaaatt gttggacaca acacacgtaa 101160
aacagcaacc acagaacgtg acccatgata ctctcaacta catctatatt tgtcattatt 101220
tcactctttc aatgaactct tagtaagcag gacgcactgt attcgatgta aggatccagc 101280
taatgacacg cccatccccc catgcaaagt caacctgaaa tttccactat tctctgcttt 101340
tgccttgctt acaaggaaga gaggaaagca cactgggcgt ggtagagtgt attgttgtcc 101400
cctccccatc cccaatattc attctgttcc ccagaagagt aaacattcct tccaattgcc 101460
atcgatgtac atagctccta cttccacgca gagtataatt cccaccccac tgtcaggcat 101520
gacaagctag gcgtgactgg ctaaggtgga tcagaaatgc atcacaaatt tccacaacat 101580
gaatgttctc ttccgctgca aaaccagtgt gtccaaaata ggagctgctt caccagggga 101640
ggcttctcca gactgcagga tgtgtggcaa tagagtctct agagccaacc catggcctcc 101700
tgtgtgcaac atgacaaaga agcaagaatt cattgttgga agccactgag atttggggag 101760
gtgtgtttcc atagcatcgc tggccagtag actaggttag cagtgattgc ccgtgctgcc 101820
tactgcaaag agggcggaag accactccct ggaataaaag ggttttacct gcccgtgccc 101880
tctcttctcc ttaaacatgt ccagctttgt gttcctccag accaaatgct acccttcata 101940
ttttatctga accacttccg gttgagctct ttgaaaattt ctattaaggg acgcctgggt 102000
ggctcagtgg atgaccatct gcctttggct cagggtgtga ccccgggatc ctgggatcaa 102060
gttctgcatg gggctcccct cagggagcct gcttttccct ctacctgtgt ctctgcctct 102120
ctctgtgtgt ctcaaataaa taaataaata aataaataaa taaataaata aaatcttttt 102180
ttaaaaaaaa gaaaatttcc attaaaagca cttacttact gaaaaatgat tctatctcac 102240
atgagattag cagccactct atgtcatagt aactttccac aaaatccaaa tatccctata 102300
cagctgagcc cctcttgatt taattttcca ttaaaaaaaa atatggtgac caagaaaagt 102360
tgtaataaac aaattttctg tagttgttga tgaatttaag caagtttaat gtagaaacag 102420
aataaagcat ataaaaatca taaccatcct tgtggaaggg gaaggagtag agccccggga 102480
ctgtcttcag agacaagagg ccaaggtcat atctcagtgg tccgcactcc ccccaacaca 102540
ccttatgaat ctttgggatg agttgtaaaa gccctgaggg tgggcaggtc aagggatcac 102600
tctgtcacac cctgccactc ctcagacacg gacactgaac atgtccaagg gagttaggac 102660
acagcagttg caaactaact tctagctctt cagttttcag tgccagtccc cacacttgaa 102720
cttccagatc ctagcaaggt tgcaccaaca agaccctctc acacagccaa ccacttgggt 102780
cttggcaaca tctgcatttt agctgttaaa gttctgcaac tcaaaggcac agtggtccct 102840
cctggccatt ctagggggtt tggcgcacct ctgtggcttg agcccgttcc tcacctcctc 102900
 
ccaaagcaac tgtctctccc agtgccagaa gcttcccctg ggccccagca tggctgggag 102960
tctcctgtaa agatgcagtc ctgcctattc tgctggagaa gatgaggaag gatgagtttc 103020
aggtcttagt cctggtttga tggttggttt tgattttggt actgttcacc atccagctgt 103080
gccaactcct cgtctgccat cagggccccc cccctgagtt tccatcctgt tctcagctct 103140
ctgagtctct gtggggaggc ctgggctctg gaaggtggga cccaaggtca ggaggcctca 103200
tctgccataa gctctgacac tctgcaggga cgtacgccac ctccctgagg tgatgatgag 103260
caccactgaa tggaggccca atgccaaggg cgacacatgc ttgcctcacc taacctcaca 103320
acaactccat ggggtacccc ctggtaccac tggaatctac agatgaggaa accaaggcaa 103380
ggagcagtta agacatttgc tcaagctcac ctgtctaaca aatggcaggg cagggatgtg 103440
aacccagaca gttgagtgct gaacgtcctc ccaaagcgga gagataaaga aagacagctg 103500
ggtccaaagt tcagtcccac aggtgcctgg gatggggatg ggcagacggt gccgcaacca 103560
cagcccctca ttttgctttc ctcctccatt cataacttta tcaactcctt acttctccct 103620
cctcttaaat accacgatgt gtccccaaag gcccccatgg gagagggtct agtacagact 103680
cttcatgcta tagatgagga aaccaaggca ggaaaagacc atgactcctg aggtacaagg 103740
agagctagca gcagagccag ctctggaact cagtctcctg gttccccagc cctgcaactc 103800
acagtctgtc ttgatgacct tctcattttc ctgtggttct ccctgggggc tgctccatcc 103860
tctcaccctt aaccacaatc ttccctgaaa agaacatcaa gtttttctcc actctgtcta 103920
tttccagtct caaaatgtag gcagcttttc actatttcag tctttgttgg aagatacact 103980
ataatgagat tttcttccca cataacaaat caaccttggt ttttccttca tgtcacccac 104040
atcgcctaaa tggctctgag tcacatgtct gcaagctgca tggggaaaga aagaagtttc 104100
ctttatcctt ccttgctaac tcaccagccc tggcttcata ttcaagatta gaacatttga 104160
aatttcaata cagattttaa gaaacttaaa acttgggttc acaagtctcc aagataatga 104220
acatgtatgt tacccaagtt ggatgtacaa aagctaaagg cagaataggt gcagggttgg 104280
gggcaaaatt ctctcccact ggccccaaaa taagcatgag tttgtcccaa acttggtaag 104340
aacttttagg aaagactgaa ctgccccatt ggaccctggg ggtggtcagt gtccttaatc 104400
cacccaacca aagcaagctt agcccatggc ctcactggtg tccatcacct cctgcccttc 104460
ccgcacattc gtctgtctcc attgcacctg gctttcttaa cacttctcca gcaccagtct 104520
gagcttgtcc tttgggcata agaaagccag gatatcagaa aactggatct tcttaaagaa 104580
aggaaaagca ccagagaagt aatacatgaa ggtaaaacca aatcttctat tgttcttact 104640
cttaatggat ataatagata attcctcaaa ataaaaatag caacagtgta tcagacagtt 104700
atcacatgtg gatatgaaat aaatggcagc aaggctgtaa ggtgcaagag agaggaattg 104760
gaaatattct aagacacctg caccgtctgt gacacagtat agtgtcattt gaaactgagc 104820
ccagattatt tgtaaagttg tatggcaaac tcttgaacaa ccatcacagt ttttaaaaga 104880
tgtataagtg atatcttgag gaaagagaaa attaagttct atcaaatgct caattaaaac 104940
cagacaaagc ataaaagtag ggagaaaaga acaaagaaca agtgtattag gtagaaaagt 105000
 
tacaaatatg gtagatatta atccatctat atcaaataat tatttaaaat aggagtggcc 105060
tacatgggcc aagggaagat agcaatcatc agagtgaatt taaaaataac ccaactaggg 105120
gcacctggat ggcacagtca tttaaacttc agactcttgg tttcagctca ggtcatgatc 105180
tcaaggtcat gggatccaga cccatgtcaa ggtcagcaca gagtccgctt gtccctctct 105240
cccttcccct ctgcccctcc tctcactcac atacacctgt gcctctctct ctctctctct 105300
ctctcaaata aataaataaa atctcttaaa aaatataacc caactctatg ttttctataa 105360
gaaccccaat ttaaatatta agacttgtat aaactgacag taaaagaaca gaaaaagata 105420
tctcatgcta atatttacca aaagaaagtt agaatacctg tcttaatttc agacaaaaca 105480
ggctttggga caaagaaaat tatcagagtt aaagagaaac atcacataag aaagaagtca 105540
gttctccaag aagagataac aacccctcag cacgtataca cctgacaaca gtgcatcaaa 105600
atacattagg caaaaactga tagaaatgca aggagaaaaa ccaaatcccc tattatgact 105660
gaagactaaa gctcctttct cagtaattga taaatcaatt aaacaaataa tcaataaaga 105720
tataagtaac agataatcag taaagttaca gatgaacatc acaatcaatt aacttggtct 105780
aattgatatt ttaaaaatac tccatcaggg atgcctgggt ggctcagtgg ttgagcttct 105840
gcctttggct cagggcctga tcccaggatc caggatcgag tcccatatcg ggctccttgt 105900
gaggagcctg cttctccctc tgcctctgtc tctgcttctg tctctgtgtg tgtgtgtgtg 105960
tgtgtgtgtg tgtgtgtgtc tcatgagtaa ataaataaat cttaaaaaat aagtaaaaat 106020
attccatcaa ataacagcag aataaacatt tttctcaaac tcgcatggaa tatattcgca 106080
aagatagacc atactttggt ccacaaacat accttagcaa atttaaaaga acagaaatcc 106140
ataggctaga gaagaaaatc atgtaatcat atcaattgat ccagacaaaa cattcaacaa 106200
aatccaacac cctctcatga taaaactctc aacaagctat aaataaatgg aaactacaga 106260
aaatctactg ctaacatcat aattagcagt gagaaactag acactttgcc cctaagacca 106320
caaacaagac aaggaagtcc tctctcatca ttcctattca acatcacact gaaagtcata 106380
gctagtccaa taagataaga aaaggaaata aaagatatac tgactgagat ggaaaaaaat 106440
agaactgtct ttgttcacaa atgacatgat tatctaagta ggaaatccca aacaaccaaa 106500
gatagcatca aaaaaaataa aatatttaga aataaatgaa aggaatgcaa aacttctact 106560
ctgaaaacag caaagcatta ttgaaataaa tcaaagaaaa attaaataaa tggaaagata 106620
cccggtgttt gtggaatgaa agacttaaca ttgttgaagt ggctctactc cccaaactga 106680
tctacagatt cggtgcaatc cctatcagaa tcctagccaa attctttgta gaaattcaca 106740
agctgattct aaaattcata gagttttgca aaggatgcaa aacagccaga acaatcttga 106800
aacagtagaa cagacccaga ggactaactc ttcccaattt tgaaactaac tacaaaacaa 106860
cagtaatcaa acggtacggg ccttgcacaa ggagagacat gcaggttaat agaacagaat 106920
gcagtacaga aataaaccca tacatttatg ttcaattgat tctcaacaaa gataccaaag 106980
caattccaag gaaaagagtc atcttttcaa tcactggtgt taggaaaccg gatctccata 107040
 
tcaaaaacaa ttaaatttga cccctacctc acatcacagt acatcatgta caaaaattag 107100
ctcaaaatga atcaaagagc taaatgtaaa agttaaaact ataaaactct tagaaaaaag 107160
ccagaggtaa gtcttcacaa ccttgattag gcaatgtttt gttttgtttt gttttgtttt 107220
gttttgtttt tttcagatac aactctaaaa gaacaacaaa ataaattgaa cttcatcaaa 107280
tttaaaagct gctgtgcttt gaggggcacc tgactggttt agtcagcaga gcacatgact 107340
cttgatcttg gggtcatgag tttgagcccc atgctgggca tagagattac ttaaaaataa 107400
aatctaaaaa aaaaaaaaag tgatatgctt tgaaagacac tttcatgtgg aaaaacatcc 107460
cacagaatgg gagaaaatat ttgcaaatca tatgtctgat tagggatttg catctagaac 107520
acgtaatgct tcaataataa caagaaaact caatttaaaa aaaaaaaaaa acgggcaaag 107580
catctgaatg gtcatgtttc caaagaccat acacagatgg ttaataagta catgaaaaga 107640
accagcgtca ctggccatca gggaaatgcg aattaaagcc acagtgagat gccacctcac 107700
aaccaccagg acgggttaga caatcaccag tgagaatgtg gagaagatga aatgctcaca 107760
cgctcactac cggtgggaat gtgaaatcgc cactttgccg ggtgcctggg aacacagtct 107820
ggcagctcct caaaaggtta aacatagagt caccctatga gccagcaatt ccactcttag 107880
cttcacatcc aagagaaatg aaaacacatc cacacaaaaa cctgcacaaa atgttcatag 107940
tagtattatt cataagatcc caaaagtaaa aacactcccg ttgatgagga gatcatgaaa 108000
tgtggtctct ccacgccctg gaatattatt caacattgaa gaggattgat acaagccacc 108060
gcgtggatga agtttgaaaa cgtgacgcta agtgaaagaa gccgatcaca gaagaccatg 108120
tattgtatga ttccgttggc atgtatagtc tgaataggcg aatcgacaga aacaaaaaga 108180
tgagggcttg cttagacctg ggatggggtg gcaggtgggg ggaggaggag gagaagagaa 108240
tagaagagga tgggatggga caagatagag ttgggtcaaa tagcatggca cggcatggaa 108300
tggaatggca taggatctca tggcatggga tgggatggga tgggatggga tgggatggga 108360
tgggatggga tgggatggga tgggatgaaa tggaatggag tggagtggag tggaatggag 108420
tcgaatggaa tggagtggga tgggatggaa ggggatggaa tggaatggca tggcatgaca 108480
tggcatgcca tgggatggaa tggagtggag tggaatggaa tggagtggaa tggaatggag 108540
tcgaatggag tcaaatggag tcgaatggag tggagtggga tgggatggaa ggggatggaa 108600
tggaacggga tgggacgcaa tgcaacagag taatagagac cctgtattaa ccaagcaacc 108660
gagaagtgtg gggcgcagcg aggataagaa gactgagcgg aggacctggg tcatagacac 108720
agctctccac tccctgaccg tgggactccg ggccacttcc tgacctgagc tccagttttc 108780
tctgttggta aaatgagaac actttgccca tattcctggt ctcgtaaggt tgtcgcggag 108840
attggttgag aggaggaagt gaaagagttc cgaaggctgc aggatggtct ggcgggggtg 108900
acaggtggag agcccggacc tgtcacagcc tctcctcctg ttctctgccc agaacacctt 108960
cagctatccc ctcctctgta acagccaacc ccgtgccccg tgtgtgtgtg tgtggagggg 109020
gggggttgtg ctcaaggttt catgttgtca cttctgtgcc ctggacgttg gcccctgccc 109080
actgctgacg ggctggggag gcggggggag ggacgagccc cggagcaggc agtgccaggt 109140
 
cccccttgca gggggcgtgg atggaggtgg gaggtgaccc ggaggaaggt gagcgcccca 109200
ggcctcggag acattggcag gcgggtgggg acgctcccgg aagatgacag ggcagtgagt 109260
aagggtggaa gcgggacaga gagccagctc agcccccacc cggccaccac agcctcagac 109320
cgggaggggc cgggcaccga ggagcagtca cagtgactgg cacgggcacg cccgccccgg 109380
ctgcccccct ggctgccccc cgccccggcc tccgaggaag ccgttagcag ggcggccgct 109440
gattcacggc ttacaggcag cagtgaagtc ctttttccac tggttgcaac agcctaggtg 109500
ctgtcaacag tgactgtgac tgtggcagtg ggggacccgc accctcccct cgcctgaccc 109560
ctccagcccc aagcaggctc ctcagcacgc cgctgcgggc ccgaaaaacc cccggtgacg 109620
ctggtgacga ctcccaggct ccccggaagg cggcaggtgg gctgcaagcc tgtgtcctgg 109680
acagacgaag aggcagaaac agaggtgtgg gtgtccccag cacgatcccc cgggtgcccg 109740
gaaactggtg acaacaggac gcagacatca ttgctaagcc cattcattca tttattcatt 109800
cattcattca tttattcatt cttcatacaa gtaagccatc tctgaggacc cgccaggagc 109860
tacagaagca aaacacagac aaatgtacat ggttctcagc acaaaggaag gctttgtctg 109920
tattctcagg gcaccctcat gtcaccttgt aattcccaag ggaagagtcc cctccaggta 109980
ggtctggcga gaagcctggc tcagacctat caccatcaag gtgttggagc ccaggaaaaa 110040
tgtcagaagc tttcccacct cacgaattcc ttagacccac agggatgggg tacatttctg 110100
cgtagtgttt agtgataagg actccaaaag ccacaccatc gggctcctca cccagcccct 110160
tggagcctgg gggagcccat cacccaacag ctgccaagtg cagctgataa aggctgtcgc 110220
ctgcagcttt ctccgcaggc ttatctttga ccaatgggag ccaccccctt ggaaatgctg 110280
ggagcttgtg cccccctcca gaaggcagac cgagagccag agagcagggg tgcaaagctc 110340
tccagggcac attccacatg tgctccagag cacatcatcc gacgcacttc cagggagcca 110400
gacccaaaag cgccgtgagc taggatgtct gcacaaaccc cagctgcacc gactcacccg 110460
ggccgggtcc tcaacctctc cagactcagc ttcctcctct gtgaagtagg ggtaatggga 110520
acccttgcca tacagtgctc gtgcagatgg tgtgtgtgcg ttaacacacg tgaagaggct 110580
ggagcaaacc ccagcctgta gaaagctcct gactgtcatt tgttcgccct gagacgggta 110640
ggtggccttt gccacgccat gtgtcaggca cccaataccc atcccttcct cttcctataa 110700
accagcaccc tcaaaccagc cacctgccta tttttcatgt taggcgtctt cagtagcatt 110760
tgctcagcca gcacgtaggt tgaaggttta tttccaggga ggaagtggga agagcggact 110820
acaagtatag gaatacacag agcaggggcg cctgggggct caggcactag cgtgcctgac 110880
tctcaacttc ggctcaggtc gcgatcgcag agcaggggcc tgggatggag ccctgcatcg 110940
ccaggctcca cgctcagctg ggagtctgct ggagattctc tgtctctctc cctctgcttc 111000
tctctctcta aaataaatct ttttgaaaaa atgttttaag gggatccctg ggtggcgcag 111060
cggtttagca cctgcctgca gcccagggca tgatcctgga gtcccgggat cgagtaccgc 111120
gtcgggctcc ctgcatggag cctgcttctc cctctgcctg tgtctctgcc tctgtgtgtg 111180
 
tgtgtgtgtg tgtgtgtgtg tgtctcatga ataaataaat atttaaaaac atttaaaaat 111240
attttaaaat atttaaaaga atgttttaaa ataaacacgc acagccttcg caggctgctc 111300
tgagcagggc agcgattgta aacagggctg tgggtcagag tcccccgggg gctttcatta 111360
aaatgtcagc tcccctctga tgccacctgt caccettgaa tgtctaattc aaccacttac 111420
cgagaacccc ccgcccccca ccaagcaccc aggaagacgg gggtcgtaag gccttcctgg 111480
aggagaagcc tcctgaactg ggtctgcagg gacctgtcac agttgtccga tgggtagcag 111540
ctgcgaaggg gacgcttcaa gcggggggac atgagagcag agggatgggc cctggggggc 111600
cgtgcgaccg gaacgtggtg ggggtggcag gtctggtggc aggcgggagc tggctgtggt 111660
ggctcacgat gcccacgacg gtcatgcttc ccctccagcc cctctcagag gctcccacag 111720
tgccccccca cacactccct gcttcctgtc tccccatggc atgccacggg gtaacccacg 111780
atgccccaca gtgggaccag acctcaagag gccactagaa cttagcaatg actctgaaga 111840
ccttggatca tcttctagaa tcttcactcc ccaccatcct tcatcccatt atgaccctca 111900
ggggcctcct ttgctggatg agagggggct ggacaaaggt gggggcttct tccccctgag 111960
atccccacac tcccatcccc ggggaggggg gtgtcccccc agcaggctgc acaggggtgc 112020
ggttcttcct cctgtagacc cgcagatgga tgatgggagc ccagggcctt atccagccac 112080
tgcagctgct gagatcattt acaacaggga accttgagga aagggggtcc acgcaggtac 112140
acaggacgat acagcattca ctctgcgttg ttcacctgga atttaccatt agtcagcaca 112200
ggccaggagc agatgtcaca acacagcaaa cagacacagc cccagccatg gcagaaattg 112260
aaatatgata aaacacagga aaagaccaaa aaaaaaaaaa gtgagtctat aattgggact 112320
ttgtgcgtgt gtgtgtgtgt gtgatacagc tgcgcttcaa gttggtaatg tctaagatca 112380
ttggaattga tgctgtgggc acatcatggt gttatttaga tagaaacact gcagcagcct 112440
tagcggagac agaggtgtgg aatgcgcact gcacaaggtc tgcagcaaag cttaacatcg 112500
tgttactctg ttgcctctga gttatggcaa gaggcaatga tacatatggg ttttttttta 112560
agatattatt tatttattta tttatttatt tatttattta tttatttatt tatgagagtg 112620
cacacacatg cacaggccgg ggctgggggg aggcagaggg agagggagaa gcagactccc 112680
cgccgagcag ggagcccgac accgagctcc accccaggac cccgggatcg tgacctgagc 112740
cgaaagggag acgcttcacc gactgagccg cgcaggtgcc tggacacacc tgagttttta 112800
ggaatgactt tcactatcaa aggaggttac aaaaccaccg taagctacgc gcactcccgg 112860
ttcttcgggc ccctgagagc gcaggatggc aggggcgccc gggtgatgca gcctgagccc 112920
ggccatctcc ctggtggccg gagagcacct cctggggccc cgacctcccg gccccggaag 112980
tgcctccttc accccgcatc cagagacttg gggcgcagct acagaaaggc atccggagac 113040
catgttctac agacgggtgc ttcaggggtt gtctcggaac ccacgtggtc agttctttct 113100
ataattttta agaaatctta cttctcttga tctcgatctt ataacccctc ctcccagcag 113160
atctcttctt ttctctcatt ccaattacct ttcataaagg gttacgtgtg ggcgcctggg 113220
cggctcagct ggttacgcga cccactcctg gtttcaggtt tttaggatgg agcctcattg 113280
 
ctcagcaggg agtctgcttg atattctctc tctccctctg cccctcaccc cgctcaccct 113340
ctcgtgctct ttctctcaaa aaattaaatt aaatgaaatt aacaaataaa gggttttggg 113400
tgtatggggt caggaatctc aatcgcttta ggaaaactca gtatgtaaat tctaaaatat 113460
tatgtatgtc atatataata acatataagt atgataacat aaatataaca tgtataaata 113520
tataacatat tatgcattta ttatattata tgacattatg ttatactaac caatttttca 113580
tttgattaaa ttggatccct gggtggtgca gcggtttggc gcctgccttt ggcccagggc 113640
gcgatcctgg agacccagga tcgaatccca cgtcgggctc ccggtgcatg gagactgctt 113700
ctccctctgc ctgtgtctct gcgcctctct ctctctctct gtgactatca taaataaata 113760
aaaattaaaa aaaaattcat ttgattaaat ataatacgca ggttctcaca accagttgta 113820
tacaatcatc cctgggacgt atcctctcag gatacgtgct gatgaaggtg tccgtagctt 113880
tctggaaccc tggaggattt caggcggagg cccagctgct cagggttggc cacgtccttc 113940
cttccccgtg ggttcggtct gacatggcac aagggttgag gaggtccact tctactcttc 114000
tcacccctac gggagtcctt acatctgagt ccaggctccc agactggctc cagttacacc 114060
agttataccc accagggaga cagacagaca catagacatg catacctacg gacacacaca 114120
cagacctgca tgtcaggtat gcctgtctat gcctcccaca gacagctttg gggctccggg 114180
atgctggcct ccctccctcg ggctccaccc ccattcccag tgtctacagc tccccgaagt 114240
taattcgagg tgggaaccac tcagggtaac acccgcggcc ctgcagcgat gtgagctcac 114300
ggtggccaga atcttccagc atttcgagaa aagctgcaaa acctcatttt ttgtgggaca 114360
tcgaattttc aaatatagga gtaaatattc cttggaacga aatgggtcct gcaggcgtcc 114420
gggcttcacg tctgcttcgc agatgatcca gctcacacag cagagcgcct cctcaccagc 114480
gagctggccc cgcaccaccg agggctcccc atctttcagg cccctcccgg ctgcactccc 114540
cccgccccca aaagcctgcc tgaaatgttc taactccctc ttgatgtaat ttattagagc 114600
attagctgaa ttataaatgt gtctattcaa acaggggcct ggccagtgaa attttaacca 114660
actaccagat ttccaactgt tttccattga cccacatatt aaaatagtag aagccgttgg 114720
tctcatgagg tttctggaca accctggatc cagcaaaaat gacgctccag ttgtggaatt 114780
tcactaggca acaggcccca aacagaaaat gtcagcactt ctgaggaata gtgcagcgtt 114840
tctggtacac tctctcccac cccgtgtctt tatccagttt ctgttctttt atttttacca 114900
ctccttttaa gggatcgtgt gtgtttttat tactataagc agtacttacc tccctaaaat 114960
gaccctttct gactcatcct gcatcaccta aaattatcat gctgtcccct tctgtggaat 115020
caggctctac tctgcggaaa tgcccaccct ggaagggcaa tggacttgtg ctttcctcac 115080
gggggtctgg gttttaagga gtacagacaa ctagtcaacc cagaggaagg ccagctcagg 115140
ggagagcaag gccagtgtca aggtccagcc aagggtcctc gagcctggag gaagactcag 115200
gcaccagcat catccaacgg gagccatcaa ggtacatcca ggagaagcca gatttccaac 115260
cgttttacat tgacccacct attaaaatag ttgaagccaa catgaagttg gtctcataag 115320
 
gtttctggac aacctcgggt ccagcaggaa tgaccctttc tgtgccacac agaatacgag 115380
catctgctaa atgaatagaa attgagagag acccccttca agcagcttat tatccttcca 115440
ttcggtgtca cagagcataa tagctccctg gaggcccaag aagtggctgt ttcccctggt 115500
cttctccgac aagtctctgc agagaactca ctctacgggg atttaggttg gaaaggggac 115560
atgtcagggg tttagggagt atagtgggtc cagtggtggc ccccccaatg atatgcccac 115620
atcctaagcc ccagaaaccc agaacgtgac tttatttggg aaaacgtctt tgcacatata 115680
attaatttca aggttttgag atggaaacac cttggattac cggtgggccc tgggcaatga 115740
acagccctta taaaggattg aaggctgaag agccaggaga ggaggacttg gctgagaaga 115800
agggactgat gtgccacaaa ccaagaaaca cctggaacca gagccccaga agctgcaaga 115860
ggcaggggag gaccctccta agagcctctg gggggccacg gccctgctga catcttgatt 115920
tcagacatcc aacctccaga acggtgagaa agtaaacttc tgtcatttct agaccatcaa 115980
gtttgtggcg atggttacag cggcccggga aaatgcaagg gggagctgcc ctcagaaggg 116040
ggctcacggg cccgatgccc agccaggacg aggatgcgag gtcacttggg gtgtggacgg 116100
tcaggtgtga ggatcgacag caagggtccc taacaaacaa gctgcccacc tccaggtcac 116160
tctgcgcagg accttccgtc ggcatcccaa ggctccactc cacatacgtg cctgacacag 116220
cgatccaaga gcacagaaag ccaacacgtg tagggaaaag ggaaaggaga caagagggtg 116280
atggagactc tgcataaaat ctcagagaac aagataaaat aacagattct taaaacaact 116340
aaactactcc aaaacattaa aacaacaata gagtaatcat gatcactgga gagttaaata 116400
tatgaggatg agaaccaaaa aaccccccaa aagtgataga aacaaagttg aggggcacct 116460
gggttggctc agtgggttga gcgtctgcct ccggctcagg acatgcccct agggtcctgg 116520
gatcgagtcc cacatcgggc tacctgctca gcggggagtc tgcttctccc tctgcccctc 116580
cccctgcatg tgctctctct ctctcaaata aataaataaa atcctatttt attattaaaa 116640
aaaaaaagaa acaaaattga ggaaattacc aagacggtgg aatagaacta aaaggacttt 116700
gcaaccaagg caccaagatg agaccatgaa caggtcagtc aaccccagaa gtcttgacac 116760
agagaaggga aattgtgaaa gaagtggctc ggggagacgc cagaactgag ggcctgcagt 116820
cgacagaccg gggcctgggg ggagccccgg cccttggggc ccccccgaag cccgcgcggt 116880
gagcagatgg tcctgctcgg tccctctgcc cctccctgcg cccgctcggc tccgccggag 116940
gcctcagcac tctcatttgg ggactacaca gtaataacat ttacatgctt tcccgttgag 117000
taaacgatcc cgaaatcgaa tctaaaagtt aaacaggccc gcgcccgcac cacgtagtct 117060
ccctccacgc ttgtccttct agaagccaac agtcccaggg caagggcacg cacgccccgg 117120
acgcggggtc cacaccccac ctgctgcccc cgcatcccag ggacgccccg cttcccgccc 117180
gttccctccg gcgccctggc ctccctcccc aggcctcctc gaggacgcag gccggcctcc 117240
ttctccagcc tcccctactc aggggtttgt ggtccagtgg cttcctgcgg aaaggggcgg 117300
caggtaagct cccgaggcct gagcgggacg cagggcacct gtcactccct catcccctag 117360
cagctgctga gcgtgtgtgc aacacggaca gacagccgct ctccctccca cctggaggcg 117420
 
ctatccctgg gactcccggg ggccccccac cccacgggac tgctgctggg acctgctgcc 117480
ggtctggcgc ttgagttctt tgcagggcat cttccccccc tttctctgct tggcgctgcc 117540
tctcctctcc ctctgccctg cctgctctcc cctgctggcc tctctggccg ggaggacaaa 117600
tggaaacagg aggagaggag gcccgggagc cccagctgca gcagggggtc ccccggtggc 117660
ctgtgctgtg ctgctcacgg cagctctgga ccaagacccc cttgcccccc agcccccygg 117720
cctgctcacg gggatgggcc aggacctgtg cacctgcagc tcccccaccc tccgcgcagg 117780
aaccttctca aaggcaccag ctcaccagcc cagccaggaa gccctgcrgg gtgggggggc 117840
caccagggag cccacgggga gcaggaaggc tgtccccact gtcagttccc caggccactg 117900
ctagtcacac cggcgagctc ctacacggac cggaccgtgg gcctcttctc cctttgtctc 117960
tgtttctttg tcttctgaac tgttttccag aggattcctt ctccctctca acctctcctt 118020
ctgactccac ggcacttgct ggctgtggct gctctgctct cataggtctg tgttcatgtt 118080
tcatggatgc atcacgtttt ctctaattca ccgaagacgc taacgaagct gggtttgggt 118140
tagagttttt tatgtctttt tacttttttc ttctgatctt gatgggttgt tggttttttt 118200
gttttttttt tctttttttt tttttttaat ttttattgtg tcggttggat catctgcaaa 118260
gcagagttta ggaatgaatg acacgggttg gcagcaaagc ctatgatggt taaaatccaa 118320
gcaggcgagc atttccagct ggcctccaga taccaagcaa gctcaggctg cctcctcctt 118380
ctgcaggtcg tatcacacca cccgcagggc cagagatgcc agctggagga ggggcgcccc 118440
tcccactgtg gcaggtgacg gagagggggc ctggtgcccc ctggtcctgc ccagctccat 118500
cccagaagca ccatttccac agtagaatgg actcccccac cccaccaccc caggcacacc 118560
caacttcgtg tcatggtggg tgatagaaga aggacgaacc ctcttaaaga tcacgttctt 118620
aaaatcttca aaggcagcgc agacggtgcc aacccaccca gcttccggaa ggtgacattg 118680
tttttgattc actaccttgg cctaaggggg gtgaggggac tttccagcac ttccacttgg 118740
tcttcccgtt atggttgctc tttctctgca ttccaagggt ccagctcagg attgacccct 118800
ctgtacgggt ccccctgcgg tcgtgaatgc agcacagtaa taaccaacca tttctgctgt 118860
cctcatatta ttgcctccct atttctagca gactgactga tcacacttgt ccaggcgtcc 118920
ccgtcgctgg gacaatctct caagtcacca ccattaaaac tgttggcatc tgggcccccc 118980
gtcttgtgcc aggccccatc cacagcccac ataccactag ggatgggagc ccccttgcca 119040
accaggctgc aagccatctc tcccctaaac ctaattcttg ggtcctctgc aaagcagaga 119100
cgtgcaagag atttattggg cgccaacacc tgtgaaaaat aaaagggggg aggaagccag 119160
aagtgtgaag agccctcaga tggtgatgca ggtctagctc ctgtggaagg agaggggaaa 119220
aaggaccagg aggggacgcc ccccaccctc caccccatgg gccatgcagc cctgagcaca 119280
tctcagccgg gcccttggga gtctggggca caggtcaccc tcttagagga ctcccacgca 119340
ggctggaacc agtgaacccc gtacccccga tggtgcccat ccctgggctg acctcggctg 119400
gggtgtttct gaagggagat cagagggcac ccctcccacc cctctctggg cgctctttcc 119460
 
ttctgcattt gggtgtgagt gtgagggagt gtgagagtga gagtgcccta cttcgctcct 119520
ctccagcagg ttgccagctc agctcgcctt ccatggccgg cagggccggc cggcaagagc 119580
ggactgccgg ggcctggccc tggggtgccc agcacagccg tgccaacgtc acccctccca 119640
aaataactcg ttaggtgtct tcagagcaga tgctcggtcg gccggtgggc gtgtgggctg 119700
agtttgcagg ggaggtggag gggcctggga ggggagcgag gcttacactg cagatattct 119760
gggagtcagg gggtcagggg ggttcatctc cacttagccc cccagttcag atccgcagcc 119820
cccccacagg gccgccctga gccccgggca tcccctcggt actgcccccg tgcgcgtgtc 119880
cgtgtgagga ccctccagcc gctctccaaa ggcagagaaa tctcgcctcc tcggaggcac 119940
cccctcccat tctctttctt ttcctgagtt gataaggtca cctccaatcc cacttcggaa 120000
ataatcaact cacaaaattt gtatttctcg gactgcagag gcgaagctta caaatcttgc 120060
tctctggccg cgtcccctcc cccccccccc gactctgctg ttggctgcac cccgagcact 120120
gccccacagc tcatcccccc ctgctgactc tgaaacggga tcggggcctc gtgtaccctg 120180
ttccacatgc aaaaaaatcc tccacctgcc aagacgttag cagaagcagc ggagatgtcc 120240
actgctggat gtctcgggat tgcccgggag aaagcagtca cgactccccc aaacgcccct 120300
aaaataatcg tcagcctggc tcccagtgcg ctgccgtgct agggggctcc tgccccacat 120360
cctgtccatg cagccccctc agagcgcctc cctccttcct ctgcacgccc ccaccttggg 120420
gcctcctcca cggggccaca gcgccccaca attcactaaa gcccactcac tgatgaagtg 120480
tcgactaaag ccagcatcgt cccacttgtc ccagagacgc agcgtggctg gggggtgggg 120540
gggccttggg gatgccagag ttggcccact tggacccctg aggtctccag ccggagacgg 120600
ccagtgaggc ccaaagtcac cagtgtccag cggggaggac tgtcccagcg gaagaggaga 120660
cagtgtccat ggaggtctca atatccaccc ggactcaggg cctggcaaaa cagaccagag 120720
ggctggggac actggctggc atctccgtcc tacacgtgtg tcccacctcc ggccccacgg 120780
tccggacaga gataagggtc acacacgtga gaagttaaat gcgttagttt tcatgccagc 120840
actggaagaa gctgcatcct gggccttgtc taaatcccca gatttgcatg taattatatg 120900
cacaattatg cagcatgcca ttctttccgc cccacgaaat gtggcttaag tgaacattcg 120960
agcggcgcca tccgtcccat actgagcaca tgcaagatga aggtgactca caaacccttt 121020
cggcttgtgt ctggactgcc ccagcgagga gaggattgct cagctccagg agacacagac 121080
ccctgcaggg ggcggctcgg cgcccccctg ggacggcatg ttcctgcaca gcgcactctc 121140
cggggtgggg aggctcggta tggggctctg gtacaacgag caacagcccc aagcaccacc 121200
cacagtcact cagcaggaac gagagctgtg ggtgccccat cggggtcctc tcccgggacc 121260
gtgaacagct gacaggtgtg tgagggccca cgacctggct gggcctgggg ggccccagat 121320
gcggcacagt gggaactaag cagggtcgcc cctgggtccc ccaccctcct gctggccctg 121380
acgcctcccc caccccagct tcctgtgccc caggcctctg gctgactctg accctgcctc 121440
gcacctggac cgttgccacc cctaccctgg cattgcgaga tgggggagcc cgacatagcc 121500
aagggcttgg gaccacgtgg gagcacgatg tcttccttgg gtaaccaaca gctgggaaga 121560
 
gctcaggggg caggatgggt ggatccttag tggggctcct gacacctgcc ctcaccaggg 121620
cctcagcgat gctgtgggcc tgaccaccgt gtagagtacg gttctttgga aaaaatgcat 121680
tttttccacg tattgaacaa cacccagtcc tgattctcca actaagattt ccacagatat 121740
tcctctgggc ttgattattg cagtgagtcg gagcagctga gtcggagcaa aggtattcag 121800
gggcatgggt agggagcctg atgagcccac ggagggaaca cctggctgag gcaaactatg 121860
aatcacaatg agacgagctt caccaggaca gtgttcccgg ctgtgggagg tggggggcag 121920
aggaagaagg agagaaagag atggagagag gaggaaggaa agagggaagg aaggaaggat 121980
gggaggaggg aaggaaagaa gggagggagg gagggaagga ggaagtcact tctgctaggt 122040
gtggatttga cagtaagacg ctcaggaagc agccctgagc agtcgtaggt gagggcagga 122100
gcctgatatc ctccaaccag gggtcatttt catgtctctg cccttaagtc tcacaacacc 122160
ctgtttaaat cttcatactt tactctaaca cgctttgctg gtccagtcct cccgagaagc 122220
tgacacccag acgagattag ccatccagga gggtgcttgg gggaagccga gcacgacaag 122280
gacagggtac agggggaggt gggtgagtct gcagacaggg aggctgccca actccacacg 122340
aggagaaggg aggaagagct tcagactcca gtgtgccaag agcatcttag ccagcttggg 122400
ggcatgtcct tgtgccaaag tgtcccttgg agggggttgc acagctctca gaaactagcc 122460
tgtgacaggg ccccacgcaa gtgcccagag cagccatgga cgtgtgatct tttttgtttg 122520
tttgtttgtt tgtttgtttc ttagatttat tttttacact tttttaattt aaattcaatt 122580
tgccagcata ttgtataata gccagtgttc ctcccatgaa gtgccctcct caatgcctgt 122640
cacccagtca ccccatatcc cagcccacct cccattctgt aaccctttgt gtttcccaga 122700
gttaggggtc tcttatggtt tgtctccctc tctaattttt cccactcagt tcccttcctt 122760
tcccttataa tccctatcac tatgtcttat attccatgta tgagtgagac tatatgatga 122820
ttgtccttct ccgatggacc tatccatcag agatggatgc atgatcttac tgagtgagaa 122880
gccccatagc agggagcctg atcccaattg ttcacctgaa aacattacca tccgaaagaa 122940
ttcttttctt tttttaatga ttttatttat tgattgatga gagacacaca gagagaggca 123000
gagggagaag caggctcctt gcaggaagcc tgaagcgaaa ctcgatcctg ggaccccggg 123060
atcacaccct gagccagacc cagatgttca accactgagc cacccaggtg tccccaaaag 123120
gatccttttc atgtaatttg cttgcctgtc ccttggtgtt cccctccata caagtacagg 123180
ctccccaggc tccccaaggc tgggaccttt ctttacctat tgcatgaacc cctggcccct 123240
gacccagggc cagacaccga gtaggtattc aaatgtttgt ggagcaggag aatgcatgga 123300
ggcagatcgt ggcttgacac attattatat tgcaacagag attttgaaac cagggcaatt 123360
taagtcgcca aatcagaata agaaatattc agaatttttt ttcttgcttg ctatctcaaa 123420
aaaaaaaaaa tcacttaatt gatagtaaga aacaaaagtg tcgcaggagc tcatgaagag 123480
tgtagccatc gtgatccctg gtgctgggga cgctctcaat gtgagccttc tcagcagaga 123540
aggtttgtga tacacaaaac tacgcgtggt attattttcc tacgtctgtt aggctttcca 123600
 
aaggaaaaca ttccttaatt gaggaggggg gaaaaaggat tggatagcga gccccgcagc 123660
ctgatttttg gcttaaggat acagaagtca tgtagatggg agctcggtgt ccgtgcacat 123720
taatcgcctc cggtaccaag cagggctagg acataagcct acagctgttt caccgtataa 123780
tgttatataa acagagccct ctgcacacta gggcaaaaga taaacatttg ggacagaata 123840
attagaaaaa cctgaaacca ccataatcag aaacagcccc cgaaagtgcc taattttaag 123900
acagcattct gttacgggtt ttactaagag gttttctttt ttaacgaggg tgggggtggg 123960
taatccaaat gcgtgtgcta ctaagtctaa caatgttcaa agactttttc atgacactcg 124020
gtgttttaat cattcctgag ggacacggga gtccttgaca cacaaaggac acaggttggg 124080
gggggattcc agctcgcccc acaacgacaa caaggttatg cttggaagtg gctggctttg 124140
attacccgtg tggtgcttgc tttttttttt tttttttttt ttttttttta agattgtagg 124200
aaatctccac acccaccgtg gggctcgagc tcaccacccc gagttcgagt cgcagggtct 124260
tctgacggag ccagtgggcg cccctgcttg taaggctttt gatttcaaag cgggaggcag 124320
agcagacgtg gcctgactcg cctggacctg agcagccccc gaccagcatc tccgcccccc 124380
catccgctcc tctcctgcaa ggtcgacctt gtagacagat gtcgggggac cacgtgccgc 124440
cccctctgca gcagccctgg gtgacgggca gacaaagcgg atgctgggag acacacctgc 124500
tcacacacct gagcgtcccc ggtgaggaac aaccacgcgt gcccgggcag gtgggcccgt 124560
gctcctgtct gcccggcctc cggacctccg ccaacgacct cttagccatc atggcctgta 124620
gctcccaagg tttcccaact gggaaatgga agccagaaag gtcgggaggc agacctggaa 124680
tcagcaaccg ccaggcagtg cccaatccct gcccgggcct ggaggttcca cgggacttga 124740
atccgcacac cttgcttcag tctacactca ctctgcaact gggggtgcgg ggacattgtt 124800
tatgcgtcca tcagtgactc ttgctggagc tgctctaggc cggaaggaca caggggtgaa 124860
caaaccaggg atgaccccag caccccaccc cagcatcccc gtgggaggac agatggccca 124920
gacacaaagc acccagtcca cggagctcgg gctggcacgg gacgaggagg gggcctgacc 124980
cgtcctccac ctcacctcct cgagctgcac ctctctcctc ccccagagct cgccagccat 125040
ccgctctccc tctgtgtctg agcctggctg tcccctggcc acctgcccgg gcaggtgatg 125100
aagctgacgg ggagggacaa acgacaggga ggccgggcca ggtgcaggcc cagctggggc 125160
aaagaaggag aaggaggccg cccggccgtc ccgctgcctc tggaagctgc cgcactcacg 125220
ggatgcttct cggcccccga tctgagcaga cttttaccag gctggaagag aacacccaga 125280
agagaaggaa aataaaactc tcccttcaga atgccaagat ggggaaattt atctttgcac 125340
ttaaaaaaaa ttttttttgg aataacatga tatgtctgag gcccgtcttg gagtgggaag 125400
ttgtatttat ccaactgttc cttgttatct cccttcccac catgacaggg tgagggcgag 125460
tctggtctgt gggactctca gagtggtggg tgggacagca gtgcgaggtc aaggccccgt 125520
ccctaccctg caagacccct ccctacaagc ccgtctctct ccccctcttc ccctctcttc 125580
ctccctcctc caggagagca ttttgcatgc agccccatcc tcccatttcc actcagccaa 125640
gggacacgtc atggcccgac atacatgatc ctggaaaaag agcgtgcgtg cggggcctcc 125700
 
atggaatctc cacgtctcgc tctggtttcc caacatcccc atccagatct tataggatga 125760
cacttctgtc aaagatttaa aaaccattct caggaaaccc gagggttcct atttgaccca 125820
ggctgggcta acacatccca ccgggggcag agaatggggc tcaagatgct catgtgggga 125880
gaaggcagcc cggagggaaa ggcgtccagg gctcccggtg tctgcacagg gctgagccca 125940
gcacctccat ccacggcctc ttcctccata aacaacccga acgccagcct ggagcagtcg 126000
tcctcagggg cctccagcca gggacactgc cctgcctcct tcctgacgcc atgaaggccg 126060
tgtcatgtcc caggcatggt cccgtgctcg gcctgagctc gatcactcgt tcctcacgca 126120
tgtggaggac cttctccatg ttggctactc ttctgcgatg ccctggagac ggacaggcca 126180
ggaccctggg cctcgggggc ctcccctgca gaggaggagc cagccccacg tctgcatcag 126240
tacgtgaaca cctgtgatgt tcactggggt agtgaaacca gcctctgtgg ggcttgagag 126300
gagtcaggga cctgactgga gtcatgaccc ggaaagctcc agaaggttgg gccattactc 126360
cgagtgcaag gagatgactt gaggcccgag ggcagaggaa gcgcaaggtc gggctggagg 126420
gagggtggtg ggggatggaa accagccagg gcgaggacac ggtcagggag gcgagacggg 126480
ccggggccag gccaacaagg ggtcacacta cgccccgttc taatgctgcc aagtggcagt 126540
gaccaactgg gtcctggtct ccggtggaca aagtcgatct tttcaaatcc cagagtgaac 126600
acgctcaggc cacgttcagg ccacatgtag agtcaataca tttctgccca aaacattgct 126660
tctagcaggt gtcatggagt tggggaaaag catacccggc cacagcacag gcccgaggag 126720
gttgcagggc cctgcaccat cgtggccagc agctggccag gtcctcgccc ctgaggaggg 126780
atgatgacaa ggctgatgtc agaggcacaa tgagctgaaa tgcagaggga ctggccacct 126840
ccccaacgcc cagtcatgcc ggccccctcc taggccatgg tctgcttcac catgcacggc 126900
gagggcagca ggacagcacc ggcccagaac gtgcacacca gccaggcgcc cccccccccc 126960
cccacaacca cgttcacact gctctggggg caggtgggac cgacgcccat ctctctccct 127020
cctgggctct ctggggcact cttggctgca gccgagatcc cctggcatct ggcccgcact 127080
gtcctggggt ccctgagaca tccctggcca tgtggggtga gcactggtgc agggaggggg 127140
ctcggcaatc aggccaagac cccgggccgg ccgggcgtgc ccccctggtg gccgggaggg 127200
aggcccttgc ggctgaggct cccagaccag gcagcgccag cgaccgctcc cttcactgca 127260
gaaggttctc cgggcgggcg cggtgcccag cacggctctc gggggccatc actctgcgga 127320
cactctccgc aagagcgctc gccgtgttcc aagccggccc tccatccggc ccgtccttca 127380
tgcaccttgt gccccggtcc gccgcctgcc ttctcgtctc cttggcggat ccttctatag 127440
agcgtaagtt ttccttccca cggggtctaa ttcatcaatt ctttcctccc acggggcaca 127500
ctgtccaatg ccaaatctca gagctcttcc cctacccgtc ggtctctgtt tttttctacg 127560
agtctcatgg ttttctattt tacatctcag ggtgtgattc attttgggtt aaatgcctta 127620
taaggtatca ggacgggtca gggttcactt tctgtccaca gacatcggat cgctccagtg 127680
ccagtcgtgg ggaaggcggc cttcctccgt tggcttgctt ctgcggcgtc gtcaggagcc 127740
 
aggcgggcac gttggggctc atattggtga ttagctcgtg gggttggtga gttttacgtg 127800
tgggatatgt ggaatgctcg gctgagtgcg ggcgaatggg gtgggcacac cttagcagga 127860
ttacggagca aagcccatgg ccctcctagg cctcgtctca gcctccggca gcctgagagt 127920
gatttcttct tccagccgca ccacgtccct gcaggaacac acgcccccat aatgccaccg 127980
gggctccccc aaaactctgc ccatacagcc agcctcactc cttcatcaca caggacccgc 128040
tgttagtctg atgacagagg cacgtgggga ggatgaggag ggatgtccct gacttccttg 128100
tcctggggat caggctcggg cacccccccc atgagctctg ctgtcaagct ggagggatcc 128160
tgacccctct cttcagagcc cacattgctg ggtccccaaa gagtccctcc tagcatctgt 128220
ccctggagcg gccgcatctc cttcattttt aactgaagac ccccctcctg cagctgagac 128280
ccatttcctg ctcaggggcc ccccacgcaa atgaacaaca gctgggaaac atctctgccg 128340
gggcttcgag cgctgcacca cccccacccc ttcctggctc attaactgct gcatgggggc 128400
aggttccatc ctccccctgc agcctaggcc ccttggctgc tctgctgagc cctttcaaac 128460
tgcctcctct tgtggccaca aaggaaaacc agccttgggc agagagaggg aggtggccct 128520
gcagaggagt cacctcgatc cctgctcggt gagtgcccac cgcgtgccag gcaggttctc 128580
tgtgccccac gacctcggag atgtccacaa agtcatgcat ccttcctccc cagcaaggaa 128640
atttggcccc aaaggcagcc aggccagggg ggtggggaga ggagggacag tcatgctggc 128700
tggagggcga gggacatcct ggagcctgaa gctgcctagg ggagtgcgtt ccctgagaga 128760
aggaaggagg cacctggacc aggagagaaa ggggtgagac aggggctggg gagcgggtaa 128820
ggtgggcaca ttggccaggg agggactcct ccatgggttt ggaggagcca gcccccaccc 128880
caactttaca accttctgat gtgagccctg cttgtgtgtg gcgctagagt cctcacgcag 128940
acgggacagg cccggtgaga cctcgagggc tcaatccgtc ccactgcgct agagcgcggc 129000
cgccctcaaa gccaccacca ctgaaaacat ttcgtcatta gtgaaatggc aacagattac 129060
gtaacagaca gacatcacgt gcaccccaag gctggatgac caggcatcag gggcctttcc 129120
tccagcccct gagcccagac ttctgacacc ccgactgctc attcactgcc ttccccaaag 129180
caggtatttg gaggcaacgc atgagaactc cagattttac taagaagctg caaaagcgcc 129240
cccgagagaa gccacccatg aggtcaccag cgggagtcag ttcctcgagg gctgaggatg 129300
gatcacagag tccggcaaac ctgatcactc tgaggtgcca gaggggaggg gcagggaggg 129360
cacctcgagg tggggagagg gctctctctg gacacaagag cgtgcacagc ccccagcgtg 129420
caggacaaga gtgccggcgc cagcggtctg ccagcagccc tcaatcaatg acctgcttgc 129480
tgaagaagca gaagaaagtc ctcacctcct cgcctctaga gagggacaat tctggggaca 129540
tccagcatgg tcccacgggg ataatcaggg tgaccgcatg ccctgtctcc ctgcctcctg 129600
gaccacctcc cagaaacatg acttgcagcc acattcccat ctcggggtct gcttctgtgc 129660
aacctccaaa atgatgctct gtacgtactg acaagctgtt ttccataaag caaatataag 129720
taaattctag aaaataacta aattatatac caacaccctc caattcagat ggcaaagagt 129780
agtgcgctgc ttcccaccct gcgccctcgg agacagacat tgctaattga ttgaggtccc 129840
 
ttttggaggc tgtacaagcc cagcaagaca gcatcatgat cacttagtca tgtctgccac 129900
tggtaggatc agtggagaac gcagcaaggg ccttgctact cgccatccct gctccagccc 129960
attggtttcg gagcccataa agctactcaa caaatgtcac tgtcataatg tcaccagttc 130020
ccaaaatggc taattttcct tccgccgccc attgccagcc ctgctaagag tcacatgtgc 130080
acaagtatct gctggaaaat gccaagtgct cagctcgcct cgccaccgac aaaggaaaca 130140
gagatgccga atgcagaagg cagttagcac ccacctgggt gagtgaggca gctgaaaata 130200
ggcatgactc acacttggca gggagggcca aaccgcagtt ccagcaagcc ctcccccgcc 130260
ctaaatgcca ccaaaggtgc ccagggagca gaactgggcg tcctgtaggt agagctcggt 130320
gtgtgcaggc cttgcagaca cacagcctcc cagaccccgc tcccccgaga gtcacgcggc 130380
tcaaggaagg ccttagggct ccagtgctca tgttacacag agtacagcca gaaccatctg 130440
gccaggcccg gagcccactc ggagtccctg cataaacaca tcccgtcact taggaaaaca 130500
gaattacatt ttccatagtg aagaaataaa tctctagggg gtatttacga aaatgtatac 130560
tctgctaagg gacagtgtgt cttttgccaa ttaactagaa cagggtcttt gcacaacata 130620
acaaaacaca agagactgag gaaaggaaag tccagttagg tacacattca aaataaacaa 130680
tctttacagt tacagatggc ccctggtgtg tacacaggtc aagtccctgt gttaccaagt 130740
cacttgtttg gagcttgaca tgtactttcc catagaaact atgtcccttg gtcataaagc 130800
caacccacgg ccgtctcttc ctctgatagt gatggaagca tgttactgcc ttcacatctc 130860
cacttccctg cagcatcgcc tctgcgttgc ctctgtgaga agataaatcc tgtgtccaga 130920
gcggaattgg ggaaggcccg gtggagtgtg ggcaaaggca ctaagatttg gtggcctttg 130980
gcttctggtg tgaatgccag caccccttgt gctctggcaa gatttgtaac ctctctcagc 131040
cctgatcacc tcacctctga acagttccca ccttccattg ttgctattgg gacagggaag 131100
tggcagagag gatgtagagt acaagttacg gaacatggcc ccaaacaaga gcttgaggga 131160
gaagctgcgc tgtgcaaaag agaacctgac tatgaaatct gagcatttca gggtttgagg 131220
gaattcactg agtgctccca acaaagggca agaccatatc tgttttaccc taaaatgacc 131280
cccagcccag gcatggccca tggcaagctt taacaaggat tggatggatg gatggatgga 131340
tggatggatg gatggatgga tggatggatg aacagatgca tgaatggatg gatggatgga 131400
tggatggatg gatggatgga tggatggttg gatggacagg cagatgattg ggtggataga 131460
tggatggatg gatagatggg aaaaggtgga tggatggatt gatggatgga taaataggta 131520
ggtggacaag tggatgggtg ggattgatga gtagactggt gggtgggtgg atggacgaca 131580
aaaagatgga ccaatgagta aatgtataaa gatcaaaacc attctttctt tccccttacc 131640
attgccactg gcttgagtcc aaggccttcc tctcaggtcc ccagagagct ttgtcaccca 131700
ttctaccacg gggagacaca tgtatacaag atgcagtcta tgaagcagga agctctcatc 131760
aaacactaaa tctgtcagca cattggtatt tgactttcca gcctccagaa ctgtgagaaa 131820
tatactgtct gtcatttata agccacccag acttgggtat ttggttatag taactcaaac 131880
 
agactaaaac aggtgtggac actaggcgga gtggctcact ggggccacct tcaaagatta 131940
gcaaccacat ccattcaggc ctcagtcctc agcatgtcta gctagcatca tttgccatgt 132000
gtggtctcca aggtcagcat ggcagaggat aagaggaaga gagagagcta gagaaagtac 132060
agtgattttt aaatgtctct gttcatcggc cagaactagt cctatgaccc caacataggt 132120
cctgactgta aggaaagctc gcgaatagag aggactgggt gagtatttgg tcagccccag 132180
tggcccctgc cccatcatct gtttcctcta aggatggtcc ttatgctggt gagcacaagc 132240
aagactccca ttcactgtgg gtgggtgtgt aaaccgggac agcctctgct tgagggcagt 132300
gtgacctgcc cagcttcctg cttgctcctg gctgggtgtg gagaccacag catgcatggg 132360
cctcggggct ctaggactca gtgggcatga ctaagccaca gaagacaagg gctgaggaac 132420
atcaggacac cagcctgctg tcctctttcc agagcatctt gtggtgttgg ccagaataga 132480
gaggaactca ttcctctcaa gggtggaggc cactggtgtg accctgctgg ttcccaccta 132540
gttaaggaga acacacagca tcattgtggg aggcagaatt ggtcgcattg ggcaagttga 132600
tgtgtggagc cttctcacac actgtgccca attcagggtg ccatattagc acaaaggtgg 132660
agaaaccaga tagaaatcca agaacaacaa gaccacttga cctataagtg aaggtggaag 132720
ggactgggat taactcacca aatgaacagg aaatgactca gcatcaaatg cctgcgagca 132780
gcaagtcaag agtgtggaca tgggtgtcga atggacctgg cttggatcac tggctccatc 132840
gtcaaccagc tgtggggcat tagtcatgtt acttaacctc tctgaatgtc acgttcctca 132900
tctgtaaagt ggccaaagtc atagtagctc cctcacagag ttcttgtgac cattaaatcg 132960
aatcatgagt gctctgggct cctggtgaga gctcaggaaa tgtgggtggt gttcttgctg 133020
ttgatgttat tatcactatt tccagctatg ttggaatgac atgacagtct ctacaagcta 133080
ttagattaac tgagagggaa atcagtgata gtccaatagt gacctggaca aatccttagc 133140
gtggcattgc aaaccctcct agcccagccc ctgtcgacct ctccaccacg atcctacctc 133200
acaccacaca gcccaaccct gatgaaggac ttgcagtttg aactggtcag ggttcttctg 133260
gatgaacctg gatgaactta cgcaaactga agatgtactg aaatccaaac actagagaag 133320
caggactcgg gggcgcctgg atggctcaat tggacaggca tctgccttca gctcaagtca 133380
tgatctcagg gtctcgggat cgagccccat gttgggctcc ctgttcatcg aggagtctgc 133440
ttctccctct gcccctcatc catctagtgc tctctctctc tatcgctctc tctctccttc 133500
tcaaataaat taataatatc tttcaaaaaa aataaggaaa gaagcagaac tcaggaagtg 133560
caagcaccag gcagccctta gagaggaaag gacttcaaag gccatgccac atcctctggg 133620
tttcaaattc acatctaaga tgacctgggc ccacagagaa ccaatggcct ggcctgggat 133680
gggtacagtg taattcaggg aagcgctgct gaggagcaaa tgatgcagga tcagtctttc 133740
tggcaatctg ccttagcaca atccgatcag cattgcctgg aatgaagcac atgtgccacc 133800
gagtgcaaga catgatccgt gttttatttt gcgttaggaa agaccctgct gagttcacca 133860
aactgttggt tttatggaga ttactgccta ggaaacatat aaaagaggcc ttcactttaa 133920
aagagcccat ttgaaagttt caagcaaatg gaggtggcac ctggatatag agcaggtcac 133980
 
aaaggagctt cgagaaaaaa aaaaaaaaaa aaaaaaaaca aaggagcttc gaggattagc 134040
gcggtttaag gaacgtggcc tgaaagaggg agtcgtagag agctcattat aaataaaagc 134100
gttctgagtc accggagtgg ctcagtcagt tttcaccagc tgccttgggc tcaggtcatg 134160
atctcagggt cctgggatca agccccacgt tgggctcctc gctcagcagg gagtctgctt 134220
ctccctctcc tctgtcccta ccccactcat gcgctctttc tctctctctc tctccctctc 134280
tcaaataaac aaagcctttt taaaaaataa atagaagtgt tctaatgagt cctgtgtcct 134340
aaatgcaaag gttttcgggg atgagacagg aaacctgagt acagtgagtc aggtgtgacc 134400
cctgaggaag cagaggaggt ggtggcagac cagaggggac accatccatc cagccgccac 134460
aggggggacg tgcaggccct gtcctgtcag acctctgctt tcaaagagag gttggagaca 134520
ttttggattt ttacgaacaa tttttcagat ttttaaatgc aagtaactaa ctcaaaagct 134580
gcaaatcaat gggcgagcca aacctagcat gtcacggact gatgtgcacc cctaggcttg 134640
cagcctctga ccccactgac gtgcaacaga gacccgaacg cacagcacgg ggggcacacc 134700
actcctctct ctcctctcgg tgcagagaaa gacaaggctg cgtaaccacg ctcagggaga 134760
aatggtgaac ccagaaagct acctgcttct tctccctcaa agatgaaaat ggtgtttaaa 134820
gagggtctct ctctctctct ctctttttaa caatacatca ttacaaaaat gttcagacgt 134880
agagcaaagg tgaaaaaatc ttacagggaa tgtccatatc tctaccaccc acattctgct 134940
gttactgttc accgcaggag cttcatcacg cacggatcca tctgtccatc cctccagcca 135000
ccatctacga gactcaagat gagcttcctg gcaaattggc agggatgagg ttttggaagt 135060
tgtcctggtt cccttcacat tcatctcccc acttttcctt cttctcctct gtactgaaga 135120
gaagtagaga ccgcaggatc tgatgtcctc tagtttgtgg gtccaggtgg acactggaga 135180
ggcgcacagg caggagagaa attcgggcat ttctacttcg gtctctctct gtttgaagca 135240
acccccagct tgactacaag ggacctgcta gtcatccctc tggggtccca gctccgacct 135300
gacaggcctg caaccactgc agcatatacc aggtagcccg agaccctggg gctccagcaa 135360
cacctcccct cctccctttg tctctgtagg cctcaagggg aaagtgactg tctacccttg 135420
ctcatgtctg cctgccttat tctgaattcc acctgttatc tctttccact ttcctgcacg 135480
gtgtcctcta gctgaaataa ccagcatgga ttctgttgtc ttgattggaa cctggctgaa 135540
ataagacgtc agcaagtttg tgacctccgg tcttctgagg gcataggcca tgatgcgaga 135600
tgcttattgt ggacaatacc tgtaaaagaa ggaggggagg gagctgtaaa ggaggagaca 135660
aactctgacc cagcatgatg ggggcagtct gacaccagcc agcatcgggg aggacagcgt 135720
ggagagagcc tcagatgacg gctgtgctgg agcaggtgaa ccagccctga tggtcctaca 135780
tggggtggaa agggccaggc tcgaatctcc cactgtgccc agaacctgtc aggagctact 135840
gacaagagca gcctcgaccc atacatacct cagaatgggt cctaaagatg tcaaagccag 135900
agacttcccc caaaactcac tcctccagct ttccagcaag ttcttccttg aagggagagc 135960
caagtgatac acctccatat ctgcaacagc aagttactct ttagtaaaaa atgtctaaat 136020
 
tatatcttaa tttcaactcg aaggaaccct gcttgaattg agaagtaaac aagaatggag 136080
tctaatctca aaaaattgat gcatatatgc ccagcgccaa agtttctcct ccatggacac 136140
ctgtattatt tttttaaata tttgattttt aagtaatctc cacacccaac atggggctca 136200
gactcatgac cctgagatca agagttgcat gctctaccaa ctgagccagc cagacatccc 136260
ctccatggac acctttaaat aagtgattaa tcacatgggt cagagtggat gggatggtta 136320
gggcccagct ggaatgagtt aaatgcagag aattgttttt aagaaataaa tctcatccag 136380
ttgtcaactg gaacatggga aactatgctg taaaattgct gtctaggggc gcctgggtgg 136440
ctcaattggg taatcatcca actcttgatt ttggctcagg tcttgatctc agggttgtga 136500
gatcgagccc tgtgttgggc tccatgctgg gtgtggagcc tgcttaagat tctttctctc 136560
catctctccc tctgcctctg cccttccgac cctctaagaa ttaggggtgg tggaagggga 136620
ggagggtggg agggtggggg tgaatgggtg acgggccctg aggggggcac ttgacgggat 136680
gagcactggg tgttattctg tatgttggca aattgaacac caataaaaaa taaaattatt 136740
attaaaaaaa taaaaaataa aatagtacat taaaaatgaa aaaaaaataa gaaataaaat 136800
taaaataaaa taaaaagaca tttgtaacca gttctagcca ttaggcccaa aacctcctat 136860
tcacgtttag gaaacccctt tttccttaaa agattttatt tatttattca tgagaaacag 136920
agagagagag aggcagagac acaggcagag ggagcagcag gctctctgca aggagtccaa 136980
tgtgggactc gatcccggga ccccaggatc atgacctgag ccagaggcag acgctcaacc 137040
actgagccac ccaggtgccc taagaaaccc ttttttaatc agatataatt tacatctaat 137100
aaaatgcact catttaaatg tacatttcaa tgatttttga taaatgttta tacctatgta 137160
tatgaagtat acagcttgat gaattacaac aagcgcagcc ttgcagcctt gcagccacca 137220
tctcaatgac gatatagaac atctccattg cccccaaaag ggccccccgt tcagcaccag 137280
acagctacat actcaaacta aatttaattt ctctattcca gagtttccta taaacggaag 137340
cttactccct ccatactgca tgcttttgtg tctggcttcc ttcactcaat ataacggcta 137400
tgagattcac tcaagttgct gtgagtaccg tcagcttgtc actttttatc gaggatcagt 137460
tagtattcca tttatgaatc atcacattgt gtttacccag tcacctgttg acagatattt 137520
gcattattcc cagttgcggg caatgacact atgcactgtt gttaccagtt ttcatttctc 137580
ttcaactcat tcatttttaa aattttttac ttttgcgtaa gggtaattgt agactcacat 137640
gtagttgtaa gaaataatac aaaaagaccc aaatgtcctt taccccactt ccatcaaagg 137700
cagcatcttg cataaccata gaaccctatt agagccaaga aactgacctt gatacacttt 137760
atccacctta ttctgattta ccaattatgt acctactcat gtctgtgcat gtgcttgtgt 137820
ttacttctgt gcagttttat cacatgtata gattcatgtg accccccacc accaccacca 137880
ccaccgcaat ggagataggc cagctccaaa acaggatccc ttttatagcc acagcctctc 137940
cctattcccc tccagatcct ggacaatcac tcatctgtct tccacctcga taaatttgtc 138000
acttcaacaa cgctatttaa atggaatcat gcaatttgtc gtctatggag acatttttca 138060
tgcagcataa ttcccttgag acccatcccc acttgttaac atgtttcaac agtttgtacc 138120
 
ttttttccgc tgggaagtat ttcaagatac agatacatga tttggtttaa cctctcaccc 138180
cctgaaggac aagaagttgt tctcagttta ggactaatac aaataaagct tctatgaaca 138240
tttgtagaca gatttttgtg cgaacacaag ttttcaaatt tctggaagaa atgcccaacg 138300
gagcaattgt agggccattt agaaagggca cattgagcct tccaggaaat tgctatattc 138360
ttttccagag gagctgtacc atcttacact tctaccagaa agtgtaggtg actcatcctt 138420
gccagcattt ggtgttatca caattctttg ttttagccat tctcacaggc aatgatatct 138480
cattgtggtt ttcttttaca tttgccccat gactaagatg ccaagaacat tttcatgtgc 138540
tcatttactc tctttggtga aatgtctgtt tatatcttta gcccattttc taattggatt 138600
gtttggattt aaaaaaattt ttttttcact gttgagtttt taaaagttct ttatatactc 138660
tagatactaa tcctttgttg cgagtatgtt cccccagtgg attgcttttc ttttccttct 138720
cttttctttt tttgtaagat tctatttatt tattagtgag agacacacag agagagagag 138780
agaggcagag acacagacag agggagaagc agactcctca caaggagcct gatgtgagac 138840
tcgatcctgg gaccaggatc acaccctgag ccaaaggcag atactcaact gctgagccac 138900
ccaggtgtcc ctcttttcct tctcttaata gaatgtttca cagagtaaga gtttttagtt 138960
ttgatgcagt cccatttatc ccatttgtcc attttttctt ttgatgtcag gtcaaacaac 139020
taatgcttca cctcgctctg gtttaaactt ttttttcaca aaaatgtagt ggttttatat 139080
tttacattta agtctgtcat ccattttgag ttgagtattg tgtaatgtat gagatttagg 139140
ttaaggttca tctctttgcc tccggatgtc cacttgtttt cacaccattt gttgaagact 139200
atcctgcctt cgttgggctg ctctcacacc tttggcaaat atcaattggg catatttgtg 139260
tagctctatt tctgggttct ctattttgtt ctattgatct atgtgtctat ccctctgcca 139320
attccatcta gtctcgattg ctgtagttac attatgaccc taacatcata gcccattctt 139380
taaaaaaaaa acagtaatat gaactaaatt tctgttaatc ttctctcaca tgctctgaag 139440
gtccctctaa aagatgagac acaagtgaga gtcaagaaag tagcaaagat ggtcctgagc 139500
ccagccattg caacgggtcc aatccttgag cagaaaggct cacttcccac agggtggcag 139560
tggggtaggg ctttagtcac agggcaggaa gttttagccc tgccagtgga ctagaccaac 139620
cccaggacac aaggtacatg aggaaattac agcagagggg tgttcagtgc cacatttacg 139680
tgaacaagga gccattaatg gaggtaggtt actggtaacc ccaagactct gtgcctgagg 139740
catatttcct gaaaagagga gcctcaaaca acaacagaga gttgttggat aaaatcccag 139800
cttcctcacc caaggctagg ataactctaa ggcctgttct cccttacctc cctgaccccc 139860
agcaggcatg ggctcctctt gcccatagtg gtaaccaact tgaccataca ctgtcttatg 139920
tttcccactc ccctgcaggt gctttttagg gtggcctcca aaaataaatt atttgcatca 139980
aatgcactca gggtctgctt ctaggggaca caaaccatcc ctgggtggcg tagcggtttg 140040
gcgcctgcct ttggcccagg gcgcgatcct ggagacctgg gatcgaatcc catgtcgggc 140100
tcccggtgca tggagcctgc ttctccctct gcctatgtct ctgcctctct ctctctctct 140160
 
gtgactatca taaataaata aaaaataaaa aatatatatt taggggacac aaaccaagag 140220
ggggtcccag agttgacttt cagctggctg cccttggttg gtgccatatt gctttcactg 140280
tgtgtctaga ttcttacaaa ccaaccactc actaaaattt ccagttctcg tatgatgaaa 140340
ccgcctgtgt cagctgacaa ctgcagttta ggcatcttaa cctggagagc tggggaatgc 140400
tgtgtcttat tcttacctca tacatatgag tagtgctgtg aactatgccc cctgcaaaat 140460
tcatatgcta accccccaca ttgtgaccat attggatatt ggacttataa ggaggtctat 140520
attttttagt aaggtcatca gggtgggcct ctgatctaat aggatcgttg tccctataca 140580
aagaaaagag agaccacaga tctcagacac acacacacac acacacacac acacagtgag 140640
gtaaggccat gtgaggacac agcaagaaga gaaacatctg caagccagga aggaagtcct 140700
ctccaggaac caaactgatg gagaccttgg tcttgaactt ctgacctcca aaactatgag 140760
aaaataaaat tctgttgttt aagccacccg gcgtatggtg ttttgttata gcggcccaag 140820
cagactaata caggtagtaa atagacaggg aggggaggga cggggagggg ggagacggga 140880
ggggtggggg ggatggagac gggataagga gggagggagg agctcatcca gctgaagtct 140940
tcttaatgca aaacaacaga gacagtgatg gaactcttta gaaaggcgaa gcagggagtc 141000
agctttgatc aacactcata agaccaaagg agaagaacca aattaaacaa cacgttagaa 141060
tattagaaac gcttcgctta agatctaaaa ggagagacat gactatgtca ttgtgccatc 141120
ctttctaggt cctgtggaat ccactccact ccaccaggaa gcttccgatc accctagctg 141180
acagcgggag agggcggatg tgcccagacc tgagcagcac tggggacaga gctggcccag 141240
ctgcgccatc agggctctgc actcagtttt atgcagggac acaaggactc acctgacagt 141300
ctattgccac ctgacttggg gcgctctccc gggaggagtg cttcccaggc tggcacacat 141360
cattccagat tttacacagg ggatgttttg gcagagccac tgctgactct agcatccaga 141420
cgtctgaaat cattctgcct gtgaaatcaa gcatagacgt tataaggatt gcaaagacca 141480
tctagttgaa accagatacc taaagacgtg gcaagccgtt gagcaaaaga atataaacat 141540
taaagtccta aaccatgcca cttaggtctg ctcaggagaa atctgtgctg gtaggtgtct 141600
cggatgctaa tggagaccag atgggcagcc ttacagccac ccacgctcct cgcggtgggt 141660
ggggagggtt gcacagactt ctctgtgggc tccagctcag ggcagatggg tacattaagc 141720
agataggggt atgctgtggg gccggtagga agagctgggt gtgtgtggtc actcttcttg 141780
aggaggcatg atcctgagtc cccaacccaa ccccaatccc atccctgggg aactccaggc 141840
ctgcagagtc agcctcagta actggctctg gccagtggag ggcatcagag gacagcagga 141900
agcagcgggg tgtgcccagg tgccagctgg ccctagagcc tgtctttaaa ggactgagtg 141960
ggctcagaag gaagctgtcc ccggcgcctg gagacgccgc tggacagagg cgggcagagt 142020
cgtcgggctc ctgcagcagg tgccctttgg gacactggct cttccctcca ttccaggagg 142080
agccaccagt gggcacagac cgcagggtca gacctgcgtg ctgctctccc tccgcaagtc 142140
ctgaagggcg gtcgcctgct acatgccaag gccaagggag ccgccagcct gatgcacagg 142200
ggagccccgc tctgaaggct cagcactgca gggccagagt ctgagcatgt ggcagggaca 142260
 
agacgcaaag cagggctcag atggagcccc ggggggccag gccatggagc tgggggaccc 142320
gggggcctag tgaacgggga agggctaagg gggaggagat gtgcatacca gggggcgcct 142380
gggtctgcct tgggctcagg ccgtgatccc ggggtcccgg gatcgagtcc tacactgggc 142440
tccctgcatg gagcccgcct cccctgcctg tgtctctgcc tcactctgtg tctctcatga 142500
atcaataaat aacaccttta aaaaaaaaaa gtgtgtgtat cagaagctcc ctagcagaga 142560
ggtgaggtgt gtggctgccg ggctgagaaa ggcaggcagc tctgggaatc tctcggggtc 142620
catcccaggt ggggaagctg gggcccggat caggccgtgg tgggggggtt gggaggagga 142680
tctccaaggg gagcgtatta gagaggcagc catcgagttc tggtgcctcc cagggggccc 142740
cccagccggg gcctggccca atcaccacca acctggggct cgtcgacttc ccaaggacca 142800
cagcacgggt gcccgtggcc tggcggcctt tcaggagcag cctcacagcc aggaggcagg 142860
gaggccacac tcggctctga gacagagctg gttgggagcc cccgggccgc ctgcagcgtc 142920
cagcagcctt tgggaagtca cttatccgac atccccaccc cttgagcctc agttttgtga 142980
tccataaaat gggggtgttt gcagcagtgc ctaccccacg agcgtgctgt ggggactgaa 143040
tgagatccta gaagtgaccc tagacacagg gctgtgcatg gtttaaaggc ttgggggctg 143100
ggagccaccg agcatcacat ggctgtcact tgttgggcag caggagcacg tgcctatcag 143160
ctgggccctg ggcacggagg ctgcgcttta ccccctgcac ttcggggccc tggggacagc 143220
tcttgggttg cccctcgccc cctcccagcc taaggacata ttccttcctg aagaggctgc 143280
cgtgctctcg ggcttgccag cgaggcccag gaccctcctg atttattgcc cccacgagcc 143340
tgggccgagc gccaaccacc cacagatgcc attcgaggcc ccggcacgcg gctgggcagg 143400
aagccaggtg gggccgtgag acgcacgggg acgggaagcc tctgtccaga ggccgctcgg 143460
gtggccaggg cactcggggg accccccggg gctggggctg cgagggacac gtggggcgat 143520
cctgcgcagg acccccgtgc gcccctgcgg agctgctccc accccagggt cccggactct 143580
gacccccgca gcccacatct tctgaggccc cgcctgccgc ctggctcagg gtgccggggc 143640
ccgggctctc ttccagcctc cttctagaaa ccacccctgt gccccttaag gcccaaggtg 143700
gcaatggctt ccccagcctc ccctgctggc tcccctaact gccctcccct ccgccggggg 143760
agcctgggcg acaacccctc cggctgcttc ctgcgtagac cctggccggc agggagcagg 143820
tgcccaggga ggcccgtggg gcgcccagcc cggagccccg agaggccagg gtaagggggt 143880
gtgggctctg ctctcgggcg aggagccctc aggttactaa gtcctgctgg gcgacagcag 143940
acagcaaaac ccagggcgtg aggacagagg tcccagagct gccccggctc agactcgccg 144000
tggatcgtga ggctgaggga cacccacccc gcagggaagc ccctcagggg tagctgctgg 144060
gtgggaacgg gcagggagaa gggaccctcc gcagcttcgc tacaggtccc tcactcgacc 144120
gctggagccc aggacgcatg ttcccacagc cacggcggcc tggccccgcg ccagaaacca 144180
cacaacccat gacacgacag agaggccagc agagacagcc agtttcccat tgttccctgg 144240
gaaatggcag gcccggctcc cacccctttg cgtcctgaga attagaaata atgcctggcc 144300
 
ccttctccca gccagccccc cggggccacc cactcgccgt cctcctctgc cctacagctg 144360
cggccagatg ggctcccctc ggggctctgc gccacggggg gaggcagggg ccgggcttct 144420
gctggaggct caggccagaa agggtgtcgc tggaggttcc tagcagtagc tgctaactga 144480
gggtgggaaa agggggcagc ggtgggtcgg cctctgcagg tctggggctg tgatggtagc 144540
tcgacccagt acccgggatg gggggaaatc ccagaactcc catccccctg ctcccacccc 144600
ctcccctgaa agtgcaagga ttccctaagc tgcaccctgg ggtcagatag tcggtgttgc 144660
aaccctataa cagccctgct gggaggcgac tccccaaggg gcccagcacc aggaggcttg 144720
caccacccac aaaccagaca ggggcaccrg gagatgagat ttctttttaa ttggatattg 144780
aaaacaaaaa ataacaacaa caaaaaagct ttcaagtaac tgtcgtggat acaaaactct 144840
gggctgcggg gccctgtgct aaatttccct gacttagggg cgcccggtgg ggctcaggtg 144900
cggttaagct tcggactcct ggtttcagct caggtcatga tctcaaggtc ctgggatcga 144960
gccccacatc gggctccctg ctcagctcag agtctgcttc tccctcgatc tctgcctcca 145020
ccccgtgctc tcgcgctctc actctctcaa ataaataaaa tctttaaatt aaaataaatt 145080
cccataactt atctacacct cagtctggtt ttacattgaa tcacacagtg ggaagggagt 145140
aagcgcagga tcacctaata tcgttctggg ctcacaggtc ccagttgcca ctgatgcacc 145200
aataaagcac gatttgtcac gagggtctcc tggtaaaagg gcgtgaggct gggggctccg 145260
cgtcccgagg agtcagacgg ggggtccgca gggaaggact agagagcaga gggcgagcat 145320
tcgagggctt ctctgctccg gccgggctcc tctggggtgg gggccacccc gggccacagt 145380
cagccctacc caaggggcag atgtgtgagg gtggggcacc tccagcagga accgcatctc 145440
tgcctttaat taagatgcct tttaattatg cgtttgagtt aaagtggctg gaagcttgtt 145500
ggctgcaaaa ggaaaccctt tgaggcccca gccaggcccc agttgggcga ttgggtgagg 145560
ggaggctgag ggggaggtgg gtgtgaggag atgcagtggc ccacattgca cggaaatctc 145620
attggctcgg cacggggccc tcatcagagc tgcaggggac ccccatccac gttccacctt 145680
tgctgaaggc tagcccgtgc cctctgagct cagaagccag ggagcagaaa tgtgtcccag 145740
ggccccaatg tggggatgcc gaggccgcaa ggggcaggag tgtgggctcc cgtggcagct 145800
ggccttggcg ggttgactgt cctggccctg cctgcccctt tagggcagga ggtcggccac 145860
taagtgccct ggggtccact ggccataggc ctctctcctt ccctctcagt acagaggagt 145920
cacagcagga aatggcccgg cggcaggctt gggactgctc cactgttgtc cctaaggctc 145980
tgccttgcgg ggctccttcc cacgctgcca tccaggcagg acgtctggag ggagcagccg 146040
ggagcccggg accattgggg ccagccctgg gggatgcctc caagcttcat ccgttgctgt 146100
gtctccagcc cttggcaaag gtagacacat ggtccgaagg caggacgagg gcagaaagga 146160
gagagaggga gtgaggacgt ctggatgtga tgagcaccgt cctgagggtt tttacacaaa 146220
cccttaaaat ccttgaaaga caaagataaa atagtagccg ctaatgtttg tggarcctga 146280
tgccaggagg gtggggtgta tgcactcctc ctccccctcc acatagctgg catctcagac 146340
aagaaccccc ctcctccatt cggggctctc tgaaatgggg accgctctgg caccccgccc 146400
 
gggggggcag aggggacggc ctgtgaccac gtgggtaaat cctgtaggac agcccccggc 146460
gcgcagagaa ccgtcaccag catcctagag taacaggtca acaacctaga acccttccaa 146520
gaccacacaa ccagtgggga caccctggcc cctgattctc actattcatg tttcataagc 146580
caacgatttg taattgattg ttgaagcgct tttgtgacac tgatttatga ggcctagtta 146640
tgacggctgc ctccaaaccc ttgtcagatc actccagcat caggtccatc taagtcctgg 146700
catctactca ctgtccttgc ccggttccgg tggttttcct ggatcttggt acgattgtga 146760
tgtatgatct aatgatttg tgctttatcc tggacttttt ggttattaca ttaggagact 146820
tgatcgcaac gaaatctatt ttagcagatt attacctgct ttggttgagg tgtaggctct 146880
ggcctgcttt gtgggcttca gctccaagga cagcccggtt tcctgagccc atgccatgtc 146940
attcaggtgt gcttcattct tccagtgttt tttttttttt tttaagattt tatttattta 147000
ttcattaaag acacagagag aggcagagac acaggcagag ggagaagcag gctccatgca 147060
gggagcccga cgtgggactc gatcccgggg ccctgggagc aagccctgag ccaaaggcag 147120
atgctcaacc actgagcctc ccaggtgccc ccattcttcc agtgttggtg gggctctgtc 147180
cctctgtccc tgctggtgtg gtgtgtcgag gcaagaggtg gtcccctgag ccacctgggt 147240
cactggtcac tctgcaggaa gggacagaag ccactgggtc caggtctccc tatactgggt 147300
ggcgggcagg gaggcacacg gcttcacccc tcccgctggt gtgggcagca tctgtgggca 147360
ggtggacccc ccggggaccc agttgtggag aggctgggac agtcactgct cacagatcgg 147420
actgcctgct gggacttggt tgttgagtga ggctggggct gttcacagag gctccattgg 147480
gctccctttt cccagtccct tggccagaga aaatgggctt ttttgaggtt ttcttttaac 147540
ctgtgcttgg ggaagggggg gttctgacca caggtctttc tggcgcccag ataaaaggaa 147600
aacccaggga gcatccttcc tctggcctga gatctccagc ccttccacct tcttccacgc 147660
gagctcatag tcctccgatc attgtcggtt gaataacttc caaggtatta agttattttc 147720
cagatagagg agcaggggaa agggagtccg gcatcttttt tcagaatcag cattctaagt 147780
cctttataat cacacgttcc aactcacctc tcaccaacgc ccactaatcc ccccaaacac 147840
acgctagttc caccaggatg gttggtcttc tccggggggt ggttccatac gaagagctca 147900
tcatcagtcc aatacgcagc aaaagataga gttaggtcag gcttggtgag aggaagcccg 147960
ttccgctggg ccacggtgca gacatccttg cagcatctgg tatcagcctc ctgctcgccg 148020
gttcctgaat tcttctgcat gtaccagtca agcaatatct tttattctat ttatttattt 148080
atttatttat ttatttattt atttatttat atttacgata gtcacacaca cagagagaga 148140
gagagaggca aagacacagg cagagggaga agcaggctcc atgcaccggg agcccaatgt 148200
gggattcgat cccgggtctc caggatcgtg ccctgggcca aaggcaggcg ccaaaccgct 148260
gcgccaccca gagatcccca gtcaagcaat atcctagttg gatgcttgtc ttttgagtcc 148320
cgagaacacc atggtctatt aaccagcccc accgatacca atgggccaaa gggccatggt 148380
cacctttctg gatttttctg ccattatggc aactttgccc accttgcctc tgatagctga 148440
 
gcacctccac ctaaactcta tttggtgatc ccggcatccc cgctgacact aaggagtgaa 148500
gttctacggc ataagctact gtcaatacta ccagcatcaa gacgcagaag cacgtctcat 148560
aggtgtgttc cctgccgcct tggtgaggga agtgtcctct ggaccctccc agggaacacg 148620
gtcaaggggt agagcctcca gcctcacacg ataaatccat ttacccatcc ctacctcccc 148680
aaaccagctc gccgacgcca tgcccaagct ttgcagggcc acttcagcgg tgtatctgcg 148740
tgcctcccga gtatccttgc cgggaggtca gaccctgacc catggccaag ctctctcaaa 148800
tcagtaaact caatccattg ccttcaaagt ccgctcctac acgtttcccc tgttgctcgt 148860
atgtgacaca tacaagccag ggcctgtgct cacgtccatg aagacgccat tgatatccag 148920
agcaggaatt ttattttccc acttgggctt tgttgagacc tggccctagt ccctggtcta 148980
gaggaaagaa ggaccgggtg ggacatgtcc ttgagaataa gcatctgatt gtcagacagc 149040
tagctcaggg gacgtgttcg aatggtgtcc aaacaccagc gctgctgggc caacgggaga 149100
gcctgcttcc ttatgcccag agtgttctgg agaatctggg tgttcacgct cagactcttc 149160
cacccaagag tccccatccc aggtcacagg atgccaccac tgccctatca aggacccgtc 149220
tactgcacag aaaacctgtc ccgctctggc acacatttgc caggtgtcaa tacaaaatgg 149280
caaattaaac ttgaagttca gaaaaacaaa tgaatatttt agtaaaaata gctatgcctc 149340
atgaaatact tagaacatac ttatacttga aaaaaaaaaa acccattcat ggttacccta 149400
aaattaagat ttaaccaggc agcccatatt tcacctggaa accctacatt ggcatcctta 149460
tgattaaatg ctgggtctga ttatcagcac agtctgcccc aaggtgtcag gggatgcaga 149520
tctctttaaa catcgctgga gaccttctgg cttccacagc aggccctgag agacacctgc 149580
acctgccgtt ctcattctca agtacgcgtt aggtttttat tacaggtttt aaatgacttt 149640
aaaaaataat ttaaaaaaat gactttaagt acctgaaaac tttaatcagt gtctccagaa 149700
ccccgaaata tatttatgaa ataattttca aaaccggtga cctgacccag gctcctgggc 149760
aaggatcctt ggaaggctat cttgagagca gggaggacaa gagggaaggc ccagaagcag 149820
gtggacacca aggtctcgtc ctggaccgaa ggcatatcca tgctccctga aaccagcgtg 149880
cccatctggg cctggcgggc aggagccacc ggagaggggg ctgggcactt gagctgggtt 149940
tggggtgcag gcagctagtc aacgtgaaga ctggggttga gtccacaggg agggggaggg 150000
cagggcgagg gccagtgggc gcagggtggg ccgcggggtg gagggaggac gggaccacgc 150060
tggcctggaa tctcatactc cgggttctgg gctggtttcc agctggcaca gagctccgag 150120
ccctcgccgg cttcaggatg acccggaggg tccggctggc agcggctccg cagagtcagc 150180
gtgcaacaga tctcctaggc aacaccgcaa acgttcagga acattcggac aagagtgtaa 150240
catgaagtgt gaagtgcagc tcatgctgct gaaatggaaa cgaggtcagc ctgcgaggtg 150300
crggcgtgaa gagggggtgg gggagccctt gtggggacgc ctggtgcaac cgggctgctc 150360
cgtccgcccc aggctgcgct gccctgatgg agcccccaac cccgctgtgc tgggggccgc 150420
ggctggccgg ttccgggaca cccccacccg ggcccaaacc cgggctcccc gctctaactg 150480
tgcagacccg gcatctcagc ttcctcacct gcacaaccca ggccacgccg catgggtcag 150540
 
gcacccaaca cgagagctgt caacacgtca ggcaccccca gtgcctgctc agcacacagt 150600
agktcttcgg gggtgggggg accagctctt ctcactttgg aagggggtct cctgagagga 150660
gaacatataa cctgtcctga attcttgggg catccagggg ggctcagcgg tgaagcattc 150720
acctttggcc cggggcatga ctccggggtc ctgggattga gtcccgcatc gggttccctg 150780
cgtggagcct gcttctccct ctgcctgtgt ctctgcctct ctctgtgtct tgtgaataaa 150840
taagtaaata atctttaaaa aaaataaaac aagaaaacaa aacaaaacat gaaaattttc 150900
ctgaattctt tcccttgact ccaaccattt ctgctcagtt cctgaatcct cagaagtgaa 150960
agcccctaat taagctcatc tagagaaggc caagggctca ttggctcatg cctcacatga 151020
cccgaagctt ccagaaccct gaaagcctgc gtggtctgat gattttagag tcacgggctt 151080
cattcctcag gagatttctc tctctcttct ggcttggcct ctctgcctaa ttagaactgc 151140
ttctaagcat atcgaaatct aactcctaac gtgttcagat gaggaaaaag cacgttgcca 151200
aaacggattt gcggatcgta tgttcctctg agcccttgaa cacaggggag gaacaggcaa 151260
atgaaattct gtagggcttc aagtggaagg agactgtttc ttgccctaga acattctggg 151320
gagggaaagc caggatgacc tcacaccaac tcaagagaga ggaggaggcc gagctgagtt 151380
gggggccagc gtctcggagc gcagtaaggg ccggtgagtg acagaagcaa gaggaggggg 151440
cagcagaaag ttctggcttc agtgagggcc agaggagggg ccgcagaccc ctttccccgg 151500
aggctgccag gagcgccggg tgccgggggc agagctctgt ttggagaatg cccatcagtc 151560
agagcaaaat cacgggcatc atcatgtgca aggatatgga cagagctgga cagtataatg 151620
ctaagcmaga gcagtcagag agagacacac acmagatgat ttcactccta cgtggaatwt 151680
gagaaacaaa cgagcaaagg gtgggggggg gggacagaaa aaggcacacc gagaaccaga 151740
ctcttagctc tagagaactc actgagggtc accagagggg agggctgggg ggctggggga 151800
gcaggtgatg gggatgaagg agcacacctg tcgtgatgag caccggctgt tgtagggaag 151860
ggctcaatcc ctatactgta cgcccgaaac taatactaca ctgcagttaa ctacctggaa 151920
tttaaattaa aaccttttta aaaaaatcaa gggcaccatc raaacagata attatgatta 151980
tcaaatactg tttattcaac agttatcaaa ggcctactat gtaccaagca ccatgtaagg 152040
cagcagacac gtcagggtga acagcacggt cccagggctc ccagagctcc gaacagcgga 152100
gcaaagacaa gaaccaggtc accacacaca tggcaggtac actgtgggag gggccctgca 152160
ggaacgtgta cagaataaca gaatccgatg gcggggaggg ctggccgctc agaagatgct 152220
ctctctagac tgttgaacca agaactgaaa agggaggagg accagaggag tggcgggggt 152280
gggggggacgc catcgacaag gctggacctc agagagcaag gggagggagg gggtggttgg 152340
aagacagcag acaggtgctt cgctgtggtc ctgaccccat gggaaacaca gctcagggtc 152400
agtgcgtgtc ccccgatgtg tgggggacag tcacagggcc ccgtgcactg cagagctggc 152460
tgaagcagcg tcaggtgaca cctcctcaaa ggccctgtga ttccctgcct ggccccacaa 152520
ccacggaacc agaagaatgc ttccaaaaat gggatttttc aaaaaaaaaa aaaaaaaagg 152580
 
gatttttccc aactttccag attcataaaa gaaaaaaaaa aaaggtcatt cagtatctga 152640
tgaaaacata attctcttgt aaggtacagg tagtactggg catagctggg gcttggggca 152700
gtattttgca tgtattcaat atcctgtaca ttcatctcag aaacccagat gcccctgtca 152760
gcccaatgcc aaagtggccc agaggattta gacaggggag aaggaggtat ggagtggaac 152820
agctgcctgc agtgagctag aggacccagg aggggcccgt ggctcgggga cagcaggcct 152880
ttctgcgcaa atcctggacc ggcccatctc acagatattg tagaacactc cctggtgggc 152940
caggggcctc ctttccccct gcggccccca cctacgagca gagatcaggc taagtccaag 153000
cccaccagtc agtgaaagca gcctgcatag cgagccctgg agatgggggc ctcccaaacc 153060
gtgaagacca aggatccccc tctgaaggcc atcttccacg ctgcgcttcc tgctggagtg 153120
gtggagacaa ggctctttcc ctccttgtcc catctgcccc taaatcccag caaaggcaag 153180
gtctccagac aggcactagg gagacgagga ggtggggagg agccctcgac tgccctctgg 153240
cgtccaccca tctaccagct ttcaggagag tccaggatgg cttagtgaga acgcgctaga 153300
gaggtgggag gtctctaacc cactgtgtga ccctgggtgt actccacctc ttctctgggc 153360
atcagctcct caccagtaag agacagaaaa cactaaatca aaaagatgta tgcaccgcta 153420
cgtttactgc agcattgtta acaatggcca agatgcggaa gcagcctaag tgtccatgaa 153480
tagatgaatg gattaaaata tatatatatg tgtgtgtgtg tgtgtaaaaa tacacacaca 153540
cacactggaa tattactcat tcatgaaaac aatgagaatt tactatgtgt aacaacatgg 153600
gtggacctac agcgtattat gctaagagaa ataagtcaga caaacaccgt atgatttcac 153660
ctctgtgtgg actctaaagc acaaaacaaa cagaaagaga ctcataaata cagagaacaa 153720
acacgttgat gccagagggg aggggacagg gagatggcca aacaggtgaa ggggattaag 153780
aggtacacac ctgcagttat aaactaaata agtcatggga tgaagaggac agcatgggga 153840
ctatacggta ttgtaataag gttgtacggg acaggtggca actatacttg gtggtgagtg 153900
tagcttaacg tacagagtcg tcgaatcacc acgttgcaca cctgaaacta atgtaacact 153960
gtatgttgac ggctcttcca tcaaccagcc aatcaagaga caagatggaa ccacaaagtg 154020
ttgagaaaga gcaatccagg ccctgagcaa gagaacaagg ggcaggggca aactgtcagg 154080
gagccagagg gtaccaccaa ggggacacaa ggagccctca gaagctgggg agcggccgag 154140
gtagccagaa tcccaaaggt gaggcccagg agaggcagga atgtcaggct gaggggaaac 154200
aaccagctcc acccatcctg gccgggagcc accaagcgct acagctaacg tctcccccct 154260
ctcctccctg acctctccca gcttccccga aatagctaat gtaggaaatt aagatactct 154320
aaatgggacc ctttgcaata aaagatatca tggagacgac tgccaaaatt tgcaacggtt 154380
cttgtgatta gagacagcca tgtatcgcga ttttgatggt aatgcggttt tttagaaaac 154440
tgcccgcgtt tgtataaaat acacaccaga gaaaaatcat gtcagcaact tacaaatgga 154500
agagagcaga tatttgtgtc atgcatgcaa atttctgtaa ttttcagtgc tacaattaaa 154560
aattttttta aagattttat ttatttattc gagacacaga ttgagagaga gagaggcaga 154620
gacacaggca gagggagaag caggctctct gcaaggagtc tgatgtggga cttgatcccg 154680
 
ggtctccagg atcacacccc gagcccgagg caggcactaa accgctgagc cactcaggga 154740
tcccccagtt aaaaattttt taatccttgg gatccctggg tggcacagcg gtttagcgcc 154800
tgctttggcc cagggcgtga tcctggagac ccaggatcga atcccacgtc gggctcccgg 154860
tgcatggagc ctgcttctcc ctctgcctgt gtctctgcct ctctctctct ctgtgtgact 154920
atcataaatt aaaaaaaata aaaaaaataa atttttaatt ccaaaatttc ctcgtagaaa 154980
ttgcatacat tctttgactt agcaattcaa tttcaggatt tcattctata aacatatcgg 155040
cataaatcta tgtgtacaat gataggtatg cacgtacagt aatctttttg aaacattgta 155100
aatatacgtg tcatcgatac catgtattcc ctacaatacc cataggacag aaatttttta 155160
agtagcaaaa aaatcacaag ttaactgtcc atcaatggga ggctgtaaaa taattacaat 155220
aaactcatgc aaccatttag aagaatgaat tgggtctgta ctaagagcaa ctagcattta 155280
ccaaatgcca cgtggcaggg ctcatccaag agctttgtgt atatggattc agaacaaccc 155340
attatgtaga tcctattgct atccccacaa tatagaagaa gaagtgaacc ccggggaggt 155400
taagtaacat gcacaggtta acacagctgg cccctcatac acccagctgg gagtcgggct 155460
cttgcagttg gatgcggagt ccaagtcctt aaccacaccc acggtaatcc cggaacaata 155520
cccaagacac cattagcaat gattgccttt ggggagtggg gttcgagcag gaacaagaac 155580
aaatttaact tttgatttca tactgtttat attctttacc atatattttt atcaggtatc 155640
atcatgtgtc acttctgtac attttttaaa acccactcat ttaagtgacc actaaagtat 155700
atatttaaaa aaaaaaaaga ttaaaaaaaa accaccactt ctgcccttaa ggaacttaca 155760
gtcctattgc ttttaagaga tttattaatt ttacaagcaa tagtaaaata catggtaagg 155820
aaatgaaaag catggcaacc gttggaaact cagctttaag ctgaaactat atccagtgtt 155880
tgcacacgca ctggtgcgtg cacacacgag cacgggcagg cacacaacca gttgcagatg 155940
tttggtgaga gctcccatcc taaggcagcc caagggcctg aaatacccaa attcacttgc 156000
acggcttctt ggggcgccag gcaaacgctc taactgcagc agcattgcgc ggctttcgaa 156060
ataagtcgat cagagaagga caggcattgt atggtctcac tcatttgggg aatgtaaaaa 156120
atagtgacag ggaatgaagg ggaaaggaga gaaaatgagt gggaaatgtc agaaagggag 156180
atggaacatg agagactcct aactctggga aacgagctag gggtgggggt gactgggtga 156240
cgggcactga ggggggcact tggcaggatg agtactgggt gttattcttt atgttggaaa 156300
actgaacacc aataaaaaat taattaaaga aaagaaaaat ttacaaatca tctaatgtga 156360
cgtcacaatg aacctctaag gctgagtccc ctactctgca aaaacggagc cggtagtgtg 156420
tgttcaccta acagtatcga ttatgcattg attaaccatc ccctccccat tttgtagctg 156480
tgggggagac ctcccatcct aaggcggccc aagggcctgc aagacccaaa ggtagggcct 156540
ggggccgggg gcgatggact cttgggttca gtatttttta atgataagca agagggagga 156600
ggggagcgcg ctccaatgcg tccaactcca gaagagaggg gaagacgggg tcccccaggg 156660
gtccccggga gatccgagga ggcacggggg tgtttgggct ggggatgggg acctgcagag 156720
 
tcagtcgagc cctgagcgcc gagtccaggc agggagggtg cggggagggt gcggggggcg 156780
ccgcggaggg tctccccaag cgcatcctcc aagcactccc cgcggggtcc gcgcgccccg 156840
agcgcagccc gtgcgccctg caggacggtc ccggggcccc aaggccaact taccactggg 156900
cacccccact tagcagtcgg gggcttcccg agcctcggtt ttcccatcgg taaagccggg 156960
gtcaactgcg ttcccctgca gaattgggta ggaatccaaa gaattaagag aagccagtgc 157020
cgggcgcgcg ctccgagggc gctgtcaggc cggcggggcc ctggctgggg gcggaggcac 157080
ggcagccacg gcacccggca ctcccgcgag tggcctcaag ccctgccctc cccaccctcc 157140
tcccgaggct cctggggcgc cgaccccgga gcatcccccc cagccccccg cacctgcgcc 157200
accacctgcg cggcacccgg cacccggagg cgcgcccgga gctgggcggg gcgggctcgg 157260
cccatgtgac ttcgcccgcc cgcgccccgc gcccccccca ggtcaggtgc ccggtgcccc 157320
gcgccccgcg ctcagcctgg ccccggccat cctgcgtcgc cccagtgcgc ccggaccgac 157380
gccccgcctg ctcgccttcc ttcccctgta ggaccggaga gagcagcccc aggggcaggg 157440
ccagggcctg cccgccccgg gaaaggagcc ggtgagtgca gccagcgcag ggaatgggga 157500
ggccaggggg gcgccctggg actgggcacc caggcataag gagggtccac ggtgatgggt 157560
gcagctcccc ccgtgtgcaa gtgcccccag cggctcccag gccttggtcc aggctggtgc 157620
agagggcact ccctttcccc caaacacagg cctcagggct accccgcgtc akcctggcct 157680
gggttctcag ggagctcatt ttaggcaaaa cagtgatttc gctcagagcc agctgtgggt 157740
cccagtgtgg actgggccga ggcttgggcc aacccagcag acacagggct agacccgcgg 157800
ggcccaaggc catctgatga aagtcttcac ctgcactgca gtgccgattt ggggggacag 157860
agcgggctgg tgttttgcgg tgggaggagg gtcctggcct tcctctgggc tcctaaagtc 157920
ccagctttat tagaaacatc ttttgtttac caacagcact ccccgccgcc cgtccaccta 157980
gttctagaga tgtgggacgg attggtgggt gatgggcatc gcagagtggg cgctggtccc 158040
aggtcctggc ctgagctaaa ccccacttat aaatcctcta aagtacttta ggcttagaca 158100
cctgtttcct accaacctta atcaaatatt gaatatgcca ttttcagctg ggttggggct 158160
tttacatttt ctttttttta tgtggtaggg tttttttttt ttaaatcttt acatatttcc 158220
ttctgagagg ttagaattgt aatgggggag gcgaggcccc cagaagccca ttatgtgcaa 158280
aggcagaccc tggctgtggc tgtgaagtcc acagaaaggg caagggcttg gagaccgtgg 158340
gcacctcagc agtgacactg gcatttgggt ccagctcatt cacctctttc ctggacgacc 158400
gtgcctcccc ctcattgttg ggccacacag ctgagcttag caaggccatg actttcctgg 158460
gcaagagccc ccccccccct gcaaatctcc aggacctcat gccttagaat ccaagtacca 158520
attctaggtt cctgtgctct cccccatccg aaaaatgggc acagtggtag gtgccctgga 158580
gacagttctg ggcacacaga tgagaagtca gggggtccta ctgattaggg gattagaaac 158640
taaaaagtta aaaccagcag agtaggtctg gtggggggct tggggtggta aacaggtttc 158700
agagccctga aggctaatgg gaaaggtgac tctggtgtcc ttagatgaca gcgtggtgtg 158760
tgcctgtgtg tgcaccacct caggtggctg ggtcagcctt ctgggatgac tgtcctgttc 158820
 
atttcagaag caagttctgg ggccaccggg gtggttcagt cagttaagtg tctgccttca 158880
gctcagggtg tgacccctgg gtcctgggat cgagtcccgt atcgggctcc ccatagggag 158940
cctgcttctc cctctgcctg tgtctctgcc tctctccgtg tgtcttatga ataaataaat 159000
aaaatcttta aaaaaaaaaa aaaaaaagag gaagcaacct ctggaggaag agctctgctc 159060
ctccacccca ccctagagcc tcccctttaa caacctggca ttgtgcacag ctggagaggg 159120
ctagagagct cacggagccc aaccctgtcc gtggcacaga tgggaaagct gagacccaga 159180
gagacggtga ccctgtcctg ggtgacagag ccagctggtg cttgagccag gatgggaagc 159240
cagctccaga ctcttggccc tgtcgccgtc ccgcagcccc agccggggaa ccattcttgc 159300
tgtggacgct gagctgaggc cttggctgca catgcagagc ttgtgtccgt gtgtcagtga 159360
ggatcaccag caactgccca gagacctgag ggagcagaga gaaagggcag gtgggcgggc 159420
aagctacaga gtgccccccc tcccccccgt gcggttgctg ctggagcaac aggtgtgcct 159480
ggtgtgcttt gagccggggt tggcggaagc ttctgggtgg aaacgcgctc agtgcacagc 159540
tggccagggc tggggctttg gttgttccta tcgggcggcc aggcccaccc gcagaagtaa 159600
ccgcaccggc cagctcagag cgggctgttt gagaagcagc cggcccctcg cctggcacat 159660
ccaccgtcgc tcaggaatgt gcctgaacct ttatcggcaa gaccacctcc tcctcgacgg 159720
aggcagagag cattcctgcc tcctttggga gctgggcatt gagaggaaac tccgactggc 159780
ttcccagttt tatttgccca ggcgggtgcc cactgctaat caccctggct cccaggctca 159840
gagggcagct gacccaggag gtggtcgagc cacatgtcac ggacgaggag cctgccccgc 159900
ccaggcttct ctttaactgg gcccaaacat gacctcgggt ctctaacaga taaagacgca 159960
ccagcgaaat tcagcagtct gcgcccaggg ttttccattt tacaaaaccc gtcccgttct 160020
gtcttcccaa ctgtcttccc aactcatgaa cccccatgtc cacccttgct tttctccgtt 160080
acgaccctga cacaattagg gagggctaac agtctcgggg attaaaaatg tgattaaatc 160140
tgagcagcag aacaggcaaa attgaaagcg tttcacgcag gaacctcatg actaggagcc 160200
tagtggagtc gaccctcctc ccacacagac acagacgcgt gagcacggtc accctgggag 160260
gttccccggg gagggggggg caggggcaga agcagccctg ctcctggatg gggtcagcag 160320
gtgccctgtc ctcatgcccc cctgtgactg ttgtgggtgg aggggatcca ggggcagctc 160380
agggtctcgg ggagcaagcc cggtgtgtag tgatgggaga cgggaaaatt ggctgaaggg 160440
ctgttgcttc catgagcccc cgtaggtccc ccggtagagc cgcctgcatc agcgcaccag 160500
cctgaagcca cccagagggg ctgctagcga tgcctgcccc aggtgcccgt gtggtatatg 160560
ccttgtaaat gctgaagtct agtatagtct ccattcaatt tgagatcact ttttaaaggc 160620
agttttcaaa aagcactaaa atcattaatt cctaagtggg aagattcggt cacagtagat 160680
aacgacaggg atccccgggt attggctccc aattcctggc tgtgctgcga cacgctgccc 160740
tagggaccct caccgacacc cccaccccca cctcccggca ctgcggcaca gcacagcggg 160800
cgaatgtgga ctttggaatc cctcgtcctt ccctcccctc cctgcacccc cctcccagct 160860
 
cccctggagg ttgcatcgga gctcaacata agcacaagag taaaacgtct caggcgacgt 160920
ggaaccgtgt gtgacacatg cagttcttgg ttctcggctt ggcggtgtca gatgcccgtt 160980
aacaatccag atcccatcag ctctggcttc gtgaaacagc acagtgaaca gatgtcgctt 161040
cccttaataa ctcgggctca cgaccggaac tcccaagagg gtgctaagcc actgtgcctc 161100
ggtttccgca ggggcaggtg tggccgcctg ccgccctcgg cctgctggcc tcccctgttc 161160
gcatctcagc atgtttctga ggggctcacc ccctcctggc aggcaccccc tccccagagg 161220
tgcccagacc ctctgggtcc agcacgcatc tggtccccgg ggccttggtg gtctttgctg 161280
tcggagctgg gaaaccaagg ggccatcctt acgagcaaga agcttccaag agaaggaaca 161340
gtctgagaag gggagggggg agacatgtcc tcagatctcg gggattgggg ggcgagggag 161400
gcagctctga ctggctagcc gccttcccat ccatccgctt actggccgct cacggggcgt 161460
gctctaagat aggtgggctt gcagggtaag aagagtatac tggtttcctt ttgttttgca 161520
tttctccctc attctttact atcttttttt ttttttttta agattttatt catttgtttg 161580
ggagaaagag caccagagag agcacatgtg cacgagcagt ggggaggggc agaggagagg 161640
caggctcccc gcagagcagg gagcccgatg cggcactcga tcccgggacc cgggaccatg 161700
acctgagaca aaggcagaca cgtaacctgc tgagccaccc aggcgcccca ccatctaagt 161760
ttcctaaagg aagcctggaa tgacgtccct ttggagagga gacgtggtaa cgagtcagct 161820
gatggtcacg gctctctcat ctgctctgaa aatagaactt caatgaacgt gcttcttagg 161880
atacaatttt tttttccaaa agtggtttaa agaaatgaat aaaccccagt catccatcac 161940
aaaagaactt tccccccgat gcttcaaaaa ccagaaattg cgtattccag gagaacggca 162000
acagtgaatt ttgttttaag gttttcttgt ccaaaatcta aactatccgt gaaaatagtt 162060
tgaatgactg gcacagcaag gcaactcccg aatgtgtcat tcacggtcag cgtcgtgccc 162120
gtagggggaa cccctgaggg tttggtgcaa aaaatattaa gaaatgactc ttcaccaatt 162180
taaggcagag gtcaggggac attttagttg ggaactctga gaaagcgctc gtgttaggtg 162240
gtgtctggct gccctggaaa tgtcgcttgt cactaattaa ccccttgtga aacacatcag 162300
agtcctctct ggcccagagc agtttttgtt gacgtctccg cggcgaacat gcaaatgtga 162360
acagcacggg gcggggtggg gggggtgctc cccagaactc agaagcaacg tcacgtcttt 162420
cggttggcct gaagggttcc cagggggagg agacagttgg gacccccttc tccgatgtcc 162480
ctgagacacg ggtgacagtc ctcggtgaag gcttgttgaa gtcacacagg ttctcacccc 162540
acaagggtcg cccaaaccat gttttcccct gagcctcggt gcccagagaa gagctgatgt 162600
gggtgtagcg gcctcgtcct cgggccgggg cgctgggagc acggaacggt gcctgcggtg 162660
tgccagggga aatgacaaag tctccgtcct cgtggagctg gacagggacg agggacagca 162720
gccgtcacac tagaacgtgc aaagaaatgc tccaaggatg agcatgaagg gggtggggct 162780
gagcagggtg ggcccctctg ggcagcaccc cgaccccgtg tgctcgaatc cccagtgaag 162840
tcctcccagg tgtggtaatg cctcaacttt gtacccagtg taacggcagc gtcagcatcc 162900
gtgaggcccg agtgactcct ctgcaggaag aaccagcagg ggggcccgtg tccatcctca 162960
 
gtctcgctca gccaacccct tcactgtccc tgtcctgctt tgctagagct ttgccgctca 163020
gaggggtccc gggaccagcg acaccggcca cctccagtgt gagcacggcc acctgggaat 163080
ctccttaaaa tatgaattct gaatcaggag gtctggggtg gggcctgaga agtctgcatt 163140
tctagcaagc tcctcggaaa tgctgctggt cctcagaggc acactgttcg gtttgggggt 163200
ttgtttggtt ttttttaagg cttaatttat gtatttgaga aaggcagttg ggggacaagc 163260
agggggagac gcagaggcag agggagacac agactccccg ctgagcaagg agctggacac 163320
gggactcgat cccaggaccc ctggatcatg acctgagcct caccgactga gccaccccgg 163380
cgccccctgc gaggcaagcg cccagcgcta ctccgttaag cagctgagtg aagtcagggt 163440
ctcggaggcc agcgctggcg gtgaacctaa catgcgccca ggctcgagcc ccacggccga 163500
cccccctcgg gtacacccgc tcccgctgac catgttgtct gcgggcctgg tccccactcc 163560
tctgccggca ggtgggctcc accctgggaa gtatgtgcag gccccccatc ccccccgcag 163620
acacagtgac ctcaggacat cgggctgggg cgacctctca gggggccagg gcatgcacgg 163680
tgactccaca gcttgaccat gaatctgagg ggaagttgcc tggaaacagg acaggatcac 163740
cctccaccag gagctggaag gcgagaagcc ccggggatgg agcccttctg cagcgtttcc 163800
ccagcgtttc tggggtcccc actgcagcgc gtgaccctgg ggcaggggaa agatactgac 163860
gtcaccgaca ttatgtcaca gcggagggac aaagggcccc ggggctacgt gtgggagttc 163920
caagtgtgca ggaacgtcac acataaacct tacaccgttt aaacaacaca agtttccaga 163980
caggcatttg tctgttctag aaaacccttc cgtccggagc caaagtacct gggaactcgg 164040
cgtgtcccgc ggcccctctt gtggggcctt gtgatgtagc ctctgtccac gggctttcgg 164100
ggtgaccacc tgtgatcctg tcctattcca ggtgtgatcc tgacaagcag agatggggaa 164160
aaaactggat ctttccaagc tcacggacga agaggccaag cacgtctggg aagtggttca 164220
gcgggacttt gacctgagaa ggaaagaaga ggaacgattg gagtaagtgt gtggggccga 164280
gccggtccct ccggcgtcct caggaggggc ctcaccgggg cgccctcggg ggaggggggc 164340
agacagcagg aagcacaggt gcaggacgct ggatgggaag ccgggcgctg ggagacgtcc 164400
tgcgttccgg gctcggcttc cttctgagta aaacgagggg atctgacctt ccagcctgca 164460
ttcgggtgtc ccctgaatgg ttttagttcc tcttgatcca gcttcaggct cagggtcccg 164520
tggtctcctg agttcaggga tgcttcggcc acctggcgag ctgaaggtga taaaataggg 164580
aaacacccgg aatgtcggga tggagggatt tcagcgtcaa ggctggcttc gcctctcttt 164640
cccgcrygtt gggtggcgcc catcctcgag tgggtggccg tgraccgagc caacaccctc 164700
tgccagtatt tacccgcggc ccctcctgtg acattagctg tgccccgagy tcctttatga 164760
tcctttcttg gtgtccrtgt gggccggagg cctatcgaga aggagacccc ggagcaaagg 164820
atcacggagg aggagggcag cccagcgaat ggccgaaaga gcaccccagg cggaggcgga 164880
gaggagctag tgaggagcgg ggtgccaggc ggcaggtcct ggaggacctc tctggggctt 164940
gagccttctg agccccggca gccgtcaagc cggggaaccc caagacccgg cttctgctct 165000
 
gcaaccctac ggctcccggg gaatggtcac acgtacctcg agggcagagc cagcaagttt 165060
gcccacgact ggatgtgcgg gtgccgaggg cgaggccagg gtcgtggcct gagtcaccgg 165120
ggggatgggt tgtcgccatc tcccccctcg gaagacgagg cggagcgggt ttgggggacg 165180
ccgggaaagg gttccgacac gttgcggtta ggtgcctggt aggcgtcagg tgggccggtg 165240
gcctccacgt gagctggccg ggagctcggg gactgtgaga gttttcacca atagatgaca 165300
ttttatatcc gtttcctggg gctgctgtaa cgctataaat ttagcggctc acagggcata 165360
cgtttctcag gggttctgga ggtcagacgc tttaagatca aggtctcggc aggcctgcgt 165420
tccttctaga agctccaggg aggagcctgt ttccttgcct ctcccagcct cccgggcacc 165480
tggccctgcc tctgtcctca gggcggtcag cctggcatcc ggcccccacc cctcgcctcc 165540
tcctcctccc tctgcccctg tgatgacatc gggcccaccc agatagtcca gggtaatctc 165600
gctgcctcga ggtccttaat cacatctgct aagtcccttt tgccctgtga ggtgatgtct 165660
ctacaggtcc cgggaattag gatcatagac catttatttt cttttctttt cttttattta 165720
ttttttcatg agagacacag agagaggccg agacacaggc agagggagaa gcaggctcca 165780
tgcgggaagc ccaatgtggg acttgatcct gggtctttgg gatcatgcac tgggctgaag 165840
gtggcactaa accgctgggc cacccgggct gccccataga ccattttcat gtgggaagga 165900
gagcattatt caactgaccc caacatgtag tcgccagaca ggaggagacc ccccaggagg 165960
gagggagtcc agagagagaa agacgggccc ccccctcccc cgggagacga ggaggaccag 166020
gggcgaggga tgctctgggt cccaacacag aacaaagccc acataatggc cgagccacta 166080
agagaagcaa gcagggtccc cccaatgcct gcagcgcccg cagggggggc ccggtcagtg 166140
ccgccagagg gtccctgctg cctcttggtg cagcccattc cgccctgggc ccctgggtca 166200
cccccgggct ttggtcatga agttgctgcc tccgtgctgg cctccgggga ccgtcagccc 166260
ctaacatacc cacacgagac ctcagactgg agcccacggc aggccttgac ctgacctgac 166320
ttccgagtac cgcgcaggtg cccgagctgc cacggggccc tccgcccctt ctctccctgc 166380
ctctgaggtc tccccattct gaggtcccct ccctctcttc ccttcgtcct gtgaacactg 166440
cagcgtcctc tcgctctgtg acccagagca gctccctgcc cggccgccgg gctctgccca 166500
gggccgcgat gctcgcacca gcaaagccag cgcagccttc cctcctgaag gcaccttctt 166560
ccccagggat ggacccagcc ctgcctccgg gcagcccagc tgttgtcaac ttttattttc 166620
agtctataga aacacagata agtgagaagg agggagccca gctctccagg gagcagaggg 166680
aggaccacct gggccaggtg ccaagaggca gcctccttcc cagagaccct tgagctgggc 166740
tgctcctctg tctcggcagc cagccggagt gggtgtccca gaaatgcctg tttcttgcat 166800
gaaggcgagt cccttctgat gtccaccatt ccgtagtgtc ctattcctct tgccacgagg 166860
agacaggccc aggtgacctc tcaccgctgt tggaaacgga aggctcccac aaggatgcgg 166920
accgtgacgc cgcgggccag gtccccgggc cagtcacggt cagggcggct tcctcattca 166980
gaagtgcccg aagccgtctg ctcagaagtg ggacccggtg ggggggtgat gggggggatc 167040
ggccctctct gctccgggta cttggcctgc cgtgtggcgg ggggtgggct gattaaatcc 167100
 
ccccatcaga cgggaggagg tcggatgggg aatgttgacg cagtggaggg tcgtacagcc 167160
ctgggaacgc gagcgtgcag agaggccgcg gcgtggagag tctgtgtccc gggagcgtta 167220
ggtcaagagc agcgaagccc aaaaggcata acctgggctg gttgttgttg tgcaaacatg 167280
cgtggagcag gtggctcagg aagtgacgag tcactgtggg agggaggagg aagggaaggg 167340
tttttatatt tcttatcgtt tctgacggtt ttgtctcttg gtaactatgc tgctagttag 167400
atacgcaaca gaaaggaaag ctgcctctga ccgtgaggga ccttttctcc cgtcaccccc 167460
agctgcccgg gggcagccgc gggacccaga catgtgctcc cgagcacgag ggtgggcgtg 167520
atgagcaccg tgaggcccga acgcgtgggt ccaggttctg cccagatggg cagctctgct 167580
tagaaggggc ggctaaaccc agcatgaaaa tgaagaccct gagttcttga caccagaggt 167640
gattctacag agcagttggc taaaatgcca gcccccccag gttccctgat ccatgaagca 167700
gtcatgggcc aagaggagaa agtggtcacc cagtggctgt gacaaaccca gcggctcggg 167760
aatcgctcag acgggtttct catgtctttg ccatcctcca aaagggagac cgagtgcaca 167820
gctcagctgg aggaagcttc cacagtcagt gatagaacca agttaatggg ggaaagaagt 167880
ggaaacccaa gaaacacgtt cactaaatgg agagaacttt cctgccctga atatgcatcc 167940
ggtggacaca gtgcatgggc ctgattcgcc gggagcagat ttgccaaggg gcagtttgct 168000
aaacagtcag ctcaccaaat ctaccagttt gaaaacaaac cacaaaaata tttcttttaa 168060
tgtctacgag ttaacagaaa tgtgtatttg gtggatttct ccccagatgt ggaaatgcca 168120
atcaactgta gaggatgcta ggacagggcc tgggccgaga ctccggtacc tggtccctcc 168180
ctgtaggagc agcgcagctt ggggtctcct ctgtcgaagc acgggacaaa tgagctggcc 168240
cagatcgctg atatttggga ggacgaaggc cacatggctg tgagactggt ttccatgaca 168300
cctgcaggac cagaggtaga gcatcccctc caaatactga cgcccctgat ccctcacccg 168360
ggctgtgtga ggacattcag agctccatgg gggacacagg ggccatccca gctgtgagag 168420
gcgagggccg cagggtcttg gccactgcct cagcaggagc ctcttgatgt ggctttccgg 168480
atagaaaaca cagctgcctc tccgctgaat cgaccagaac gagttttttc agggtccagc 168540
tcccgagtgg atggcaccac atagagagca tcccgagtgg gtggctttcc ttgcaggccg 168600
tttctggaag acgacaagaa ggaagaaaga ggccatgctg tctttgattt agccccaggc 168660
acgtagcagt ctctccctgg agttggcgtg tgggaccagg gactcgggag tggggtgttt 168720
acagtacaga gtgtcctgtt attcctggct tcccatcagg acgtgacact gcactccaaa 168780
gaatcaaaac ctcaaagaca aacgaaatta ggatgttaac aacatgggca ccagtgaggt 168840
gctttaggat acctttaact tactacctgc ttcccaccat cgagtcctga gccgtgggct 168900
ttgtaacgag ggcatatgtc ttgctgtgga tgaacctcga gcctcagtgg agggcagatc 168960
ctcgtgtgag ggaatggccc tctttacccc gagcctgcta cccagtgcaa ggcctgggct 169020
gtgagccttc ccgggtgtta gggagatgga acataggact atctcagtgg gagcatgctg 169080
tttctgataa ttctgttaaa ttaataaaat agaaataaaa atacttattt ttttaagatt 169140
 
tttatttata tattcggcag agagaacaca agcaggggaa cagcaggcag agggagaagc 169200
aggctccctg ctgaacaggg agcccaatgt ggggctcgat cccaggaccc cagggccatg 169260
acctgagctg aaggctgatg cttaactgac tgagccaccc aggcgcccta aaaatacttt 169320
cctaacatga tgaaagtatc tctcaaataa tagtcatcag gatatattaa tgttagaaac 169380
aagaagagga tttctatcat cagcactcat cccgctaatt tcgctctgct ctcgatgggc 169440
tggccagagc gattagagaa ggcagcagaa taaatcccgg agatacaaac ctgttatctg 169500
cagatgtgaa aacccaaggt agttctccgt aaagccaagg taaaagagaa tgtagtagtc 169560
tggctaatta ccaaaaaata taaaagaaaa atcagtgtcc ccttcttaga cattggaaat 169620
aatcaattag aaaatatata ggaaaaataa ggaattagaa aatatatagg aaaaataatc 169680
aattagaaaa tatataggaa caaaacttat ttacaataaa aatacgaggt acccagaaat 169740
atacaaatca gaaatgtgca aaagcactat ggagaattgc acaaaacatg gtggagcctc 169800
acatgaaaat gttaaataaa tagcaagtga tgccgtatgc tcgagattgc acaagcatct 169860
caatggccgg agaatcagcc aactgggacc ctggtgagat tcccaaggca ctttagggaa 169920
catgagataa tggtacagaa aaacaaaaat gtaaggtaaa gcagttcaaa taaaaatagt 169980
aaaaaggcct tcgttccttg agatcattta tttcagtgct acgttaatta aaataaagcg 170040
ggccctgcta ttgtggacat tgctgctata aacattgggg tacatttgcc ctttcgaatc 170100
aatcgatcaa tcaatcaagt gggccttcac agattaatca atgaaacaac atggaaaggt 170160
ctaaagaaca accaagggta acagttccta ggacgataaa ggtaacattt cataccagta 170220
gaaaggtcag ttgggggaca gctagccacc ggcaggggtg agggttaacc tcagattttg 170280
aagtgagatg ttaaaaatgt aaaatatgta actaggaaaa ttcctgaaag agaatatagg 170340
taaatatgtg tatggtcttg aagtgggggc tcagttggta aagcgtctgc cttcagctca 170400
ggtcataatc ccggggtcct gggatcgagt cccgcgtcgg gctcttgctc ctcggggagt 170460
ctgcttctcc ctctccctct gcctgccact cctcttgctt gtgtgctctc tctgtctttc 170520
aaataaataa ataaaattca aaaaaataat aatcttgagg cccctggtgg ctcagtggtt 170580
gagcatctgc ctttggctca gggcgtgatc ccagggtcct gggatcaagt cctgcatcgg 170640
gctccctgca gggagcctgc gtctccctct gcctgtgcct ctgcctcccg gtctgtatct 170700
ctcatgaata aataaataaa atcttaaaac aataataata atcagtttcc agtggaagga 170760
cttacgtaca gcaatgcagg cagaaatcac aacagaaaaa aaagagaaaa aaaaggaaaa 170820
aaaaaaagaa atcacaacag aagttatcag taagtattga ctaaaataca tatatatgaa 170880
atttaacaga aaatatttat ttttattttt ttaaagattt catttatttg agagaaatgg 170940
cgagagaggt ttaattttta aaaaagaaga aacagggatc cctgggtggc acagcggttt 171000
ggcgcctgcc tttggcccag ggcgtgatcc tggagaccca ggatcgaatc ccacatcggg 171060
ctcccggtgc atggagcctg cttctccctc tgcctgtgtc tctgcctctc tctctctctg 171120
taactatcat aaataaataa aaattaaaaa aaatattaaa aaaaaagaag aaacaattta 171180
gtgtaagtgg agaggattca tatccttcat atgtaaggag cttcttagaa atatgagtaa 171240
 
gttttccaaa tgagaaaacc ccaaaaagaa aaaaacaaca aaaaaaaacg atgaaatagg 171300
aaaaggatat gaagaggaaa ttccccaaat aagtaatgca catatccaat aaacctgaag 171360
aaataccagg agaaatacag actaatcaaa ataacgtttt cacgtatttc caaagatgaa 171420
cgagaacccc aggggtcagc ccggctgtgg ggcgcaggca aatgcagggc tgattagagg 171480
gataaatcag ttagaccttt gcggaggcca gtctggggca acataaccaa accctaaacg 171540
ggtgcccgcc ctttgcgcca gcaagctctt tgctaaaagt catgccacgg agagaagcaa 171600
gagaggaaat tcctcacaac aaggatccta agagtgaaga actagaaaga ccgtaacgtc 171660
caaaattaca agctttggct acataactga cttttaccgt gttaccgtga atccaacaag 171720
gcatcgcctg gcgcccattt aaaataaagc tgcagcaata aatcttcagg accagctcac 171780
gaggtattaa agtggaaaaa gcagactgtg gaaatgtatg gttatatggc atttcatttt 171840
tttaacatgc acggaaaagt ctaatagtaa caggggctcc gcgcttcttt tttttttttt 171900
ttaacatttt tttctttatt tatttatgat agtcacagag agagagagag aggcagagac 171960
acaggcagag ggagaagcag gctccatgca ccggaagccc gatgtgggat tcgatcccgg 172020
gtctccagga tcacgccctg ggccaaaggc agacgctaaa ccgctgcgcc acccagggat 172080
cccggctccg cgcttcttgc tctgctccac cacgctcgtg cctgggagtc cagcctcttc 172140
tggttttgtt ttggtttttt tgttttgttt tgtttttttg gggttttttt kgctgatcct 172200
cctgcccccc acccccaccc agtattcatt ttcrgggggt ccggggcccc cacctcagtt 172260
ctcctctgtg cacaccccct ccctgaggga tgcgcacctg ctccacggtg ctaacaccct 172320
ccagacctgt gccacctgtg gggcaggaac tgagctacgt ctgcccacca gggacgcgac 172380
ctggagccac ctgcgtctgt taagcacttg aaatgtgccc gatgcaaatg ggaactgact 172440
ttcgtctttt tttttttctt cattataact aaacatagat agccacatgt gccgcgtggt 172500
gactgtattg gacagtgcag gtctagacag caagtcccag ctgtatgccc tcagccgaga 172560
actttcctct gaccccacag aagcaggtaa atttgtctgc tgagcgcctc agctggtttt 172620
tttttttttt taagatttta tttatttatt catgagacac agagagagag agagagagag 172680
gcagagacac aggcagaggg agaagcaggc tccatgcagg gagcccgatg caggacttga 172740
tcctgagtct ccagggtcac accctgggct gaaggccgcg ctaatccgct gagccacttg 172800
ggctgccctc agcggggggt ttaatcagca tctctaaccc agcgtgtcta aggcaggtct 172860
tgatttcccc ccccagggtc cccacttccc cgtcttggag tcagcctggg ggccgaggcc 172920
aagtgcccag caatcacagt catttccact cctcccctct catcccacat ttcacacccc 172980
cacgagcccg gttggctcaa ccctcagaat aagtcccagc tgtggctcac cagccacgcg 173040
gccacccggg ttcatgccac gccatctctc tgctggacgc ctcccctcca ccctccctgc 173100
cccgttgtct tcctccccgt ggcccccagg gaggcacctt tcctcccctg gtctccttgg 173160
ctccctcctt cacatcacgc gcatcccttc tcagacgccg cctcctccaa gaagcctccc 173220
cgggtgccct ttctaaaaca gtcaccaccc tcccaacact tctctctgct tagcctgctc 173280
 
gatccccctg cacggccctt agggctcatc tgttatccta tgagtcgtgc acttgctgag 173340
tagaatctgg ctcggcaccc gtgtcaggcc cagcgagggc tccagtctgt cactagcacc 173400
cagcacagtg cctgaccgtc ggaagactcg cagctgtctt gagtgtgcac agatgagcaa 173460
cgtgcacttg gggctttgtc tgtgttgcat cgtttacccc ctagaccagc ttgccacgcg 173520
gcttctgtta ttatctgcat ttccaggaga ggacgctgag gatgggccag cgtccccagc 173580
ggcgaggaat gtccaagaat gtccagaagt cgctaactgt gggactgcgt gggggagggg 173640
agatgttgct ccgcgtcgct ctgcattatt gctcctaagt gacattcgtc ctcgttactc 173700
gggtatgaaa aagtcgtagc tacagaaaag gcgcacgtga tgttttcatg tcccgtaagt 173760
actgagtggc acctaagcaa ccccccttga agggaacggg caggtaatgg ccccgggtgg 173820
gtcttcagct gcctccgcct ggggaagagc caggcctggg gacacctgca tgctggcccc 173880
gacggccgat gaggtcctga gtttgcaggt tgctgcgctc ctgtcgatgg aggctcagga 173940
tggtggcctc tcgactgcgt ctgtgccgga aagggttgag ttatcctggg taatcccgag 174000
gtgcaggggg agcgagggcg ctcggcgcag gcagtgactc atgcccctgg gcgaggaccc 174060
agcttaccta aatagaacac cctgtttcca agggcactgc ccgaaaatca aatattaggc 174120
ctgtgaaacc ttcctcagaa aggtaaacag gtggagatgt ggatttgcga tctctgccag 174180
cagatggggg ctcctgagat actctctgaa agctcccccg gcatgagacg ggggcgaacg 174240
tggctcactt taaaaatgct gctaccgggg gacatctggg ggggctcagt ggcttggcgc 174300
cagcatttgg cccaggccgt gaccccgggg tctcgggatc gagtcccatg tcaggctccc 174360
tgcatggagc ctgcttctcc ctctgcctgt gtctctgcct ctctgggtct cacgaataaa 174420
taaataaaat cttaaaaaaa aaaaagatcc acatacttgc tcttcagtta tcattatgct 174480
acactgtcct gttcagttgt gactgaccgc cctgcttctt tggggtgccg tgctttccat 174540
acttcagcat cctgctcctg aggctcatcg gtgcctgcag gccccgacac ccggttccta 174600
gggtcacacc tggggtccgt ctggagcacc cctgcaggtt aatttctcag gaagggcccg 174660
ggctgtggag tttacatttg ggacgctctc ggctgggcat gatatcccct ctcgcacaac 174720
ttgccgttcg gaactctggc acaataacgt gcccccccgg agacgcccgc atcctgatcc 174780
acggggcctg tgattatgtc aggttatgtg acaaaagggg gatccgcttg ccgattgcag 174840
ttaaggctgc tcatcacatg gcctggactt ggggagatgg tcctgggtga cccggctggg 174900
cctggggtga tcgcaagagg ccctgcaaga agaaaggagg gacgcaggac agtgtgagag 174960
ccagagcgct tcactgcgag gtgcaccccg cccgccctgc tggctctgac ggtagaggaa 175020
gggccagcga tacgccctcg ggacgctgga aagagccaga gcacagagcc tcccccacgg 175080
cctcccggga ggctcacggt tcctgccaac accttgattt tggcccagtg acacccacgt 175140
cagacttctg acctccagag ccatcaggta aaaagttggt gttattttaa gccacgaaag 175200
gtgtgggcat ttgctacagc agcatccgga aactgccccg tggcttccag ctctctccac 175260
ctgccggccg acccttgctc ccccagcgcg agcctgccgc gccctcaccg agcgatgcca 175320
gcagcccgcg tgtgtgtgtg gcctgcaacc cagcacctcc taatctgcag atctggctct 175380
 
ttttccctcc tggttaggag agttttcatg gtttctccca gtgatcaccg cttccgaggc 175440
atggtcaaaa gtcacagcca accagggtgt gcagagaaga gcagttcatt tattagccat 175500
ctaaggtcgc tcgtgagccc ccaccgcgcg agcagaagat gcttcagctt gttggtgtct 175560
ggtatccgag accccgctcg ctggcacgga cccttcctgg cttaggcctg tctttcagag 175620
ccccccatct ccacaccccc tgcttttggc cttcgtgggg tggacggggt tgctggggtc 175680
ttgattccat acaccgccac tggttcgtgg agtgtcagtc aggtcagcta acaggatggg 175740
gaccatcgct cacgtgtgaa ttgtcctggc cgggcgttgg gagcccctgg cacccacccc 175800
tgagatcctc gatcccagcc tcgcatgctg cggcctcttt gccccaacct caggcctccg 175860
ggtccccagc ccctcgtgct gtccggcacc taaggatccc ctaacgcagg aggcagagag 175920
cagaagccct ggtccgagct catccctctc tcagtggctg ctggggcacg ttggtgctgt 175980
ggccaccgcg cgctcctccc tagctgaccc tggagggtgc accaccgacc cctgggtccc 176040
aggctgtccc caggactcat gcgtttccac tctgcttccc tccagattcg cccctaaggg 176100
gctggggagc aaggacacgc gggtctgact gcctcacgtc gcctccagtc ctcctccctt 176160
ccacacccag ttgttacctc gaggggactt cgctgccccg tcccatcttc aaagggcttt 176220
ccttgcaccc gaccccaggc cactacacct ctcttgagga cgacgggcat ccgccctcca 176280
ctctgggctc ttgatgttgg cgcccaggcc gagctggagg ccggctggca gagcaaggtg 176340
ctgtgggttt gtggtcctgg gggcacccct cgcggcactc ctccgtctgt ccctgattcc 176400
acggccgccc cctccctcct cgtccccgtc ccttcagccc ttggctctgc gtctggggca 176460
gcagacttgc ttccgaatca gccgttctca agcattttgg cttcaggctc cctttacatt 176520
cttgaattat cgagaacccc aaagagctta cgttcatatg gattatgtat gtcggtgtgt 176580
accctatcag aagttagagc cagggcagcc cgggtggctc agtggtttag caccaccttc 176640
agcccagggc ctgatcctag agacccggga tcaagtccca cgtcgggctc cctgcatgga 176700
gcctgcttct ccctttgcct gtatctctgc ctctctctgt gtccctcatg aataaataaa 176760
taaaaatcta atacataaat aaacaaacaa acagtaatgt tttaaaaaaa aaagaaaaaa 176820
atgagagcca ggcatttcaa aaacagtcat ccattcactt aaaaataaca gcagtaaacc 176880
gcttacatgt taacataagt aacgttgtta gggaaagtaa ctttttccaa aacaaatgag 176940
gagactggca tgttctgtgt ttttggaaat cccttcagtg gctggggtaa tagaagatgg 177000
ttgaattgtc gtcttgcttc tgttttgaat gtgttgtaat atgttgtttt ggctgaagga 177060
tttgaagaaa acctgtcctc gtgcagatac gtagttgggg gggaagggca tgtttcagtt 177120
gtcttcccag atgattctgg acatcgtgct ttgatactat gtgggaaccc agcagcaact 177180
ggtgattttt tttttcaaga ttttatttat ttattcatgg gagatacaga gagagaggca 177240
gagacacagg cagagggaga agcaggctcc ctgtggggga gcctgatgcg ggattggatc 177300
ccaggacccc agggtcacac cctgggccca aggcagatgc tcaacggctg agcccccagg 177360
tgccccctgc atggtggaat ctgaaagcaa acataacaat gaacttttcc ttccccgtta 177420
 
ggttaaaatc cattactctg tcctgcactc gaagtgcatc ttgtatccat gtgcgatttg 177480
gtcgcaccat atacagtttc caaatggtga cacatttcat ccgttacccc cccaaaaaaa 177540
tcacacgtga tggacgctgt caagctcaca gtggcagacc tacatttccc gacattcttc 177600
ttcatgcttg aaagcttgag ttttgtcatg gccacaaacc ttgtcacttg ctttcctcga 177660
ggtgacgggc tccctgtgtt catttctaag ggaacgtctg acaaacgccc aagtcagaat 177720
agttcatctt ccagttgttc ttcgaagtga aaatgaggct tcgtgaaaaa agaaaaattc 177780
tcgttcacgt tggagctcca gcagtggcct gcttccttgc accaagacct tgggggtggt 177840
tcggggtgta gcagaaatgc tttgagtgca tactaattcc ttcacgccgg atatggaaaa 177900
gacgtgctca agggtcaagg ttgagtaaga tgaacagctg ttactgcagc atcaaggacc 177960
tacctttgat caggacacgc ttcatcaaag acatgaccat ctttaccttt gtggcacgta 178020
ctgcaagggc tgctgggacg gtctgcacct ctgccttgac ccctgctaag gtcccgccgt 178080
tgcttctgca ccactggtgc aggtgccgaa gagcaaccga gagaagtacc atctcagcat 178140
tgttactaaa atagtgggga ccaagtggag cctctgagag ggtctcaaga gcctccagca 178200
gtccctggac caccccccga gaaccactgt tcttagtcat gggacttaag atgctgcgat 178260
ggtcattctg cccgtcttcc tccccgtcgt cagtggccgt cccttgccat ctttgtttgg 178320
gccgcctctc ctccacccca gcgaaccagc ctgtggtctt cccatctcag cctggttcct 178380
tccctcccgc ctcctctctc tgaatcccaa ccacactggt ttctagtctt ctggttctga 178440
ccgtcgaccg catgcagagt gtcgcttctc cctgagggtg acgggaaccc acagctgcac 178500
ggggtgggct tttgcctgcc ggcgcaccga gtggagcgac atctggtcaa cccagtgttt 178560
gcaataagcg ggatgtgcgg agggccctgg cccggctcgc ttcctcctcc tcgtgcagct 178620
ccgggtcggg gtgactgccc cgtggctggc aggcctcatc tgctgctact gtgctggacc 178680
cgcgtggttc tctccttccc tgctctccag ccacgacgag gagaagttcc gctttagaga 178740
atctgaggcc gagtgtaatg atcccttccg ccgtagtcag ctcctggaat acgtcggtgt 178800
ccaggcgcca gctgcggggc ccctatttca gaggacttcc cgtcctgggg tgcagagtgt 178860
cctgtgggtc cccagccttc ttcctgctgg gctgggggtg tgcttctgcc tggatacaga 178920
atgagggaga gaggcaaagg ggtggcctcc agattttctg gctgatgcag atagagtccc 178980
aggggaaacg gaggaggcta ggtccgcccc acccacgtgg ctccgtgaca cctctccccc 179040
tggatagccg ccagcattag gccatgtttc cttggttttc aaagctgaga tgagagcttt 179100
gtttgtgggc ctaaattccc caagaatgag gagcgggaag cccatgaagt ccaggaaatg 179160
ccacttaccc ccacatgtgg tgtcccagag cttcccagaa ccgctgcctt gtctccagcc 179220
tccacctgct ctggggtgtc ctgctcagaa accacaggcg cggtgttccc acaagacggc 179280
ggcacccagg gtccggctga ttcctgccac taagattgca tcaagttgat ctcgtctaaa 179340
actaaaaata cggcacttgg cagacattct ctagaatcca gaaccaaagg aatgtgtccg 179400
tggtgtgggg gtacacttcc cagtcgacgc aatttggcga gcacgccggg tagatctggg 179460
ataactgcgg ttttcccggg acaccctggt gctggatgtt tcctgtgctg gacgccctcc 179520
 
aaaccaggct attgcagcct tgctctgtgg gaaccaaact tagggagggc agggcggggg 179580
gcagctcagc ttgctcccct gctgcaggac cccaaaactg cttgtgagga ggtgcctccc 179640
atcccagtcc ccaggaccct cgtgacctcg ggggagtgca gcgacccatc actttctctg 179700
gagggtgcca acccaacccc cacctacctg gagctgcctt ccttgcaggg ccgttagctc 179760
acccagggtg cagggcaggg gcgactggcc ggggcctcac agccttgccc gtctatgagc 179820
ttggtctctg ccttccctgt ggcatctgca gagcactgac cagcccgtga accacttgga 179880
tttttctctc ctctaatctc ctctcaaaca attagactgg gcaatagatt ggtttgaaat 179940
aaccatgtgg cctgaccagg tgcaccccca grmccccacc tgcccccggc cccactgggc 180000
tccccagggc ctctcacctc atcccccgac acacaactga ggaaggttgg atgctcggtg 180060
ctaatgactc aggtcctccg gggaaacagg gtgccgaatg aagcacagtc tccgagctct 180120
gggctggrca tcaggaaagc tgcgggttcc agctccgagc tggagttcgg gacccctgtc 180180
cttcctctat tagtatgagt tagctgaggt ggtgcaggca gcggcagcaa ctgtcccagc 180240
ctgtgattct gagcctcgat gggccgcggc cctttggtgc tggatgacac tcctcggtag 180300
gcagcctgga aaaatgtaca attgcaaata gtcacagaaa tgttgaaatg cgattttgtt 180360
tctataaaaa taaagtattt cagttccagc ctacccagac tgcacgtgat tgaacccaac 180420
ttattgggct tccccctcgg gaaagtggga gcattgagct ggcgacttta ggtttctgga 180480
cacgagaaag atcctgtgtg tgtgtatatg tgtgtgtgca cacacatgcg tgcaagcatt 180540
taaaacctgt tgcagggacg cctgggtggc tcagcatttg agcatctgcc tttggctcgg 180600
ggtgtgatcc tggagaccca ggatcgagtc ccacatgggg ctcaccgcaa ggagcctcct 180660
cctccctctg cctgtgtctc tgcctctctc tgtgtctctc atgaataaat aaataaaatc 180720
tttaaaaaaa ttaaaacctg ttgcaaaaac aaggaaggtg tctaatagat ttcctgtttc 180780
tgatattttc ctaagggggt tgaagggcaa gattaagaag gaaagctccc agcgggagct 180840
gcttgcggac tccgcgcacc tgaatgagac ccactgtgcc cgctgcctgc agccctaccg 180900
gcttctcgtg acccccaaaa ggcaatgcct ggactgtcac ctcttcacct gccaagactg 180960
cagccacgcc cacccggagg agcagggctg gctttgcgac ccctgccacc tggccaggtg 181020
agccagtggg tgttagggcc tgacccgtcc cctcggtggg gctggctgag gaagggcggg 181080
cacggacgtg tgctgcgcgg ggcagtgccg cgggcctgag ctccgtgtct ccgcagagtt 181140
gtgaagatcg gctcggtaga gtggtaccac aagcacctga gggcccgctt caagcggttc 181200
gggagtgcca aggtgatccg gtccctgtgc ggacggctgc agggtgcagg tgaggagtgc 181260
gcctctcccg ggggctcttt gtgttcccag gcatgtttta tgttttaaga ccaagcagat 181320
cctttggaat gtgagtgtgc acctgcctgt ccgtttcagg tgttttaggc gaggttggct 181380
cctttccgtg ggatgcaaac atctgcaccc ccctggcctc gccccctggg aagtcctgca 181440
tgctcttgac acgcccacta tttgaaatca tatttttcca tgctttctag caggttgaga 181500
actgacagct aaggtctcaa actccgtggc cggtcgaata gccaggcgct cgcgggctaa 181560
 
tacgacacat tctccaggcc tttcctcccc gggagcccag tttggggcct aggctttctc 181620
ttttcgggaa atatcccctg tgcatctgat gattatcaag actcaggggt cctgatggta 181680
acgcagcagt gtgttttaat attttgaatc caatatttat tccataatga acttgaaacc 181740
tagactgtga tcttgcacta atcgtacgtt cacctgaatt gtccactttt aaggtatttg 181800
ggaagggaga tcatttcagg ggaaggacga agtttggaaa ttgaacagag atgactgagc 181860
agtgtttgca ggggacaagt gccttgtccc tgccacttga gttccaggct ctgccattaa 181920
gcctggccaa gcatttgggc tggcacagcc catcaggctg gtgtccgggg gcggggcgcc 181980
ttgagtctct ccaagtggag gaccaatgca agggcccgag gcgctggagc ctgtgcacgt 182040
taagagcaga tgctgtgctt cttggcttag tgtcctcatc acatcccagc ctcaggcctt 182100
ccagggaggg gccttacatg tgtcttgtgt cctataaagt ctcacattga taagcaggat 182160
ctgaagttgg gcagggccac ccccagagca ctagggcttt cactggacca ggtgcttctc 182220
aggtatctca gcacattatt gaatttagga caaggacccc acccccaccc cagaggcccc 182280
ctcagctgtc atggctcgta gcacctggag cagggaacca gggggtgaga ggcttcttgg 182340
ttcttggggg aagaaggcac aagataaatc gtccgaatca gaacattttc aagagtaaaa 182400
ggaggctctc ctggtaactg tgctgaggca acagatggga ccacggacac gggagccgct 182460
gccacccgat tctttaaggc tgtgtctgtc tcagcctgga gtccgtcagg aaagcccatg 182520
cccccctgcc cgctcgggca gccaggagcc ggccgagagg gtctctccct gcaaccccag 182580
gagctgagtc cggccccgct gcaggcccga gggtgggaac ctcagggagc gcgacagaaa 182640
gccgcacagc agagcgttcc agaacctcaa ccccgaaggc ggctccgttc acagatgcct 182700
cacgggatcc cctgaggacg tggcctccgt ctgggaggtg ccgatctcga tctccaggca 182760
ccgggtgggc tgcgggaaag gctgtgggat ggtgacaccc agccaggccg gagccccggg 182820
gccaacccca gccgggacct gccgatttca gttgtcacga ctgagggcca cgaggtggcg 182880
ctgtggggca gcaggaacca ggctgcatgg ggtcggggtg gaggggggca ccctgcgtcc 182940
aggggtgatc tcactgagtc acggagggtg caggggagca aagttgcgtc ttttttctca 183000
ggtagtcaaa ctgaaaaact agaagtgatg gaggtcactg tttagagacc agccagggcc 183060
tggtccaggc cggtcccctg gggtcagggg gcaccaggtc ttctccgggc gacccagcag 183120
agccacgggg ccccgcagca ggagaccgtc cagggccggg agggagggga gaggacctgt 183180
cctcgggcac ctggtcccag gaggcagtcc cttcccaccg cctcttcttt ggtcccaggt 183240
tcgcctgaca gcatgcgcag ctcgcctgac aaccacagta agtgcctcgt gcccccgggt 183300
ggggtttgtt gccacctccc tgtcaagggc gcccgtgcac cctctcccgc gacagcagcg 183360
gggaggcctc tgggctttgt taagaatcac tggctctgga gagggtgggg gccktgcaaa 183420
ccctccccag cggggacacg cgcaaggagc tggtaaccag caggggcgca gggctcctgt 183480
tcctgttcgg tcctggggac aggccacgtg cgtgtgcata cacaagcaca ctcatgcatg 183540
caaaccgtgc acatcgtgca caagcataca cgggagcatg cacacaacct cacatatgca 183600
catgcatgca cgaatacata tgcacacacg ggcatgcacg ctgcacacat gcacacacac 183660
 
atgcatgcac actcatgcac atgcacgctc aggggcatgc acacatgcac atgccccagg 183720
cctccggaag ccacgtccac atttgctcag ttcagctcag tgtctttcct ctgtctggtg 183780
gtctagggtg agtttcctca aggctcactg tccagagagt gaagataatt acccggtctg 183840
tccgctgcac gggtctgagt ccaggggacc cagaaaggct ttcagggtgt tctcaaggac 183900
actaaatgcc ctgctgttca cacttgagac ctccccccac ccaaaggcac aaggagtgag 183960
ggccggggcg gcacaggctg gggtgtgcag tggcagctcg tgcagggccc agccccacag 184020
cccaagctcc tcattgtggc ccctgaggga aggtggaaga tccatgccct aaatcccaag 184080
agtccatttc ctggattcct ttctggtgac ctaacaggag accaggggcc aggccagcat 184140
gagtgggaga acagagggcc agggccacct gggtgctcag gtgagggcct ggccagggca 184200
gagaaagggg gggcgggatc agcagatgaa atgactgaga aaacatgact tagctctggc 184260
ctccttcccc caccacaagg ccagggggtg agattgccca agaagcagtc caggaatgga 184320
gggcaaatgc ctgaaggtgt ggccctccac aaaattgttg caaaggaata gtttcctgct 184380
gggattcaga atgggtctaa atgatgcttg gataggtctc tgtgaggcca gcagatgagc 184440
ttgcctggat ggatggatcc acagcgggta gatgggtgga tggagaatga atgggtgggt 184500
ggatgagtgg taggtaggta gacgggtgga cagatgggtg gggtagatgg gtgtgtggat 184560
gggctgtaga tgggcggggt ggttggatgg atgggtaggt aggtggatgt gtgggtggtg 184620
gatgaatggt ctgcccccag tctttctcty cccgggtgga gtcatgcagg tgtctgttgc 184680
aggtgggccg gagccaagtc ctggcgaggg aagtggagac agtgaacaga cagatgagga 184740
tggagaactg ggcacagtgg cccaggccca gcccctcggc agcacagtaa gtcctcccca 184800
gacatcccca ctgccaaacg ggaggcttgg gtcctgattt cctaaaaatc tccgtgctct 184860
ccacgactct cctgggtgct tcagcatgag cacaagaccc cgcctccctt gccgtgcctg 184920
gacagtctga gactgagcac gaaagaggag ctgctttcac acgtggtctc accataagaa 184980
accccaaagc aaggtgtggg gttggcagag gctgcagaga agcctgcctg acccgtccag 185040
ccagagtccc gggggagggg gtgggcacgc ttgtgaggtt gtgagggcag gcaggggtcc 185100
cagagcaccc tgcatctttt agggaagggg accccgttta gactccttcc ctgtgaacat 185160
tttacttttc cttataggtg accacgagcc ttttattcgt gttggtctcc atcttcattt 185220
attttatgac tttttttaag attttactta ttcatgagag acacacacag agagagagag 185280
agagagagag agagagaggc agagacacag gcagagggag aagcaggctc catgcaggga 185340
gcccgatgtg ggatcaggcc ccaggccgaa ggcaggcgcc aaaacactga gccacccagt 185400
tttatgccca ttttatgact tatttttgat gatagatgag caaagcacgg cccagcgttt 185460
ctgggtggga tgacggaagg gggcaggttg gggcaggggt ggatgatgag tttagtcggg 185520
agacacgggg aggatgctgg ggtccttgag actcggcacc ggccagatct ggccagtcta 185580
cagcaagaaa attgtattcc tgccactctg agcttgaaga gtactagcca gtgtccggaa 185640
cattccctat gcgatacctt taaaggatca agatcgtcgt gggggaggat ttcagtgggc 185700
 
tggtgctctt catctgaaca ccagggagct ctcctccaag tctgcatccc tgtacagcgg 185760
gaggcccctc atttgcatga accgcccccc cccccatatg cacctctgaa gggagagaag 185820
cagccatcag ccccggagaa gaccgccgcc ccctccccgg ccccccaccc gacacccgga 185880
ggcctcagca ctccggggcc tgtgtcctca gcacttctga gcagccgcat gcccctcggg 185940
ccgagcaggt gtttccttct gctctaagag atgctgagga tgcagatctt gcrgggaatt 186000
cggggcaggg gggcctcygg accccttgga gctccttcgg atttgtttct agtgtaggag 186060
gtagatgagc ctctggggac gcccccctcc tgctgcccag ggccgagggg cccccggtcc 186120
tctctgtgag gctgactctg actctcctcc tcttgcccct gcctgcacct gtgaagaaaa 186180
agcgcctctc cttccacgac ttggactttg aggcagactc tgacgactcc acttggtctg 186240
gaagtcaccc cccccactcg tccccagtct cagtggccac agacagcctg caggtcagtg 186300
ggctcatttc tggcccccca gccttcccag gataacctga gcgacaggta cgtgggcccc 186360
aggtggggga cggggcgctc tgggaaggag tccgatggcc atagcaggct tcggggcacg 186420
acaggcctgg ggagagtcag ggtgagggca cagggtccag aagcttctca ctccccatct 186480
ctgcagagac ggggctggca gcttggagaa gtggtgtacg ggatgggaac gtgggaaccg 186540
ggctggacag atgaggggcc cagggatttc ggggtgactg cccatcccag gaataaggtt 186600
tctaccccgt gactcgggag ggcaggcccg aggaccggcg tctgctctgg agtcagggcc 186660
tcacgtgtgc ctggatgtcc atgcagtggc accaggccag agctggggtg gggatgcctc 186720
tgcccaggcg gatgcacaga ccactttggg ggcggcgggc tggctcccca gacgcctttg 186780
ggccacaaga ccagcccctg cggtctccac ttcccgtggc ctccagggcc tgacgactgc 186840
ttccttcctg tcccactcac gctggaaaag agccctggag catgtggctt ccaggagccc 186900
tctgcacctt gtttccttgg ggacaatggc acaagagaga gaccccggcg ggtagaagag 186960
gtgctggggy ggcacccagg ggcccccggg agccccaccc acactcccac tcgcttttct 187020
gggagtatta gggtgtacac agaacaatgc attccccaaa aacagtcacc tccccatcgg 187080
cctccttggg gctttgggct gcagctgagt cggagccccc gaggctgtca agtgggtctc 187140
aggtccctcg tgtgatgagt ggcctagggc atcgggaacc tcagcccccc acggaygcat 187200
gatgcagagt agtcagcaag cgggttcagc gggatttctg aaagcaaagg gcagtgagtt 187260
gcgaggggcc ggagctgagg ggtgtctggc gtttgctcag atgatgcctg gaaaccctcg 187320
agactgggac tgggaaggac ccacgtgcca aggagctgac cctcctcgta aaaccgcagg 187380
tcccgtgtgc ccaggccctc acgcatggtc ctcgtgcaga ggacgcctcc caggaggccg 187440
ccgtcctgga ggaggcggat gttgatgccg ctgggtgccg tgcgcatcca gaagagcaga 187500
tggacagcct ctcacctgcc ggacgggacg ccctcgccga gccctgcctc cctgcggggt 187560
cctgcagcac agccgcagct ggtaggtccc tcgatgggct ctgcggggga ggaggcsggg 187620
ggggggggga gcctgcctgt tgccccgacg ggtgcagacg cgctggctca ctcatagcaa 187680
cccctgcctt cactctgact gtccaacacc cgccgtcctg gtggacgcca tccacacctg 187740
cccccacccc cccgtccctg cacccacccc accccgaccc cggagctggg tgcttctctt 187800
 
gtaatcctca gaagttccag cgctaatgcc tcaactggcc gcacagctcg ctgcggcctt 187860
tagggaagat cctgagctgt tttataggga caagcaggtg tccctgaagg gattaggatc 187920
accggtcacc tggaccgccc atcgtgatcc aggaccgggc agctccaaac ctggggggac 187980
ggctcccctg gcccgccgtc ggctcctagc tgggaatcgg gaggcctgtg tgtgccgaga 188040
acctggattt gctcggttta atctcgttgg cagacagtgt gcttcataat gaggtcctga 188100
atggctgtct tccctgtgcc ctgtgggagg gtgggttcag aagttcagag cccctactga 188160
tgcgggagcg gagcagaccg cagaggcagc ccgccgtccc gctaacgagc cgggggccag 188220
ctgcccggaa atgtggttaa gacactgtct gccaggaaat cggtccactt tccgtttttc 188280
cttttcatta tatcctaggg gccataatct caaggtgcac agggcagtta ggatgctgga 188340
ggcatttcgg ggggaacatg gggctttact gccccccgaa cagtgaataa gtgaacctac 188400
atcgaacaag tcataggatg gggatgtgca cagacttcat gcccccctcc ctgccgccac 188460
cgccgtgacc cctcaccacc ccgagcaccg gactgtgggg cggcgggcag ggcgggacag 188520
aggcccaggg agacggccgg tgcggagttc cgagcccagc cattctgccc aaatagcaca 188580
gtctccacct catcccttaa actgggggca tctcccagtg acctgtcggg gtttgacggg 188640
cagcgccgtc cttcagggtg cctcagacaa cctgcaggtg cggcgggacg cggcacgccc 188700
ctcgaccaac ccgcttagcc gttgtgactc agtttctcct ctctgcgaag agggattacc 188760
gcgtaacgag cggcgtgctc ataggcaccg cacgggatca taggatcaca accgtatcgg 188820
cttaaagccg cgcatgtcca ccctgtcgcc gtgggctggg agtccgggca gggctgcact 188880
gcgccctcag cacacggcgt cccgggctgg aacccgcgtt tcacggggcc ggagtgtccc 188940
tggcgcccgg ggccctcctg gcagcttgtt caggggctca acaggactcg tcgtgcccgt 189000
ggagtgcaga agtcgctgtg cgcggtgact tcctgccgca ggagctcccg ggcggcacct 189060
cccggcaagg gtcacccccg gatgcagctc cttgtccccc accaggtccc ggcgtggccg 189120
ggcgccgtgc catccaggct gcctggagcc ccgggaggga cctcttgggg aggccgtggg 189180
gacagtaagc cccccgcagc cggagcccag gacaagcatt tccaggccca cgggagggga 189240
cccgtgccca cagaaggtct ggcgtgagaa aaagcccata gtgacgagtg gaaggacttc 189300
ctaaagtcac ctctgctcgt ggaagctccc gggggccccg tccgcctgcc ccgagcagca 189360
gccctggggt gctgtgagcc tcctgcgcac acggctgtct ccctgcagcc caccgaggct 189420
gcgccttgcc tgaagctcca cccgcctttg ccttaagctc tgggctcccc ctccgcaggg 189480
gccgccggca cgtctcggcg gctgtaatct ctcctttttc tttatcgggg aaagcgtgtc 189540
cactggcttt ccgcttttcg ctctaaataa gtcgaaccca ctctcacccc aaagggacgc 189600
acgccatcgg gagggagccc ctccgctccc agcatctggc cgacgcggac atctccgtct 189660
ccgaagacga gggcaccggg gctccgggca ccacctctca gcatccccga cggggaggcc 189720
acactccggc tgagagccag gtaaggccga gggccaggta agggcaaggg ccaggtaagc 189780
gcaagggcca ggtaaggccg ggggccaggt aaggctgggg gccaggtaag gcgtctggga 189840
 
agctcctcct tgcagccgct tggggaggag cctttttagc ttaacatcaa gtcctggtgg 189900
agaaatcaga gccttcaaaa tacctcaagt attgaattta aaaccctaca tttgcacaaa 189960
ccgacgtgcg ttgcggtatc atcaaaatat ccatcgtttc gttgaagtga aatccacaca 190020
atataaaatt agccattgcg aagcggacgc tcgctcgcac ttaggacgtt cacagtgtca 190080
cgcagccgcc accacgaggg tcccaccaaa acgccattgc cccagaggga aacttcccat 190140
tagcgtcgct ccccgccccc tccccccagg ccctggccgc caccagcctg cttctctcgt 190200
ctcctggatt tgccctttcc gggctttcca cgtagacaga agcatcccct gcaggacctt 190260
ccgtgcccgg ctccctgcgc gcaggaatag tgctttgagg tccgtcatag tctagcttgt 190320
gtcaccgctt ggctcctttt gatgccggta ttcactctcc actccaccaa gtatcctcag 190380
gtcacctaca agaggaggac atgctccact gctttgtacc ttcagcagac tttacgcagg 190440
ttttaagaaa atttagaaga cgtaacacct gttaaattag tgagaccccc ctcacactga 190500
agactccgat ttagggaata aatcacaaga ttcctctgag tgccccaggt tcccttcaag 190560
gagttgaaac cgtggtcccg tttgcagata attggcttag gtctgagcaa gtacacggta 190620
ccggggggaa ggcgacagtg ctttctagga ggagagcagg accaccaggt ggatacagac 190680
gcaccgaccc cagtaaggcc tgactgccac ggagggggaa gtgggattgg ggctgtttga 190740
gagtgaggaa cccagaggca ggacaccgag cggagagccc ctgctccggg tgggggagcc 190800
tgaatgtagc tcagccaagg cgggtctcgg gcacgtttgt ctgcacccgg aggtgtggca 190860
cagagctcct cccgggaccc tccccatggg gtgggggtgt tggagggccg ggggatgcac 190920
ctatggagcc tccccaacgg ggacggagct ctgggccgtc aggtggaagg gctcaccaac 190980
atccagcgtg tccagaaagg gcgggaagca gctgctaatg tgcagtggtg ctttgctgcg 191040
gttgcacagg cagacggggg gacagggggc ccgcggggcg gcagggggct ggagctggga 191100
ggggctgtct gcagggagct aggccttgcc cgccccgtac cactgtgtgc gcccagacca 191160
cccagaggag cagggccgac aacccccccc caggcctggc tggccctaca ccggcactgc 191220
ctggggccat cggtgaccag ggggtggccc agtggggccc aggtttgctg agcgctctgt 191280
ccccatcagc ccgagcagca gcggatgtgc ccgcgcgggc caggacgtgc acgaggacgg 191340
cagggacggc tgagggtgag tctcagccga cgtagggcag gttacaggct ctgccaacct 191400
ccatcctccc ccgaccccag gaagtgatct gccaaaaaca agcagcaggc ttgcaggagg 191460
agcccgccct tcccgcgtgg cttttgagga accggggtac gtttttttgt tctaaagttg 191520
gttgggctga aaaacgtcag caggacagaa ttgtggagtc tttgggctcc gcgtctgtct 191580
ccctgcccgg agtccgtgcc cgcaggcaca gctgcctctc cttcctggca tgtcaccctg 191640
aagcatcctg caggaggagg tgtgctcgcc ctttgccctg agcacggcag ggtgggaaag 191700
gccctgagaa ggtctgcagg ggaaagccgt ctaccgttct gcacccaggg tggcgctcgc 191760
ttcttggggc ctgacccccc cccccctttt accgaatagt gaggaaaaca ctacacgggg 191820
aggtgacctt tgatcagacc gaggtgaggc ccccgggagg gctggggcgg tggtggaggt 191880
gcaataaggc ccgggcaggg acctcccagg gcaggccaga gccacggtgg gcagaggagc 191940
 
cccggtcaga agacaggaga tgaagactag aattgtccat ttgcagaaaa tttctttaag 192000
aggtggcaac tttagtaaca ttcagtaaaa cctgtgccct ggcatccagc cctgcaggta 192060
ctagctgccc ccgccctgtg agtacctgct gcccctgccc tgagggtacc ccctgcccct 192120
gccctgaggg tacctgctgc ccctgccctg agggtaccca ctacctctgc cctgagggta 192180
ccccctgccc ctccccaagg gtagccactg cctctgctct gcaggtacct gctgcccctg 192240
ccctgagggt accccctgcc cctcccctga gggtagccac tgcctctgct ctgcaggtac 192300
cygctgcccc tgccctgagg gtacccsctg cccctgccct ragggtacct gctgcccctg 192360
ccctgagggt acctgctgcc cctgccctaa gggtacccgc tgcccctgcc ctgcaggtac 192420
ccgctgcccc tgccctgagg gtacctgctg cccctgccct gagagtaccc actgcccctg 192480
ccctgagggt acctgctgcc cctgccctga gggtacctgc tgcccctgcc ctgagggtac 192540
ctgctgcccc tgccctgcag gtacccgctg cccctgccct gagggtacct gctgcccctg 192600
ccctaagggt acctgctgcc cctgccctga gggtacctgc tgcccctgcc ctgagggtac 192660
ctgctgcccc tgccctgagg gtacccactg cccctgccct gagggtaccc gctgcccctg 192720
ccctgtgagt acctgctgcc cctgccccac cggtaccccc tgtccttcga gcctccttaa 192780
acagttctca tctctgactc cacgtccacc aacgtctcac cttgcttgtg tcctgtgcat 192840
ctttgtgtaa aggcaagcgg gcagcccgcc cggtgcacca ttgaaaacca gggcagactc 192900
ttacagcggc cctggggagg gcccccggga gtaagggagg aagccccccc cacctgccca 192960
ccctcagaga cggcttctaa acccagaccg acagacacac cctggggagg gggatgagag 193020
ttctccagaa tgacgccgtg tcgcgcccgt ctgaatgtcc cgctcttccc tgttgcctgg 193080
ctccgcgctt gtagtcaggg tcaggacttc ctggatgagg gctgctcggg ggaggctcat 193140
ctacaattgc tcacaccaga gccccgcaca gcatgtgtgc gaaggggctt ccgtggggct 193200
gggttccaga aggccgggaa acccagccct ggcatgcccc ccctcccgtg gtgggcgtgg 193260
gcccgcactc ccaaaagccc cccccaaggc catcggtgct gtgctcagcc tgcagacgct 193320
gactcggttt ccccgcggaa gcctcacccg tgccaggtta tcgtggttca tttcaatcca 193380
cggcctaggt ctgttcccaa tttaaaaatg gtaaattttg gtttggttgc acgcaagctg 193440
aaagcaccaa gagctcatgc ccrgttttct gtcggcgtgt gtctaagtaa ggtgggaagg 193500
ctcacagtcg gagctcacac ggggcaggcg ccagtgccga ggtggttgcc gctttgggca 193560
gaaaggacag ggccaccagc ttcgtggagg tcgctcacga ggacctgaga agcaggcgtt 193620
ctgcttgggg tgggaggggc agtgccttga taccagatgg aaaaccatgg tggtggtgta 193680
tttaccgtgt ccgcgccgcc ctggggccct cctgcctcct cctcggagcg cccagagctg 193740
ccagggcctg gctcagggca gcactggacc cgcagctcat gcccgggctg ctgcccgccg 193800
ccctctccct tctctgtggc tccctgccgc ccctggctcc tgcctctcct ccaaccccag 193860
atgagagctc tccagctcct tgtcctggga tgcttttctt tcctttccat gctcgagagg 193920
acatttgaca gggacagtgc cgggtgctgg ctggggtctg cagtgacagc cacgtgtcct 193980
 
gcacgtgcag aggcagttgg tgagcctccc cccgcccggt atccatcgct gcctctggac 194040
tccccgggta gccagagggg ggctcgatga ccctcagaca gaggggggct tgatgaccca 194100
cagctgggcg aggggctgat gaccggcagg agggggacgg ggcttgatga cccgcagcca 194160
gagagggggc tgggtggatg acctgcaccc agaggggggc tcgatgaccc gcagccagag 194220
gtgggggagg ctcaatgacc cacatccggg gggggacaat gacccataac cgggggggaa 194280
cccgatgacc tgcaaccagg ggggctgctc gacccacagc cagaaggggg ctcgatgacc 194340
cacagccaga ggtgggggag gctcaatgac ccacatccgg gggaggggac aatgacccat 194400
aaccgggggg ggggggaccc gatgacctgc aaccaggggg tggctcgacc cacagccaga 194460
gggggaccca atgacccaca gccagagggg ggggcctcga tgacccgcag ccagaggggg 194520
ggctcgatga cccacagctg ggggggggac tcgatgaccc tcagccaggg tcagggggag 194580
gcttgatcac cgtcagccag agagggggct caatgacccg cagccggggg ggggccgatg 194640
acccgcagcc gagcccctcg tgttgacgtt tcccggctct ccgttcttct tggggcagat 194700
tcgccccctc ggagccctgg gtgcgtgagg atcgagctaa ttacctgccg aacaaagcag 194760
gaacatctcc ctgctcccca agtgccattt ccctttgaaa acaagcgagc cacagaggac 194820
gtaatggcag gaaagacgcc tctgctgtgc ttggaagcag aacggaggca tcgatttttc 194880
tgggtcccga tctgggcgcc gtggactccg gtgactcagg ggaagaggaa gtggtacttc 194940
cagtcactgg gaggctgtta cctggtgcct ccaggcccac ggtgacccaa ggccgcggcc 195000
agctaacgac tcgtagttcg agcagcctgg cacratcgat ccgcgccttc ctgtgcccgt 195060
caggagcgcc ggtgacagcg gctgcccagg ggtaatgacg cgtccgacgc ccgtcagctt 195120
cggcgtcgct gtcacctccc gcccccgccc tctcttctgg tcccactccc agctttcact 195180
tggtgttcca gaactctcgg gcatcaaaat gggcttcagg gccgcccgcc ccaggtcacg 195240
tgacccgtgg tggcctcggc tttcctgcca ctgcttggga atctggtgct tgtgcagagc 195300
tgggctgctg acggcggggg gcactccctc ggctcgggga ccccatctgg ggtcagaggg 195360
aagaactgtt ggtggtggaa gtgtccacga aggagcgctc agtgtctcgc tcaccgtggg 195420
tgggccttgg cctgggtgcg tcaggatcgg caggtgtgcc cccgccgcca cccctgcaga 195480
gcctggctcc tgagcccggg actggcccag cacgagctct tcgggccagc tcctctgggc 195540
agtggccgcg gacccctccc agagccccct gcggcccggt gcccacggat gacccgctga 195600
acgcggatct ccttgcccgc tgcagtgtct cgccggccgc gagcccaccg acgccgacag 195660
agaagaggag accctcagga ggaagctgga ggagctgacc tgccgcgtca gtgaccaggg 195720
cgcctcgtcc gaggaggcgg ggagcgagga ggaagggtca gacctggcca ggagcccctc 195780
ctcgcaggac ctccccgggg cagccccaga ggtgaggcgc cccggtgggc tcagcgggac 195840
ctgggcagag gtggggccag gcgtccagag gttgtgacga agccgctgcc cccacccgta 195900
ccccccccca ggcaggcagc ccacccccgg tgggccctgg gcccccacaa gagcggcagc 195960
gagtgcccag ccccccgggc ccacaggaag gggtgtctct gggatgggcc cgtcttgggg 196020
aggccttgcc gtgtctcctg caggtggtca cgttccatgc ttccctgtcc caggtgtgcg 196080
 
cggctgcggg ccaaacgcac ggacgggaca cgaccccctg gggccctcag gacctcgtgc 196140
agcccggcag gaccacagac gaggagctgc tgcagctgga ggacagagtg gccctgacgg 196200
cctgtgaggt ccagcaggtg gagagcgagg tagcaccagc cccaggggca ggggctcccg 196260
ggaggaggag tggcggccgt ggacagtcca cccgcagctc cggaacagga gcttggaaat 196320
gcccgcccgc ctgcagcctc ggtgagaggc aggtgggcag ggtgttcagc cccgcgggca 196380
tgatgctgac gcgtgccctc gccagtctca ggacgcgggg gcttctccag aagcaggctc 196440
aggtttcagg acagcccccg ggggtggggg ggctcaggac cagactctga cccgagggtc 196500
accctgggag cccaagcgtg ggcccaggac ctgagttccg gtgagcgttg tgtccgtagt 196560
ctacagaggt caaggacgga gccagggccc ctcggggaca gcctaggtga ctggggactg 196620
ctcccccctg gggacccgtg gagaagagag ggtctgcccc tcaggctgca aggggagcca 196680
gcacccacgg cccattctcg cttctgctcc taagcaagtg cccacaagga gtgaggagca 196740
cgtcacctga gctgtgcacc gagcacactc atcagggggg gacaagccag cctgacagcc 196800
aaggagctcg gttcttaaag agagctgtgc agacaggaag tgagggtggc tcagtggagc 196860
ccccaggtgg gctgcccagg aagggatagg acaggaagaa ggcaacacac atgtgcaaag 196920
gtcctggggt ggtcttgaac ttggcatgtt cgggaacaag cagtgaaacc ctgagccagg 196980
ggcagagggt gtgagggggg agagggtggg aggcaggtcc gagaggaggg tggggagcca 197040
ttggagggag cagaacaggg ggccttgttt ctgtttctag aagtttcctc tggctgccat 197100
gggagtcggg ggtgggggcc catttaaaat gatgccagaa aaaataataa taaaataaaa 197160
taaaataaaa taaaataata aaatataaaa taaataaaat aaaataaaat aaaataaaat 197220
aaaataaaat aaaaaagtaa tgccagaggc agacgccgac caagtctttg cccgtcaagt 197280
gtagccctgt cccttagagc tacagcgatg ccgaccccag tcgctgcccc tccttccatc 197340
cctggtggga accagaattc ctaatgggcc agaaagtgtg cagggccccc ctccctccca 197400
cctggggcga cctgcagcag ccccctcccc ggcagccacg tgtgacaacg ggctccccgg 197460
aagcgcatcc gctgggaccc ccgtcttaag ggaaccgtaa caatgcaggc gaatcggcgg 197520
ctgggtcggc acatctgaat gaggtccgcc tgtctgtccc tctttctgcc aggtgtccaa 197580
catcaagtcc aagattgcgg ccttgcaggc tgcggggctc acggtgaagc cctcggggaa 197640
gccccggagg aagtccaacc tcccggtgag tgcgggcgcc cagcgcgggg gctttgtgcc 197700
cggagaccgg ccagagcgca ggcccacccg gtgggcctgg aaccaagagg ccgccgcctg 197760
ccccgccaac ccatggccgg cccgggagct gtgccccgcc ggagttctgg ctgaagaaat 197820
gctttttaag gcgagcttgg cctttcctgt gagaacttgg gacgcatgca cacggcctca 197880
agtccaaaag aagcagcctg aagggtggcg tcctgcacgt gccgtcagag ctgggcgcga 197940
caggaagagc aggtcggggg cgggtctggg gccagtacct gtctccccag attcgggctg 198000
gggtccccag gcaggtcatg ccggtggggt agcctggcag gcaaacagag ggtggtccca 198060
ggagagcccc cggaggtcca gccgcagggg atacacggcg gggccagtgt tctgggagga 198120
 
cggccatcct tctgtcccgc gggcccacct gtgacggggt tggacaagca gcaagcaggt 198180
acaggcttcc tccttgtcct ccagatattt cttccccgac ttgctgggag ttacgaccag 198240
aggcctaagg atccgaatgc agagccttcg gacgaggtac gtgtcattcc aagaggaagg 198300
ctggcgcagc tcttccagaa gcgtgacgag tcccgggagc ggtggggctg aaatgtgccc 198360
tgtgtaagct cggccagccc cgcaggggac cccagggcgt ccccagaggc gtgcgctgag 198420
acggggctgg catcatcgtg ttggggaaat ccaggaaaac ccagctgggg aggcaggacg 198480
gtgggcagag gcctcggcct cagccttggc aaaccccacg cacggtgtct ttggctctgg 198540
gtaaggcagc gggagtggtt cagcatgggg gtgggaggcc gcacgacccc tcctcgcccg 198600
tcagtcacag accacctcac tccgggctct cccccgtggc cctgcacagg caggcgggct 198660
ccagcggcct ggggtcggcc cccgggaggt gcaggtgtca gctgtggccg cgtgggcatg 198720
aggagccagc gtgcctgcag gggcaccggg acgggggtgg gtctgagacg gcggccccat 198780
ccctgcgtgc agaggaggtg ccggcccggg agggagcctg gtctccgagg cgtcaggggt 198840
accaccccca tgccccagga agagcagcat ctcttccccg tcctgcctgc tgccttgtgc 198900
cccttcggag ccacagcgca ccctggtcac tcctgttcat cgtgcagtga cgctgggcag 198960
ctctctacag tgtaaacagt gtttttcccc tccttccatt tggggctgac aaacctggcg 199020
agtgagcgtt gggattaacc ctgtggcccc catgagcaaa ccggggctcg gggagctgga 199080
cttacttgat ggagatcacg caggccacgc gtgatggagg gatgtcggcc ccggcaccct 199140
ggccccggtg cccaggcccc gacctcccgc cctgctcccg gggccgtggt gcttctgacc 199200
tgctcgggga gccccagccc tggaggcctc tgcatcaggt ccccgggtgc agcccacaaa 199260
acacgcccgg caggacccca gggagacgct gggcactttc caggggaatt ccaggccacc 199320
tgctcctgcc cgaagaggca aaacagggag cagacgatcc gcgtcgtcac agggaggcag 199380
tccacgggac agtgagagcg aaggggcctc agccacgaga gccggggacg tgcgaggagc 199440
gggcggtcat ccagtatccc cgagaagggg ctactggccc attttccaga tgagttcaga 199500
gaggacacgc aggtcgtcca cgtcaccccg cgggtggtga cacccccagg tctgcggggc 199560
tgagagcgtg ctgtccgtgc ctcccaccca tgggggacaa acggaagcac ctcctttatg 199620
ctctggcact gggcgcttct ttcactgata ccacatattt taacattgac caatattcac 199680
atactgccaa aggacagaca gctgacctgg ttcactagag tccggaagaa ccccgggaag 199740
tcccgggtta gctcccagtg tggctaagaa ttacagcaat ttttttttaa agattttatt 199800
tatttattca tgagagacac agagagaggc agagacacag gcagagagag gagcaggctc 199860
cctgcaggga gcccgatggg ggactccatc ccgggacccc ggggtcatgc cctgagctga 199920
aggcagatgc tcaaccactg gctcaaccca ggggccccag aattgcagca atgtcgtgaa 199980
gcccagagac ttctttcttc taggtgactg cgccctatct cctcagaagg aagttcagta 200040
attctccgaa aagccaaggt aagagggagc ggctcccctc gtccccgcag ggttttccac 200100
accctgcgcc gtaagctggt ctaatgcggt acgcagctcc ccaccttctc atgcgaagca 200160
cagaacgtga cctagtgtcc ctctgtgtct ccgtgtgaca aggacacgga ccttgttccg 200220
 
ggcagaaagg ctggaaggcc tggctgcctc gtgcacggtg cacagccggg ccggcgggga 200280
gccgtgtgct cgtgcgtgga tcccctcgca gcccggcagc gggtcaccag agggaagcag 200340
cccccttcct aggtcagggc ccggtccccc cgctgctccg tcagccctct aagccccggt 200400
ggcgtgtccc gtcgtccctg cgtcggagct ctgtcctcac cacccatcgt gacagccagg 200460
gccccggcac agctctgtgt ccctcctctg ctcccgaaca atcattggcc cccgcctgcc 200520
tgcagaatga gtgggatttc ttagcacagc cctccagacg ctcacttcct gccccacgca 200580
gggccctggg tggacggcac acacctcgca ggcccctggg gtctcagagc tctgcgcaaa 200640
cacgcacttc cgtacctttg cctgggccgg gcctcctgcc cagagctctt ctcgatccct 200700
tccccgatgg ccgggtgcag tgtcccccct cagaggttcc ccaccacttc cagaatgttt 200760
tcctccacct cctcagggca cgtctttgca caccccggtg tgcacccagc tcccctccct 200820
accagctccc tctctgcacc cctgagctcc ctgtaccccg agaggtcagc ccagagacag 200880
agctggtcct gaggcccatt gaggggaacc cgcgacccag ggccaccagc cagggtatgg 200940
actttgccag tggggccagc tgccacgggg tggccaggag gtttctggag cgcacgcact 201000
ggcacttggg gagcttccgg gaggcctcgt ggctgaaacc caagtgaaag ctcccgctca 201060
cagcaggagg acaggggtga gccccttgcc ctcagaggcc gctcacccgg tcccgggcct 201120
ggatacctca ccgccccagg cccccccaga gctggcgggc tttcccacgg aggctggtgg 201180
cctgcacctg cctctcctgc caccatgacc cgcctccgtg gagtggcttc tcatcccgcg 201240
tccccaggcc caccaccccc agggacaggt ggggagggcc aggggtgtcc tccctcccca 201300
ccccgggatc gctggatcgg cagtcccctc agtagggttg ggtcccggag caggaacagg 201360
tggcagatcc cggccccgcg tgccctgccc ggaacggagc tcctcagctc ccctggcaag 201420
atgcgtcaca cagccctact tggtggcttc cgtggcgtga acacctgcac ctggtggatt 201480
tgcagccacc gccgcgatgt cacggagccc gggactggga gggggagacg ggcccagcac 201540
cccccaggga gcaggtgcca ggcctcaggc aggcagacaa ggccgctggc cgccctcccc 201600
cgggatccac tcgctgtggc cacaagtggt tggctaggat gtgagagcga gggttgggag 201660
ctgtgaggcc ccgcggccca cggcacgtca cagagtaaag gaaaggcaac ggcgcttgct 201720
gttttgccgg agggagcctt tccgacggga ccgtgaggct cgctcccagg ccgcggagaa 201780
cagtggagcc cacggggtgt gcacatccct aaccgcttcc ctgcccccga aggtagagcc 201840
gccgactctg ctcggccgtc ggcgtcagcg taccgcggat ccctgacgca gaggaacccc 201900
aacagcagga agggggtggc cgcccacagc ttcgcagtaa gctttcctct tgcttcctga 201960
ggcgcgggca cctcggtggc ctggcgggtt tacgtggtga cggtgacgtt aggaccccag 202020
agcacatggg gggtgggggt gactgggtga cgggcactga ggggggcact tgacgggatg 202080
agcactgggg tgttattttg tatgttggca aattgaacac caataaaaaa taaatttatt 202140
aaaaaaaaga ccccagagca cagaccgggg gagcactaac gggtaaaatc ggccaaagat 202200
ttaaaggaaa attgaaaaca tttcttaaag gtaccagaat gtaccagtgg caggggccac 202260
 
tgctcatcct ggggtgggtc ttggcttggg gcagaggaca ccccgggagg ggacagtcca 202320
tgggcaggga ggacacccag ggagggacaa cccacagacg aggaggacac tggggagggg 202380
acagcccgtg gatggggagg tcacctgggg aggggacagc ccacggacac ccagggaggg 202440
aacaggtgcc ccgacaaggg gatgcacgtg gggggagacg agaggataga ggtgcccgca 202500
ggccccacct taactgacgg cggtrgcacg accgtggccg gagctccgcc gtccccaccc 202560
agggctgatg actgtcccgg aggaagttgc cagcggggac ttgagaactg acgggctttc 202620
cgattagaat atccctcccc gcgcttcaga gcctgaaatt ctcactctca tccgtgcacc 202680
ctctgatgag ggagtggcca gtgcacacaa ctgtgtgtgt gtgtgtgtgt gtgtgtgcgt 202740
gtgtgtgtcc cgcagaagcc tgtgatgacc cagcagccct gaaggggccc gacggagcca 202800
cccgggcccg cctcccgtct ccctgcacct cagcttgtgc tcctccaccc gccctgcacg 202860
tcgtgctgcg cacagtccgg gggacacggg acaagacgga gccccgtccg aaagcgagcc 202920
tcctgcgagg tgacagcgct ccagacccgc cgctaccccc cacccctgct ccctgacgcc 202980
ctcggccacc gcccctgccg gcctgcccga tccctgagaa acgccgaggc tgacgtggct 203040
ttcctgtacg acggcgttcc acgcacgtat tgaaggccac ggagaagaat gctccagcct 203100
gatcttttgt ccgtttccga gccggcctgg ccagagcggg cctggccccg cgcagcacct 203160
gccatgtgct ccagcctggg tgggagcggg gcaggcgtgc gtgtccccgt gagtgcgcgt 203220
gcgctgtcgg accgggcaac tgcgcatcga ggcgggcgac ggacccctga ccagccgacc 203280
tccccgcccc cttctgcccc ccgttctgag aggccacctg ggattcacag accgggggca 203340
accccgctgc ggtccaggac acagcacgca caaggcagca ggggagcctc ggatccatgt 203400
cggggggcgg aggacctgct ggggaggcgg gcagcagggt gggttccagc ctggcgctgt 203460
cactcgtgca ggggccagcc tgtgggaccg agaggccagg accaactagg gatgcccgct 203520
agccacctgc catcagctct ggaggggccg agtcctcctc cacctggggc agcctgggag 203580
tcagggttgc ctctgggtga ctgggtggct catccggttc taaggggagc ccacggggac 203640
cctggggggg gctgcctcgg aacatggtaa ttgtaattgc acacgcccct ccccccggct 203700
gcacattgtg acccgcccca cggggactcc tgtgtgcaat cttggcttga atttaagcct 203760
attttgtgga cgaggacacg gagccctggg tgcaaggcag cgtccccctg gagcgagagc 203820
tgactgcggg cacagctcca tgcacccccc ctccccgcgc ccacccagga ccccgagcct 203880
tgcacctcca ccgagcccca ggtgcagccg ccaccgggct ggcccacggg cttcgggaac 203940
cagcttctgg gtggatccgg ctgccggggc tgccggggtg tgtagaagag acaggtctga 204000
cacccctgct ccgggcggcc acctggctcc tgacccaaag ccgagtgatg gaaggcgtcc 204060
tacagacgaa aggggatccg tttctttgtt ggcgcgcccc tccctgttca cagagtacct 204120
taaagtcggt gtgagttcac aggagtccga gcagtgagtc tgctggccct gggtgtgtcc 204180
ccgagggttg acagtgggtc cccagtttac ctgctcgtgt cccagaacct cactagccag 204240
gctcctccct ggctgatctg agctccgaga agctcaaact cccagcagcc gggtgtcccc 204300
tgtcagggtg gcctcgaggg acatgtgagc tgtcatcggc accttcggac acgtgccttc 204360
 
actccctcag cggccccgca ccgagacccg acagtgcggg aggctggggg gtggggggtc 204420
cttccccgcc cctgccctgg tcccgccggc ccagcccccc actctggagc tccttctcca 204480
tccccatctc gccccaaggc ctcgggcccc tggacgtgtg gccgattcca ccccagagag 204540
cagccaccac caccgcctct ggggctcaga gtcaccttcc agctgaactt ggtctcacgc 204600
cccagttgct tgctgtgccc tctagtgtcc cctccagcct tccaggggct ccggccacgg 204660
ctcgctcgcc ttcgccccca cctcctgtca ggagaacgtg gctatttgga gtgtatcttt 204720
aggccaaaca cttccactcg gttcttttcc acgatgtctg ggtctcgttg ctcgtgcgac 204780
acatctgccc gtgtcttgat aaacccagga gctgggtcta tgcctgtgtc acggtgcatc 204840
agtgccgtct gtgctctcgg gaaccgtggg ccattgctcc atggacacct tcgtcgtgac 204900
ttgtgacagc gtcctcaggg agcactggct gcttgggctg ccgttgtggg gggcaggagg 204960
gtcaccgtga gccccactgc ccagacccct tgccttccaa acaagcccct gggaagcttg 205020
agaagaccca tcccccgagc ctgggggcca ccaggcctga cagttttgag ggggctgctc 205080
ccaaggcagg ccagccccac ctcctttctc caaagtcccc gttgcccctg cgtcctcccg 205140
aggacacctg ccccatggcc caggcctgag tccatggatc gggccacatc tctgtgcttc 205200
cttgtctgtg atgcttttgt gctacaacaa cagagttggg tcgttaccat ctgaccccgt 205260
atgggacaag gttgccgact cccggcctgg gcccttgtcc cggcttctgc aaggggccgt 205320
ccccaggcag cggctggcag ggcgagagga gcacaccagc ttcctccagt ccggcccccc 205380
cacggccccc tacccagcca ccctgcccac tccacagccc tccacccctg cactccatcc 205440
ccggcttgtg ctgggagagg tgcggcaccc acgccagcgc gcaccgtctt ggcaaccggg 205500
atgcattgca tcctggaaca gggggtggca gccagcccag acctcgaggg gccccggggc 205560
tgctcctaac aaattctcac ccgccagcaa cggaccgtct cagggtggag gccggaagtc 205620
tgggatcggc gtgtgggcag gccctgctcc ctgcagctgc tctcggagag actcccttct 205680
ctgcctgccc gagcctcggg ggcccccgcc gtcctgagcc tgtggctgca acatcccaca 205740
tcctgagcct gtggctgtgt catcccacgt tgtggccccg tcctcacagg acccactgtg 205800
tccgcgtcac atctccctct gcccttctct tacaaggatg ctcatccctg gatttgggac 205860
ccacctcagt tatccggggt aatctcatca gacatcttca ccctccagac gtgtacaaag 205920
accctttttg caaataaaat caccttccca ggttccaggc tctggaatga gagtttgggg 205980
agccaccatt caaccccctg tagctaagtt gggaaaccta ttatgatccg ctgacagagc 206040
ggactccccc ccatttacca ccaccatcca gaccccgaaa gggagctcga ggcgacactc 206100
acaggccagc ggccgcctcc ccagcctctc agggagcccc gggcacagtc acatgcttca 206160
ccggagtgcc gcaggggccc aggcgacagg ccaccctccc cgtcccgtcc agtggccctg 206220
ccacactgcc ccttgccctc ccgacccccg tcttcacgga aacccccacc ccacggctat 206280
ccttacttca cctggtgcag actcagcacc cactgcccca ctcgaggcca gagcacctgt 206340
tttgtgcagt aaacccccag ttccgccagt tcagggaaga gagactggga gggggtaggc 206400
 
gagcacctgt ggccgtgacc agacatgatg ccccggggcc cggccagcac tgacacagtg 206460
gagagcttcg ggtacgacgt gcatggggcc tcgccaggta gtaacttggt ggcccaagcg 206520
ccggactagc ctgcaatggc ctacctgggg cccagggcag cacccggcac ggacacgcag 206580
ccgccagact gcccgttcac ggggctgcgg acgtgagtga ccatctcccc gaggcagggc 206640
cccggataac tgaggtgggt cccaaatcca ggcagccaag cattccgcag acagacgggg 206700
caggctcccg tggcggcacg ctggcaggtt cagggtgccc ccccgaaccg cgcacccctg 206760
gctggccttc cagggctccc actactgctc acgctccaga caggaccccg aggctgcatc 206820
cccggcgggg ggaggagggt gctggcgcac ggggagggct tccctgggaa tcacgcaggc 206880
ggcagagacg agagtcagcc tgtgtcccca agggctggcc agggggaggg tttcctgcca 206940
cctccgagtg ggacccttgc cgggctgctc cccatgcagg ccgtcactcg agctaggacc 207000
tgaatccacc gtgctttgct gggaggcagg tgcctgcgtc ctcaggagat acggagcgcc 207060
cacgccgaca gctgtgctct ggctcccgtg gcatcaagtg aggggggatc gttttcgtag 207120
ttcattcccc ccacactacc tgtaaattgc actatttgaa aggcttctcc tggggcaccc 207180
gggcggctca gtcggtcaga cccctgcctt caactcaggt atgatcccac gtcgggctcc 207240
ctgcctcgcc ctctgcctgc cactcctctt gcttgtggtg tcgctctctc tgtgtcagat 207300
aaataaataa aatcttgaaa gaaagaattc tcctgagtcc ctttttctag gagatggtca 207360
ttcgtgagac accttccctg gaagctgaat agagacaggc cagggacccg tgaccctggg 207420
gctcacctgc ttctaaccaa gagcaggaga agtcagtggt ttgattatga ctccagatgg 207480
atattagagt cagcatttaa aatttctccc gagtggggac aaccgggtgg ctcagctggc 207540
taagcatctg attcctattt tttagctcag gtcgtgatct cagggtcggg agatggagcc 207600
ctgtatcggg ctctgtgctg agcgtggagc ctgcttagga tgctctctct ctctctctct 207660
ctctctctct ctttctctac ccctctgcac tcactctctc tcttaaaaaa aattaactta 207720
atttttaaaa agttgaaatt tccccagact tttattcccc agcaatcttc tttattaggg 207780
aaaagagaaa aatctccatg aggaggtgaa tgcacggatc ccccgaggat cttccatggt 207840
ttaaagtgag ccaggaccta gcgtcctatg cactggggtc cagggctggt ccgctcggct 207900
cccggcaggt ggctggggaa atacacagcc tcccaggctg tcccctgtgc atcaggcccc 207960
aagccaaccc cgggccagtg ttccttcacc gcagcgatac agcaagccac gatttctgtc 208020
tcagagcacg gcgaggaaga gcccacttct caggcggccc tgtgataact caccccaaag 208080
gcaccatagg attcctgtgc gcctgctttc tggtccccag atacctggca tccggcacac 208140
gggccagcca agtgaccttt aaacgtgccc ccgaggccca tagcaccaga gttgtgtttg 208200
cacggaatta ttattacctg tgagctatct ttgtgttgtg ttgggaagaa aacagaaaat 208260
gtagatacac taaggaaaca agaacagtca gccccgtctc tcaaccagca gcccccgcca 208320
cccctacgga catccatccg tacattcgct ttcaacttaa aactcctgca ggtgaattca 208380
ccggcgagag gtgaagccgc ctctggatgt taccccactc ctgtggcttc tcaggaaaga 208440
gctggctggg gaaagaaggg ttttcccctc tcctgctgct ggattcccct ccagacgcct 208500
 
acagcaaagc ctgccagagg cagcctcaca ggttccaaat aagccacatg ccctccaacc 208560
tcccaagtat gtccacacct gcagctcagc ggtgcccagg ctcagagtcc caaggccatg 208620
gtcatgctgg agacagtagc atttgactac atccctgcaa atgcttgtaa cagccggtga 208680
tggtcagtct ttttttttta agattttact tattcatgag agagagagaa agaggcagag 208740
acacaggcag agggagaagc aggctccatg cagggagctc gacatgggac tcaatcccgg 208800
gtcacgccct gggctgaaga tggcgcttaa ccgggctgcc caatggtcag tcttttgtat 208860
ctacttgact aggtcacagt gctctgatgt ttggccaaac accagtctag atgttgctgt 208920
gacaatattt tttagacgtg actaacattt gcgattagct gcctttgagt aaagcagatt 208980
ctcctctgca atgtgggtgg gcctcatcta atcagttgaa agtcttaaga gagcaaggtc 209040
tcccaagagg gaattctgtc ccaggactgg cttccaactc tggctacaac atcatccctt 209100
ccttgagtcc ccagcctgtc atcttacccc gtagatttca ggcttgccag cctcataacc 209160
acaaaagcca gttccttaat cctctctctc tctccacaca cacacacaca ctattggtac 209220
catttctctg gagaaccctg actaatgcac ggttcccaca taagagctgc gtctaacatt 209280
tcccacactg gaaactcaga aaccagctgg atgggagtca acgtgcaagc gttggagaac 209340
accatttccg ttgaccgcaa acgcgatgcc aagttcccag agccgctgcc tctcggggca 209400
ggaagatgct cccactgctg ctcatctctc actaaaactt gatccctgct cccaggaact 209460
tcatttccca catgacacat actttctaac ttaaagatga gtcctttaac atgttctcaa 209520
gaatttggat tttcagtggg cagaaacatg aagagacgtt tctccaaaag accaaacttt 209580
tggacctaca tgtgtaggtc ttctttggaa ggtcttcttc ttcgttggaa gatcttcttc 209640
ttccaaccta catgtatagg tcttctttga agaagatctt ccaaagaaga cctacacatg 209700
gccaacagac acatgaaaaa atgctccaca tcaccacgcg gcatcaggga gatacaaatc 209760
caagcgcaac gagataccac ctcacggctg tcagagtggc taaaattaac cactcatgac 209820
gcaacaggtg ttggcgagga tgtggagaaa gggcaacaac cttcttacat tgttggtggg 209880
gatgcaaact ggtgtagccc ctccggaaaa caggatggag ggtcctcaaa aagttaaaaa 209940
tagagccacc ctgcgatcca gcaattctgc tactggctat ttacccccaa agatacaaat 210000
gaggtgatga gaaggggcac ctgcacccag atggaaagag cccagatgtc catcgacaga 210060
tgactggaca aggaagatgt ggtctatatc cccacaatgg gatattactc agccatcaga 210120
aaaataaaac cttgccattt gcaatgccac agatggaaac ggagggtatt atgctaagtg 210180
aaatgagtca gtcagagaaa gacaatttat gatctcactc ctatgtggaa tttaagaaac 210240
aaaacacatg aacacagggg aagggaagga aatataaaat aagacagaag cagagagaag 210300
gagacaaacc ataagagcct cttaactcta gggaacaaat agggttgctg gaggggaggc 210360
agggaggacg atggggtaac tggggggtgg gcaggaagga gggcacgtga tgggatgagc 210420
actgggtatt gtacacgact gatgaatcac tagattttac ctctgaaaat aataatgcac 210480
ctttgttaat taaatcgatt ttaaatttaa aatattttta gaaaagaatt tggggagtac 210540
 
ctgggtggct ccgttgctta agcatctgcc ttcagctcag gggacctgga attgagcccc 210600
acattgggct ccctgctcag cggggagtct gcatccccct ctgtccctcc ctgctgcttc 210660
tgctcattct ctctctctct ctgtcaaata aataaataaa atcttaaaaa aaataattgg 210720
atgttcagaa gccttctctg tcagaagctg ctttgctgtt gaccagtggc cccattcgcc 210780
acggccactc tgcagctagg acactggtgg ccccttcctt ccagaagctc tgagggagac 210840
gccgtgtcat cctcaagcca agctcctggt gtcggtcgcc tgtccttggc atgtgcccac 210900
atcactcccc catctctgac tccgtcttca cagcaccttg ccctgctgtg tctctgtgtg 210960
tctgtcctct cttggacacc aatgactgga ttcagggccc acctcaccca gcatgacctc 211020
aacttcatgt ggttgcatct gcaaagaccc tttttcccca taaggtcaca ttctgaggtt 211080
tctggctgac acgaatcgtg gagggacacc gctcagcccg ttgcaccatt gcagtccaaa 211140
ggtcaagccc cggaacagcc gcccgtcatc gtgctacctg ccgggtatgc ccgggaatcc 211200
accgtgttga gggtaaaagt tcaaagatct caaaaccatc aagctccttc caaaaagtgg 211260
gatcctcttc ctcgtgctaa catgaagaac gaggagggga gagctcctcc cgaggtcctt 211320
cgatgtgggg ctcccctaat agcaaggagc agaagccact caaatgggcg caagtgagat 211380
ggagagggaa tgagacctgg ggtgctctcc ttcaggatgg tgggaaccca aggcccagcc 211440
cgggggtggc cggtgctcag gggcggttat gcccctgtga cctcttggag gcatcccttg 211500
tgaccgtgaa ccactcactg gacttcacgg caggtggccc agcggcctct cctggcccgg 211560
tcctgagtgt tcccctgccg tggtcgtggc tcacggctcc agacacaagg agacaagcgt 211620
gcagggccgg gtgcgtccca ggtgctggcc atcccagggg gacggggacc ggccagatct 211680
gcacgaggag ccccccagct cggttagcag gggagcaggt catgtgatcc ccacacatgg 211740
gtcacagcga ccgcctaggt ttgggcctca gaaaccagac tacggtctct gcaccagggt 211800
cggagggcct ccctctaggg cgcaccttcc gcctacacct gtaaggagtc gactgcctgc 211860
cagccacccg gggggcccaa cccagcccgc ggcctggttt cgtgaaagtc cgccgcaaac 211920
attcacgttc cggtcacctg tggctgccac agagactctg gccctcgagg ctgaggaggg 211980
gtgccaagcc ctgacagacc acggtgtgct gtgggcaggg ggcaggacag tgcaggggtt 212040
tgagctctcg tgtgcagggc ggccaaggat gggagggcgg atgggaggga ggccgcgatg 212100
cccgtgccac ctcccgcccc agctgccagc catcacgtgc ccggggcccc acagtggccc 212160
ggcaccaatg ccacgctgtg acaaagctac ttagctaaaa ggggaggatt cactatcccc 212220
cacacacaca catgcacgtg caggcactgt acacacacac acacacacac acacgcgcgc 212280
gcatgtacag gtgcatgcac aggcaggcac acatgcacat gcgtacatgc acacgyaggc 212340
atgtgcacac acgtgcatgc atacatacat ggtgcacgtg cacacacgca cacgtacaca 212400
tatgcacaca cgagcccatg cacacacgct cccacagtgg tgcccaggaa gctgcccacg 212460
gctcccgtga tctcagttgc tcttgctcca ggtgctcaca tctctgggga ccccrccccc 212520
agacaaggcc acaccccacg agagcaccct ccatcatcat caccctcttt cacagacact 212580
gctaccgggg attcagcaga atcagcccag tgcctcactc gcagcccagg tggagcctgc 212640
 actgccctca ccccggggtc tcagcgcccc tcacagtgcc gggtccgaca ccggag 212696
<210> 2
   <211> 21
   <212> DNA
   <213> artificial sequence
<220>
   <223> forward primer
   <400> 2
   gtcaccgaca ttatgtcaca g 21
<210> 3
   <211> 19
   <212> DNA
   <213> artificial sequence
<220>
   <223> reverse primer
   <400> 3
   caggctggaa ggtcagatc 19